# EUROPEAN PATENT APPLICATION

(11) **EP 3 702 465 A1**
(43) Date of publication of application: **02.09.2020**
(21) Application number: 18870157.7
(22) Date of filing: 16.10.2018
(51) Int. Cl.: C12N 15/85, C12N 1/15, C12N 1/19, C12N 1/21, C12N 5/10, C12N 15/12, C12Q 1/02

(54) **CONSTRUCT FOR EXPRESSING G-PROTEIN-COUPLED RECEPTOR OR SUB-UNIT THEREOF, AND USE OF SAID CONSTRUCT**

(30) Priority: 23.10.2017 JP 2017204687
(71) Applicant: Japan Tobacco Inc., Tokyo 105-8422 (JP)
(72) Inventor: SHIMOMURA, Ippei, Tokyo 130-8603 (JP); YAMAMOTO, Takeshi, Tokyo 130-8603 (JP); JOTAKI, Masafumi, Tokyo 130-8603 (JP); TOBITA, Naoya, Tokyo 130-8603 (JP)
(74) Representative: Hoffmann Eitle
(86) International application number: PCT/JP2018/038391
(87) International publication number: WO 2019/082721

(57) **Abstract**

An purpose of the present invention is to provide a construct for expressing a G protein-coupled receptor, and the use of said construct. The construct is used for expressing a G protein-coupled receptor protein or a subunit thereof, wherein the construct is characterized by including a nucleic acid that encodes G protein α subunit protein; a nucleic acid that comprises an IRES sequence; and a nucleic acid that encodes the G protein-coupled receptor protein or a subunit thereof in the stated order from the 5' side in a single vector.

SEQUENCE LISTING

<110>  JAPAN TOBACCO INC.

<120>  Construct for expressing G-protein coupling protein or its
       subunit or use thereof

<130>  FA0092-18121

<150>  JP2017-204687
<151>  2017-10-23

<160>  81

<170>  PatentIn version 3.5

<210>  1
<211>  900
<212>  DNA
<213>  Homo sapiens

<220>
<221>  CDS
<222>  (1)..(900)

<400>  1
atg cta gag tct cac ctc att atc tat ttt ctt ctt gca gtg ata caa      48
Met Leu Glu Ser His Leu Ile Ile Tyr Phe Leu Leu Ala Val Ile Gln
1               5                   10                  15

ttt ctt ctt ggg att ttc aca aat ggc atc att gtg gtg gtg aat ggc      96
Phe Leu Leu Gly Ile Phe Thr Asn Gly Ile Ile Val Val Val Asn Gly
            20                  25                  30

att gac ttg atc aag cac aga aaa atg gct ccg ctg gat ctc ctt ctt      144
Ile Asp Leu Ile Lys His Arg Lys Met Ala Pro Leu Asp Leu Leu Leu
        35                  40                  45

tct tgt ctg gca gtt tct aga att ttt ctg cag ttg ttc atc ttc tac      192
Ser Cys Leu Ala Val Ser Arg Ile Phe Leu Gln Leu Phe Ile Phe Tyr
    50                  55                  60

gtt aat gtg att gtt atc ttc ttc ata gaa ttc atc atg tgt tct gcg      240
Val Asn Val Ile Val Ile Phe Phe Ile Glu Phe Ile Met Cys Ser Ala
65                  70                  75                  80

aat tgt gca att ctc tta ttt ata aat gaa ttg gaa ctt tgg ctt gcc      288
Asn Cys Ala Ile Leu Leu Phe Ile Asn Glu Leu Glu Leu Trp Leu Ala
                85                  90                  95

aca tgg ctc ggc gtt ttc tat tgt gcc aag gtt gcc agc gtc cgt cac      336
Thr Trp Leu Gly Val Phe Tyr Cys Ala Lys Val Ala Ser Val Arg His
            100                 105                 110

cca ctc ttc atc tgg ttg aag atg agg ata tcc aag ctg gtc cca tgg      384
Pro Leu Phe Ile Trp Leu Lys Met Arg Ile Ser Lys Leu Val Pro Trp
        115                 120                 125

atg atc ctg ggg tct ctg cta tat gta tct atg att tgt gtt ttc cat      432
Met Ile Leu Gly Ser Leu Leu Tyr Val Ser Met Ile Cys Val Phe His
    130                 135                 140

agc aaa tat gca ggg ttt atg gtc cca tac ttc cta agg aaa ttt ttc 480
Ser Lys Tyr Ala Gly Phe Met Val Pro Tyr Phe Leu Arg Lys Phe Phe
145                 150                 155                 160

tcc caa aat gcc aca att caa aaa gaa gat aca ctg gct ata cag att 528
Ser Gln Asn Ala Thr Ile Gln Lys Glu Asp Thr Leu Ala Ile Gln Ile
                165                 170                 175

ttc tct ttt gtt gct gag ttc tca gtg cca ttg ctt atc ttc ctt ttt 576
Phe Ser Phe Val Ala Glu Phe Ser Val Pro Leu Leu Ile Phe Leu Phe
            180                 185                 190

gct gtt ttg ctc ttg att ttc tct ctg ggg agg cac acc cgg caa atg 624
Ala Val Leu Leu Leu Ile Phe Ser Leu Gly Arg His Thr Arg Gln Met
        195                 200                 205

aga aac aca gtg gcc ggc agc agg gtt cct ggc agg ggt gca ccc atc 672
Arg Asn Thr Val Ala Gly Ser Arg Val Pro Gly Arg Gly Ala Pro Ile
    210                 215                 220

agc gcg ttg ctg tct atc ctg tcc ttc ctg atc ctc tac ttc tcc cac 720
Ser Ala Leu Leu Ser Ile Leu Ser Phe Leu Ile Leu Tyr Phe Ser His
225                 230                 235                 240

tgc atg ata aaa gtt ttt ctc tct tct cta aag ttt cac atc aga agg 768
Cys Met Ile Lys Val Phe Leu Ser Ser Leu Lys Phe His Ile Arg Arg
                245                 250                 255

ttc atc ttt ctg ttc ttc atc ctt gtg att ggt ata tac cct tct gga 816
Phe Ile Phe Leu Phe Phe Ile Leu Val Ile Gly Ile Tyr Pro Ser Gly
            260                 265                 270

cac tct ctc atc tta att tta gga aat cct aaa ttg aaa caa aat gca 864
His Ser Leu Ile Leu Ile Leu Gly Asn Pro Lys Leu Lys Gln Asn Ala
        275                 280                 285

aaa aag ttc ctc ctc cac agt aag tgc tgt cag tga 900
Lys Lys Phe Leu Leu His Ser Lys Cys Cys Gln
    290                 295

<210> 2
<211> 299
<212> PRT
<213> Homo sapiens

<400> 2

Met Leu Glu Ser His Leu Ile Ile Tyr Phe Leu Leu Ala Val Ile Gln
1               5                   10                  15

Phe Leu Leu Gly Ile Phe Thr Asn Gly Ile Ile Val Val Asn Gly
            20                  25                  30

Ile Asp Leu Ile Lys His Arg Lys Met Ala Pro Leu Asp Leu Leu Leu
            35                  40                  45

Ser Cys Leu Ala Val Ser Arg Ile Phe Leu Gln Leu Phe Ile Phe Tyr
        50                  55                  60

Val Asn Val Ile Val Ile Phe Phe Ile Glu Phe Ile Met Cys Ser Ala
65                  70                  75                  80

Asn Cys Ala Ile Leu Leu Phe Ile Asn Glu Leu Glu Leu Trp Leu Ala
                85                  90                  95

Thr Trp Leu Gly Val Phe Tyr Cys Ala Lys Val Ala Ser Val Arg His
            100                 105                 110

Pro Leu Phe Ile Trp Leu Lys Met Arg Ile Ser Lys Leu Val Pro Trp
        115                 120                 125

Met Ile Leu Gly Ser Leu Leu Tyr Val Ser Met Ile Cys Val Phe His
    130                 135                 140

Ser Lys Tyr Ala Gly Phe Met Val Pro Tyr Phe Leu Arg Lys Phe Phe
145                 150                 155                 160

Ser Gln Asn Ala Thr Ile Gln Lys Glu Asp Thr Leu Ala Ile Gln Ile
                165                 170                 175

Phe Ser Phe Val Ala Glu Phe Ser Val Pro Leu Leu Ile Phe Leu Phe
            180                 185                 190

Ala Val Leu Leu Leu Ile Phe Ser Leu Gly Arg His Thr Arg Gln Met
        195                 200                 205

Arg Asn Thr Val Ala Gly Ser Arg Val Pro Gly Arg Gly Ala Pro Ile
    210                 215                 220

Ser Ala Leu Leu Ser Ile Leu Ser Phe Leu Ile Leu Tyr Phe Ser His
225                 230                 235                 240

Cys Met Ile Lys Val Phe Leu Ser Ser Leu Lys Phe His Ile Arg Arg
                245                 250                 255

Phe Ile Phe Leu Phe Phe Ile Leu Val Ile Gly Ile Tyr Pro Ser Gly
            260                 265                 270

His Ser Leu Ile Leu Ile Leu Gly Asn Pro Lys Leu Lys Gln Asn Ala
        275                 280                 285

Lys Lys Phe Leu Leu His Ser Lys Cys Cys Gln
    290                 295

<210>  3
<211>  951

<212>  DNA
<213>  Homo sapiens

<220>
<221>  CDS
<222>  (1)..(951)

<400>  3
atg atg gga ctc acc gag ggg gtg ttc ctg att ctg tct ggc act cag      48
Met Met Gly Leu Thr Glu Gly Val Phe Leu Ile Leu Ser Gly Thr Gln
1               5                   10                  15

ttc aca ctg gga att ctg gtc aat tgt ttc att gag ttg gtc aat ggt      96
Phe Thr Leu Gly Ile Leu Val Asn Cys Phe Ile Glu Leu Val Asn Gly
            20                  25                  30

agc agc tgg ttc aag acc aag aga atg tct ttg tct gac ttc atc atc      144
Ser Ser Trp Phe Lys Thr Lys Arg Met Ser Leu Ser Asp Phe Ile Ile
        35                  40                  45

acc acc ctg gca ctc ttg agg atc att ctg ctg tgt att atc ttg act      192
Thr Thr Leu Ala Leu Leu Arg Ile Ile Leu Leu Cys Ile Ile Leu Thr
    50                  55                  60

gat agt ttt tta ata gaa ttc tct ccc aac aca cat gat tca ggg ata      240
Asp Ser Phe Leu Ile Glu Phe Ser Pro Asn Thr His Asp Ser Gly Ile
65                  70                  75                  80

ata atg caa att att gat gtt tcc tgg aca ttt aca aac cat ctg agc      288
Ile Met Gln Ile Ile Asp Val Ser Trp Thr Phe Thr Asn His Leu Ser
                85                  90                  95

att tgg ctt gcc acc tgt ctt ggt gtc ctc tac tgc ctg aaa atc gcc      336
Ile Trp Leu Ala Thr Cys Leu Gly Val Leu Tyr Cys Leu Lys Ile Ala
            100                 105                 110

agt ttc tct cac ccc aca ttc ctc tgg ctc aag tgg aga gtt tct agg      384
Ser Phe Ser His Pro Thr Phe Leu Trp Leu Lys Trp Arg Val Ser Arg
        115                 120                 125

gtg atg gta tgg atg ctg ttg ggt gca ctg ctc tta tcc tgt ggt agt      432
Val Met Val Trp Met Leu Leu Gly Ala Leu Leu Leu Ser Cys Gly Ser
    130                 135                 140

acc gca tct ctg atc aat gag ttt aag ctc tat tct gtc ttt agg gga      480
Thr Ala Ser Leu Ile Asn Glu Phe Lys Leu Tyr Ser Val Phe Arg Gly
145                 150                 155                 160

att gag gcc acc agg aat gtg act gaa cac ttc aga aag aag agg agt      528
Ile Glu Ala Thr Arg Asn Val Thr Glu His Phe Arg Lys Lys Arg Ser
                165                 170                 175

gag tat tat ctg atc cat gtt ctt ggg act ctg tgg tac ctg cct ccc      576
Glu Tyr Tyr Leu Ile His Val Leu Gly Thr Leu Trp Tyr Leu Pro Pro
            180                 185                 190

tta att gtg tcc ctg gcc tcc tac tct ttg ctc atc ttc tcc ctg ggg      624
Leu Ile Val Ser Leu Ala Ser Tyr Ser Leu Leu Ile Phe Ser Leu Gly
        195                 200                 205

agg cac aca cgg cag atg ctg caa aat ggg aca agc tcc aga gat cca      672
Arg His Thr Arg Gln Met Leu Gln Asn Gly Thr Ser Ser Arg Asp Pro

210                 215                 220

acc act gag gcc cac aag agg gcc atc aga atc atc ctt tcc ttc ttc      720
Thr Thr Glu Ala His Lys Arg Ala Ile Arg Ile Ile Leu Ser Phe Phe
225                 230                 235                 240

ttt ctc ttc tta ctt tac ttt ctt gct ttc tta att gca tca ttt ggt      768
Phe Leu Phe Leu Leu Tyr Phe Leu Ala Phe Leu Ile Ala Ser Phe Gly
                245                 250                 255

aat ttc cta cca aaa acc aag atg gct aag atg att ggc gaa gta atg      816
Asn Phe Leu Pro Lys Thr Lys Met Ala Lys Met Ile Gly Glu Val Met
            260                 265                 270

aca atg ttt tat cct gct ggc cac tca ttt att ctc att ctg ggg aac      864
Thr Met Phe Tyr Pro Ala Gly His Ser Phe Ile Leu Ile Leu Gly Asn
        275                 280                 285

agt aag ctg aag cag aca ttt gta gtg atg ctc cgg tgt gag tct ggt      912
Ser Lys Leu Lys Gln Thr Phe Val Val Met Leu Arg Cys Glu Ser Gly
    290                 295                 300

cat ctg aag cct gga tcc aag gga ccc att ttc tct tag                  951
His Leu Lys Pro Gly Ser Lys Gly Pro Ile Phe Ser
305                 310                 315


<210>  4
<211>  316
<212>  PRT
<213>  Homo sapiens

<400>  4

Met Met Gly Leu Thr Glu Gly Val Phe Leu Ile Leu Ser Gly Thr Gln
1               5                   10                  15


Phe Thr Leu Gly Ile Leu Val Asn Cys Phe Ile Glu Leu Val Asn Gly
            20                  25                  30


Ser Ser Trp Phe Lys Thr Lys Arg Met Ser Leu Ser Asp Phe Ile Ile
        35                  40                  45


Thr Thr Leu Ala Leu Leu Arg Ile Ile Leu Leu Cys Ile Ile Leu Thr
    50                  55                  60


Asp Ser Phe Leu Ile Glu Phe Ser Pro Asn Thr His Asp Ser Gly Ile
65                  70                  75                  80


Ile Met Gln Ile Ile Asp Val Ser Trp Thr Phe Thr Asn His Leu Ser
                85                  90                  95


Ile Trp Leu Ala Thr Cys Leu Gly Val Leu Tyr Cys Leu Lys Ile Ala
            100                 105                 110


Ser Phe Ser His Pro Thr Phe Leu Trp Leu Lys Trp Arg Val Ser Arg

115                 120                 125

Val Met Val Trp Met Leu Leu Gly Ala Leu Leu Leu Ser Cys Gly Ser
    130                 135                 140

Thr Ala Ser Leu Ile Asn Glu Phe Lys Leu Tyr Ser Val Phe Arg Gly
145                 150                 155                 160

Ile Glu Ala Thr Arg Asn Val Thr Glu His Phe Arg Lys Lys Arg Ser
                165                 170                 175

Glu Tyr Tyr Leu Ile His Val Leu Gly Thr Leu Trp Tyr Leu Pro Pro
            180                 185                 190

Leu Ile Val Ser Leu Ala Ser Tyr Ser Leu Leu Ile Phe Ser Leu Gly
        195                 200                 205

Arg His Thr Arg Gln Met Leu Gln Asn Gly Thr Ser Ser Arg Asp Pro
    210                 215                 220

Thr Thr Glu Ala His Lys Arg Ala Ile Arg Ile Ile Leu Ser Phe Phe
225                 230                 235                 240

Phe Leu Phe Leu Leu Tyr Phe Leu Ala Phe Leu Ile Ala Ser Phe Gly
                245                 250                 255

Asn Phe Leu Pro Lys Thr Lys Met Ala Lys Met Ile Gly Glu Val Met
            260                 265                 270

Thr Met Phe Tyr Pro Ala Gly His Ser Phe Ile Leu Ile Leu Gly Asn
        275                 280                 285

Ser Lys Leu Lys Gln Thr Phe Val Val Met Leu Arg Cys Glu Ser Gly
    290                 295                 300

His Leu Lys Pro Gly Ser Lys Gly Pro Ile Phe Ser
305                 310                 315

<210>  5
<211>  900
<212>  DNA
<213>  Homo sapiens

<220>
<221>  CDS
<222>  (1)..(900)

<400>  5
atg ctt cgg tta ttc tat ttc tct gct att att gcc tca gtt att tta          48

Met Leu Arg Leu Phe Tyr Phe Ser Ala Ile Ile Ala Ser Val Ile Leu
1               5                   10                  15

aat ttt gta gga atc att atg aat ctg ttt att aca gtg gtc aat tgc      96
Asn Phe Val Gly Ile Ile Met Asn Leu Phe Ile Thr Val Val Asn Cys
            20                  25                  30

aaa act tgg gtc aaa agc cat aga atc tcc tct tct gat agg att ctg      144
Lys Thr Trp Val Lys Ser His Arg Ile Ser Ser Ser Asp Arg Ile Leu
        35                  40                  45

ttc agc ctg ggc atc acc agg ttt ctt atg ctg gga cta ttt ctg gtg      192
Phe Ser Leu Gly Ile Thr Arg Phe Leu Met Leu Gly Leu Phe Leu Val
    50                  55                  60

aac acc atc tac ttc gtc tct tca aat acg gaa agg tca gtc tac ctg      240
Asn Thr Ile Tyr Phe Val Ser Ser Asn Thr Glu Arg Ser Val Tyr Leu
65                  70                  75                  80

tct gct ttt ttt gtg ttg tgt ttc atg ttt ttg gac tcg agc agt gtc      288
Ser Ala Phe Phe Val Leu Cys Phe Met Phe Leu Asp Ser Ser Ser Val
                85                  90                  95

tgg ttt gtg acc ttg ctc aat atc ttg tac tgt gtg aag att act aac      336
Trp Phe Val Thr Leu Leu Asn Ile Leu Tyr Cys Val Lys Ile Thr Asn
            100                 105                 110

ttc caa cac tca gtg ttt ctc ctg ctg aag cgg aat atc tcc cca aag      384
Phe Gln His Ser Val Phe Leu Leu Leu Lys Arg Asn Ile Ser Pro Lys
        115                 120                 125

atc ccc agg ctg ctg ctg gcc tgt gtg ctg att tct gct ttc acc act      432
Ile Pro Arg Leu Leu Leu Ala Cys Val Leu Ile Ser Ala Phe Thr Thr
    130                 135                 140

tgc ctg tac atc acg ctt agc cag gca tca cct ttt cct gaa ctt gtg      480
Cys Leu Tyr Ile Thr Leu Ser Gln Ala Ser Pro Phe Pro Glu Leu Val
145                 150                 155                 160

act acg aga aat aac aca tca ttt aat atc agt gag ggc atc ttg tct      528
Thr Thr Arg Asn Asn Thr Ser Phe Asn Ile Ser Glu Gly Ile Leu Ser
                165                 170                 175

tta gtg gtt tct ttg gtc ttg agc tca tct ctc cag ttc atc att aat      576
Leu Val Val Ser Leu Val Leu Ser Ser Ser Leu Gln Phe Ile Ile Asn
            180                 185                 190

gtg act tct gct tcc ttg cta ata cac tcc ttg agg aga cat ata cag      624
Val Thr Ser Ala Ser Leu Leu Ile His Ser Leu Arg Arg His Ile Gln
        195                 200                 205

aag atg cag aaa aat gcc act ggt ttc tgg aat ccc cag acg gaa gct      672
Lys Met Gln Lys Asn Ala Thr Gly Phe Trp Asn Pro Gln Thr Glu Ala
    210                 215                 220

cat gta ggt gct atg aag ctg atg gtc tat ttc ctc atc ctc tac att      720
His Val Gly Ala Met Lys Leu Met Val Tyr Phe Leu Ile Leu Tyr Ile
225                 230                 235                 240

cca tat tca gtt gct acc ctg gtc cag tat ctc ccc ttt tat gca ggg      768
Pro Tyr Ser Val Ala Thr Leu Val Gln Tyr Leu Pro Phe Tyr Ala Gly
                245                 250                 255

atg gat atg ggg acc aaa tcc att tgt ctg att ttt gcc acc ctt tac     816
Met Asp Met Gly Thr Lys Ser Ile Cys Leu Ile Phe Ala Thr Leu Tyr
            260                 265                 270

tct cca gga cat tct gtt ctc att att atc aca cat cct aaa ctg aaa     864
Ser Pro Gly His Ser Val Leu Ile Ile Ile Thr His Pro Lys Leu Lys
        275                 280                 285

aca aca gca aag aag att ctt tgt ttc aaa aaa tag                     900
Thr Thr Ala Lys Lys Ile Leu Cys Phe Lys Lys
    290                 295

<210>  6
<211>  299
<212>  PRT
<213>  Homo sapiens

<400>  6

Met Leu Arg Leu Phe Tyr Phe Ser Ala Ile Ile Ala Ser Val Ile Leu
1               5                   10                  15

Asn Phe Val Gly Ile Ile Met Asn Leu Phe Ile Thr Val Val Asn Cys
            20                  25                  30

Lys Thr Trp Val Lys Ser His Arg Ile Ser Ser Ser Asp Arg Ile Leu
        35                  40                  45

Phe Ser Leu Gly Ile Thr Arg Phe Leu Met Leu Gly Leu Phe Leu Val
    50                  55                  60

Asn Thr Ile Tyr Phe Val Ser Ser Asn Thr Glu Arg Ser Val Tyr Leu
65                  70                  75                  80

Ser Ala Phe Phe Val Leu Cys Phe Met Phe Leu Asp Ser Ser Ser Val
                85                  90                  95

Trp Phe Val Thr Leu Leu Asn Ile Leu Tyr Cys Val Lys Ile Thr Asn
            100                 105                 110

Phe Gln His Ser Val Phe Leu Leu Leu Lys Arg Asn Ile Ser Pro Lys
        115                 120                 125

Ile Pro Arg Leu Leu Leu Ala Cys Val Leu Ile Ser Ala Phe Thr Thr
    130                 135                 140

Cys Leu Tyr Ile Thr Leu Ser Gln Ala Ser Pro Phe Pro Glu Leu Val
145                 150                 155                 160

Thr Thr Arg Asn Asn Thr Ser Phe Asn Ile Ser Glu Gly Ile Leu Ser
                165                 170                 175

Leu Val Val Ser Leu Val Leu Ser Ser Ser Leu Gln Phe Ile Ile Asn
            180                 185                 190

Val Thr Ser Ala Ser Leu Leu Ile His Ser Leu Arg Arg His Ile Gln
        195                 200                 205

Lys Met Gln Lys Asn Ala Thr Gly Phe Trp Asn Pro Gln Thr Glu Ala
    210                 215                 220

His Val Gly Ala Met Lys Leu Met Val Tyr Phe Leu Ile Leu Tyr Ile
225                 230                 235                 240

Pro Tyr Ser Val Ala Thr Leu Val Gln Tyr Leu Pro Phe Tyr Ala Gly
                245                 250                 255

Met Asp Met Gly Thr Lys Ser Ile Cys Leu Ile Phe Ala Thr Leu Tyr
            260                 265                 270

Ser Pro Gly His Ser Val Leu Ile Ile Ile Thr His Pro Lys Leu Lys
        275                 280                 285

Thr Thr Ala Lys Lys Ile Leu Cys Phe Lys Lys
    290                 295

<210>  7
<211>  900
<212>  DNA
<213>  Homo sapiens

<220>
<221>  CDS
<222>  (1)..(900)

<400>  7
atg ctg agc gct ggc cta gga ctg ctg atg ctg gtg gca gtg gtt gaa      48
Met Leu Ser Ala Gly Leu Gly Leu Leu Met Leu Val Ala Val Val Glu
1               5                   10                  15

ttt ctc atc ggt tta att gga aat gga agc ctg gtg gtc tgg agt ttt      96
Phe Leu Ile Gly Leu Ile Gly Asn Gly Ser Leu Val Val Trp Ser Phe
            20                  25                  30

aga gaa tgg atc aga aaa ttc aac tgg tcc tca tat aac ctc att atc      144
Arg Glu Trp Ile Arg Lys Phe Asn Trp Ser Ser Tyr Asn Leu Ile Ile
        35                  40                  45

ctg ggc ctg gct ggc tgc cga ttt ctc ctg cag tgg ctg atc att ttg      192
Leu Gly Leu Ala Gly Cys Arg Phe Leu Leu Gln Trp Leu Ile Ile Leu
    50                  55                  60

gac tta agc ttg ttt cca ctt ttc cag agc agc cgt tgg ctt cgc tat      240
Asp Leu Ser Leu Phe Pro Leu Phe Gln Ser Ser Arg Trp Leu Arg Tyr
65                  70                  75                  80

ctt agt atc ttc tgg gtc ctg gta agc cag gcc agc tta tgg ttt gcc 288
Leu Ser Ile Phe Trp Val Leu Val Ser Gln Ala Ser Leu Trp Phe Ala
                85                  90                  95

acc ttc ctc agt gtc ttc tat tgc aag aag atc acg acc ttc gat cgc 336
Thr Phe Leu Ser Val Phe Tyr Cys Lys Lys Ile Thr Thr Phe Asp Arg
            100                 105                 110

ccg gcc tac ttg tgg ctg aag cag agg gcc tat aac ctg agt ctc tgg 384
Pro Ala Tyr Leu Trp Leu Lys Gln Arg Ala Tyr Asn Leu Ser Leu Trp
        115                 120                 125

tgc ctt ctg ggc tac ttt ata atc aat ttg tta ctt aca gtc caa att 432
Cys Leu Leu Gly Tyr Phe Ile Ile Asn Leu Leu Leu Thr Val Gln Ile
    130                 135                 140

ggc tta aca ttc tat cat cct ccc caa gga aac agc agc att cgg tat 480
Gly Leu Thr Phe Tyr His Pro Pro Gln Gly Asn Ser Ser Ile Arg Tyr
145                 150                 155                 160

ccc ttt gaa agc tgg cag tac ctg tat gca ttt cag ctc aat tca gga 528
Pro Phe Glu Ser Trp Gln Tyr Leu Tyr Ala Phe Gln Leu Asn Ser Gly
                165                 170                 175

agt tat ttg cct tta gtg gtg ttt ctt gtt tcc tct ggg atg ctg att 576
Ser Tyr Leu Pro Leu Val Val Phe Leu Val Ser Ser Gly Met Leu Ile
            180                 185                 190

gtc tct ttg tat aca cac cac aag aag atg aag gtc cat tca gct ggt 624
Val Ser Leu Tyr Thr His His Lys Lys Met Lys Val His Ser Ala Gly
        195                 200                 205

agg agg gat gtc cgg gcc aag gct cac atc act gcg ctg aag tcc ttg 672
Arg Arg Asp Val Arg Ala Lys Ala His Ile Thr Ala Leu Lys Ser Leu
    210                 215                 220

ggc tgc ttc ctc tta ctt cac ctg gtt tat atc atg gcc agc ccc ttc 720
Gly Cys Phe Leu Leu Leu His Leu Val Tyr Ile Met Ala Ser Pro Phe
225                 230                 235                 240

tcc atc acc tcc aag act tat cct cct gat ctc acc agt gtc ttc atc 768
Ser Ile Thr Ser Lys Thr Tyr Pro Pro Asp Leu Thr Ser Val Phe Ile
                245                 250                 255

tgg gag aca ctc atg gca gcc tat cct tct ctt cat tct ctc ata ttg 816
Trp Glu Thr Leu Met Ala Ala Tyr Pro Ser Leu His Ser Leu Ile Leu
            260                 265                 270

atc atg ggg att cct agg gtg aag cag act tgt cag aag atc ctg tgg 864
Ile Met Gly Ile Pro Arg Val Lys Gln Thr Cys Gln Lys Ile Leu Trp
        275                 280                 285

aag aca gtg tgt gct cgg aga tgc tgg ggc cca tga 900
Lys Thr Val Cys Ala Arg Arg Cys Trp Gly Pro
    290                 295

<210> 8
<211> 299
<212> PRT
<213> Homo sapiens

<400>  8

Met Leu Ser Ala Gly Leu Gly Leu Leu Met Leu Val Ala Val Val Glu
1               5                   10                  15

Phe Leu Ile Gly Leu Ile Gly Asn Gly Ser Leu Val Val Trp Ser Phe
            20                  25                  30

Arg Glu Trp Ile Arg Lys Phe Asn Trp Ser Ser Tyr Asn Leu Ile Ile
        35                  40                  45

Leu Gly Leu Ala Gly Cys Arg Phe Leu Leu Gln Trp Leu Ile Ile Leu
    50                  55                  60

Asp Leu Ser Leu Phe Pro Leu Phe Gln Ser Ser Arg Trp Leu Arg Tyr
65                  70                  75                  80

Leu Ser Ile Phe Trp Val Leu Val Ser Gln Ala Ser Leu Trp Phe Ala
                85                  90                  95

Thr Phe Leu Ser Val Phe Tyr Cys Lys Lys Ile Thr Thr Phe Asp Arg
            100                 105                 110

Pro Ala Tyr Leu Trp Leu Lys Gln Arg Ala Tyr Asn Leu Ser Leu Trp
        115                 120                 125

Cys Leu Leu Gly Tyr Phe Ile Ile Asn Leu Leu Leu Thr Val Gln Ile
    130                 135                 140

Gly Leu Thr Phe Tyr His Pro Pro Gln Gly Asn Ser Ser Ile Arg Tyr
145                 150                 155                 160

Pro Phe Glu Ser Trp Gln Tyr Leu Tyr Ala Phe Gln Leu Asn Ser Gly
                165                 170                 175

Ser Tyr Leu Pro Leu Val Val Phe Leu Val Ser Ser Gly Met Leu Ile
            180                 185                 190

Val Ser Leu Tyr Thr His His Lys Lys Met Lys Val His Ser Ala Gly
        195                 200                 205

Arg Arg Asp Val Arg Ala Lys Ala His Ile Thr Ala Leu Lys Ser Leu
    210                 215                 220

Gly Cys Phe Leu Leu Leu His Leu Val Tyr Ile Met Ala Ser Pro Phe
225                 230                 235                 240

Ser Ile Thr Ser Lys Thr Tyr Pro Pro Asp Leu Thr Ser Val Phe Ile

245                 250                 255

Trp Glu Thr Leu Met Ala Ala Tyr Pro Ser Leu His Ser Leu Ile Leu
            260                 265                 270

Ile Met Gly Ile Pro Arg Val Lys Gln Thr Cys Gln Lys Ile Leu Trp
        275                 280                 285

Lys Thr Val Cys Ala Arg Arg Cys Trp Gly Pro
    290                 295

<210>  9
<211>  957
<212>  DNA
<213>  Homo sapiens

<220>
<221>  CDS
<222>  (1)..(957)

<400>  9
atg gca gat aaa gtg cag act act tta ttg ttc tta gca gtt gga gag      48
Met Ala Asp Lys Val Gln Thr Thr Leu Leu Phe Leu Ala Val Gly Glu
1               5                   10                  15

ttt tca gtg ggg atc tta ggg aat gca ttc att gga ttg gta aac tgc      96
Phe Ser Val Gly Ile Leu Gly Asn Ala Phe Ile Gly Leu Val Asn Cys
            20                  25                  30

atg gac tgg gtc aag aag agg aaa att gcc tcc att gat tta atc ctc      144
Met Asp Trp Val Lys Lys Arg Lys Ile Ala Ser Ile Asp Leu Ile Leu
        35                  40                  45

aca agt ctg gcc ata tcc aga att tgt cta ttg tgc gta ata cta tta      192
Thr Ser Leu Ala Ile Ser Arg Ile Cys Leu Leu Cys Val Ile Leu Leu
    50                  55                  60

gat tgt ttt ata ttg gtg cta tat cca gat gtc tat gcc act ggt aaa      240
Asp Cys Phe Ile Leu Val Leu Tyr Pro Asp Val Tyr Ala Thr Gly Lys
65                  70                  75                  80

gaa atg aga atc att gac ttc ttc tgg aca cta acc aat cat tta agt      288
Glu Met Arg Ile Ile Asp Phe Phe Trp Thr Leu Thr Asn His Leu Ser
                85                  90                  95

atc tgg ttt gca acc tgc ctc agc att tac tat ttc ttc aag ata ggt      336
Ile Trp Phe Ala Thr Cys Leu Ser Ile Tyr Tyr Phe Phe Lys Ile Gly
            100                 105                 110

aat ttc ttt cac cca ctt ttc ctc tgg atg aag tgg aga att gac agg      384
Asn Phe Phe His Pro Leu Phe Leu Trp Met Lys Trp Arg Ile Asp Arg
        115                 120                 125

gtg att tcc tgg att cta ctg ggg tgc gtg gtt ctc tct gtg ttt att      432
Val Ile Ser Trp Ile Leu Leu Gly Cys Val Val Leu Ser Val Phe Ile
    130                 135                 140

agc ctt cca gcc act gag aat ttg aac gct gat ttc agg ttt tgt gtg      480

Ser Leu Pro Ala Thr Glu Asn Leu Asn Ala Asp Phe Arg Phe Cys Val
145                 150                 155                 160

aag gca aag agg aaa aca aac tta act tgg agt tgc aga gta aat aaa      528
Lys Ala Lys Arg Lys Thr Asn Leu Thr Trp Ser Cys Arg Val Asn Lys
                165                 170                 175

act caa cat gct tct acc aag tta ttt ctc aac ctg gca acg ctg ctc      576
Thr Gln His Ala Ser Thr Lys Leu Phe Leu Asn Leu Ala Thr Leu Leu
            180                 185                 190

ccc ttt tgt gtg tgc cta atg tcc ttt ttc ctc ttg atc ctc tcc ctg      624
Pro Phe Cys Val Cys Leu Met Ser Phe Phe Leu Leu Ile Leu Ser Leu
        195                 200                 205

cgg aga cat atc agg cga atg cag ctc agt gcc aca ggg tgc aga gac      672
Arg Arg His Ile Arg Arg Met Gln Leu Ser Ala Thr Gly Cys Arg Asp
    210                 215                 220

ccc agc aca gaa gcc cat gtg aga gcc ctg aaa gct gtc att tcc ttc      720
Pro Ser Thr Glu Ala His Val Arg Ala Leu Lys Ala Val Ile Ser Phe
225                 230                 235                 240

ctt ctc ctc ttt att gcc tac tat ttg tcc ttt ctc att gcc acc tcc      768
Leu Leu Leu Phe Ile Ala Tyr Tyr Leu Ser Phe Leu Ile Ala Thr Ser
                245                 250                 255

agc tac ttt atg cca gag acg gaa tta gct gtg att ttt ggt gag tcc      816
Ser Tyr Phe Met Pro Glu Thr Glu Leu Ala Val Ile Phe Gly Glu Ser
            260                 265                 270

ata gct cta atc tac ccc tca agt cat tca ttt atc cta ata ctg ggg      864
Ile Ala Leu Ile Tyr Pro Ser Ser His Ser Phe Ile Leu Ile Leu Gly
        275                 280                 285

aac aat aaa tta aga cat gca tct cta aag gtg att tgg aaa gta atg      912
Asn Asn Lys Leu Arg His Ala Ser Leu Lys Val Ile Trp Lys Val Met
    290                 295                 300

tct att cta aaa gga aga aaa ttc caa caa cat aaa caa atc tga          957
Ser Ile Leu Lys Gly Arg Lys Phe Gln Gln His Lys Gln Ile
305                 310                 315


<210>  10
<211>  318
<212>  PRT
<213>  Homo sapiens

<400>  10

Met Ala Asp Lys Val Gln Thr Thr Leu Leu Phe Leu Ala Val Gly Glu
1               5                   10                  15


Phe Ser Val Gly Ile Leu Gly Asn Ala Phe Ile Gly Leu Val Asn Cys
            20                  25                  30


Met Asp Trp Val Lys Lys Arg Lys Ile Ala Ser Ile Asp Leu Ile Leu
        35                  40                  45

Thr Ser Leu Ala Ile Ser Arg Ile Cys Leu Leu Cys Val Ile Leu Leu
    50                  55                  60

Asp Cys Phe Ile Leu Val Leu Tyr Pro Asp Val Tyr Ala Thr Gly Lys
65                  70                  75                  80

Glu Met Arg Ile Ile Asp Phe Phe Trp Thr Leu Thr Asn His Leu Ser
                85                  90                  95

Ile Trp Phe Ala Thr Cys Leu Ser Ile Tyr Tyr Phe Phe Lys Ile Gly
            100                 105                 110

Asn Phe Phe His Pro Leu Phe Leu Trp Met Lys Trp Arg Ile Asp Arg
        115                 120                 125

Val Ile Ser Trp Ile Leu Leu Gly Cys Val Val Leu Ser Val Phe Ile
    130                 135                 140

Ser Leu Pro Ala Thr Glu Asn Leu Asn Ala Asp Phe Arg Phe Cys Val
145                 150                 155                 160

Lys Ala Lys Arg Lys Thr Asn Leu Thr Trp Ser Cys Arg Val Asn Lys
                165                 170                 175

Thr Gln His Ala Ser Thr Lys Leu Phe Leu Asn Leu Ala Thr Leu Leu
            180                 185                 190

Pro Phe Cys Val Cys Leu Met Ser Phe Phe Leu Leu Ile Leu Ser Leu
        195                 200                 205

Arg Arg His Ile Arg Arg Met Gln Leu Ser Ala Thr Gly Cys Arg Asp
    210                 215                 220

Pro Ser Thr Glu Ala His Val Arg Ala Leu Lys Ala Val Ile Ser Phe
225                 230                 235                 240

Leu Leu Leu Phe Ile Ala Tyr Tyr Leu Ser Phe Leu Ile Ala Thr Ser
                245                 250                 255

Ser Tyr Phe Met Pro Glu Thr Glu Leu Ala Val Ile Phe Gly Glu Ser
            260                 265                 270

Ile Ala Leu Ile Tyr Pro Ser Ser His Ser Phe Ile Leu Ile Leu Gly
        275                 280                 285

Asn Asn Lys Leu Arg His Ala Ser Leu Lys Val Ile Trp Lys Val Met
    290                 295                 300

Ser Ile Leu Lys Gly Arg Lys Phe Gln Gln His Lys Gln Ile
305                 310                 315

<210>  11
<211>  930
<212>  DNA
<213>  Homo sapiens

<220>
<221>  CDS
<222>  (1)..(930)

<400>  11
atg ttc agt cct gca gat aac atc ttt ata atc cta ata act gga gaa      48
Met Phe Ser Pro Ala Asp Asn Ile Phe Ile Ile Leu Ile Thr Gly Glu
1               5                   10                  15

ttc ata cta gga ata ttg ggg aat gga tac att gca cta gtc aac tgg      96
Phe Ile Leu Gly Ile Leu Gly Asn Gly Tyr Ile Ala Leu Val Asn Trp
            20                  25                  30

att gac tgg att aag aag aaa aag att tcc aca gtt gac tac atc ctt      144
Ile Asp Trp Ile Lys Lys Lys Lys Ile Ser Thr Val Asp Tyr Ile Leu
        35                  40                  45

acc aat tta gtt atc gcc aga att tgt ttg atc agt gta atg gtt gta      192
Thr Asn Leu Val Ile Ala Arg Ile Cys Leu Ile Ser Val Met Val Val
    50                  55                  60

aat ggc att gta ata gta ctg aac cca gat gtt tat aca aaa aat aaa      240
Asn Gly Ile Val Ile Val Leu Asn Pro Asp Val Tyr Thr Lys Asn Lys
65                  70                  75                  80

caa cag ata gtc att ttt acc ttc tgg aca ttt gcc aac tac tta aat      288
Gln Gln Ile Val Ile Phe Thr Phe Trp Thr Phe Ala Asn Tyr Leu Asn
                85                  90                  95

atg tgg att acc acc tgc ctt aat gtc ttc tat ttt ctg aag ata gcc      336
Met Trp Ile Thr Thr Cys Leu Asn Val Phe Tyr Phe Leu Lys Ile Ala
            100                 105                 110

agt tcc tct cat cca ctt ttt ctc tgg ctg aag tgg aaa att gat atg      384
Ser Ser Ser His Pro Leu Phe Leu Trp Leu Lys Trp Lys Ile Asp Met
        115                 120                 125

gtg gtg cac tgg atc ctg ctg gga tgc ttt gcc att tcc ttg ttg gtc      432
Val Val His Trp Ile Leu Leu Gly Cys Phe Ala Ile Ser Leu Leu Val
    130                 135                 140

agc ctt ata gca gca ata gta ctg agt tgt gat tat agg ttt cat gca      480
Ser Leu Ile Ala Ala Ile Val Leu Ser Cys Asp Tyr Arg Phe His Ala
145                 150                 155                 160

att gcc aaa cat aaa aga aac att act gaa atg ttc cat gtg agt aaa      528
Ile Ala Lys His Lys Arg Asn Ile Thr Glu Met Phe His Val Ser Lys
                165                 170                 175

ata cca tac ttt gaa ccc ttg act ctc ttt aac ctg ttt gca att gtc      576
Ile Pro Tyr Phe Glu Pro Leu Thr Leu Phe Asn Leu Phe Ala Ile Val
            180                 185                 190

cca ttt att gtg tca ctg ata tca ttt ttc ctt tta gta aga tct tta 624
Pro Phe Ile Val Ser Leu Ile Ser Phe Phe Leu Leu Val Arg Ser Leu
        195                 200                 205

tgg aga cat acc aag caa ata aaa ctc tat gct acc ggc agt aga gac 672
Trp Arg His Thr Lys Gln Ile Lys Leu Tyr Ala Thr Gly Ser Arg Asp
    210                 215                 220

ccc agc aca gaa gtt cat gtg aga gcc att aaa act atg act tca ttt 720
Pro Ser Thr Glu Val His Val Arg Ala Ile Lys Thr Met Thr Ser Phe
225                 230                 235                 240

atc ttc ttt ttt ttc cta tac tat att tct tct att ttg atg acc ttt 768
Ile Phe Phe Phe Phe Leu Tyr Tyr Ile Ser Ser Ile Leu Met Thr Phe
                245                 250                 255

agc tat ctt atg aca aaa tac aag tta gct gtg gag ttt gga gag att 816
Ser Tyr Leu Met Thr Lys Tyr Lys Leu Ala Val Glu Phe Gly Glu Ile
            260                 265                 270

gca gca att ctc tac ccc ttg ggt cac tca ctt att tta att gtt tta 864
Ala Ala Ile Leu Tyr Pro Leu Gly His Ser Leu Ile Leu Ile Val Leu
        275                 280                 285

aat aat aaa ctg agg cag aca ttt gtc aga atg ctg aca tgt aga aaa 912
Asn Asn Lys Leu Arg Gln Thr Phe Val Arg Met Leu Thr Cys Arg Lys
    290                 295                 300

att gcc tgc atg ata tga 930
Ile Ala Cys Met Ile
305

<210> 12
<211> 309
<212> PRT
<213> Homo sapiens

<400> 12

Met Phe Ser Pro Ala Asp Asn Ile Phe Ile Ile Leu Ile Thr Gly Glu
1               5                   10                  15

Phe Ile Leu Gly Ile Leu Gly Asn Gly Tyr Ile Ala Leu Val Asn Trp
            20                  25                  30

Ile Asp Trp Ile Lys Lys Lys Lys Ile Ser Thr Val Asp Tyr Ile Leu
        35                  40                  45

Thr Asn Leu Val Ile Ala Arg Ile Cys Leu Ile Ser Val Met Val Val
    50                  55                  60

Asn Gly Ile Val Ile Val Leu Asn Pro Asp Val Tyr Thr Lys Asn Lys
65                  70                  75                  80

Gln Gln Ile Val Ile Phe Thr Phe Trp Thr Phe Ala Asn Tyr Leu Asn
                85                  90                  95

Met Trp Ile Thr Thr Cys Leu Asn Val Phe Tyr Phe Leu Lys Ile Ala
            100                 105                 110

Ser Ser Ser His Pro Leu Phe Leu Trp Leu Lys Trp Lys Ile Asp Met
        115                 120                 125

Val Val His Trp Ile Leu Leu Gly Cys Phe Ala Ile Ser Leu Leu Val
    130                 135                 140

Ser Leu Ile Ala Ala Ile Val Leu Ser Cys Asp Tyr Arg Phe His Ala
145                 150                 155                 160

Ile Ala Lys His Lys Arg Asn Ile Thr Glu Met Phe His Val Ser Lys
                165                 170                 175

Ile Pro Tyr Phe Glu Pro Leu Thr Leu Phe Asn Leu Phe Ala Ile Val
            180                 185                 190

Pro Phe Ile Val Ser Leu Ile Ser Phe Phe Leu Leu Val Arg Ser Leu
        195                 200                 205

Trp Arg His Thr Lys Gln Ile Lys Leu Tyr Ala Thr Gly Ser Arg Asp
    210                 215                 220

Pro Ser Thr Glu Val His Val Arg Ala Ile Lys Thr Met Thr Ser Phe
225                 230                 235                 240

Ile Phe Phe Phe Phe Leu Tyr Tyr Ile Ser Ser Ile Leu Met Thr Phe
                245                 250                 255

Ser Tyr Leu Met Thr Lys Tyr Lys Leu Ala Val Glu Phe Gly Glu Ile
            260                 265                 270

Ala Ala Ile Leu Tyr Pro Leu Gly His Ser Leu Ile Leu Ile Val Leu
        275                 280                 285

Asn Asn Lys Leu Arg Gln Thr Phe Val Arg Met Leu Thr Cys Arg Lys
    290                 295                 300

Ile Ala Cys Met Ile
305

<210>  13
<211>  939
<212>  DNA
<213>  Homo sapiens

<220>
<221>  CDS
<222>  (1)..(939)

<400>  13
atg cca agt gca ata gag gca ata tat att att tta att gct ggt gaa      48
Met Pro Ser Ala Ile Glu Ala Ile Tyr Ile Ile Leu Ile Ala Gly Glu
1               5                   10                  15

ttg acc ata ggg att tgg gga aat gga ttc att gta cta gtt aac tgc      96
Leu Thr Ile Gly Ile Trp Gly Asn Gly Phe Ile Val Leu Val Asn Cys
            20                  25                  30

att gac tgg ctc aaa aga aga gat att tcc ttg att gac atc atc ctg      144
Ile Asp Trp Leu Lys Arg Arg Asp Ile Ser Leu Ile Asp Ile Ile Leu
        35                  40                  45

atc agc ttg gcc atc tcc aga atc tgt ctg ctg tgt gta ata tca tta      192
Ile Ser Leu Ala Ile Ser Arg Ile Cys Leu Leu Cys Val Ile Ser Leu
    50                  55                  60

gat ggc ttc ttt atg ctg ctc ttt cca ggt aca tat ggc aat agc gtg      240
Asp Gly Phe Phe Met Leu Leu Phe Pro Gly Thr Tyr Gly Asn Ser Val
65                  70                  75                  80

cta gta agc att gtg aat gtt gtc tgg aca ttt gcc aat aat tca agt      288
Leu Val Ser Ile Val Asn Val Val Trp Thr Phe Ala Asn Asn Ser Ser
                85                  90                  95

ctc tgg ttt act tct tgc ctc agt atc ttc tat tta ctc aag ata gcc      336
Leu Trp Phe Thr Ser Cys Leu Ser Ile Phe Tyr Leu Leu Lys Ile Ala
            100                 105                 110

aat ata tcg cac cca ttt ttc ttc tgg ctg aag cta aag atc aac aag      384
Asn Ile Ser His Pro Phe Phe Phe Trp Leu Lys Leu Lys Ile Asn Lys
        115                 120                 125

gtc atg ctt gcg att ctt ctg ggg tcc ttt ctt atc tct tta att att      432
Val Met Leu Ala Ile Leu Leu Gly Ser Phe Leu Ile Ser Leu Ile Ile
    130                 135                 140

agt gtt cca aag aat gat gat atg tgg tat cac ctt ttc aaa gtc agt      480
Ser Val Pro Lys Asn Asp Asp Met Trp Tyr His Leu Phe Lys Val Ser
145                 150                 155                 160

cat gaa gaa aac att act tgg aaa ttc aaa gtg agt aaa att cca ggt      528
His Glu Glu Asn Ile Thr Trp Lys Phe Lys Val Ser Lys Ile Pro Gly
                165                 170                 175

act ttc aaa cag tta acc ctg aac ctg ggg gtg atg gtt ccc ttt atc      576
Thr Phe Lys Gln Leu Thr Leu Asn Leu Gly Val Met Val Pro Phe Ile
            180                 185                 190

ctt tgc ctg atc tca ttt ttc ttg tta ctt ttc tcc cta gtt aga cac      624
Leu Cys Leu Ile Ser Phe Phe Leu Leu Leu Phe Ser Leu Val Arg His
        195                 200                 205

acc aag cag att cga ctg cat gct aca ggg ttc aga gac ccc agt aca      672
Thr Lys Gln Ile Arg Leu His Ala Thr Gly Phe Arg Asp Pro Ser Thr
    210                 215                 220

gag gcc cac atg agg gcc ata aag gca gtg atc atc ttt ctg ctc ctc      720

Glu Ala His Met Arg Ala Ile Lys Ala Val Ile Ile Phe Leu Leu Leu
225                 230                 235                 240

ctc atc gtg tac tac cca gtc ttt ctt gtt atg acc tct agc gct ctg       768
Leu Ile Val Tyr Tyr Pro Val Phe Leu Val Met Thr Ser Ser Ala Leu
                245                 250                 255

att cct cag gga aaa tta gtg ttg atg att ggt gac ata gta act gtc       816
Ile Pro Gln Gly Lys Leu Val Leu Met Ile Gly Asp Ile Val Thr Val
            260                 265                 270

att ttc cca tca agc cat tca ttc att cta att atg gga aat agc aag       864
Ile Phe Pro Ser Ser His Ser Phe Ile Leu Ile Met Gly Asn Ser Lys
        275                 280                 285

ttg agg gaa gct ttt ctg aag atg tta aga ttt gtg aag tgt ttc ctt       912
Leu Arg Glu Ala Phe Leu Lys Met Leu Arg Phe Val Lys Cys Phe Leu
    290                 295                 300

aga aga aga aag cct ttt gtt cca tag                                   939
Arg Arg Arg Lys Pro Phe Val Pro
305                 310


<210>  14
<211>  312
<212>  PRT
<213>  Homo sapiens

<400>  14

Met Pro Ser Ala Ile Glu Ala Ile Tyr Ile Ile Leu Ile Ala Gly Glu
1               5                   10                  15


Leu Thr Ile Gly Ile Trp Gly Asn Gly Phe Ile Val Leu Val Asn Cys
            20                  25                  30


Ile Asp Trp Leu Lys Arg Arg Asp Ile Ser Leu Ile Asp Ile Ile Leu
        35                  40                  45


Ile Ser Leu Ala Ile Ser Arg Ile Cys Leu Leu Cys Val Ile Ser Leu
    50                  55                  60


Asp Gly Phe Phe Met Leu Leu Phe Pro Gly Thr Tyr Gly Asn Ser Val
65                  70                  75                  80


Leu Val Ser Ile Val Asn Val Val Trp Thr Phe Ala Asn Asn Ser Ser
                85                  90                  95


Leu Trp Phe Thr Ser Cys Leu Ser Ile Phe Tyr Leu Leu Lys Ile Ala
            100                 105                 110


Asn Ile Ser His Pro Phe Phe Phe Trp Leu Lys Leu Lys Ile Asn Lys
        115                 120                 125

Val Met Leu Ala Ile Leu Leu Gly Ser Phe Leu Ile Ser Leu Ile Ile
    130                 135                 140

Ser Val Pro Lys Asn Asp Asp Met Trp Tyr His Leu Phe Lys Val Ser
145                 150                 155                 160

His Glu Glu Asn Ile Thr Trp Lys Phe Lys Val Ser Lys Ile Pro Gly
                165                 170                 175

Thr Phe Lys Gln Leu Thr Leu Asn Leu Gly Val Met Val Pro Phe Ile
            180                 185                 190

Leu Cys Leu Ile Ser Phe Phe Leu Leu Leu Phe Ser Leu Val Arg His
        195                 200                 205

Thr Lys Gln Ile Arg Leu His Ala Thr Gly Phe Arg Asp Pro Ser Thr
    210                 215                 220

Glu Ala His Met Arg Ala Ile Lys Ala Val Ile Ile Phe Leu Leu Leu
225                 230                 235                 240

Leu Ile Val Tyr Tyr Pro Val Phe Leu Val Met Thr Ser Ser Ala Leu
                245                 250                 255

Ile Pro Gln Gly Lys Leu Val Leu Met Ile Gly Asp Ile Val Thr Val
            260                 265                 270

Ile Phe Pro Ser Ser His Ser Phe Ile Leu Ile Met Gly Asn Ser Lys
        275                 280                 285

Leu Arg Glu Ala Phe Leu Lys Met Leu Arg Phe Val Lys Cys Phe Leu
    290                 295                 300

Arg Arg Arg Lys Pro Phe Val Pro
305                 310

<210>  15
<211>  924
<212>  DNA
<213>  Homo sapiens

<220>
<221>  CDS
<222>  (1)..(924)

<400>  15
atg cta cgt gta gtg gaa ggc atc ttc att ttt gtt gta gtt agt gag      48
Met Leu Arg Val Val Glu Gly Ile Phe Ile Phe Val Val Val Ser Glu
1               5                   10                  15

tca gtg ttt ggg gtt ttg ggg aat gga ttt att gga ctt gta aac tgc 96
Ser Val Phe Gly Val Leu Gly Asn Gly Phe Ile Gly Leu Val Asn Cys
            20                  25                  30

att gac tgt gcc aag aat aag tta tct acg att ggc ttt att ctc acc 144
Ile Asp Cys Ala Lys Asn Lys Leu Ser Thr Ile Gly Phe Ile Leu Thr
        35                  40                  45

ggc tta gct att tca aga att ttt ctg ata tgg ata ata att aca gat 192
Gly Leu Ala Ile Ser Arg Ile Phe Leu Ile Trp Ile Ile Ile Thr Asp
    50                  55                  60

gga ttt ata cag ata ttc tct cca aat ata tat gcc tcc ggt aac cta 240
Gly Phe Ile Gln Ile Phe Ser Pro Asn Ile Tyr Ala Ser Gly Asn Leu
65                  70                  75                  80

att gaa tat att agt tac ttt tgg gta att ggt aat caa tca agt atg 288
Ile Glu Tyr Ile Ser Tyr Phe Trp Val Ile Gly Asn Gln Ser Ser Met
                85                  90                  95

tgg ttt gcc acc agc ctc agc atc ttc tat ttc ctg aag ata gca aat 336
Trp Phe Ala Thr Ser Leu Ser Ile Phe Tyr Phe Leu Lys Ile Ala Asn
            100                 105                 110

ttt tcc aac tac ata ttt ctc tgg ttg aag agc aga aca aat atg gtt 384
Phe Ser Asn Tyr Ile Phe Leu Trp Leu Lys Ser Arg Thr Asn Met Val
        115                 120                 125

ctt ccc ttc atg ata gta ttc tta ctt att tca tcg tta ctt aat ttt 432
Leu Pro Phe Met Ile Val Phe Leu Leu Ile Ser Ser Leu Leu Asn Phe
    130                 135                 140

gca tac att gcg aag att ctt aat gat tat aaa acg aag aat gac aca 480
Ala Tyr Ile Ala Lys Ile Leu Asn Asp Tyr Lys Thr Lys Asn Asp Thr
145                 150                 155                 160

gtc tgg gat ctc aac atg tat aaa agt gaa tac ttt att aaa cag att 528
Val Trp Asp Leu Asn Met Tyr Lys Ser Glu Tyr Phe Ile Lys Gln Ile
                165                 170                 175

ttg cta aat ctg gga gtc att ttc ttc ttt aca cta tcc cta att aca 576
Leu Leu Asn Leu Gly Val Ile Phe Phe Phe Thr Leu Ser Leu Ile Thr
            180                 185                 190

tgt att ttt tta atc att tcc ctt tgg aga cac aac agg cag atg caa 624
Cys Ile Phe Leu Ile Ile Ser Leu Trp Arg His Asn Arg Gln Met Gln
        195                 200                 205

tcg aat gtg aca gga ttg aga gac tcc aac aca gaa gct cat gtg aag 672
Ser Asn Val Thr Gly Leu Arg Asp Ser Asn Thr Glu Ala His Val Lys
    210                 215                 220

gca atg aaa gtt ttg ata tct ttc atc atc ctc ttt atc ttg tat ttt 720
Ala Met Lys Val Leu Ile Ser Phe Ile Ile Leu Phe Ile Leu Tyr Phe
225                 230                 235                 240

ata ggc atg gcc ata gaa ata tca tgt ttt act gtg cga gaa aac aaa 768
Ile Gly Met Ala Ile Glu Ile Ser Cys Phe Thr Val Arg Glu Asn Lys
                245                 250                 255

ctg ctg ctt atg ttt gga atg aca acc aca gcc atc tat ccc tgg ggt 816
Leu Leu Leu Met Phe Gly Met Thr Thr Thr Ala Ile Tyr Pro Trp Gly
            260                 265                 270

cac tca ttt atc tta att cta gga aac agc aag cta aag caa gcc tct 864
His Ser Phe Ile Leu Ile Leu Gly Asn Ser Lys Leu Lys Gln Ala Ser
        275                 280                 285

ttg agg gta ctg cag caa ttg aag tgc tgt gag aaa agg aaa aat ctc 912
Leu Arg Val Leu Gln Gln Leu Lys Cys Cys Glu Lys Arg Lys Asn Leu
    290                 295                 300

aga gtc aca tag 924
Arg Val Thr
305

<210> 16
<211> 307
<212> PRT
<213> Homo sapiens

<400> 16

Met Leu Arg Val Val Glu Gly Ile Phe Ile Phe Val Val Val Ser Glu
1               5                   10                  15

Ser Val Phe Gly Val Leu Gly Asn Gly Phe Ile Gly Leu Val Asn Cys
            20                  25                  30

Ile Asp Cys Ala Lys Asn Lys Leu Ser Thr Ile Gly Phe Ile Leu Thr
        35                  40                  45

Gly Leu Ala Ile Ser Arg Ile Phe Leu Ile Trp Ile Ile Ile Thr Asp
    50                  55                  60

Gly Phe Ile Gln Ile Phe Ser Pro Asn Ile Tyr Ala Ser Gly Asn Leu
65                  70                  75                  80

Ile Glu Tyr Ile Ser Tyr Phe Trp Val Ile Gly Asn Gln Ser Ser Met
                85                  90                  95

Trp Phe Ala Thr Ser Leu Ser Ile Phe Tyr Phe Leu Lys Ile Ala Asn
            100                 105                 110

Phe Ser Asn Tyr Ile Phe Leu Trp Leu Lys Ser Arg Thr Asn Met Val
        115                 120                 125

Leu Pro Phe Met Ile Val Phe Leu Leu Ile Ser Ser Leu Leu Asn Phe
    130                 135                 140

Ala Tyr Ile Ala Lys Ile Leu Asn Asp Tyr Lys Thr Lys Asn Asp Thr
145                 150                 155                 160

Val Trp Asp Leu Asn Met Tyr Lys Ser Glu Tyr Phe Ile Lys Gln Ile
                165                 170                 175

Leu Leu Asn Leu Gly Val Ile Phe Phe Phe Thr Leu Ser Leu Ile Thr
            180                 185                 190

Cys Ile Phe Leu Ile Ile Ser Leu Trp Arg His Asn Arg Gln Met Gln
        195                 200                 205

Ser Asn Val Thr Gly Leu Arg Asp Ser Asn Thr Glu Ala His Val Lys
    210                 215                 220

Ala Met Lys Val Leu Ile Ser Phe Ile Ile Leu Phe Ile Leu Tyr Phe
225                 230                 235                 240

Ile Gly Met Ala Ile Glu Ile Ser Cys Phe Thr Val Arg Glu Asn Lys
                245                 250                 255

Leu Leu Leu Met Phe Gly Met Thr Thr Thr Ala Ile Tyr Pro Trp Gly
            260                 265                 270

His Ser Phe Ile Leu Ile Leu Gly Asn Ser Lys Leu Lys Gln Ala Ser
        275                 280                 285

Leu Arg Val Leu Gln Gln Leu Lys Cys Cys Glu Lys Arg Lys Asn Leu
    290                 295                 300

Arg Val Thr
305

<210> 17
<211> 912
<212> DNA
<213> Homo sapiens

<220>
<221> CDS
<222> (1)..(912)

<400> 17
atg gaa agt gcc ctg ccg agt atc ttc act ctt gta ata att gca gaa 48
Met Glu Ser Ala Leu Pro Ser Ile Phe Thr Leu Val Ile Ile Ala Glu
1               5                   10                  15

ttc ata att ggg aat ttg agc aat gga ttt ata gta ctg atc aac tgc 96
Phe Ile Ile Gly Asn Leu Ser Asn Gly Phe Ile Val Leu Ile Asn Cys
            20                  25                  30

att gac tgg gtc agt aaa aga gag ctg tcc tca gtc gat aaa ctc ctc 144
Ile Asp Trp Val Ser Lys Arg Glu Leu Ser Ser Val Asp Lys Leu Leu
        35                  40                  45

att atc ttg gca atc tcc aga att ggg ctg atc tgg gaa ata tta gta 192
Ile Ile Leu Ala Ile Ser Arg Ile Gly Leu Ile Trp Glu Ile Leu Val

50                  55                  60

agt tgg ttt tta gct ctg cat tat cta gcc ata ttt gtg tct gga aca          240
Ser Trp Phe Leu Ala Leu His Tyr Leu Ala Ile Phe Val Ser Gly Thr
65                  70                  75                  80

gga tta aga att atg att ttt agc tgg ata gtt tct aat cac ttc aat          288
Gly Leu Arg Ile Met Ile Phe Ser Trp Ile Val Ser Asn His Phe Asn
                85                  90                  95

ctc tgg ctt gct aca atc ttc agc atc ttt tat ttg ctc aaa ata gcg          336
Leu Trp Leu Ala Thr Ile Phe Ser Ile Phe Tyr Leu Leu Lys Ile Ala
            100                 105                 110

agt ttc tct agc cct gct ttt ctc tat ttg aag tgg aga gta aac aaa          384
Ser Phe Ser Ser Pro Ala Phe Leu Tyr Leu Lys Trp Arg Val Asn Lys
        115                 120                 125

gtg att ctg atg ata ctg cta gga acc ttg gtc ttc tta ttt tta aat          432
Val Ile Leu Met Ile Leu Leu Gly Thr Leu Val Phe Leu Phe Leu Asn
    130                 135                 140

ctg ata caa ata aac atg cat ata aaa gac tgg ctg gac cga tat gaa          480
Leu Ile Gln Ile Asn Met His Ile Lys Asp Trp Leu Asp Arg Tyr Glu
145                 150                 155                 160

aga aac aca act tgg aat ttc agt atg agt gac ttt gaa aca ttt tca          528
Arg Asn Thr Thr Trp Asn Phe Ser Met Ser Asp Phe Glu Thr Phe Ser
                165                 170                 175

gtg tcg gtc aaa ttc act atg act atg ttc agt cta aca cca ttt act          576
Val Ser Val Lys Phe Thr Met Thr Met Phe Ser Leu Thr Pro Phe Thr
            180                 185                 190

gtg gcc ttc atc tct ttt ctc ctg tta att ttc tcc ctg cag aaa cat          624
Val Ala Phe Ile Ser Phe Leu Leu Leu Ile Phe Ser Leu Gln Lys His
        195                 200                 205

ctc cag aaa atg caa ctc aat tac aaa gga cac aga gac ccc agg acc          672
Leu Gln Lys Met Gln Leu Asn Tyr Lys Gly His Arg Asp Pro Arg Thr
    210                 215                 220

aag gtc cat aca aat gcc ttg aaa att gtg atc tca ttc ctt tta ttc          720
Lys Val His Thr Asn Ala Leu Lys Ile Val Ile Ser Phe Leu Leu Phe
225                 230                 235                 240

tat gct agt ttc ttt cta tgt gtt ctc ata tca tgg att tct gag ctg          768
Tyr Ala Ser Phe Phe Leu Cys Val Leu Ile Ser Trp Ile Ser Glu Leu
                245                 250                 255

tat cag aac aca gtg atc tac atg ctt tgt gag acg att gga gtc ttc          816
Tyr Gln Asn Thr Val Ile Tyr Met Leu Cys Glu Thr Ile Gly Val Phe
            260                 265                 270

tct cct tca agc cac tcc ttt ctt ctg att cta gga aac gct aag tta          864
Ser Pro Ser Ser His Ser Phe Leu Leu Ile Leu Gly Asn Ala Lys Leu
        275                 280                 285

aga cag gcc ttt ctt ttg gtg gca gct aag gta tgg gct aaa cga tga          912
Arg Gln Ala Phe Leu Leu Val Ala Ala Lys Val Trp Ala Lys Arg
    290                 295                 300

<210>  18
<211>  303
<212>  PRT
<213>  Homo sapiens

<400>  18

Met Glu Ser Ala Leu Pro Ser Ile Phe Thr Leu Val Ile Ile Ala Glu
1               5                   10                  15

Phe Ile Ile Gly Asn Leu Ser Asn Gly Phe Ile Val Leu Ile Asn Cys
            20                  25                  30

Ile Asp Trp Val Ser Lys Arg Glu Leu Ser Ser Val Asp Lys Leu Leu
        35                  40                  45

Ile Ile Leu Ala Ile Ser Arg Ile Gly Leu Ile Trp Glu Ile Leu Val
    50                  55                  60

Ser Trp Phe Leu Ala Leu His Tyr Leu Ala Ile Phe Val Ser Gly Thr
65                  70                  75                  80

Gly Leu Arg Ile Met Ile Phe Ser Trp Ile Val Ser Asn His Phe Asn
                85                  90                  95

Leu Trp Leu Ala Thr Ile Phe Ser Ile Phe Tyr Leu Leu Lys Ile Ala
            100                 105                 110

Ser Phe Ser Ser Pro Ala Phe Leu Tyr Leu Lys Trp Arg Val Asn Lys
        115                 120                 125

Val Ile Leu Met Ile Leu Leu Gly Thr Leu Val Phe Leu Phe Leu Asn
    130                 135                 140

Leu Ile Gln Ile Asn Met His Ile Lys Asp Trp Leu Asp Arg Tyr Glu
145                 150                 155                 160

Arg Asn Thr Thr Trp Asn Phe Ser Met Ser Asp Phe Glu Thr Phe Ser
                165                 170                 175

Val Ser Val Lys Phe Thr Met Thr Met Phe Ser Leu Thr Pro Phe Thr
            180                 185                 190

Val Ala Phe Ile Ser Phe Leu Leu Leu Ile Phe Ser Leu Gln Lys His
        195                 200                 205

Leu Gln Lys Met Gln Leu Asn Tyr Lys Gly His Arg Asp Pro Arg Thr
    210                 215                 220

Lys Val His Thr Asn Ala Leu Lys Ile Val Ile Ser Phe Leu Leu Phe
225                 230                 235                 240

Tyr Ala Ser Phe Phe Leu Cys Val Leu Ile Ser Trp Ile Ser Glu Leu
                245                 250                 255

Tyr Gln Asn Thr Val Ile Tyr Met Leu Cys Glu Thr Ile Gly Val Phe
            260                 265                 270

Ser Pro Ser Ser His Ser Phe Leu Leu Ile Leu Gly Asn Ala Lys Leu
        275                 280                 285

Arg Gln Ala Phe Leu Leu Val Ala Ala Lys Val Trp Ala Lys Arg
    290                 295                 300

<210>  19
<211>  954
<212>  DNA
<213>  Homo sapiens

<220>
<221>  CDS
<222>  (1)..(954)

<400>  19
atg ggt ggt gtc ata aag agc ata ttt aca ttc gtt tta att gtg gaa      48
Met Gly Gly Val Ile Lys Ser Ile Phe Thr Phe Val Leu Ile Val Glu
1               5                   10                  15

ttt ata att gga aat tta gga aat agt ttc ata gca ctg gtg aac tgt      96
Phe Ile Ile Gly Asn Leu Gly Asn Ser Phe Ile Ala Leu Val Asn Cys
            20                  25                  30

att gac tgg gtc aag gga aga aag atc tct tcg gtt gat cgg atc ctc      144
Ile Asp Trp Val Lys Gly Arg Lys Ile Ser Ser Val Asp Arg Ile Leu
        35                  40                  45

act gct ttg gca atc tct cga att agc ctg gtt tgg tta ata ttc gga      192
Thr Ala Leu Ala Ile Ser Arg Ile Ser Leu Val Trp Leu Ile Phe Gly
    50                  55                  60

agc tgg tgt gtg tct gtg ttt ttc cca gct tta ttt gcc act gaa aaa      240
Ser Trp Cys Val Ser Val Phe Phe Pro Ala Leu Phe Ala Thr Glu Lys
65                  70                  75                  80

atg ttc aga atg ctt act aat atc tgg aca gtg atc aat cat ttt agt      288
Met Phe Arg Met Leu Thr Asn Ile Trp Thr Val Ile Asn His Phe Ser
                85                  90                  95

gtc tgg tta gct aca ggc ctc ggt act ttt tat ttt ctc aag ata gcc      336
Val Trp Leu Ala Thr Gly Leu Gly Thr Phe Tyr Phe Leu Lys Ile Ala
            100                 105                 110

aat ttt tct aac tct att ttt ctc tac cta aag tgg agg gtt aaa aag      384
Asn Phe Ser Asn Ser Ile Phe Leu Tyr Leu Lys Trp Arg Val Lys Lys
        115                 120                 125

gtg gtt ttg gtg ctg ctt ctt gtg act tcg gtc ttc ttg ttt tta aat 432
Val Val Leu Val Leu Leu Leu Val Thr Ser Val Phe Leu Phe Leu Asn
    130                 135                 140

att gca ctg ata aac atc cat ata aat gcc agt atc aat gga tac aga 480
Ile Ala Leu Ile Asn Ile His Ile Asn Ala Ser Ile Asn Gly Tyr Arg
145                 150                 155                 160

aga aac aag act tgc agt tct gat tca agt aac ttt aca cga ttt tcc 528
Arg Asn Lys Thr Cys Ser Ser Asp Ser Ser Asn Phe Thr Arg Phe Ser
                165                 170                 175

agt ctt att gta tta acc agc act gtg ttc att ttc ata ccc ttt act 576
Ser Leu Ile Val Leu Thr Ser Thr Val Phe Ile Phe Ile Pro Phe Thr
            180                 185                 190

ttg tcc ctg gca atg ttt ctt ctc ctc atc ttc tcc atg tgg aaa cat 624
Leu Ser Leu Ala Met Phe Leu Leu Leu Ile Phe Ser Met Trp Lys His
        195                 200                 205

cgc aag aag atg cag cac act gtc aaa ata tcc gga gac gcc agc acc 672
Arg Lys Lys Met Gln His Thr Val Lys Ile Ser Gly Asp Ala Ser Thr
    210                 215                 220

aaa gcc cac aga gga gtt aaa agt gtg atc act ttc ttc cta ctc tat 720
Lys Ala His Arg Gly Val Lys Ser Val Ile Thr Phe Phe Leu Leu Tyr
225                 230                 235                 240

gcc att ttc tct ctg tct ttt ttc ata tca gtt tgg acc tct gaa agg 768
Ala Ile Phe Ser Leu Ser Phe Phe Ile Ser Val Trp Thr Ser Glu Arg
                245                 250                 255

ttg gag gaa aat cta att att ctt tcc cag gtg atg gga atg gct tat 816
Leu Glu Glu Asn Leu Ile Ile Leu Ser Gln Val Met Gly Met Ala Tyr
            260                 265                 270

cct tca tgt cac tca tgt gtt ctg att ctt gga aac aag aag ctg aga 864
Pro Ser Cys His Ser Cys Val Leu Ile Leu Gly Asn Lys Lys Leu Arg
        275                 280                 285

cag gcc tct ctg tca gtg cta ctg tgg ctg agg tac atg ttc aaa gat 912
Gln Ala Ser Leu Ser Val Leu Leu Trp Leu Arg Tyr Met Phe Lys Asp
    290                 295                 300

ggg gag ccc tca ggt cac aaa gaa ttt aga gaa tca tct tga 954
Gly Glu Pro Ser Gly His Lys Glu Phe Arg Glu Ser Ser
305                 310                 315

<210> 20
<211> 317
<212> PRT
<213> Homo sapiens

<400> 20

Met Gly Gly Val Ile Lys Ser Ile Phe Thr Phe Val Leu Ile Val Glu
1               5                   10                  15

Phe Ile Ile Gly Asn Leu Gly Asn Ser Phe Ile Ala Leu Val Asn Cys
            20                  25                  30

Ile Asp Trp Val Lys Gly Arg Lys Ile Ser Ser Val Asp Arg Ile Leu
        35                  40                  45

Thr Ala Leu Ala Ile Ser Arg Ile Ser Leu Val Trp Leu Ile Phe Gly
    50                  55                  60

Ser Trp Cys Val Ser Val Phe Phe Pro Ala Leu Phe Ala Thr Glu Lys
65                  70                  75                  80

Met Phe Arg Met Leu Thr Asn Ile Trp Thr Val Ile Asn His Phe Ser
                85                  90                  95

Val Trp Leu Ala Thr Gly Leu Gly Thr Phe Tyr Phe Leu Lys Ile Ala
            100                 105                 110

Asn Phe Ser Asn Ser Ile Phe Leu Tyr Leu Lys Trp Arg Val Lys Lys
        115                 120                 125

Val Val Leu Val Leu Leu Leu Val Thr Ser Val Phe Leu Phe Leu Asn
    130                 135                 140

Ile Ala Leu Ile Asn Ile His Ile Asn Ala Ser Ile Asn Gly Tyr Arg
145                 150                 155                 160

Arg Asn Lys Thr Cys Ser Ser Asp Ser Ser Asn Phe Thr Arg Phe Ser
                165                 170                 175

Ser Leu Ile Val Leu Thr Ser Thr Val Phe Ile Phe Ile Pro Phe Thr
            180                 185                 190

Leu Ser Leu Ala Met Phe Leu Leu Leu Ile Phe Ser Met Trp Lys His
        195                 200                 205

Arg Lys Lys Met Gln His Thr Val Lys Ile Ser Gly Asp Ala Ser Thr
    210                 215                 220

Lys Ala His Arg Gly Val Lys Ser Val Ile Thr Phe Phe Leu Leu Tyr
225                 230                 235                 240

Ala Ile Phe Ser Leu Ser Phe Phe Ile Ser Val Trp Thr Ser Glu Arg
                245                 250                 255

Leu Glu Glu Asn Leu Ile Ile Leu Ser Gln Val Met Gly Met Ala Tyr
            260                 265                 270

Pro Ser Cys His Ser Cys Val Leu Ile Leu Gly Asn Lys Lys Leu Arg
        275                 280                 285

Gln Ala Ser Leu Ser Val Leu Leu Trp Leu Arg Tyr Met Phe Lys Asp
    290                 295                 300

Gly Glu Pro Ser Gly His Lys Glu Phe Arg Glu Ser Ser
305                 310                 315

<210>  21
<211>  876
<212>  DNA
<213>  Homo sapiens

<220>
<221>  CDS
<222>  (1)..(876)

<400>  21
atg ata ccc atc caa ctc act gtc ttc ttc atg atc atc tat gtg ctt      48
Met Ile Pro Ile Gln Leu Thr Val Phe Phe Met Ile Ile Tyr Val Leu
1               5                   10                  15

gag tcc ttg aca att att gtg cag agc agc cta att gtt gca gtg ctg      96
Glu Ser Leu Thr Ile Ile Val Gln Ser Ser Leu Ile Val Ala Val Leu
            20                  25                  30

ggc aga gaa tgg ctg caa gtc aga agg ctg atg cct gtg gac atg att      144
Gly Arg Glu Trp Leu Gln Val Arg Arg Leu Met Pro Val Asp Met Ile
        35                  40                  45

ctc atc agc ctg ggc atc tct cgc ttc tgt cta cag tgg gca tca atg      192
Leu Ile Ser Leu Gly Ile Ser Arg Phe Cys Leu Gln Trp Ala Ser Met
    50                  55                  60

ctg aac aat ttt tgc tcc tat ttt aat ttg aat tat gta ctt tgc aac      240
Leu Asn Asn Phe Cys Ser Tyr Phe Asn Leu Asn Tyr Val Leu Cys Asn
65                  70                  75                  80

tta aca atc acc tgg gaa ttt ttt aat atc ctt aca ttc tgg tta aac      288
Leu Thr Ile Thr Trp Glu Phe Phe Asn Ile Leu Thr Phe Trp Leu Asn
                85                  90                  95

agc ttg ctt acc gtg ttc tac tgc atc aag gtc tct tct ttc acc cat      336
Ser Leu Leu Thr Val Phe Tyr Cys Ile Lys Val Ser Ser Phe Thr His
            100                 105                 110

cac atc ttt ctc tgg ctg agg tgg aga att ttg agg ttg ttt ccc tgg      384
His Ile Phe Leu Trp Leu Arg Trp Arg Ile Leu Arg Leu Phe Pro Trp
        115                 120                 125

ata tta ctg ggt tct ctg atg att act tgt gta aca atc atc cct tca      432
Ile Leu Leu Gly Ser Leu Met Ile Thr Cys Val Thr Ile Ile Pro Ser
    130                 135                 140

gct att ggg aat tac att caa att cag tta ctc acc atg gag cat cta      480
Ala Ile Gly Asn Tyr Ile Gln Ile Gln Leu Leu Thr Met Glu His Leu
145                 150                 155                 160

cca aga aac agc act gta act gac aaa ctt gaa aat ttt cat cag tat      528
Pro Arg Asn Ser Thr Val Thr Asp Lys Leu Glu Asn Phe His Gln Tyr

165                 170                 175

cag ttc cag gct cat aca gtt gca ttg gtt att cct ttc atc ctg ttc       576
Gln Phe Gln Ala His Thr Val Ala Leu Val Ile Pro Phe Ile Leu Phe
            180                 185                 190

ctg gcc tcc acc atc ttt ctc atg gca tca ctg acc aag cag ata caa       624
Leu Ala Ser Thr Ile Phe Leu Met Ala Ser Leu Thr Lys Gln Ile Gln
        195                 200                 205

cat cat agc act ggt cac tgc aat cca agc atg aaa gcg cgc ttc act       672
His His Ser Thr Gly His Cys Asn Pro Ser Met Lys Ala Arg Phe Thr
    210                 215                 220

gcc ctg agg tcc ctt gcc gtc tta ttt att gtg ttt acc tct tac ttt       720
Ala Leu Arg Ser Leu Ala Val Leu Phe Ile Val Phe Thr Ser Tyr Phe
225                 230                 235                 240

cta acc ata ctc atc acc att ata ggt act cta ttt gat aag aga tgt       768
Leu Thr Ile Leu Ile Thr Ile Ile Gly Thr Leu Phe Asp Lys Arg Cys
                245                 250                 255

tgg tta tgg gtc tgg gaa gct ttt gtc tat gct ttc atc tta atg cat       816
Trp Leu Trp Val Trp Glu Ala Phe Val Tyr Ala Phe Ile Leu Met His
            260                 265                 270

tcc act tca ctg atg ctg agc agc cct acg ttg aaa agg att cta aag       864
Ser Thr Ser Leu Met Leu Ser Ser Pro Thr Leu Lys Arg Ile Leu Lys
        275                 280                 285

gga aag tgc tag                                                       876
Gly Lys Cys
    290

<210>  22
<211>  291
<212>  PRT
<213>  Homo sapiens

<400>  22

Met Ile Pro Ile Gln Leu Thr Val Phe Phe Met Ile Ile Tyr Val Leu
1               5                   10                  15

Glu Ser Leu Thr Ile Ile Val Gln Ser Ser Leu Ile Val Ala Val Leu
            20                  25                  30

Gly Arg Glu Trp Leu Gln Val Arg Arg Leu Met Pro Val Asp Met Ile
        35                  40                  45

Leu Ile Ser Leu Gly Ile Ser Arg Phe Cys Leu Gln Trp Ala Ser Met
    50                  55                  60

Leu Asn Asn Phe Cys Ser Tyr Phe Asn Leu Asn Tyr Val Leu Cys Asn
65                  70                  75                  80

Leu Thr Ile Thr Trp Glu Phe Phe Asn Ile Leu Thr Phe Trp Leu Asn

85                  90                  95

Ser Leu Leu Thr Val Phe Tyr Cys Ile Lys Val Ser Ser Phe Thr His
            100                 105                 110

His Ile Phe Leu Trp Leu Arg Trp Arg Ile Leu Arg Leu Phe Pro Trp
        115                 120                 125

Ile Leu Leu Gly Ser Leu Met Ile Thr Cys Val Thr Ile Ile Pro Ser
    130                 135                 140

Ala Ile Gly Asn Tyr Ile Gln Ile Gln Leu Leu Thr Met Glu His Leu
145                 150                 155                 160

Pro Arg Asn Ser Thr Val Thr Asp Lys Leu Glu Asn Phe His Gln Tyr
                165                 170                 175

Gln Phe Gln Ala His Thr Val Ala Leu Val Ile Pro Phe Ile Leu Phe
            180                 185                 190

Leu Ala Ser Thr Ile Phe Leu Met Ala Ser Leu Thr Lys Gln Ile Gln
        195                 200                 205

His His Ser Thr Gly His Cys Asn Pro Ser Met Lys Ala Arg Phe Thr
    210                 215                 220

Ala Leu Arg Ser Leu Ala Val Leu Phe Ile Val Phe Thr Ser Tyr Phe
225                 230                 235                 240

Leu Thr Ile Leu Ile Thr Ile Ile Gly Thr Leu Phe Asp Lys Arg Cys
                245                 250                 255

Trp Leu Trp Val Trp Glu Ala Phe Val Tyr Ala Phe Ile Leu Met His
            260                 265                 270

Ser Thr Ser Leu Met Leu Ser Ser Pro Thr Leu Lys Arg Ile Leu Lys
        275                 280                 285

Gly Lys Cys
    290

<210>  23
<211>  1002
<212>  DNA
<213>  Homo sapiens

<220>
<221>  CDS

<222>  (1)..(1002)

<400>  23
atg ttg act cta act cgc atc cgc act gtg tcc tat gaa gtc agg agt      48
aca ttt ctg ttc att tca gtc ctg gag ttt gca gtg ggg ttt ctg acc      96
aat gcc ttc gtt ttc ttg gtg aat ttt tgg gat gta gtg aag agg cag      144
cca ctg agc aac agt gat tgt gtg ctg ctg tgt ctc agc atc agc cgg      192
ctt ttc ctg cat gga ctg ctg ttc ctg agt gct atc cag ctt acc cac      240
ttc cag aag ttg agt gaa cca ctg aac cac agc tac caa gcc atc atc      288
atg cta tgg atg att gca aac caa gcc aac ctc tgg ctt gct gcc tgc      336
ctc agc ctg ctt tac tgc tcc aag ctc atc cgt ttc tct cac acc ttc      384
ctg atc tgc ttg gca agc tgg gtc tcc agg aag atc tcc cag atg ctc      432
ctg ggt att att ctt tgc tcc tgc atc tgc act gtc ctc tgt gtt tgg      480
tgc ttt ttt agc aga cct cac ttc aca gtc aca act gtg cta ttc atg      528
aat aac aat aca agg ctc aac tgg cag att aaa gat ctc aat tta ttt      576
tat tcc ttt ctc ttc tgc tat ctg tgg tct gtg cct cct ttc cta ttg      624
ttt ctg gtt tct tct ggg atg ctg act gtc tcc ctg gga agg cac atg      672
agg aca atg aag gtc tat acc aga aac tct cgt gac ccc agc ctg gag      720
gcc cac att aaa gcc ctc aag tct ctt gtc tcc ttt ttc tgc ttc ttt          768
gtg ata tca tcc tgt gct gcc ttc atc tct gtg ccc cta ctg att ctg          816
tgg cgc gac aaa ata ggg gtg atg gtt tgt gtt ggg ata atg gca gct          864
tgt ccc tct ggg cat gca gcc gtc ctg atc tca ggc aat gcc aag ttg          912
agg aga gct gtg atg acc att ctg ctc tgg gct cag agc agc ctg aag          960
gta aga gcc gac cac aag gca gat tcc cgg aca ctg tgc tga                 1002


<210>  24
<211>  333
<212>  PRT
<213>  Homo sapiens

<400>  24

<210>  25
<211>  1017
<212>  DNA
<213>  Homo sapiens

<220>
<221>  CDS
<222>  (1)..(1017)

<400> 25
atg cta ggg aga tgt ttt cct cca gac acc aaa gag aag caa cag ctc 48
Met Leu Gly Arg Cys Phe Pro Pro Asp Thr Lys Glu Lys Gln Gln Leu
1               5                   10                  15

aga atg act aaa ctc tgc gat cct gca gaa agt gaa ttg tcg cca ttt 96
Arg Met Thr Lys Leu Cys Asp Pro Ala Glu Ser Glu Leu Ser Pro Phe
            20                  25                  30

ctc atc acc tta att tta gca gtt tta ctt gct gaa tac ctc att ggt 144
Leu Ile Thr Leu Ile Leu Ala Val Leu Leu Ala Glu Tyr Leu Ile Gly
        35                  40                  45

atc att gca aat ggt ttc atc atg gct ata cat gca gct gaa tgg gtt 192
Ile Ile Ala Asn Gly Phe Ile Met Ala Ile His Ala Ala Glu Trp Val
    50                  55                  60

caa aat aag gca gtt tcc aca agt ggc agg atc ctg gtt ttc ctg agt 240
Gln Asn Lys Ala Val Ser Thr Ser Gly Arg Ile Leu Val Phe Leu Ser
65                  70                  75                  80

gta tcc aga ata gct ctc caa agc ctc atg atg tta gaa att acc atc 288
Val Ser Arg Ile Ala Leu Gln Ser Leu Met Met Leu Glu Ile Thr Ile
                85                  90                  95

agc tca acc tcc cta agt ttt tat tct gaa gac gct gta tat tat gca 336
Ser Ser Thr Ser Leu Ser Phe Tyr Ser Glu Asp Ala Val Tyr Tyr Ala
            100                 105                 110

ttc aaa ata agt ttt ata ttc tta aat ttt tgt agc ctg tgg ttt gct 384
Phe Lys Ile Ser Phe Ile Phe Leu Asn Phe Cys Ser Leu Trp Phe Ala
        115                 120                 125

gcc tgg ctc agt ttc ttc tac ttt gtg aag att gcc aat ttc tcc tac 432
Ala Trp Leu Ser Phe Phe Tyr Phe Val Lys Ile Ala Asn Phe Ser Tyr
    130                 135                 140

ccc ctt ttc ctc aaa ctg agg tgg aga att act gga ttg ata ccc tgg 480
Pro Leu Phe Leu Lys Leu Arg Trp Arg Ile Thr Gly Leu Ile Pro Trp
145                 150                 155                 160

ctt ctg tgg ctg tcc gtg ttt att tcc ttc agt cac agc atg ttc tgc 528
Leu Leu Trp Leu Ser Val Phe Ile Ser Phe Ser His Ser Met Phe Cys
                165                 170                 175

atc aac atc tgc act gtg tat tgt aac aat tct ttc cct atc cac tcc 576
Ile Asn Ile Cys Thr Val Tyr Cys Asn Asn Ser Phe Pro Ile His Ser
            180                 185                 190

tcc aac tcc act aag aaa aca tac ttg tct gag atc aat gtg gtc ggt 624
Ser Asn Ser Thr Lys Lys Thr Tyr Leu Ser Glu Ile Asn Val Val Gly
        195                 200                 205

ctg gct ttt ttc ttt aac ctg ggg att gtg act cct ctg atc atg ttc 672
Leu Ala Phe Phe Phe Asn Leu Gly Ile Val Thr Pro Leu Ile Met Phe
    210                 215                 220

atc ctg aca gcc acc ctg ctg atc ctc tct ctc aag aga cac acc cta 720
Ile Leu Thr Ala Thr Leu Leu Ile Leu Ser Leu Lys Arg His Thr Leu
225                 230                 235                 240

cac atg gga agc aat gcc aca ggg tcc aac gac ccc agc atg gag gct 768
His Met Gly Ser Asn Ala Thr Gly Ser Asn Asp Pro Ser Met Glu Ala

245                 250                 255

cac atg ggg gcc atc aaa gct atc agc tac ttt ctc att ctc tac att      816
His Met Gly Ala Ile Lys Ala Ile Ser Tyr Phe Leu Ile Leu Tyr Ile
        260                 265                 270

ttc aat gca gtt gct ctg ttt atc tac ctg tcc aac atg ttt gac atc      864
Phe Asn Ala Val Ala Leu Phe Ile Tyr Leu Ser Asn Met Phe Asp Ile
    275                 280                 285

aac agt ctg tgg aat aat ttg tgc cag atc atc atg gct gcc tac cct      912
Asn Ser Leu Trp Asn Asn Leu Cys Gln Ile Ile Met Ala Ala Tyr Pro
    290                 295                 300

gcc agc cac tca att cta ctg att caa gat aac cct ggg ctg aga aga      960
Ala Ser His Ser Ile Leu Leu Ile Gln Asp Asn Pro Gly Leu Arg Arg
305                 310                 315                 320

gcc tgg aag cgg ctt cag ctt cga ctt cat ctt tac cca aaa gag tgg     1008
Ala Trp Lys Arg Leu Gln Leu Arg Leu His Leu Tyr Pro Lys Glu Trp
                325                 330                 335

act ctg tga                                                         1017
Thr Leu

<210>  26
<211>  338
<212>  PRT
<213>  Homo sapiens

<400>  26

Met Leu Gly Arg Cys Phe Pro Pro Asp Thr Lys Glu Lys Gln Gln Leu
1               5                   10                  15

Arg Met Thr Lys Leu Cys Asp Pro Ala Glu Ser Glu Leu Ser Pro Phe
            20                  25                  30

Leu Ile Thr Leu Ile Leu Ala Val Leu Leu Ala Glu Tyr Leu Ile Gly
        35                  40                  45

Ile Ile Ala Asn Gly Phe Ile Met Ala Ile His Ala Ala Glu Trp Val
    50                  55                  60

Gln Asn Lys Ala Val Ser Thr Ser Gly Arg Ile Leu Val Phe Leu Ser
65                  70                  75                  80

Val Ser Arg Ile Ala Leu Gln Ser Leu Met Met Leu Glu Ile Thr Ile
                85                  90                  95

Ser Ser Thr Ser Leu Ser Phe Tyr Ser Glu Asp Ala Val Tyr Tyr Ala
            100                 105                 110

Phe Lys Ile Ser Phe Ile Phe Leu Asn Phe Cys Ser Leu Trp Phe Ala

        115                 120                 125

Ala Trp Leu Ser Phe Phe Tyr Phe Val Lys Ile Ala Asn Phe Ser Tyr
    130                 135                 140

Pro Leu Phe Leu Lys Leu Arg Trp Arg Ile Thr Gly Leu Ile Pro Trp
145                 150                 155                 160

Leu Leu Trp Leu Ser Val Phe Ile Ser Phe Ser His Ser Met Phe Cys
                165                 170                 175

Ile Asn Ile Cys Thr Val Tyr Cys Asn Asn Ser Phe Pro Ile His Ser
            180                 185                 190

Ser Asn Ser Thr Lys Lys Thr Tyr Leu Ser Glu Ile Asn Val Val Gly
        195                 200                 205

Leu Ala Phe Phe Phe Asn Leu Gly Ile Val Thr Pro Leu Ile Met Phe
    210                 215                 220

Ile Leu Thr Ala Thr Leu Leu Ile Leu Ser Leu Lys Arg His Thr Leu
225                 230                 235                 240

His Met Gly Ser Asn Ala Thr Gly Ser Asn Asp Pro Ser Met Glu Ala
                245                 250                 255

His Met Gly Ala Ile Lys Ala Ile Ser Tyr Phe Leu Ile Leu Tyr Ile
            260                 265                 270

Phe Asn Ala Val Ala Leu Phe Ile Tyr Leu Ser Asn Met Phe Asp Ile
        275                 280                 285

Asn Ser Leu Trp Asn Asn Leu Cys Gln Ile Ile Met Ala Ala Tyr Pro
    290                 295                 300

Ala Ser His Ser Ile Leu Leu Ile Gln Asp Asn Pro Gly Leu Arg Arg
305                 310                 315                 320

Ala Trp Lys Arg Leu Gln Leu Arg Leu His Leu Tyr Pro Lys Glu Trp
                325                 330                 335

Thr Leu

<210> 27
<211> 972
<212> DNA
<213> Homo sapiens

<220>
<221>  CDS
<222>  (1)..(972)

<400>  27
atg gca acg gtg aac aca gat gcc aca gat aaa gac ata tcc aag ttc      48
Met Ala Thr Val Asn Thr Asp Ala Thr Asp Lys Asp Ile Ser Lys Phe
1               5                   10                  15

aag gtc acc ttc act ttg gtg gtc tcc gga ata gag tgc atc act ggc      96
Lys Val Thr Phe Thr Leu Val Val Ser Gly Ile Glu Cys Ile Thr Gly
            20                  25                  30

atc ctt ggg agt ggc ttc atc acg gcc atc tat ggg gct gag tgg gcc      144
Ile Leu Gly Ser Gly Phe Ile Thr Ala Ile Tyr Gly Ala Glu Trp Ala
        35                  40                  45

agg ggc aaa aca ctc ccc act ggt gac cgc att atg ttg atg ctg agc      192
Arg Gly Lys Thr Leu Pro Thr Gly Asp Arg Ile Met Leu Met Leu Ser
    50                  55                  60

ttt tcc agg ctc ttg cta cag att tgg atg atg ctg gag aac att ttc      240
Phe Ser Arg Leu Leu Leu Gln Ile Trp Met Met Leu Glu Asn Ile Phe
65                  70                  75                  80

agt ctg cta ttc cga att gtt tat aac caa aac tca gtg tat atc ctc      288
Ser Leu Leu Phe Arg Ile Val Tyr Asn Gln Asn Ser Val Tyr Ile Leu
                85                  90                  95

ttc aaa gtc atc act gtc ttt ctg aac cat tcc aat ctc tgg ttt gct      336
Phe Lys Val Ile Thr Val Phe Leu Asn His Ser Asn Leu Trp Phe Ala
            100                 105                 110

gcc tgg ctc aaa gtc ttc tat tgt ctt aga att gca aac ttc aat cat      384
Ala Trp Leu Lys Val Phe Tyr Cys Leu Arg Ile Ala Asn Phe Asn His
        115                 120                 125

cct ttg ttc ttc ctg atg aag agg aaa atc ata gtg ctg atg cct tgg      432
Pro Leu Phe Phe Leu Met Lys Arg Lys Ile Ile Val Leu Met Pro Trp
    130                 135                 140

ctt ctc agg ctg tca gtg ttg gtt tcc tta agc ttc agc ttt cct ctc      480
Leu Leu Arg Leu Ser Val Leu Val Ser Leu Ser Phe Ser Phe Pro Leu
145                 150                 155                 160

tcg aga gat gtc ttc aat gtg tat gtg aat agc tcc att cct atc ccc      528
Ser Arg Asp Val Phe Asn Val Tyr Val Asn Ser Ser Ile Pro Ile Pro
                165                 170                 175

tcc tcc aac tcc acg gag aag aag tac ttc tct gag acc aat atg gtc      576
Ser Ser Asn Ser Thr Glu Lys Lys Tyr Phe Ser Glu Thr Asn Met Val
            180                 185                 190

aac ctg gta ttt ttc tat aac atg ggg atc ttc gtt cct ctg atc atg      624
Asn Leu Val Phe Phe Tyr Asn Met Gly Ile Phe Val Pro Leu Ile Met
        195                 200                 205

ttc atc ctg gca gcc acc ctg ctg atc ctc tct ctc aag aga cac acc      672
Phe Ile Leu Ala Ala Thr Leu Leu Ile Leu Ser Leu Lys Arg His Thr
    210                 215                 220

cta cac atg gga agc aat gcc aca ggg tcc agg gac ccc agc atg aag     720
Leu His Met Gly Ser Asn Ala Thr Gly Ser Arg Asp Pro Ser Met Lys
225                 230                 235                 240

gct cac ata ggg gcc atc aaa gcc acc agc tac ttt ctc atc ctc tac     768
Ala His Ile Gly Ala Ile Lys Ala Thr Ser Tyr Phe Leu Ile Leu Tyr
                245                 250                 255

att ttc aat gca att gct cta ttt ctt tcc acg tcc aac atc ttt gac     816
Ile Phe Asn Ala Ile Ala Leu Phe Leu Ser Thr Ser Asn Ile Phe Asp
            260                 265                 270

act tac agt tcc tgg aat att ttg tgc aag atc atc atg gct gcc tac     864
Thr Tyr Ser Ser Trp Asn Ile Leu Cys Lys Ile Ile Met Ala Ala Tyr
        275                 280                 285

cct gcc ggc cac tca gta caa ctg atc ttg ggc aac cct ggg ctg aga     912
Pro Ala Gly His Ser Val Gln Leu Ile Leu Gly Asn Pro Gly Leu Arg
    290                 295                 300

aga gcc tgg aag cgg ttt cag cac caa gtt cct ctt tac cta aaa ggg     960
Arg Ala Trp Lys Arg Phe Gln His Gln Val Pro Leu Tyr Leu Lys Gly
305                 310                 315                 320

cag act ctg tga                                                     972
Gln Thr Leu

<210>  28
<211>  323
<212>  PRT
<213>  Homo sapiens

<400>  28

Met Ala Thr Val Asn Thr Asp Ala Thr Asp Lys Asp Ile Ser Lys Phe
1               5                   10                  15

Lys Val Thr Phe Thr Leu Val Val Ser Gly Ile Glu Cys Ile Thr Gly
            20                  25                  30

Ile Leu Gly Ser Gly Phe Ile Thr Ala Ile Tyr Gly Ala Glu Trp Ala
        35                  40                  45

Arg Gly Lys Thr Leu Pro Thr Gly Asp Arg Ile Met Leu Met Leu Ser
    50                  55                  60

Phe Ser Arg Leu Leu Leu Gln Ile Trp Met Met Leu Glu Asn Ile Phe
65                  70                  75                  80

Ser Leu Leu Phe Arg Ile Val Tyr Asn Gln Asn Ser Val Tyr Ile Leu
                85                  90                  95

Phe Lys Val Ile Thr Val Phe Leu Asn His Ser Asn Leu Trp Phe Ala
            100                 105                 110

Ala Trp Leu Lys Val Phe Tyr Cys Leu Arg Ile Ala Asn Phe Asn His
        115                 120                 125

Pro Leu Phe Phe Leu Met Lys Arg Lys Ile Ile Val Leu Met Pro Trp
    130                 135                 140

Leu Leu Arg Leu Ser Val Leu Val Ser Leu Ser Phe Ser Phe Pro Leu
145                 150                 155                 160

Ser Arg Asp Val Phe Asn Val Tyr Val Asn Ser Ser Ile Pro Ile Pro
                165                 170                 175

Ser Ser Asn Ser Thr Glu Lys Lys Tyr Phe Ser Glu Thr Asn Met Val
            180                 185                 190

Asn Leu Val Phe Phe Tyr Asn Met Gly Ile Phe Val Pro Leu Ile Met
        195                 200                 205

Phe Ile Leu Ala Ala Thr Leu Leu Ile Leu Ser Leu Lys Arg His Thr
    210                 215                 220

Leu His Met Gly Ser Asn Ala Thr Gly Ser Arg Asp Pro Ser Met Lys
225                 230                 235                 240

Ala His Ile Gly Ala Ile Lys Ala Thr Ser Tyr Phe Leu Ile Leu Tyr
                245                 250                 255

Ile Phe Asn Ala Ile Ala Leu Phe Leu Ser Thr Ser Asn Ile Phe Asp
            260                 265                 270

Thr Tyr Ser Ser Trp Asn Ile Leu Cys Lys Ile Ile Met Ala Ala Tyr
        275                 280                 285

Pro Ala Gly His Ser Val Gln Leu Ile Leu Gly Asn Pro Gly Leu Arg
    290                 295                 300

Arg Ala Trp Lys Arg Phe Gln His Gln Val Pro Leu Tyr Leu Lys Gly
305                 310                 315                 320

Gln Thr Leu

<210>  29
<211>  924
<212>  DNA
<213>  Homo sapiens

<220>
<221> CDS
<222> (1)..(924)

<400> 29
atg caa gca gca ctg acg gcc ttc ttc gtg ttg ctc ttt agc ctg ctg 48
Met Gln Ala Ala Leu Thr Ala Phe Phe Val Leu Leu Phe Ser Leu Leu
1               5                   10                  15

agt ctt ctg ggg att gca gcg aat ggc ttc att gtg ctg gtg ctg ggc 96
Ser Leu Leu Gly Ile Ala Ala Asn Gly Phe Ile Val Leu Val Leu Gly
            20                  25                  30

agg gag tgg ctg cga tat ggc agg ttg ctg ccc ttg gat atg atc ctc 144
Arg Glu Trp Leu Arg Tyr Gly Arg Leu Leu Pro Leu Asp Met Ile Leu
        35                  40                  45

att agc ttg ggt gcc tcc cgc ttc tgc ctg cag ttg gtt ggg aca gtg 192
Ile Ser Leu Gly Ala Ser Arg Phe Cys Leu Gln Leu Val Gly Thr Val
    50                  55                  60

cac aac ttc tac tac tct gcc cag aag gtc gag tac tct ggg ggt ctc 240
His Asn Phe Tyr Tyr Ser Ala Gln Lys Val Glu Tyr Ser Gly Gly Leu
65                  70                  75                  80

ggc cga cag ttc ttc cat cta cac tgg cac ttc ctg aac tca gcc acc 288
Gly Arg Gln Phe Phe His Leu His Trp His Phe Leu Asn Ser Ala Thr
                85                  90                  95

ttc tgg ttt tgc agc tgg ctc agt gtc ctg ttc tgt gtg aag att gct 336
Phe Trp Phe Cys Ser Trp Leu Ser Val Leu Phe Cys Val Lys Ile Ala
            100                 105                 110

aac atc aca cac tcc acc ttc ctg tgg ctg aag tgg agg ttc cta ggg 384
Asn Ile Thr His Ser Thr Phe Leu Trp Leu Lys Trp Arg Phe Leu Gly
        115                 120                 125

tgg gtg ccc tgg ctc ctg ttg ggc tct gtc ctg atc tcc ttc atc ata 432
Trp Val Pro Trp Leu Leu Leu Gly Ser Val Leu Ile Ser Phe Ile Ile
    130                 135                 140

acc ctg ctg ttt ttt tgg gtg aac tac cct gta tat caa gaa ttt tta 480
Thr Leu Leu Phe Phe Trp Val Asn Tyr Pro Val Tyr Gln Glu Phe Leu
145                 150                 155                 160

att aga aaa ttt tct ggg aac atg acc tac aag tgg aat aca agg ata 528
Ile Arg Lys Phe Ser Gly Asn Met Thr Tyr Lys Trp Asn Thr Arg Ile
                165                 170                 175

gaa aca tac tat ttc cca tcc ctg aaa ctg gtc atc tgg tca att cct 576
Glu Thr Tyr Tyr Phe Pro Ser Leu Lys Leu Val Ile Trp Ser Ile Pro
            180                 185                 190

ttt tct gtt ttt ctg gtc tca att atg ctg tta att aat tct ctg agg 624
Phe Ser Val Phe Leu Val Ser Ile Met Leu Leu Ile Asn Ser Leu Arg
        195                 200                 205

agg cat act cag aga atg cag cac aac ggg cac agc ctg cag gac ccc 672
Arg His Thr Gln Arg Met Gln His Asn Gly His Ser Leu Gln Asp Pro
    210                 215                 220

agc acc cag gct cac acc aga gct ctg aag tcc ctc atc tcc ttc ctc 720
Ser Thr Gln Ala His Thr Arg Ala Leu Lys Ser Leu Ile Ser Phe Leu

225                 230                 235                 240

att ctt tat gct ctg tcc ttt ctg tcc ctg atc att gat gcc gca aaa       768
Ile Leu Tyr Ala Leu Ser Phe Leu Ser Leu Ile Ile Asp Ala Ala Lys
                245                 250                 255

ttt atc tcc atg cag aac gac ttt tac tgg cca tgg caa att gca gtc       816
Phe Ile Ser Met Gln Asn Asp Phe Tyr Trp Pro Trp Gln Ile Ala Val
            260                 265                 270

tac ctg tgc ata tct gtc cat ccc ttc atc ctc atc ttc agc aac ctc       864
Tyr Leu Cys Ile Ser Val His Pro Phe Ile Leu Ile Phe Ser Asn Leu
        275                 280                 285

aag ctt cga agc gtg ttc tca cag ctc ctg ttg ttg gca agg ggc ttc       912
Lys Leu Arg Ser Val Phe Ser Gln Leu Leu Leu Leu Ala Arg Gly Phe
    290                 295                 300

tgg gtg gcc tga                                                       924
Trp Val Ala
305

<210>  30
<211>  307
<212>  PRT
<213>  Homo sapiens

<400>  30

Met Gln Ala Ala Leu Thr Ala Phe Phe Val Leu Leu Phe Ser Leu Leu
1               5                   10                  15

Ser Leu Leu Gly Ile Ala Ala Asn Gly Phe Ile Val Leu Val Leu Gly
            20                  25                  30

Arg Glu Trp Leu Arg Tyr Gly Arg Leu Leu Pro Leu Asp Met Ile Leu
        35                  40                  45

Ile Ser Leu Gly Ala Ser Arg Phe Cys Leu Gln Leu Val Gly Thr Val
    50                  55                  60

His Asn Phe Tyr Tyr Ser Ala Gln Lys Val Glu Tyr Ser Gly Gly Leu
65                  70                  75                  80

Gly Arg Gln Phe Phe His Leu His Trp His Phe Leu Asn Ser Ala Thr
                85                  90                  95

Phe Trp Phe Cys Ser Trp Leu Ser Val Leu Phe Cys Val Lys Ile Ala
            100                 105                 110

Asn Ile Thr His Ser Thr Phe Leu Trp Leu Lys Trp Arg Phe Leu Gly
        115                 120                 125

Trp Val Pro Trp Leu Leu Leu Gly Ser Val Leu Ile Ser Phe Ile Ile

130                 135                 140

Thr Leu Leu Phe Phe Trp Val Asn Tyr Pro Val Tyr Gln Glu Phe Leu
145                 150                 155                 160

Ile Arg Lys Phe Ser Gly Asn Met Thr Tyr Lys Trp Asn Thr Arg Ile
                165                 170                 175

Glu Thr Tyr Tyr Phe Pro Ser Leu Lys Leu Val Ile Trp Ser Ile Pro
            180                 185                 190

Phe Ser Val Phe Leu Val Ser Ile Met Leu Leu Ile Asn Ser Leu Arg
        195                 200                 205

Arg His Thr Gln Arg Met Gln His Asn Gly His Ser Leu Gln Asp Pro
    210                 215                 220

Ser Thr Gln Ala His Thr Arg Ala Leu Lys Ser Leu Ile Ser Phe Leu
225                 230                 235                 240

Ile Leu Tyr Ala Leu Ser Phe Leu Ser Leu Ile Ile Asp Ala Ala Lys
                245                 250                 255

Phe Ile Ser Met Gln Asn Asp Phe Tyr Trp Pro Trp Gln Ile Ala Val
            260                 265                 270

Tyr Leu Cys Ile Ser Val His Pro Phe Ile Leu Ile Phe Ser Asn Leu
        275                 280                 285

Lys Leu Arg Ser Val Phe Ser Gln Leu Leu Leu Leu Ala Arg Gly Phe
    290                 295                 300

Trp Val Ala
305

<210>  31
<211>  945
<212>  DNA
<213>  Homo sapiens

<220>
<221>  CDS
<222>  (1)..(945)

<400>  31
atg gcc acc gaa ttg gac aaa atc ttt ctg att ctg gca ata gca gaa      48
Met Ala Thr Glu Leu Asp Lys Ile Phe Leu Ile Leu Ala Ile Ala Glu
1               5                   10                  15

ttc atc atc agc atg ctg ggg aat gtg ttc att gga ctg gta aac tgc      96

Phe Ile Ile Ser Met Leu Gly Asn Val Phe Ile Gly Leu Val Asn Cys
            20                  25                  30

tct gaa ggg atc aag aac caa aag gtc ttc tca gct gac ttc atc ctc 144
Ser Glu Gly Ile Lys Asn Gln Lys Val Phe Ser Ala Asp Phe Ile Leu
        35                  40                  45

acc tgc ttg gct atc tcc aca att gga caa ctg ttg gtg ata ctg ttt 192
Thr Cys Leu Ala Ile Ser Thr Ile Gly Gln Leu Leu Val Ile Leu Phe
    50                  55                  60

gat tca ttt cta gtg gga ctt gct tca cat tta tat acc aca tat aga 240
Asp Ser Phe Leu Val Gly Leu Ala Ser His Leu Tyr Thr Thr Tyr Arg
65                  70                  75                  80

cta gga aaa act gtt att atg ctt tgg cac atg act aat cac ttg aca 288
Leu Gly Lys Thr Val Ile Met Leu Trp His Met Thr Asn His Leu Thr
                85                  90                  95

acc tgg ctt gcc acc tgc cta agc att ttc tat ttc ttt aag ata gcc 336
Thr Trp Leu Ala Thr Cys Leu Ser Ile Phe Tyr Phe Phe Lys Ile Ala
            100                 105                 110

cac ttc ccc cac tcc ctt ttc ctc tgg ctg agg tgg agg atg aac gga 384
His Phe Pro His Ser Leu Phe Leu Trp Leu Arg Trp Arg Met Asn Gly
        115                 120                 125

atg att gtt atg ctt ctt ata ttg tct ttg ttc tta ctg att ttt gac 432
Met Ile Val Met Leu Leu Ile Leu Ser Leu Phe Leu Leu Ile Phe Asp
    130                 135                 140

agt tta gtg cta gaa ata ttt att gat atc tca ctc aat ata ata gat 480
Ser Leu Val Leu Glu Ile Phe Ile Asp Ile Ser Leu Asn Ile Ile Asp
145                 150                 155                 160

aaa agt aat ctg act tta tat tta gat gaa agt aaa act ctc tat gat 528
Lys Ser Asn Leu Thr Leu Tyr Leu Asp Glu Ser Lys Thr Leu Tyr Asp
                165                 170                 175

aaa ctc tct att tta aaa act ctt ctc agc tta acc agt ttt atc ccc 576
Lys Leu Ser Ile Leu Lys Thr Leu Leu Ser Leu Thr Ser Phe Ile Pro
            180                 185                 190

ttt tct ctg ttc ctg acc tcc ttg ctt ttt tta ttt ctg tcc ttg gtg 624
Phe Ser Leu Phe Leu Thr Ser Leu Leu Phe Leu Phe Leu Ser Leu Val
        195                 200                 205

aga cat act aga aat ttg aag ctc agt tcc ttg ggc tct aga gac tcc 672
Arg His Thr Arg Asn Leu Lys Leu Ser Ser Leu Gly Ser Arg Asp Ser
    210                 215                 220

agc aca gag gcc cat agg agg gcc atg aaa atg gtg atg tct ttc ctt 720
Ser Thr Glu Ala His Arg Arg Ala Met Lys Met Val Met Ser Phe Leu
225                 230                 235                 240

ttc ctc ttc ata gtt cat ttt ttt tcc tta caa gtg gcc aat ggg ata 768
Phe Leu Phe Ile Val His Phe Phe Ser Leu Gln Val Ala Asn Gly Ile
                245                 250                 255

ttt ttt atg ttg tgg aac aac aag tac ata aag ttt gtc atg tta gcc 816
Phe Phe Met Leu Trp Asn Asn Lys Tyr Ile Lys Phe Val Met Leu Ala
            260                 265                 270

tta aat gcc ttt ccc tcg tgc cac tca ttt att ctc att ctg gga aac 864
Leu Asn Ala Phe Pro Ser Cys His Ser Phe Ile Leu Ile Leu Gly Asn
        275                 280                 285

agc aag ctg cga cag aca gct gtg agg cta ctg tgg cat ctt agg aac 912
Ser Lys Leu Arg Gln Thr Ala Val Arg Leu Leu Trp His Leu Arg Asn
    290                 295                 300

tat aca aaa aca cca aat gct tta cct ttg tag 945
Tyr Thr Lys Thr Pro Asn Ala Leu Pro Leu
305                 310

<210> 32
<211> 314
<212> PRT
<213> Homo sapiens

<400> 32

Met Ala Thr Glu Leu Asp Lys Ile Phe Leu Ile Leu Ala Ile Ala Glu
1               5                   10                  15

Phe Ile Ile Ser Met Leu Gly Asn Val Phe Ile Gly Leu Val Asn Cys
            20                  25                  30

Ser Glu Gly Ile Lys Asn Gln Lys Val Phe Ser Ala Asp Phe Ile Leu
        35                  40                  45

Thr Cys Leu Ala Ile Ser Thr Ile Gly Gln Leu Leu Val Ile Leu Phe
    50                  55                  60

Asp Ser Phe Leu Val Gly Leu Ala Ser His Leu Tyr Thr Thr Tyr Arg
65                  70                  75                  80

Leu Gly Lys Thr Val Ile Met Leu Trp His Met Thr Asn His Leu Thr
                85                  90                  95

Thr Trp Leu Ala Thr Cys Leu Ser Ile Phe Tyr Phe Phe Lys Ile Ala
            100                 105                 110

His Phe Pro His Ser Leu Phe Leu Trp Leu Arg Trp Arg Met Asn Gly
        115                 120                 125

Met Ile Val Met Leu Leu Ile Leu Ser Leu Phe Leu Leu Ile Phe Asp
    130                 135                 140

Ser Leu Val Leu Glu Ile Phe Ile Asp Ile Ser Leu Asn Ile Ile Asp
145                 150                 155                 160

Lys Ser Asn Leu Thr Leu Tyr Leu Asp Glu Ser Lys Thr Leu Tyr Asp
                165                 170                 175

Lys Leu Ser Ile Leu Lys Thr Leu Leu Ser Leu Thr Ser Phe Ile Pro
            180                 185                 190

Phe Ser Leu Phe Leu Thr Ser Leu Leu Phe Leu Phe Leu Ser Leu Val
        195                 200                 205

Arg His Thr Arg Asn Leu Lys Leu Ser Ser Leu Gly Ser Arg Asp Ser
    210                 215                 220

Ser Thr Glu Ala His Arg Arg Ala Met Lys Met Val Met Ser Phe Leu
225                 230                 235                 240

Phe Leu Phe Ile Val His Phe Phe Ser Leu Gln Val Ala Asn Gly Ile
                245                 250                 255

Phe Phe Met Leu Trp Asn Asn Lys Tyr Ile Lys Phe Val Met Leu Ala
            260                 265                 270

Leu Asn Ala Phe Pro Ser Cys His Ser Phe Ile Leu Ile Leu Gly Asn
        275                 280                 285

Ser Lys Leu Arg Gln Thr Ala Val Arg Leu Leu Trp His Leu Arg Asn
    290                 295                 300

Tyr Thr Lys Thr Pro Asn Ala Leu Pro Leu
305                 310

<210>  33
<211>  930
<212>  DNA
<213>  Homo sapiens

<220>
<221>  CDS
<222>  (1)..(930)

<400>  33
atg ata act ttt cta ccc atc att ttt tcc agt ctg gta gtg gtt aca      48
Met Ile Thr Phe Leu Pro Ile Ile Phe Ser Ser Leu Val Val Val Thr
1               5                   10                  15

ttt gtt att gga aat ttt gct aat ggc ttc ata gca ctg gta aat tcc      96
Phe Val Ile Gly Asn Phe Ala Asn Gly Phe Ile Ala Leu Val Asn Ser
            20                  25                  30

att gag tcg ttc aag aga caa aag atc tcc ttt gct gac caa att ctc      144
Ile Glu Ser Phe Lys Arg Gln Lys Ile Ser Phe Ala Asp Gln Ile Leu
        35                  40                  45

act gct ctg gcg gtc tcc aga gtt ggt ttg ctc tgg gta tta tta tta      192
Thr Ala Leu Ala Val Ser Arg Val Gly Leu Leu Trp Val Leu Leu Leu
    50                  55                  60

aac tgg tat tca act gtg ttg aat cca gct ttt aat agt gta gaa gta 240
Asn Trp Tyr Ser Thr Val Leu Asn Pro Ala Phe Asn Ser Val Glu Val
65                  70                  75                  80

aga act act gct tat aat atc tgg gca gtg atc aac cat ttc agc aac 288
Arg Thr Thr Ala Tyr Asn Ile Trp Ala Val Ile Asn His Phe Ser Asn
                85                  90                  95

tgg ctt gct act acc ctc agc ata ttt tat ttg ctc aag att gcc aat 336
Trp Leu Ala Thr Thr Leu Ser Ile Phe Tyr Leu Leu Lys Ile Ala Asn
            100                 105                 110

ttc tcc aac ttt att ttt ctt cac tta aag agg aga gtt aag agt gtc 384
Phe Ser Asn Phe Ile Phe Leu His Leu Lys Arg Arg Val Lys Ser Val
        115                 120                 125

att ctg gtg atg ttg ttg ggg cct ttg cta ttt ttg gct tgt cat ctt 432
Ile Leu Val Met Leu Leu Gly Pro Leu Leu Phe Leu Ala Cys His Leu
    130                 135                 140

ttt gtg ata aac atg aat gag att gtg cgg aca aaa gaa ttt gaa gga 480
Phe Val Ile Asn Met Asn Glu Ile Val Arg Thr Lys Glu Phe Glu Gly
145                 150                 155                 160

aac atg act tgg aag atc aaa ttg aag agt gca atg tac ttt tca aat 528
Asn Met Thr Trp Lys Ile Lys Leu Lys Ser Ala Met Tyr Phe Ser Asn
                165                 170                 175

atg act gta acc atg gta gca aac tta gta ccc ttc act ctg acc cta 576
Met Thr Val Thr Met Val Ala Asn Leu Val Pro Phe Thr Leu Thr Leu
            180                 185                 190

cta tct ttt atg ctg tta atc tgt tct ttg tgt aaa cat ctc aag aag 624
Leu Ser Phe Met Leu Leu Ile Cys Ser Leu Cys Lys His Leu Lys Lys
        195                 200                 205

atg cag ctc cgt ggt aaa gga tct caa gat ccc agc acg aag gtc cac 672
Met Gln Leu Arg Gly Lys Gly Ser Gln Asp Pro Ser Thr Lys Val His
    210                 215                 220

ata aaa gct ttg caa act gtg atc tcc ttc ctc ttg tta tgt gcc att 720
Ile Lys Ala Leu Gln Thr Val Ile Ser Phe Leu Leu Leu Cys Ala Ile
225                 230                 235                 240

tac ttt ctg tcc ata atg ata tca gtt tgg agt ttt gga agt ctg gaa 768
Tyr Phe Leu Ser Ile Met Ile Ser Val Trp Ser Phe Gly Ser Leu Glu
                245                 250                 255

aac aaa cct gtc ttc atg ttc tgc aaa gct att aga ttc agc tat cct 816
Asn Lys Pro Val Phe Met Phe Cys Lys Ala Ile Arg Phe Ser Tyr Pro
            260                 265                 270

tca atc cac cca ttc atc ctg att tgg gga aac aag aag cta aag cag 864
Ser Ile His Pro Phe Ile Leu Ile Trp Gly Asn Lys Lys Leu Lys Gln
        275                 280                 285

act ttt ctt tca gtt ttt tgg caa atg agg tac tgg gtg aaa gga gag 912
Thr Phe Leu Ser Val Phe Trp Gln Met Arg Tyr Trp Val Lys Gly Glu
    290                 295                 300

aag act tca tct cca tag 930
Lys Thr Ser Ser Pro

305

<210>  34
<211>  309
<212>  PRT
<213>  Homo sapiens

<400>  34

Met Ile Thr Phe Leu Pro Ile Ile Phe Ser Ser Leu Val Val Val Thr
1               5                   10                  15

Phe Val Ile Gly Asn Phe Ala Asn Gly Phe Ile Ala Leu Val Asn Ser
            20                  25                  30

Ile Glu Ser Phe Lys Arg Gln Lys Ile Ser Phe Ala Asp Gln Ile Leu
        35                  40                  45

Thr Ala Leu Ala Val Ser Arg Val Gly Leu Leu Trp Val Leu Leu Leu
    50                  55                  60

Asn Trp Tyr Ser Thr Val Leu Asn Pro Ala Phe Asn Ser Val Glu Val
65                  70                  75                  80

Arg Thr Thr Ala Tyr Asn Ile Trp Ala Val Ile Asn His Phe Ser Asn
                85                  90                  95

Trp Leu Ala Thr Thr Leu Ser Ile Phe Tyr Leu Leu Lys Ile Ala Asn
            100                 105                 110

Phe Ser Asn Phe Ile Phe Leu His Leu Lys Arg Arg Val Lys Ser Val
        115                 120                 125

Ile Leu Val Met Leu Leu Gly Pro Leu Leu Phe Leu Ala Cys His Leu
    130                 135                 140

Phe Val Ile Asn Met Asn Glu Ile Val Arg Thr Lys Glu Phe Glu Gly
145                 150                 155                 160

Asn Met Thr Trp Lys Ile Lys Leu Lys Ser Ala Met Tyr Phe Ser Asn
                165                 170                 175

Met Thr Val Thr Met Val Ala Asn Leu Val Pro Phe Thr Leu Thr Leu
            180                 185                 190

Leu Ser Phe Met Leu Leu Ile Cys Ser Leu Cys Lys His Leu Lys Lys
        195                 200                 205

Met Gln Leu Arg Gly Lys Gly Ser Gln Asp Pro Ser Thr Lys Val His

210                 215                 220

Ile Lys Ala Leu Gln Thr Val Ile Ser Phe Leu Leu Leu Cys Ala Ile
225                 230                 235                 240

Tyr Phe Leu Ser Ile Met Ile Ser Val Trp Ser Phe Gly Ser Leu Glu
                245                 250                 255

Asn Lys Pro Val Phe Met Phe Cys Lys Ala Ile Arg Phe Ser Tyr Pro
            260                 265                 270

Ser Ile His Pro Phe Ile Leu Ile Trp Gly Asn Lys Lys Leu Lys Gln
        275                 280                 285

Thr Phe Leu Ser Val Phe Trp Gln Met Arg Tyr Trp Val Lys Gly Glu
    290                 295                 300

Lys Thr Ser Ser Pro
305

<210>  35
<211>  930
<212>  DNA
<213>  Homo sapiens

<220>
<221>  CDS
<222>  (1)..(930)

<400>  35
atg aca act ttt ata ccc atc att ttt tcc agt gtg gta gtg gtt cta      48
Met Thr Thr Phe Ile Pro Ile Ile Phe Ser Ser Val Val Val Val Leu
1               5                   10                  15

ttt gtt att gga aat ttt gct aat ggc ttc ata gca ttg gta aat tcc      96
Phe Val Ile Gly Asn Phe Ala Asn Gly Phe Ile Ala Leu Val Asn Ser
            20                  25                  30

att gag cgg gtc aag aga caa aag atc tct ttt gct gac cag att ctc      144
Ile Glu Arg Val Lys Arg Gln Lys Ile Ser Phe Ala Asp Gln Ile Leu
        35                  40                  45

act gct ctg gcg gtc tcc aga gtt ggt ttg ctc tgg gta tta tta tta      192
Thr Ala Leu Ala Val Ser Arg Val Gly Leu Leu Trp Val Leu Leu Leu
    50                  55                  60

aat tgg tat tca act gtg ttt aat cca gct ttt tat agt gta gaa gta      240
Asn Trp Tyr Ser Thr Val Phe Asn Pro Ala Phe Tyr Ser Val Glu Val
65                  70                  75                  80

aga act act gct tat aat gtc tgg gca gta acc ggc cat ttc agc aac      288
Arg Thr Thr Ala Tyr Asn Val Trp Ala Val Thr Gly His Phe Ser Asn
                85                  90                  95

tgg ctt gct act agc ctc agc ata ttt tat ttg ctc aag att gcc aat      336

Trp Leu Ala Thr Ser Leu Ser Ile Phe Tyr Leu Leu Lys Ile Ala Asn
            100                 105                 110

ttc tcc aac ctt att ttt ctt cac tta aag agg aga gtt aag agt gtc      384
Phe Ser Asn Leu Ile Phe Leu His Leu Lys Arg Arg Val Lys Ser Val
        115                 120                 125

att ctg gtg atg ctg ttg ggg cct tta cta ttt ttg gct tgt caa ctt      432
Ile Leu Val Met Leu Leu Gly Pro Leu Leu Phe Leu Ala Cys Gln Leu
    130                 135                 140

ttt gtg ata aac atg aaa gag att gta cgg aca aaa gaa tat gaa gga      480
Phe Val Ile Asn Met Lys Glu Ile Val Arg Thr Lys Glu Tyr Glu Gly
145                 150                 155                 160

aac atg act tgg aag atc aaa ttg agg agt gca gtg tac ctt tca gat      528
Asn Met Thr Trp Lys Ile Lys Leu Arg Ser Ala Val Tyr Leu Ser Asp
                165                 170                 175

gcg act gta acc acg cta gga aac tta gtg ccc ttc act ctg acc ctg      576
Ala Thr Val Thr Thr Leu Gly Asn Leu Val Pro Phe Thr Leu Thr Leu
            180                 185                 190

cta tgt ttt ttg ctg tta atc tgt tct ctg tgt aaa cat ctc aag aag      624
Leu Cys Phe Leu Leu Leu Ile Cys Ser Leu Cys Lys His Leu Lys Lys
        195                 200                 205

atg cag ctc cat ggt aaa gga tct caa gat ccc agc acc aag gtc cac      672
Met Gln Leu His Gly Lys Gly Ser Gln Asp Pro Ser Thr Lys Val His
    210                 215                 220

ata aaa gct ttg caa act gtg atc ttt ttc ctc ttg tta tgt gcc gtt      720
Ile Lys Ala Leu Gln Thr Val Ile Phe Phe Leu Leu Leu Cys Ala Val
225                 230                 235                 240

tac ttt ctg tcc ata atg ata tca gtt tgg agt ttt ggg agt ctg gaa      768
Tyr Phe Leu Ser Ile Met Ile Ser Val Trp Ser Phe Gly Ser Leu Glu
                245                 250                 255

aac aaa cct gtc ttc atg ttc tgc aaa gct att aga ttc agc tat cct      816
Asn Lys Pro Val Phe Met Phe Cys Lys Ala Ile Arg Phe Ser Tyr Pro
            260                 265                 270

tca atc cac cca ttc atc ctg att tgg gga aac aag aag cta aag cag      864
Ser Ile His Pro Phe Ile Leu Ile Trp Gly Asn Lys Lys Leu Lys Gln
        275                 280                 285

act ttt ctt tca gtt ttg cgg caa gtg agg tac tgg gtg aaa gga gag      912
Thr Phe Leu Ser Val Leu Arg Gln Val Arg Tyr Trp Val Lys Gly Glu
    290                 295                 300

aag cct tca tct cca tga                                              930
Lys Pro Ser Ser Pro
305

<210>  36
<211>  309
<212>  PRT
<213>  Homo sapiens

<400>  36

Met Thr Thr Phe Ile Pro Ile Ile Phe Ser Ser Val Val Val Val Leu
1               5                   10                  15

Phe Val Ile Gly Asn Phe Ala Asn Gly Phe Ile Ala Leu Val Asn Ser
            20                  25                  30

Ile Glu Arg Val Lys Arg Gln Lys Ile Ser Phe Ala Asp Gln Ile Leu
        35                  40                  45

Thr Ala Leu Ala Val Ser Arg Val Gly Leu Leu Trp Val Leu Leu Leu
    50                  55                  60

Asn Trp Tyr Ser Thr Val Phe Asn Pro Ala Phe Tyr Ser Val Glu Val
65                  70                  75                  80

Arg Thr Thr Ala Tyr Asn Val Trp Ala Val Thr Gly His Phe Ser Asn
                85                  90                  95

Trp Leu Ala Thr Ser Leu Ser Ile Phe Tyr Leu Leu Lys Ile Ala Asn
            100                 105                 110

Phe Ser Asn Leu Ile Phe Leu His Leu Lys Arg Arg Val Lys Ser Val
        115                 120                 125

Ile Leu Val Met Leu Leu Gly Pro Leu Leu Phe Leu Ala Cys Gln Leu
    130                 135                 140

Phe Val Ile Asn Met Lys Glu Ile Val Arg Thr Lys Glu Tyr Glu Gly
145                 150                 155                 160

Asn Met Thr Trp Lys Ile Lys Leu Arg Ser Ala Val Tyr Leu Ser Asp
                165                 170                 175

Ala Thr Val Thr Thr Leu Gly Asn Leu Val Pro Phe Thr Leu Thr Leu
            180                 185                 190

Leu Cys Phe Leu Leu Leu Ile Cys Ser Leu Cys Lys His Leu Lys Lys
        195                 200                 205

Met Gln Leu His Gly Lys Gly Ser Gln Asp Pro Ser Thr Lys Val His
    210                 215                 220

Ile Lys Ala Leu Gln Thr Val Ile Phe Phe Leu Leu Leu Cys Ala Val
225                 230                 235                 240

Tyr Phe Leu Ser Ile Met Ile Ser Val Trp Ser Phe Gly Ser Leu Glu
                245                 250                 255

Asn Lys Pro Val Phe Met Phe Cys Lys Ala Ile Arg Phe Ser Tyr Pro
            260                 265                 270

Ser Ile His Pro Phe Ile Leu Ile Trp Gly Asn Lys Lys Leu Lys Gln
        275                 280                 285

Thr Phe Leu Ser Val Leu Arg Gln Val Arg Tyr Trp Val Lys Gly Glu
    290                 295                 300

Lys Pro Ser Ser Pro
305

<210>  37
<211>  900
<212>  DNA
<213>  Homo sapiens

<220>
<221>  CDS
<222>  (1)..(900)

<400>  37
atg ata act ttt ctg ccc atc ata ttt tcc att cta gta gtg gtt aca        48
Met Ile Thr Phe Leu Pro Ile Ile Phe Ser Ile Leu Val Val Val Thr
1               5                   10                  15

ttt gtt att gga aat ttt gct aat ggc ttc ata gcg ttg gta aat tcc        96
Phe Val Ile Gly Asn Phe Ala Asn Gly Phe Ile Ala Leu Val Asn Ser
            20                  25                  30

acc gag tgg gtg aag aga caa aag atc tcc ttt gct gac caa att gtc       144
Thr Glu Trp Val Lys Arg Gln Lys Ile Ser Phe Ala Asp Gln Ile Val
        35                  40                  45

act gct ctg gcg gtc tcc aga gtt ggt ttg ctc tgg gtg tta tta tta       192
Thr Ala Leu Ala Val Ser Arg Val Gly Leu Leu Trp Val Leu Leu Leu
    50                  55                  60

aat tgg tat tca act gtg ttg aat cca gct ttt tgt agt gta gaa tta       240
Asn Trp Tyr Ser Thr Val Leu Asn Pro Ala Phe Cys Ser Val Glu Leu
65                  70                  75                  80

aga act act gct tat aat atc tgg gca gta acc ggc cat ttc agc aac       288
Arg Thr Thr Ala Tyr Asn Ile Trp Ala Val Thr Gly His Phe Ser Asn
                85                  90                  95

tgg cct gct act agc ctc agc ata ttt tat ttg ctc aag att gcc aat       336
Trp Pro Ala Thr Ser Leu Ser Ile Phe Tyr Leu Leu Lys Ile Ala Asn
            100                 105                 110

ttc tcc aac ctt att ttt ctt cgc tta aag agg aga gtt aag agt gtc       384
Phe Ser Asn Leu Ile Phe Leu Arg Leu Lys Arg Arg Val Lys Ser Val
        115                 120                 125

att ctg gtg gtg ctg ttg ggg cct ttg cta ttt ttg gct tgt cat ctt       432
Ile Leu Val Val Leu Leu Gly Pro Leu Leu Phe Leu Ala Cys His Leu
    130                 135                 140

ttt gtg gta aac atg aat cag att gta tgg aca aaa gaa tat gaa gga     480
Phe Val Val Asn Met Asn Gln Ile Val Trp Thr Lys Glu Tyr Glu Gly
145                 150                 155                 160

aac atg act tgg aag atc aaa ttg agg cgt gca atg tac ctt tca gat     528
Asn Met Thr Trp Lys Ile Lys Leu Arg Arg Ala Met Tyr Leu Ser Asp
                165                 170                 175

acg act gta acc atg cta gca aac tta gta ccc ttt act gta acc ctg     576
Thr Thr Val Thr Met Leu Ala Asn Leu Val Pro Phe Thr Val Thr Leu
            180                 185                 190

ata tct ttt ctg ctg tta gtc tgt tct ctg tgt aaa cat ctc aag aag     624
Ile Ser Phe Leu Leu Leu Val Cys Ser Leu Cys Lys His Leu Lys Lys
        195                 200                 205

atg cag ctc cat ggc aaa gga tct caa gat ccc agt acc aag gtc cac     672
Met Gln Leu His Gly Lys Gly Ser Gln Asp Pro Ser Thr Lys Val His
    210                 215                 220

ata aaa gtt ttg caa act gtg atc tcc ttc ttc ttg tta cgt gcc att     720
Ile Lys Val Leu Gln Thr Val Ile Ser Phe Phe Leu Leu Arg Ala Ile
225                 230                 235                 240

tac ttt gtg tct gta ata ata tca gtt tgg agt ttt aag aat ctg gaa     768
Tyr Phe Val Ser Val Ile Ile Ser Val Trp Ser Phe Lys Asn Leu Glu
                245                 250                 255

aac aaa cct gtc ttc atg ttc tgc caa gct att gga ttc agc tgt tct     816
Asn Lys Pro Val Phe Met Phe Cys Gln Ala Ile Gly Phe Ser Cys Ser
            260                 265                 270

tca gcc cac ccg ttc atc ctg att tgg gga aac aag aag cta aag cag     864
Ser Ala His Pro Phe Ile Leu Ile Trp Gly Asn Lys Lys Leu Lys Gln
        275                 280                 285

act tat ctt tca gtt ttg tgg caa atg agg tac tga                     900
Thr Tyr Leu Ser Val Leu Trp Gln Met Arg Tyr
    290                 295


<210>  38
<211>  299
<212>  PRT
<213>  Homo sapiens

<400>  38

Met Ile Thr Phe Leu Pro Ile Ile Phe Ser Ile Leu Val Val Val Thr
1               5                   10                  15


Phe Val Ile Gly Asn Phe Ala Asn Gly Phe Ile Ala Leu Val Asn Ser
            20                  25                  30


Thr Glu Trp Val Lys Arg Gln Lys Ile Ser Phe Ala Asp Gln Ile Val
        35                  40                  45


Thr Ala Leu Ala Val Ser Arg Val Gly Leu Leu Trp Val Leu Leu Leu
    50                  55                  60

Asn Trp Tyr Ser Thr Val Leu Asn Pro Ala Phe Cys Ser Val Glu Leu
65                  70                  75                  80

Arg Thr Thr Ala Tyr Asn Ile Trp Ala Val Thr Gly His Phe Ser Asn
                85                  90                  95

Trp Pro Ala Thr Ser Leu Ser Ile Phe Tyr Leu Leu Lys Ile Ala Asn
            100                 105                 110

Phe Ser Asn Leu Ile Phe Leu Arg Leu Lys Arg Arg Val Lys Ser Val
        115                 120                 125

Ile Leu Val Val Leu Leu Gly Pro Leu Leu Phe Leu Ala Cys His Leu
    130                 135                 140

Phe Val Val Asn Met Asn Gln Ile Val Trp Thr Lys Glu Tyr Glu Gly
145                 150                 155                 160

Asn Met Thr Trp Lys Ile Lys Leu Arg Arg Ala Met Tyr Leu Ser Asp
                165                 170                 175

Thr Thr Val Thr Met Leu Ala Asn Leu Val Pro Phe Thr Val Thr Leu
            180                 185                 190

Ile Ser Phe Leu Leu Leu Val Cys Ser Leu Cys Lys His Leu Lys Lys
        195                 200                 205

Met Gln Leu His Gly Lys Gly Ser Gln Asp Pro Ser Thr Lys Val His
    210                 215                 220

Ile Lys Val Leu Gln Thr Val Ile Ser Phe Phe Leu Leu Arg Ala Ile
225                 230                 235                 240

Tyr Phe Val Ser Val Ile Ile Ser Val Trp Ser Phe Lys Asn Leu Glu
                245                 250                 255

Asn Lys Pro Val Phe Met Phe Cys Gln Ala Ile Gly Phe Ser Cys Ser
            260                 265                 270

Ser Ala His Pro Phe Ile Leu Ile Trp Gly Asn Lys Lys Leu Lys Gln
        275                 280                 285

Thr Tyr Leu Ser Val Leu Trp Gln Met Arg Tyr
    290                 295

<210>  39

<211>  930
<212>  DNA
<213>  Homo sapiens

<220>
<221>  CDS
<222>  (1)..(930)

<400>  39
atg ata act ttt ctg ccc atc att ttt tcc att cta ata gtg gtt aca      48
Met Ile Thr Phe Leu Pro Ile Ile Phe Ser Ile Leu Ile Val Val Thr
1               5                   10                  15

ttt gtg att gga aat ttt gct aat ggc ttc ata gca ttg gta aat tcc      96
Phe Val Ile Gly Asn Phe Ala Asn Gly Phe Ile Ala Leu Val Asn Ser
            20                  25                  30

att gag tgg ttc aag aga caa aag atc tct ttt gct gac caa att ctc      144
Ile Glu Trp Phe Lys Arg Gln Lys Ile Ser Phe Ala Asp Gln Ile Leu
        35                  40                  45

act gct ctg gca gtc tcc aga gtt ggt tta ctc tgg gta tta gta tta      192
Thr Ala Leu Ala Val Ser Arg Val Gly Leu Leu Trp Val Leu Val Leu
    50                  55                  60

aat tgg tat gca act gag ttg aat cca gct ttt aac agt ata gaa gta      240
Asn Trp Tyr Ala Thr Glu Leu Asn Pro Ala Phe Asn Ser Ile Glu Val
65                  70                  75                  80

aga att act gct tac aat gtc tgg gca gta atc aac cat ttc agc aac      288
Arg Ile Thr Ala Tyr Asn Val Trp Ala Val Ile Asn His Phe Ser Asn
                85                  90                  95

tgg ctt gct act agc ctc agc ata ttt tat ttg ctc aag att gcc aat      336
Trp Leu Ala Thr Ser Leu Ser Ile Phe Tyr Leu Leu Lys Ile Ala Asn
            100                 105                 110

ttc tcc aac ctt att ttt ctt cac tta aag agg aga gtt aag agt gtt      384
Phe Ser Asn Leu Ile Phe Leu His Leu Lys Arg Arg Val Lys Ser Val
        115                 120                 125

gtt ctg gtg ata cta ttg ggg cct ttg cta ttt ttg gtt tgt cat ctt      432
Val Leu Val Ile Leu Leu Gly Pro Leu Leu Phe Leu Val Cys His Leu
    130                 135                 140

ttt gtg ata aac atg aat cag att ata tgg aca aaa gaa tat gaa gga      480
Phe Val Ile Asn Met Asn Gln Ile Ile Trp Thr Lys Glu Tyr Glu Gly
145                 150                 155                 160

aac atg act tgg aag atc aaa ctg agg agt gca atg tac ctt tca aat      528
Asn Met Thr Trp Lys Ile Lys Leu Arg Ser Ala Met Tyr Leu Ser Asn
                165                 170                 175

aca acg gta acc atc cta gca aac tta gtt ccc ttc act ctg acc ctg      576
Thr Thr Val Thr Ile Leu Ala Asn Leu Val Pro Phe Thr Leu Thr Leu
            180                 185                 190

ata tct ttt ctg ctg tta atc tgt tct ctg tgt aaa cat ctc aaa aag      624
Ile Ser Phe Leu Leu Leu Ile Cys Ser Leu Cys Lys His Leu Lys Lys
        195                 200                 205

atg cag ctc cat ggc aaa gga tct caa gat ccc agc atg aag gtc cac      672

Met Gln Leu His Gly Lys Gly Ser Gln Asp Pro Ser Met Lys Val His
    210                 215                 220

ata aaa gct ttg caa act gtg acc tcc ttc ctc ttg tta tgt gcc att         720
Ile Lys Ala Leu Gln Thr Val Thr Ser Phe Leu Leu Leu Cys Ala Ile
225                 230                 235                 240

tac ttt ctg tcc ata atc atg tca gtt tgg agt ttt gag agt ctg gaa         768
Tyr Phe Leu Ser Ile Ile Met Ser Val Trp Ser Phe Glu Ser Leu Glu
                245                 250                 255

aac aaa cct gtc ttc atg ttc tgc gaa gct att gca ttc agc tat cct         816
Asn Lys Pro Val Phe Met Phe Cys Glu Ala Ile Ala Phe Ser Tyr Pro
            260                 265                 270

tca acc cac cca ttc atc ctg att tgg gga aac aag aag cta aag cag         864
Ser Thr His Pro Phe Ile Leu Ile Trp Gly Asn Lys Lys Leu Lys Gln
        275                 280                 285

act ttt ctt tca gtt ttg tgg cat gtg agg tac tgg gtg aaa gga gag         912
Thr Phe Leu Ser Val Leu Trp His Val Arg Tyr Trp Val Lys Gly Glu
    290                 295                 300

aag cct tca tct tca tag                                                 930
Lys Pro Ser Ser Ser
305


<210>  40
<211>  309
<212>  PRT
<213>  Homo sapiens

<400>  40

Met Ile Thr Phe Leu Pro Ile Ile Phe Ser Ile Leu Ile Val Val Thr
1               5                   10                  15


Phe Val Ile Gly Asn Phe Ala Asn Gly Phe Ile Ala Leu Val Asn Ser
            20                  25                  30


Ile Glu Trp Phe Lys Arg Gln Lys Ile Ser Phe Ala Asp Gln Ile Leu
        35                  40                  45


Thr Ala Leu Ala Val Ser Arg Val Gly Leu Leu Trp Val Leu Val Leu
    50                  55                  60


Asn Trp Tyr Ala Thr Glu Leu Asn Pro Ala Phe Asn Ser Ile Glu Val
65                  70                  75                  80


Arg Ile Thr Ala Tyr Asn Val Trp Ala Val Ile Asn His Phe Ser Asn
                85                  90                  95


Trp Leu Ala Thr Ser Leu Ser Ile Phe Tyr Leu Leu Lys Ile Ala Asn
            100                 105                 110

Phe Ser Asn Leu Ile Phe Leu His Leu Lys Arg Arg Val Lys Ser Val
        115                 120                 125

Val Leu Val Ile Leu Leu Gly Pro Leu Leu Phe Leu Val Cys His Leu
    130                 135                 140

Phe Val Ile Asn Met Asn Gln Ile Ile Trp Thr Lys Glu Tyr Glu Gly
145                 150                 155                 160

Asn Met Thr Trp Lys Ile Lys Leu Arg Ser Ala Met Tyr Leu Ser Asn
                165                 170                 175

Thr Thr Val Thr Ile Leu Ala Asn Leu Val Pro Phe Thr Leu Thr Leu
            180                 185                 190

Ile Ser Phe Leu Leu Leu Ile Cys Ser Leu Cys Lys His Leu Lys Lys
        195                 200                 205

Met Gln Leu His Gly Lys Gly Ser Gln Asp Pro Ser Met Lys Val His
    210                 215                 220

Ile Lys Ala Leu Gln Thr Val Thr Ser Phe Leu Leu Leu Cys Ala Ile
225                 230                 235                 240

Tyr Phe Leu Ser Ile Ile Met Ser Val Trp Ser Phe Glu Ser Leu Glu
                245                 250                 255

Asn Lys Pro Val Phe Met Phe Cys Glu Ala Ile Ala Phe Ser Tyr Pro
            260                 265                 270

Ser Thr His Pro Phe Ile Leu Ile Trp Gly Asn Lys Lys Leu Lys Gln
        275                 280                 285

Thr Phe Leu Ser Val Leu Trp His Val Arg Tyr Trp Val Lys Gly Glu
    290                 295                 300

Lys Pro Ser Ser Ser
305

<210>  41
<211>  960
<212>  DNA
<213>  Homo sapiens

<220>
<221>  CDS
<222>  (1)..(960)

<400>  41

atg ata act ttt ctg ccc atc att ttt tcc att cta ata gtg gtt ata 48
Met Ile Thr Phe Leu Pro Ile Ile Phe Ser Ile Leu Ile Val Val Ile
1               5                   10                  15

ttt gtt att gga aat ttt gct aat ggc ttc ata gca ttg gta aat tcc 96
Phe Val Ile Gly Asn Phe Ala Asn Gly Phe Ile Ala Leu Val Asn Ser
            20                  25                  30

att gag tgg gtc aag aga caa aag atc tcc ttt gtt gac caa att ctc 144
Ile Glu Trp Val Lys Arg Gln Lys Ile Ser Phe Val Asp Gln Ile Leu
        35                  40                  45

act gct ctg gcg gtc tcc aga gtt ggt ttg ctc tgg gtg tta tta cta 192
Thr Ala Leu Ala Val Ser Arg Val Gly Leu Leu Trp Val Leu Leu Leu
    50                  55                  60

cat tgg tat gca act cag ttg aat cca gct ttt tat agt gta gaa gta 240
His Trp Tyr Ala Thr Gln Leu Asn Pro Ala Phe Tyr Ser Val Glu Val
65                  70                  75                  80

aga att act gct tat aat gtc tgg gca gta acc aac cat ttc agc agc 288
Arg Ile Thr Ala Tyr Asn Val Trp Ala Val Thr Asn His Phe Ser Ser
                85                  90                  95

tgg ctt gct act agc ctc agc atg ttt tat ttg ctc agg att gcc aat 336
Trp Leu Ala Thr Ser Leu Ser Met Phe Tyr Leu Leu Arg Ile Ala Asn
            100                 105                 110

ttc tcc aac ctt att ttt ctt cgc ata aag agg aga gtt aag agt gtt 384
Phe Ser Asn Leu Ile Phe Leu Arg Ile Lys Arg Arg Val Lys Ser Val
        115                 120                 125

gtt ctg gtg ata ctg ttg ggg cct ttg cta ttt ttg gtt tgt cat ctt 432
Val Leu Val Ile Leu Leu Gly Pro Leu Leu Phe Leu Val Cys His Leu
    130                 135                 140

ttt gtg ata aac atg gat gag act gta tgg aca aaa gaa tat gaa gga 480
Phe Val Ile Asn Met Asp Glu Thr Val Trp Thr Lys Glu Tyr Glu Gly
145                 150                 155                 160

aac gtg act tgg aag atc aaa ttg agg agt gca atg tac cat tca aat 528
Asn Val Thr Trp Lys Ile Lys Leu Arg Ser Ala Met Tyr His Ser Asn
                165                 170                 175

atg act cta acc atg cta gca aac ttt gta ccc ctc act ctg acc ctg 576
Met Thr Leu Thr Met Leu Ala Asn Phe Val Pro Leu Thr Leu Thr Leu
            180                 185                 190

ata tct ttt ctg ctg tta atc tgt tct ctg tgt aaa cat ctc aag aag 624
Ile Ser Phe Leu Leu Leu Ile Cys Ser Leu Cys Lys His Leu Lys Lys
        195                 200                 205

atg cag ctc cat ggc aaa gga tct caa gat ccc agc acc aag gtc cac 672
Met Gln Leu His Gly Lys Gly Ser Gln Asp Pro Ser Thr Lys Val His
    210                 215                 220

ata aaa gct ttg caa act gtg acc tcc ttt ctt ctg tta tgt gcc att 720
Ile Lys Ala Leu Gln Thr Val Thr Ser Phe Leu Leu Leu Cys Ala Ile
225                 230                 235                 240

tac ttt ctg tcc atg atc ata tca gtt tgt aat ttt ggg agg ctg gaa 768
Tyr Phe Leu Ser Met Ile Ile Ser Val Cys Asn Phe Gly Arg Leu Glu
                245                 250                 255

aag caa cct gtc ttc atg ttc tgc caa gct att ata ttc agc tat cct 816
Lys Gln Pro Val Phe Met Phe Cys Gln Ala Ile Ile Phe Ser Tyr Pro
            260                 265                 270

tca acc cac cca ttc atc ctg att ttg gga aac aag aag cta aag cag 864
Ser Thr His Pro Phe Ile Leu Ile Leu Gly Asn Lys Lys Leu Lys Gln
        275                 280                 285

att ttt ctt tca gtt ttg cgg cat gtg agg tac tgg gtg aaa gac aga 912
Ile Phe Leu Ser Val Leu Arg His Val Arg Tyr Trp Val Lys Asp Arg
    290                 295                 300

agc ctt cgt ctc cat aga ttc aca aga ggg gca ttg tgt gtc ttc tag 960
Ser Leu Arg Leu His Arg Phe Thr Arg Gly Ala Leu Cys Val Phe
305                 310                 315

<210> 42
<211> 319
<212> PRT
<213> Homo sapiens

<400> 42

Met Ile Thr Phe Leu Pro Ile Ile Phe Ser Ile Leu Ile Val Val Ile
1               5                   10                  15

Phe Val Ile Gly Asn Phe Ala Asn Gly Phe Ile Ala Leu Val Asn Ser
            20                  25                  30

Ile Glu Trp Val Lys Arg Gln Lys Ile Ser Phe Val Asp Gln Ile Leu
        35                  40                  45

Thr Ala Leu Ala Val Ser Arg Val Gly Leu Leu Trp Val Leu Leu Leu
    50                  55                  60

His Trp Tyr Ala Thr Gln Leu Asn Pro Ala Phe Tyr Ser Val Glu Val
65                  70                  75                  80

Arg Ile Thr Ala Tyr Asn Val Trp Ala Val Thr Asn His Phe Ser Ser
                85                  90                  95

Trp Leu Ala Thr Ser Leu Ser Met Phe Tyr Leu Leu Arg Ile Ala Asn
            100                 105                 110

Phe Ser Asn Leu Ile Phe Leu Arg Ile Lys Arg Arg Val Lys Ser Val
        115                 120                 125

Val Leu Val Ile Leu Leu Gly Pro Leu Leu Phe Leu Val Cys His Leu
    130                 135                 140

Phe Val Ile Asn Met Asp Glu Thr Val Trp Thr Lys Glu Tyr Glu Gly
145                 150                 155                 160

Asn Val Thr Trp Lys Ile Lys Leu Arg Ser Ala Met Tyr His Ser Asn
                165                 170                 175

Met Thr Leu Thr Met Leu Ala Asn Phe Val Pro Leu Thr Leu Thr Leu
            180                 185                 190

Ile Ser Phe Leu Leu Leu Ile Cys Ser Leu Cys Lys His Leu Lys Lys
        195                 200                 205

Met Gln Leu His Gly Lys Gly Ser Gln Asp Pro Ser Thr Lys Val His
    210                 215                 220

Ile Lys Ala Leu Gln Thr Val Thr Ser Phe Leu Leu Leu Cys Ala Ile
225                 230                 235                 240

Tyr Phe Leu Ser Met Ile Ile Ser Val Cys Asn Phe Gly Arg Leu Glu
                245                 250                 255

Lys Gln Pro Val Phe Met Phe Cys Gln Ala Ile Ile Phe Ser Tyr Pro
            260                 265                 270

Ser Thr His Pro Phe Ile Leu Ile Leu Gly Asn Lys Lys Leu Lys Gln
        275                 280                 285

Ile Phe Leu Ser Val Leu Arg His Val Arg Tyr Trp Val Lys Asp Arg
    290                 295                 300

Ser Leu Arg Leu His Arg Phe Thr Arg Gly Ala Leu Cys Val Phe
305                 310                 315

<210>  43
<211>  900
<212>  DNA
<213>  Homo sapiens

<220>
<221>  CDS
<222>  (1)..(900)

<400>  43
atg atg tgt ttt ctg ctc atc att tca tca att ctg gta gtg ttt gca      48
Met Met Cys Phe Leu Leu Ile Ile Ser Ser Ile Leu Val Val Phe Ala
1               5                   10                  15

ttt gtt ctt gga aat gtt gcc aat ggc ttc ata gcc cta gta aat gtc      96
Phe Val Leu Gly Asn Val Ala Asn Gly Phe Ile Ala Leu Val Asn Val
            20                  25                  30

att gac tgg gtt aac aca cga aag atc tcc tca gct gag caa att ctc      144
Ile Asp Trp Val Asn Thr Arg Lys Ile Ser Ser Ala Glu Gln Ile Leu

35                  40                  45

act gct ctg gtg gtc tcc aga att ggt tta ctc tgg gtc atg tta ttc          192
Thr Ala Leu Val Val Ser Arg Ile Gly Leu Leu Trp Val Met Leu Phe
    50                  55                  60

ctt tgg tat gca act gtg ttt aat tct gct tta tat ggt tta gaa gta          240
Leu Trp Tyr Ala Thr Val Phe Asn Ser Ala Leu Tyr Gly Leu Glu Val
65                  70                  75                  80

aga att gtt gct tct aat gcc tgg gct gta acg aac cat ttc agc atg          288
Arg Ile Val Ala Ser Asn Ala Trp Ala Val Thr Asn His Phe Ser Met
                85                  90                  95

tgg ctt gct gct agc ctc agc ata ttt tgt ttg ctc aag att gcc aat          336
Trp Leu Ala Ala Ser Leu Ser Ile Phe Cys Leu Leu Lys Ile Ala Asn
            100                 105                 110

ttc tcc aac ctt att tct ctc cac cta aag aag aga att aag agt gtt          384
Phe Ser Asn Leu Ile Ser Leu His Leu Lys Lys Arg Ile Lys Ser Val
        115                 120                 125

gtt ctg gtg ata ctg ttg ggg ccc ttg gta ttt ctg att tgt aat ctt          432
Val Leu Val Ile Leu Leu Gly Pro Leu Val Phe Leu Ile Cys Asn Leu
    130                 135                 140

gct gtg ata acc atg gat gag aga gtg tgg aca aaa gaa tat gaa gga          480
Ala Val Ile Thr Met Asp Glu Arg Val Trp Thr Lys Glu Tyr Glu Gly
145                 150                 155                 160

aat gtg act tgg aag atc aaa ttg agg aat gca ata cac ctt tca agc          528
Asn Val Thr Trp Lys Ile Lys Leu Arg Asn Ala Ile His Leu Ser Ser
                165                 170                 175

ttg act gta act act cta gca aac ctc ata ccc ttt act ctg agc cta          576
Leu Thr Val Thr Thr Leu Ala Asn Leu Ile Pro Phe Thr Leu Ser Leu
            180                 185                 190

ata tgt ttt ctg ctg tta atc tgt tct ctt tgt aaa cat ctc aag aag          624
Ile Cys Phe Leu Leu Leu Ile Cys Ser Leu Cys Lys His Leu Lys Lys
        195                 200                 205

atg cgg ctc cat agc aaa gga tct caa gat ccc agc acc aag gtc cat          672
Met Arg Leu His Ser Lys Gly Ser Gln Asp Pro Ser Thr Lys Val His
    210                 215                 220

ata aaa gct ttg caa act gtg acc tcc ttc ctc atg tta ttt gcc att          720
Ile Lys Ala Leu Gln Thr Val Thr Ser Phe Leu Met Leu Phe Ala Ile
225                 230                 235                 240

tac ttt ctg tgt ata atc aca tca act tgg aat ctt agg aca cag cag          768
Tyr Phe Leu Cys Ile Ile Thr Ser Thr Trp Asn Leu Arg Thr Gln Gln
                245                 250                 255

agc aaa ctt gta ctc ctg ctt tgc caa act gtt gca atc atg tat cct          816
Ser Lys Leu Val Leu Leu Leu Cys Gln Thr Val Ala Ile Met Tyr Pro
            260                 265                 270

tca ttc cac tca ttc atc ctg att atg gga agt agg aag cta aaa cag          864
Ser Phe His Ser Phe Ile Leu Ile Met Gly Ser Arg Lys Leu Lys Gln
        275                 280                 285

acc ttt ctt tca gtt ttg tgg cag atg aca cgc tga                          900

Thr Phe Leu Ser Val Leu Trp Gln Met Thr Arg
    290                 295

<210>  44
<211>  299
<212>  PRT
<213>  Homo sapiens

<400>  44

Met Met Cys Phe Leu Leu Ile Ile Ser Ser Ile Leu Val Val Phe Ala
1               5                   10                  15

Phe Val Leu Gly Asn Val Ala Asn Gly Phe Ile Ala Leu Val Asn Val
            20                  25                  30

Ile Asp Trp Val Asn Thr Arg Lys Ile Ser Ser Ala Glu Gln Ile Leu
        35                  40                  45

Thr Ala Leu Val Val Ser Arg Ile Gly Leu Leu Trp Val Met Leu Phe
    50                  55                  60

Leu Trp Tyr Ala Thr Val Phe Asn Ser Ala Leu Tyr Gly Leu Glu Val
65                  70                  75                  80

Arg Ile Val Ala Ser Asn Ala Trp Ala Val Thr Asn His Phe Ser Met
                85                  90                  95

Trp Leu Ala Ala Ser Leu Ser Ile Phe Cys Leu Leu Lys Ile Ala Asn
            100                 105                 110

Phe Ser Asn Leu Ile Ser Leu His Leu Lys Lys Arg Ile Lys Ser Val
        115                 120                 125

Val Leu Val Ile Leu Leu Gly Pro Leu Val Phe Leu Ile Cys Asn Leu
    130                 135                 140

Ala Val Ile Thr Met Asp Glu Arg Val Trp Thr Lys Glu Tyr Glu Gly
145                 150                 155                 160

Asn Val Thr Trp Lys Ile Lys Leu Arg Asn Ala Ile His Leu Ser Ser
                165                 170                 175

Leu Thr Val Thr Thr Leu Ala Asn Leu Ile Pro Phe Thr Leu Ser Leu
            180                 185                 190

Ile Cys Phe Leu Leu Leu Ile Cys Ser Leu Cys Lys His Leu Lys Lys
        195                 200                 205

Met Arg Leu His Ser Lys Gly Ser Gln Asp Pro Ser Thr Lys Val His
    210                 215                 220

Ile Lys Ala Leu Gln Thr Val Thr Ser Phe Leu Met Leu Phe Ala Ile
225                 230                 235                 240

Tyr Phe Leu Cys Ile Ile Thr Ser Thr Trp Asn Leu Arg Thr Gln Gln
                245                 250                 255

Ser Lys Leu Val Leu Leu Leu Cys Gln Thr Val Ala Ile Met Tyr Pro
            260                 265                 270

Ser Phe His Ser Phe Ile Leu Ile Met Gly Ser Arg Lys Leu Lys Gln
        275                 280                 285

Thr Phe Leu Ser Val Leu Trp Gln Met Thr Arg
    290                 295

<210>  45
<211>  930
<212>  DNA
<213>  Homo sapiens

<220>
<221>  CDS
<222>  (1)..(930)

<400>  45
atg atg agt ttt cta cac att gtt ttt tcc att cta gta gtg gtt gca      48
Met Met Ser Phe Leu His Ile Val Phe Ser Ile Leu Val Val Val Ala
1               5                   10                  15

ttt att ctt gga aat ttt gcc aat ggc ttt ata gca ctg ata aat ttc      96
Phe Ile Leu Gly Asn Phe Ala Asn Gly Phe Ile Ala Leu Ile Asn Phe
            20                  25                  30

att gcc tgg gtc aag aga caa aag atc tcc tca gct gat caa att att      144
Ile Ala Trp Val Lys Arg Gln Lys Ile Ser Ser Ala Asp Gln Ile Ile
        35                  40                  45

gct gct ctg gca gtc tcc aga gtt ggt ttg ctc tgg gta ata tta tta      192
Ala Ala Leu Ala Val Ser Arg Val Gly Leu Leu Trp Val Ile Leu Leu
    50                  55                  60

cat tgg tat tca act gtg ttg aat cca act tca tct aat tta aaa gta      240
His Trp Tyr Ser Thr Val Leu Asn Pro Thr Ser Ser Asn Leu Lys Val
65                  70                  75                  80

ata att ttt att tct aat gcc tgg gca gta acc aat cat ttc agc atc      288
Ile Ile Phe Ile Ser Asn Ala Trp Ala Val Thr Asn His Phe Ser Ile
                85                  90                  95

tgg ctt gct act agc ctc agc ata ttt tat ttg ctc aag atc gtc aat      336
Trp Leu Ala Thr Ser Leu Ser Ile Phe Tyr Leu Leu Lys Ile Val Asn
            100                 105                 110

ttc tcc aga ctt att ttt cat cac tta aaa agg aag gct aag agt gta 384
Phe Ser Arg Leu Ile Phe His His Leu Lys Arg Lys Ala Lys Ser Val
        115                 120                 125

gtt ctg gtg ata gtg ttg ggg tct ttg ttc ttt ttg gtt tgt cac ctt 432
Val Leu Val Ile Val Leu Gly Ser Leu Phe Phe Leu Val Cys His Leu
    130                 135                 140

gtg atg aaa cac acg tat ata aat gtg tgg aca gaa gaa tgt gaa gga 480
Val Met Lys His Thr Tyr Ile Asn Val Trp Thr Glu Glu Cys Glu Gly
145                 150                 155                 160

aac gta act tgg aag atc aaa ctg agg aat gca atg cac ctt tcc aac 528
Asn Val Thr Trp Lys Ile Lys Leu Arg Asn Ala Met His Leu Ser Asn
                165                 170                 175

ttg act gta gcc atg cta gca aac ttg ata cca ttc act ctg acc ctg 576
Leu Thr Val Ala Met Leu Ala Asn Leu Ile Pro Phe Thr Leu Thr Leu
            180                 185                 190

ata tct ttt ctg ctg tta atc tac tct ctg tgt aaa cat ctg aag aag 624
Ile Ser Phe Leu Leu Leu Ile Tyr Ser Leu Cys Lys His Leu Lys Lys
        195                 200                 205

atg cag ctc cat ggc aaa gga tct caa gat ccc agc acc aag atc cac 672
Met Gln Leu His Gly Lys Gly Ser Gln Asp Pro Ser Thr Lys Ile His
    210                 215                 220

ata aaa gct ctg caa act gtg acc tcc ttc ctc ata tta ctt gcc att 720
Ile Lys Ala Leu Gln Thr Val Thr Ser Phe Leu Ile Leu Leu Ala Ile
225                 230                 235                 240

tac ttt ctg tgt cta atc ata tcg ttt tgg aat ttt aag atg cga cca 768
Tyr Phe Leu Cys Leu Ile Ile Ser Phe Trp Asn Phe Lys Met Arg Pro
                245                 250                 255

aaa gaa att gtc tta atg ctt tgc caa gct ttt gga atc ata tat cca 816
Lys Glu Ile Val Leu Met Leu Cys Gln Ala Phe Gly Ile Ile Tyr Pro
            260                 265                 270

tca ttc cac tca ttc att ctg att tgg ggg aac aag acg cta aag cag 864
Ser Phe His Ser Phe Ile Leu Ile Trp Gly Asn Lys Thr Leu Lys Gln
        275                 280                 285

acc ttt ctt tca gtt ttg tgg cag gtg act tgc tgg gca aaa gga cag 912
Thr Phe Leu Ser Val Leu Trp Gln Val Thr Cys Trp Ala Lys Gly Gln
    290                 295                 300

aac cag tca act cca tag 930
Asn Gln Ser Thr Pro
305

<210> 46
<211> 309
<212> PRT
<213> Homo sapiens

<400> 46

Met Met Ser Phe Leu His Ile Val Phe Ser Ile Leu Val Val Val Ala
1               5                   10                  15

Phe Ile Leu Gly Asn Phe Ala Asn Gly Phe Ile Ala Leu Ile Asn Phe
            20                  25                  30
Ile Ala Trp Val Lys Arg Gln Lys Ile Ser Ser Ala Asp Gln Ile Ile
        35                  40                  45
Ala Ala Leu Ala Val Ser Arg Val Gly Leu Leu Trp Val Ile Leu Leu
    50                  55                  60
His Trp Tyr Ser Thr Val Leu Asn Pro Thr Ser Ser Asn Leu Lys Val
65                  70                  75                  80
Ile Ile Phe Ile Ser Asn Ala Trp Ala Val Thr Asn His Phe Ser Ile
                85                  90                  95
Trp Leu Ala Thr Ser Leu Ser Ile Phe Tyr Leu Leu Lys Ile Val Asn
            100                 105                 110
Phe Ser Arg Leu Ile Phe His His Leu Lys Arg Lys Ala Lys Ser Val
        115                 120                 125
Val Leu Val Ile Val Leu Gly Ser Leu Phe Phe Leu Val Cys His Leu
    130                 135                 140
Val Met Lys His Thr Tyr Ile Asn Val Trp Thr Glu Glu Cys Glu Gly
145                 150                 155                 160
Asn Val Thr Trp Lys Ile Lys Leu Arg Asn Ala Met His Leu Ser Asn
                165                 170                 175
Leu Thr Val Ala Met Leu Ala Asn Leu Ile Pro Phe Thr Leu Thr Leu
            180                 185                 190
Ile Ser Phe Leu Leu Leu Ile Tyr Ser Leu Cys Lys His Leu Lys Lys
        195                 200                 205
Met Gln Leu His Gly Lys Gly Ser Gln Asp Pro Ser Thr Lys Ile His
    210                 215                 220
Ile Lys Ala Leu Gln Thr Val Thr Ser Phe Leu Ile Leu Leu Ala Ile
225                 230                 235                 240
Tyr Phe Leu Cys Leu Ile Ile Ser Phe Trp Asn Phe Lys Met Arg Pro
                245                 250                 255
Lys Glu Ile Val Leu Met Leu Cys Gln Ala Phe Gly Ile Ile Tyr Pro
            260                 265                 270

Ser Phe His Ser Phe Ile Leu Ile Trp Gly Asn Lys Thr Leu Lys Gln
        275                 280                 285

Thr Phe Leu Ser Val Leu Trp Gln Val Thr Cys Trp Ala Lys Gly Gln
    290                 295                 300

Asn Gln Ser Thr Pro
305

<210>  47
<211>  900
<212>  DNA
<213>  Homo sapiens

<220>
<221>  CDS
<222>  (1)..(900)

<400>  47
atg ata act ttt cta tac att ttt ttt tca att cta ata atg gtt tta      48
Met Ile Thr Phe Leu Tyr Ile Phe Phe Ser Ile Leu Ile Met Val Leu
1               5                   10                  15

ttt gtt ctc gga aac ttt gcc aat ggc ttc ata gca ctg gta aat ttc      96
Phe Val Leu Gly Asn Phe Ala Asn Gly Phe Ile Ala Leu Val Asn Phe
            20                  25                  30

att gac tgg gtg aag aga aaa aag atc tcc tca gct gac caa att ctc     144
Ile Asp Trp Val Lys Arg Lys Lys Ile Ser Ser Ala Asp Gln Ile Leu
        35                  40                  45

act gct ctg gcg gtc tcc aga att ggt ttg ctc tgg gca tta tta tta     192
Thr Ala Leu Ala Val Ser Arg Ile Gly Leu Leu Trp Ala Leu Leu Leu
    50                  55                  60

aat tgg tat tta act gtg ttg aat cca gct ttt tat agt gta gaa tta     240
Asn Trp Tyr Leu Thr Val Leu Asn Pro Ala Phe Tyr Ser Val Glu Leu
65                  70                  75                  80

aga att act tct tat aat gcc tgg gtt gta acc aac cat ttc agc atg     288
Arg Ile Thr Ser Tyr Asn Ala Trp Val Val Thr Asn His Phe Ser Met
                85                  90                  95

tgg ctt gct gct aac ctc agc ata ttt tat ttg ctc aag att gcc aat     336
Trp Leu Ala Ala Asn Leu Ser Ile Phe Tyr Leu Leu Lys Ile Ala Asn
            100                 105                 110

ttc tcc aac ctt ctt ttt ctt cat tta aag agg aga gtt agg agt gtc     384
Phe Ser Asn Leu Leu Phe Leu His Leu Lys Arg Arg Val Arg Ser Val
        115                 120                 125

att ctg gtg ata ctg ttg ggg act ttg ata ttt ttg gtt tgt cat ctt     432
Ile Leu Val Ile Leu Leu Gly Thr Leu Ile Phe Leu Val Cys His Leu
    130                 135                 140

ctt gtg gca aac atg gat gag agt atg tgg gca gaa gaa tat gaa gga     480
Leu Val Ala Asn Met Asp Glu Ser Met Trp Ala Glu Glu Tyr Glu Gly

145                 150                 155                 160

aac atg act ggg aag atg aaa ttg agg aat aca gta cat ctt tca tat       528
Asn Met Thr Gly Lys Met Lys Leu Arg Asn Thr Val His Leu Ser Tyr
                165                 170                 175

ttg act gta act acc cta tgg agc ttc ata ccc ttt act ctg tcc ctg       576
Leu Thr Val Thr Thr Leu Trp Ser Phe Ile Pro Phe Thr Leu Ser Leu
            180                 185                 190

ata tct ttt ctg atg cta atc tgt tct ctg tgt aaa cat ctc aag aag       624
Ile Ser Phe Leu Met Leu Ile Cys Ser Leu Cys Lys His Leu Lys Lys
        195                 200                 205

atg cag ctc cat gga gaa gga tcg caa gat ctc agc acc aag gtc cac       672
Met Gln Leu His Gly Glu Gly Ser Gln Asp Leu Ser Thr Lys Val His
    210                 215                 220

ata aaa gct ttg caa act ctg atc tcc ttc ctc ttg tta tgt gcc att       720
Ile Lys Ala Leu Gln Thr Leu Ile Ser Phe Leu Leu Leu Cys Ala Ile
225                 230                 235                 240

ttc ttt cta ttc cta atc gtt tcg gtt tgg agt cct agg agg ctg cgg       768
Phe Phe Leu Phe Leu Ile Val Ser Val Trp Ser Pro Arg Arg Leu Arg
                245                 250                 255

aat gac ccg gtt gtc atg gtt agc aag gct gtt gga aac ata tat ctt       816
Asn Asp Pro Val Val Met Val Ser Lys Ala Val Gly Asn Ile Tyr Leu
            260                 265                 270

gca ttc gac tca ttc atc cta att tgg aga acc aag aag cta aaa cac       864
Ala Phe Asp Ser Phe Ile Leu Ile Trp Arg Thr Lys Lys Leu Lys His
        275                 280                 285

acc ttt ctt ttg att ttg tgt cag att agg tgc tga                       900
Thr Phe Leu Leu Ile Leu Cys Gln Ile Arg Cys
    290                 295

<210>  48
<211>  299
<212>  PRT
<213>  Homo sapiens

<400>  48

Met Ile Thr Phe Leu Tyr Ile Phe Phe Ser Ile Leu Ile Met Val Leu
1               5                   10                  15

Phe Val Leu Gly Asn Phe Ala Asn Gly Phe Ile Ala Leu Val Asn Phe
            20                  25                  30

Ile Asp Trp Val Lys Arg Lys Lys Ile Ser Ser Ala Asp Gln Ile Leu
        35                  40                  45

Thr Ala Leu Ala Val Ser Arg Ile Gly Leu Leu Trp Ala Leu Leu Leu
    50                  55                  60

Asn Trp Tyr Leu Thr Val Leu Asn Pro Ala Phe Tyr Ser Val Glu Leu

65                  70                  75                  80

Arg Ile Thr Ser Tyr Asn Ala Trp Val Val Thr Asn His Phe Ser Met
                85                  90                  95

Trp Leu Ala Ala Asn Leu Ser Ile Phe Tyr Leu Leu Lys Ile Ala Asn
            100                 105                 110

Phe Ser Asn Leu Leu Phe Leu His Leu Lys Arg Arg Val Arg Ser Val
        115                 120                 125

Ile Leu Val Ile Leu Leu Gly Thr Leu Ile Phe Leu Val Cys His Leu
    130                 135                 140

Leu Val Ala Asn Met Asp Glu Ser Met Trp Ala Glu Glu Tyr Glu Gly
145                 150                 155                 160

Asn Met Thr Gly Lys Met Lys Leu Arg Asn Thr Val His Leu Ser Tyr
                165                 170                 175

Leu Thr Val Thr Thr Leu Trp Ser Phe Ile Pro Phe Thr Leu Ser Leu
            180                 185                 190

Ile Ser Phe Leu Met Leu Ile Cys Ser Leu Cys Lys His Leu Lys Lys
        195                 200                 205

Met Gln Leu His Gly Glu Gly Ser Gln Asp Leu Ser Thr Lys Val His
    210                 215                 220

Ile Lys Ala Leu Gln Thr Leu Ile Ser Phe Leu Leu Leu Cys Ala Ile
225                 230                 235                 240

Phe Phe Leu Phe Leu Ile Val Ser Val Trp Ser Pro Arg Arg Leu Arg
                245                 250                 255

Asn Asp Pro Val Val Met Val Ser Lys Ala Val Gly Asn Ile Tyr Leu
            260                 265                 270

Ala Phe Asp Ser Phe Ile Leu Ile Trp Arg Thr Lys Lys Leu Lys His
        275                 280                 285

Thr Phe Leu Leu Ile Leu Cys Gln Ile Arg Cys
    290                 295

<210>  49
<211>  957
<212>  DNA
<213>  Homo sapiens

<220>
<221>  CDS
<222>  (1)..(957)

<400>  49
atg aat gga gac cac atg gtt cta gga tct tcg gtg act gac aag aag      48
Met Asn Gly Asp His Met Val Leu Gly Ser Ser Val Thr Asp Lys Lys
1               5                   10                  15

gcc atc atc ttg gtt acc att tta ctc ctt tta cgc ctg gta gca ata      96
Ala Ile Ile Leu Val Thr Ile Leu Leu Leu Leu Arg Leu Val Ala Ile
            20                  25                  30

gca ggc aat ggc ttc atc act gct gct ctg ggc gtg gag tgg gtg cta      144
Ala Gly Asn Gly Phe Ile Thr Ala Ala Leu Gly Val Glu Trp Val Leu
        35                  40                  45

cgg aga atg ttg ttg cct tgt gat aag tta ttg gtt agc cta ggg gcc      192
Arg Arg Met Leu Leu Pro Cys Asp Lys Leu Leu Val Ser Leu Gly Ala
    50                  55                  60

tct cgc ttc tgt ctg cag tca gtg gta atg ggt aag acc att tat gtt      240
Ser Arg Phe Cys Leu Gln Ser Val Val Met Gly Lys Thr Ile Tyr Val
65                  70                  75                  80

ttc ttg cat ccg atg gcc ttc cca tac aac cct gta ctg cag ttt cta      288
Phe Leu His Pro Met Ala Phe Pro Tyr Asn Pro Val Leu Gln Phe Leu
                85                  90                  95

gct ttc cag tgg gac ttc ctg aat gct gcc acc tta tgg tcc tct acc      336
Ala Phe Gln Trp Asp Phe Leu Asn Ala Ala Thr Leu Trp Ser Ser Thr
            100                 105                 110

tgg ctc agt gtc ttc tat tgt gtg aaa att gct acc ttc acc cac cct      384
Trp Leu Ser Val Phe Tyr Cys Val Lys Ile Ala Thr Phe Thr His Pro
        115                 120                 125

gtc ttc ttc tgg cta aag cac aag ttg tct ggg tgg cta cca tgg atg      432
Val Phe Phe Trp Leu Lys His Lys Leu Ser Gly Trp Leu Pro Trp Met
    130                 135                 140

ctc ttc agc tct gta ggg ctc tcc agc ttc acc acc att cta ttt ttc      480
Leu Phe Ser Ser Val Gly Leu Ser Ser Phe Thr Thr Ile Leu Phe Phe
145                 150                 155                 160

ata ggc aac cac aga atg tat cag aac tat tta agg aac cat cta caa      528
Ile Gly Asn His Arg Met Tyr Gln Asn Tyr Leu Arg Asn His Leu Gln
                165                 170                 175

cct tgg aat gtc act ggc gat agc ata cgg agc tac tgt gag aaa ttc      576
Pro Trp Asn Val Thr Gly Asp Ser Ile Arg Ser Tyr Cys Glu Lys Phe
            180                 185                 190

tat ctc ttc cct cta aaa atg att act tgg aca atg ccc act gct gtc      624
Tyr Leu Phe Pro Leu Lys Met Ile Thr Trp Thr Met Pro Thr Ala Val
        195                 200                 205

ttt ttc att tgc atg att ttg ctc atc aca tct ctg gga aga cac agg      672
Phe Phe Ile Cys Met Ile Leu Leu Ile Thr Ser Leu Gly Arg His Arg
    210                 215                 220

aag aag gct ctc ctt aca acc tca gga ttc cga gag ccc agt gtg cag          720
Lys Lys Ala Leu Leu Thr Thr Ser Gly Phe Arg Glu Pro Ser Val Gln
225                 230                 235                 240

gca cac ata aag gct ctg ctg gct ctc ctc tct ttt gcc atg ctc ttc          768
Ala His Ile Lys Ala Leu Leu Ala Leu Leu Ser Phe Ala Met Leu Phe
                245                 250                 255

atc tca tat ttc ctg tca ctg gtg ttc agt gct gca ggt att ttt cca          816
Ile Ser Tyr Phe Leu Ser Leu Val Phe Ser Ala Ala Gly Ile Phe Pro
            260                 265                 270

cct ctg gac ttt aaa ttc tgg gtg tgg gag tca gtg att tat ctg tgt          864
Pro Leu Asp Phe Lys Phe Trp Val Trp Glu Ser Val Ile Tyr Leu Cys
        275                 280                 285

gca gca gtt cac ccc atc att ctg ctc ttc agc aac tgc agg ctg aga          912
Ala Ala Val His Pro Ile Ile Leu Leu Phe Ser Asn Cys Arg Leu Arg
    290                 295                 300

gct gtg ctg aag agt cgt cgt tcc tca agg tgt ggg aca cct tga              957
Ala Val Leu Lys Ser Arg Arg Ser Ser Arg Cys Gly Thr Pro
305                 310                 315


<210>  50
<211>  318
<212>  PRT
<213>  Homo sapiens

<400>  50

Met Asn Gly Asp His Met Val Leu Gly Ser Ser Val Thr Asp Lys Lys
1               5                   10                  15


Ala Ile Ile Leu Val Thr Ile Leu Leu Leu Leu Arg Leu Val Ala Ile
            20                  25                  30


Ala Gly Asn Gly Phe Ile Thr Ala Ala Leu Gly Val Glu Trp Val Leu
        35                  40                  45


Arg Arg Met Leu Leu Pro Cys Asp Lys Leu Leu Val Ser Leu Gly Ala
    50                  55                  60


Ser Arg Phe Cys Leu Gln Ser Val Val Met Gly Lys Thr Ile Tyr Val
65                  70                  75                  80


Phe Leu His Pro Met Ala Phe Pro Tyr Asn Pro Val Leu Gln Phe Leu
                85                  90                  95


Ala Phe Gln Trp Asp Phe Leu Asn Ala Ala Thr Leu Trp Ser Ser Thr
            100                 105                 110


Trp Leu Ser Val Phe Tyr Cys Val Lys Ile Ala Thr Phe Thr His Pro
        115                 120                 125

Val Phe Phe Trp Leu Lys His Lys Leu Ser Gly Trp Leu Pro Trp Met
    130                 135                 140

Leu Phe Ser Ser Val Gly Leu Ser Ser Phe Thr Thr Ile Leu Phe Phe
145                 150                 155                 160

Ile Gly Asn His Arg Met Tyr Gln Asn Tyr Leu Arg Asn His Leu Gln
                165                 170                 175

Pro Trp Asn Val Thr Gly Asp Ser Ile Arg Ser Tyr Cys Glu Lys Phe
            180                 185                 190

Tyr Leu Phe Pro Leu Lys Met Ile Thr Trp Thr Met Pro Thr Ala Val
        195                 200                 205

Phe Phe Ile Cys Met Ile Leu Leu Ile Thr Ser Leu Gly Arg His Arg
    210                 215                 220

Lys Lys Ala Leu Leu Thr Thr Ser Gly Phe Arg Glu Pro Ser Val Gln
225                 230                 235                 240

Ala His Ile Lys Ala Leu Leu Ala Leu Leu Ser Phe Ala Met Leu Phe
                245                 250                 255

Ile Ser Tyr Phe Leu Ser Leu Val Phe Ser Ala Ala Gly Ile Phe Pro
            260                 265                 270

Pro Leu Asp Phe Lys Phe Trp Val Trp Glu Ser Val Ile Tyr Leu Cys
        275                 280                 285

Ala Ala Val His Pro Ile Ile Leu Leu Phe Ser Asn Cys Arg Leu Arg
    290                 295                 300

Ala Val Leu Lys Ser Arg Arg Ser Ser Arg Cys Gly Thr Pro
305                 310                 315

<210>  51
<211>  948
<212>  DNA
<213>  Homo sapiens

<220>
<221>  CDS
<222>  (1)..(948)

<400>  51
atg cag cca gaa tct ggg gcc aat gga aca gtc att gct gag ttc atc        48
Met Gln Pro Glu Ser Gly Ala Asn Gly Thr Val Ile Ala Glu Phe Ile
1               5                   10                  15

ctg ctg ggc ttg ctg gag gcg cca ggg ctg cag cca gtt gtc ttt gtg 96
Leu Leu Gly Leu Leu Glu Ala Pro Gly Leu Gln Pro Val Val Phe Val
            20                  25                  30

ctc ttc ctc ttt gcc tac ctg gtc acg gtc agg ggc aac ctc agc atc 144
Leu Phe Leu Phe Ala Tyr Leu Val Thr Val Arg Gly Asn Leu Ser Ile
        35                  40                  45

ctg gca gct gtc ttg gtg gag ccc aaa ctc cac acc ccc atg tac ttc 192
Leu Ala Ala Val Leu Val Glu Pro Lys Leu His Thr Pro Met Tyr Phe
    50                  55                  60

ttc ctg ggg aac cta tca gtg ctg gat gtt ggg tgc atc agc gtc act 240
Phe Leu Gly Asn Leu Ser Val Leu Asp Val Gly Cys Ile Ser Val Thr
65                  70                  75                  80

gtt cca tca atg ttg agt cgt ctc ctg tcc cgc aag cgt gca gtt ccc 288
Val Pro Ser Met Leu Ser Arg Leu Leu Ser Arg Lys Arg Ala Val Pro
                85                  90                  95

tgt ggg gcc tgc ctt acc cag ctc ttc ttc ttc cat ctg ttc gtt gga 336
Cys Gly Ala Cys Leu Thr Gln Leu Phe Phe Phe His Leu Phe Val Gly
            100                 105                 110

gtg gac tgc ttc ctg ctg acc gcc atg gcc tat gac cga ttc ctg gcc 384
Val Asp Cys Phe Leu Leu Thr Ala Met Ala Tyr Asp Arg Phe Leu Ala
        115                 120                 125

atc tgc cgg ccc ctc acc tac agc acc cgc atg agt cag aca gtc cag 432
Ile Cys Arg Pro Leu Thr Tyr Ser Thr Arg Met Ser Gln Thr Val Gln
    130                 135                 140

agg atg ttg gtg gct gcg tcc tgg gct tgt gct ttc acc aac gca ctg 480
Arg Met Leu Val Ala Ala Ser Trp Ala Cys Ala Phe Thr Asn Ala Leu
145                 150                 155                 160

acc cac act gtg gcc atg tcc acg ctc aac ttc tgt ggc ccc aat gtg 528
Thr His Thr Val Ala Met Ser Thr Leu Asn Phe Cys Gly Pro Asn Val
                165                 170                 175

atc aat cac ttc tac tgt gac ctc cca cag ctc ttc cag ctc tcc tgc 576
Ile Asn His Phe Tyr Cys Asp Leu Pro Gln Leu Phe Gln Leu Ser Cys
            180                 185                 190

tcc agc acc caa ctc aat gag ctg ctg ctt ttt gct gtg ggt ttt ata 624
Ser Ser Thr Gln Leu Asn Glu Leu Leu Leu Phe Ala Val Gly Phe Ile
        195                 200                 205

atg gca ggt acc ccc atg gct ctc att gtc atc tcc tat atc cac gtg 672
Met Ala Gly Thr Pro Met Ala Leu Ile Val Ile Ser Tyr Ile His Val
    210                 215                 220

gca gct gca gtc ctg cga att cgc tct gta gag ggc agg aag aaa gcc 720
Ala Ala Ala Val Leu Arg Ile Arg Ser Val Glu Gly Arg Lys Lys Ala
225                 230                 235                 240

ttc tcc aca tgt ggc tcc cac ctc act gtg gtt gcc ata ttc tat ggt 768
Phe Ser Thr Cys Gly Ser His Leu Thr Val Val Ala Ile Phe Tyr Gly
                245                 250                 255

tca ggt atc ttt aac tat atg cga ctg ggt tca acc aag ctt tca gac 816
Ser Gly Ile Phe Asn Tyr Met Arg Leu Gly Ser Thr Lys Leu Ser Asp

260                 265                 270

aag gat aaa gct gtt gga att ttc aac act gtc atc aat ccc atg ctg         864
Lys Asp Lys Ala Val Gly Ile Phe Asn Thr Val Ile Asn Pro Met Leu
        275                 280                 285

aac cca atc atc tac agc ttc aga aac cct gat gtg cag agt gcc atc         912
Asn Pro Ile Ile Tyr Ser Phe Arg Asn Pro Asp Val Gln Ser Ala Ile
    290                 295                 300

tgg agg atg ctc aca ggg agg cgg tca ctg gct tga                         948
Trp Arg Met Leu Thr Gly Arg Arg Ser Leu Ala
305                 310                 315

<210>  52
<211>  315
<212>  PRT
<213>  Homo sapiens

<400>  52

Met Gln Pro Glu Ser Gly Ala Asn Gly Thr Val Ile Ala Glu Phe Ile
1               5                   10                  15

Leu Leu Gly Leu Leu Glu Ala Pro Gly Leu Gln Pro Val Val Phe Val
            20                  25                  30

Leu Phe Leu Phe Ala Tyr Leu Val Thr Val Arg Gly Asn Leu Ser Ile
        35                  40                  45

Leu Ala Ala Val Leu Val Glu Pro Lys Leu His Thr Pro Met Tyr Phe
    50                  55                  60

Phe Leu Gly Asn Leu Ser Val Leu Asp Val Gly Cys Ile Ser Val Thr
65                  70                  75                  80

Val Pro Ser Met Leu Ser Arg Leu Leu Ser Arg Lys Arg Ala Val Pro
                85                  90                  95

Cys Gly Ala Cys Leu Thr Gln Leu Phe Phe Phe His Leu Phe Val Gly
            100                 105                 110

Val Asp Cys Phe Leu Leu Thr Ala Met Ala Tyr Asp Arg Phe Leu Ala
        115                 120                 125

Ile Cys Arg Pro Leu Thr Tyr Ser Thr Arg Met Ser Gln Thr Val Gln
    130                 135                 140

Arg Met Leu Val Ala Ala Ser Trp Ala Cys Ala Phe Thr Asn Ala Leu
145                 150                 155                 160

Thr His Thr Val Ala Met Ser Thr Leu Asn Phe Cys Gly Pro Asn Val

165                 170                 175

Ile Asn His Phe Tyr Cys Asp Leu Pro Gln Leu Phe Gln Leu Ser Cys
            180                 185                 190

Ser Ser Thr Gln Leu Asn Glu Leu Leu Leu Phe Ala Val Gly Phe Ile
        195                 200                 205

Met Ala Gly Thr Pro Met Ala Leu Ile Val Ile Ser Tyr Ile His Val
    210                 215                 220

Ala Ala Ala Val Leu Arg Ile Arg Ser Val Glu Gly Arg Lys Lys Ala
225                 230                 235                 240

Phe Ser Thr Cys Gly Ser His Leu Thr Val Val Ala Ile Phe Tyr Gly
                245                 250                 255

Ser Gly Ile Phe Asn Tyr Met Arg Leu Gly Ser Thr Lys Leu Ser Asp
            260                 265                 270

Lys Asp Lys Ala Val Gly Ile Phe Asn Thr Val Ile Asn Pro Met Leu
        275                 280                 285

Asn Pro Ile Ile Tyr Ser Phe Arg Asn Pro Asp Val Gln Ser Ala Ile
    290                 295                 300

Trp Arg Met Leu Thr Gly Arg Arg Ser Leu Ala
305                 310                 315

<210>  53
<211>  939
<212>  DNA
<213>  Homo sapiens

<220>
<221>  CDS
<222>  (1)..(939)

<400>  53
atg gat gga ggc aac cag agt gaa ggt tca gag ttc ctt ctc ctg ggg      48
Met Asp Gly Gly Asn Gln Ser Glu Gly Ser Glu Phe Leu Leu Leu Gly
1               5                   10                  15

atg tca gag agt cct gag cag cag cgg atc ctg ttt tgg atg ttc ctg      96
Met Ser Glu Ser Pro Glu Gln Gln Arg Ile Leu Phe Trp Met Phe Leu
            20                  25                  30

tcc atg tac ctg gtc acg gtg gtg gga aat gtg ctc atc atc ctg gcc      144
Ser Met Tyr Leu Val Thr Val Val Gly Asn Val Leu Ile Ile Leu Ala
        35                  40                  45

atc agc tct gat tcc cgc ctg cac acc ccc gtg tac ttc ttc ctg gcc      192

Ile Ser Ser Asp Ser Arg Leu His Thr Pro Val Tyr Phe Phe Leu Ala
    50                  55                  60

aac ctc tcc ttc act gac ctc ttc ttt gtc acc aac aca atc ccc aag      240
Asn Leu Ser Phe Thr Asp Leu Phe Phe Val Thr Asn Thr Ile Pro Lys
65                  70                  75                  80

atg ctg gtg aac ctc cag tcc cat aac aaa gcc atc tcc tat gca ggg      288
Met Leu Val Asn Leu Gln Ser His Asn Lys Ala Ile Ser Tyr Ala Gly
                85                  90                  95

tgt ctg aca cag ctc tac ttc ctg gtc tcc ttg gtg gcc ctg gac aac      336
Cys Leu Thr Gln Leu Tyr Phe Leu Val Ser Leu Val Ala Leu Asp Asn
            100                 105                 110

ctc atc ctg gct gtg atg gca tat gac cgc tat gtg gcc atc tgc tgc      384
Leu Ile Leu Ala Val Met Ala Tyr Asp Arg Tyr Val Ala Ile Cys Cys
        115                 120                 125

ccc ctc cac tac acc aca gcc atg agc cct aag ctc tgt atc tta ctc      432
Pro Leu His Tyr Thr Thr Ala Met Ser Pro Lys Leu Cys Ile Leu Leu
    130                 135                 140

ctt tcc ttg tgt tgg gtc cta tcc gtc ctc tat ggc ctc ata cac acc      480
Leu Ser Leu Cys Trp Val Leu Ser Val Leu Tyr Gly Leu Ile His Thr
145                 150                 155                 160

ctc ctc atg acc aga gtg acc ttc tgt ggg tca cga aaa atc cac tac      528
Leu Leu Met Thr Arg Val Thr Phe Cys Gly Ser Arg Lys Ile His Tyr
                165                 170                 175

atc ttc tgt gag atg tat gta ttg ctg agg atg gca tgt tcc aac att      576
Ile Phe Cys Glu Met Tyr Val Leu Leu Arg Met Ala Cys Ser Asn Ile
            180                 185                 190

cag att aat cac aca gtg ctg att gcc aca ggc tgc ttc atc ttc ctc      624
Gln Ile Asn His Thr Val Leu Ile Ala Thr Gly Cys Phe Ile Phe Leu
        195                 200                 205

att ccc ttt gga ttc gtg atc att tcc tat gtg ctg att atc aga gcc      672
Ile Pro Phe Gly Phe Val Ile Ile Ser Tyr Val Leu Ile Ile Arg Ala
    210                 215                 220

atc ctc aga ata ccc tca gtc tct aag aaa tac aaa gcc ttc tcc acc      720
Ile Leu Arg Ile Pro Ser Val Ser Lys Lys Tyr Lys Ala Phe Ser Thr
225                 230                 235                 240

tgt gcc tcc cat ttg ggt gca gtc tcc ctc ttc tat ggg aca ctt tgt      768
Cys Ala Ser His Leu Gly Ala Val Ser Leu Phe Tyr Gly Thr Leu Cys
                245                 250                 255

atg gta tac cta aag ccc ctc cat acc tac tct gtg aag gac tca gta      816
Met Val Tyr Leu Lys Pro Leu His Thr Tyr Ser Val Lys Asp Ser Val
            260                 265                 270

gcc aca gtg atg tat gct gtg gtg aca ccc atg atg aat ccc ttc atc      864
Ala Thr Val Met Tyr Ala Val Val Thr Pro Met Met Asn Pro Phe Ile
        275                 280                 285

tac agc ctg agg aac aag gac atg cat ggg gct ctg gga aga ctc cta      912
Tyr Ser Leu Arg Asn Lys Asp Met His Gly Ala Leu Gly Arg Leu Leu
    290                 295                 300

gat aaa cac ttt aag agg ctg aca tga                                    939
Asp Lys His Phe Lys Arg Leu Thr
305                 310

<210>  54
<211>  312
<212>  PRT
<213>  Homo sapiens

<400>  54

Met Asp Gly Gly Asn Gln Ser Glu Gly Ser Glu Phe Leu Leu Leu Gly
1               5                   10                  15

Met Ser Glu Ser Pro Glu Gln Gln Arg Ile Leu Phe Trp Met Phe Leu
            20                  25                  30

Ser Met Tyr Leu Val Thr Val Val Gly Asn Val Leu Ile Ile Leu Ala
        35                  40                  45

Ile Ser Ser Asp Ser Arg Leu His Thr Pro Val Tyr Phe Phe Leu Ala
    50                  55                  60

Asn Leu Ser Phe Thr Asp Leu Phe Phe Val Thr Asn Thr Ile Pro Lys
65                  70                  75                  80

Met Leu Val Asn Leu Gln Ser His Asn Lys Ala Ile Ser Tyr Ala Gly
                85                  90                  95

Cys Leu Thr Gln Leu Tyr Phe Leu Val Ser Leu Val Ala Leu Asp Asn
            100                 105                 110

Leu Ile Leu Ala Val Met Ala Tyr Asp Arg Tyr Val Ala Ile Cys Cys
        115                 120                 125

Pro Leu His Tyr Thr Thr Ala Met Ser Pro Lys Leu Cys Ile Leu Leu
    130                 135                 140

Leu Ser Leu Cys Trp Val Leu Ser Val Leu Tyr Gly Leu Ile His Thr
145                 150                 155                 160

Leu Leu Met Thr Arg Val Thr Phe Cys Gly Ser Arg Lys Ile His Tyr
                165                 170                 175

Ile Phe Cys Glu Met Tyr Val Leu Leu Arg Met Ala Cys Ser Asn Ile
            180                 185                 190

Gln Ile Asn His Thr Val Leu Ile Ala Thr Gly Cys Phe Ile Phe Leu
        195                 200                 205

Ile Pro Phe Gly Phe Val Ile Ile Ser Tyr Val Leu Ile Ile Arg Ala
    210                 215                 220

Ile Leu Arg Ile Pro Ser Val Ser Lys Lys Tyr Lys Ala Phe Ser Thr
225                 230                 235                 240

Cys Ala Ser His Leu Gly Ala Val Ser Leu Phe Tyr Gly Thr Leu Cys
                245                 250                 255

Met Val Tyr Leu Lys Pro Leu His Thr Tyr Ser Val Lys Asp Ser Val
            260                 265                 270

Ala Thr Val Met Tyr Ala Val Val Thr Pro Met Met Asn Pro Phe Ile
        275                 280                 285

Tyr Ser Leu Arg Asn Lys Asp Met His Gly Ala Leu Gly Arg Leu Leu
    290                 295                 300

Asp Lys His Phe Lys Arg Leu Thr
305                 310

<210>  55
<211>  939
<212>  DNA
<213>  Homo sapiens

<220>
<221>  CDS
<222>  (1)..(939)

<400>  55
atg atg att aaa aaa aat gca agt tcg gaa gac ttc ttt att cta ctt        48
Met Met Ile Lys Lys Asn Ala Ser Ser Glu Asp Phe Phe Ile Leu Leu
1               5                   10                  15

gga ttt tct aat tgg cct cag ctg gaa gta gtt ctc ttt gtg gtt atc        96
Gly Phe Ser Asn Trp Pro Gln Leu Glu Val Val Leu Phe Val Val Ile
            20                  25                  30

ttg atc ttc tac ctg atg aca ctg aca gga aac ctg ttc atc atc atc       144
Leu Ile Phe Tyr Leu Met Thr Leu Thr Gly Asn Leu Phe Ile Ile Ile
        35                  40                  45

ctg tca tac gtg gac tcc cat ctc cac aca cca atg tac ttc ttc ctt       192
Leu Ser Tyr Val Asp Ser His Leu His Thr Pro Met Tyr Phe Phe Leu
    50                  55                  60

tca aac ctc tca ttt ctg gat ctc tgc tac acc acc agc tct atc cct       240
Ser Asn Leu Ser Phe Leu Asp Leu Cys Tyr Thr Thr Ser Ser Ile Pro
65                  70                  75                  80

cag ttg ctg gtg aat ctc cgg ggc ccg gaa aag acc atc tcg tat gct       288
Gln Leu Leu Val Asn Leu Arg Gly Pro Glu Lys Thr Ile Ser Tyr Ala
                85                  90                  95

ggt tgc atg gtt caa ctt tac ttt gtt ctt gca ctg gga atc aca gag 336
Gly Cys Met Val Gln Leu Tyr Phe Val Leu Ala Leu Gly Ile Thr Glu
            100                 105                 110

tgt gtc cta ctg gtg gtg atg tca tat gat cgt tat gta gct gtg tgt 384
Cys Val Leu Leu Val Val Met Ser Tyr Asp Arg Tyr Val Ala Val Cys
        115                 120                 125

aga cct ttg cat tac act gtc ctc atg cac cct cgt ttc tgc cac ttg 432
Arg Pro Leu His Tyr Thr Val Leu Met His Pro Arg Phe Cys His Leu
    130                 135                 140

ttg gtt gcg gct tct tgg gta att ggt ttt act atc tca gca ctt cat 480
Leu Val Ala Ala Ser Trp Val Ile Gly Phe Thr Ile Ser Ala Leu His
145                 150                 155                 160

tcc tcc ttt act ttc tgg gta ccc ctt tgt gga cat cgc cta gtg gat 528
Ser Ser Phe Thr Phe Trp Val Pro Leu Cys Gly His Arg Leu Val Asp
                165                 170                 175

cac ttc ttc tgt gaa gtt cca gca ctt ctg cgt tta tca tgt gtt gac 576
His Phe Phe Cys Glu Val Pro Ala Leu Leu Arg Leu Ser Cys Val Asp
            180                 185                 190

acc cat gca aat gag ctg acc ctc atg gtc atg agc tcc att ttt gtt 624
Thr His Ala Asn Glu Leu Thr Leu Met Val Met Ser Ser Ile Phe Val
        195                 200                 205

ctc ata cct ctc att ctc att ctc act acc tat ggt gcc att gcc cgg 672
Leu Ile Pro Leu Ile Leu Ile Leu Thr Thr Tyr Gly Ala Ile Ala Arg
    210                 215                 220

gct gta ctg agc atg caa tca acc act ggg ctt cag aaa gtg ttt agg 720
Ala Val Leu Ser Met Gln Ser Thr Thr Gly Leu Gln Lys Val Phe Arg
225                 230                 235                 240

aca tgt gga gcc cat ctt atg gtt gta tct ctc ttt ttc att cca gtc 768
Thr Cys Gly Ala His Leu Met Val Val Ser Leu Phe Phe Ile Pro Val
                245                 250                 255

atg tgc atg tat ctc cag cca cca tca gaa aat tct cct gat cag ggc 816
Met Cys Met Tyr Leu Gln Pro Pro Ser Glu Asn Ser Pro Asp Gln Gly
            260                 265                 270

aag ttc att gcc ctc ttt tat act gtt gtc aca ccg agt ctt aat cct 864
Lys Phe Ile Ala Leu Phe Tyr Thr Val Val Thr Pro Ser Leu Asn Pro
        275                 280                 285

cta atc tac act ctc aga aac aag cat gta aaa ggg gca gcg aag aga 912
Leu Ile Tyr Thr Leu Arg Asn Lys His Val Lys Gly Ala Ala Lys Arg
    290                 295                 300

cta ttg ggg tgg gag tgg ggg aag tga 939
Leu Leu Gly Trp Glu Trp Gly Lys
305                 310

<210> 56
<211> 312
<212> PRT
<213> Homo sapiens

<400>  56

Met Met Ile Lys Lys Asn Ala Ser Ser Glu Asp Phe Phe Ile Leu Leu
1               5                   10                  15

Gly Phe Ser Asn Trp Pro Gln Leu Glu Val Val Leu Phe Val Val Ile
            20                  25                  30

Leu Ile Phe Tyr Leu Met Thr Leu Thr Gly Asn Leu Phe Ile Ile Ile
        35                  40                  45

Leu Ser Tyr Val Asp Ser His Leu His Thr Pro Met Tyr Phe Phe Leu
    50                  55                  60

Ser Asn Leu Ser Phe Leu Asp Leu Cys Tyr Thr Thr Ser Ser Ile Pro
65                  70                  75                  80

Gln Leu Leu Val Asn Leu Arg Gly Pro Glu Lys Thr Ile Ser Tyr Ala
                85                  90                  95

Gly Cys Met Val Gln Leu Tyr Phe Val Leu Ala Leu Gly Ile Thr Glu
            100                 105                 110

Cys Val Leu Leu Val Val Met Ser Tyr Asp Arg Tyr Val Ala Val Cys
        115                 120                 125

Arg Pro Leu His Tyr Thr Val Leu Met His Pro Arg Phe Cys His Leu
    130                 135                 140

Leu Val Ala Ala Ser Trp Val Ile Gly Phe Thr Ile Ser Ala Leu His
145                 150                 155                 160

Ser Ser Phe Thr Phe Trp Val Pro Leu Cys Gly His Arg Leu Val Asp
                165                 170                 175

His Phe Phe Cys Glu Val Pro Ala Leu Leu Arg Leu Ser Cys Val Asp
            180                 185                 190

Thr His Ala Asn Glu Leu Thr Leu Met Val Met Ser Ser Ile Phe Val
        195                 200                 205

Leu Ile Pro Leu Ile Leu Ile Leu Thr Thr Tyr Gly Ala Ile Ala Arg
    210                 215                 220

Ala Val Leu Ser Met Gln Ser Thr Thr Gly Leu Gln Lys Val Phe Arg
225                 230                 235                 240

Thr Cys Gly Ala His Leu Met Val Val Ser Leu Phe Phe Ile Pro Val

                245                 250                 255

Met Cys Met Tyr Leu Gln Pro Pro Ser Glu Asn Ser Pro Asp Gln Gly
            260                 265                 270

Lys Phe Ile Ala Leu Phe Tyr Thr Val Val Thr Pro Ser Leu Asn Pro
        275                 280                 285

Leu Ile Tyr Thr Leu Arg Asn Lys His Val Lys Gly Ala Ala Lys Arg
    290                 295                 300

Leu Leu Gly Trp Glu Trp Gly Lys
305                 310

<210> 57
<211> 963
<212> DNA
<213> Homo sapiens

<220>
<221> CDS
<222> (1)..(963)

<400> 57
atg gac caa agc aat tat agt tct tta cat ggt ttt att ctg ctt ggc 48
Met Asp Gln Ser Asn Tyr Ser Ser Leu His Gly Phe Ile Leu Leu Gly
1               5                   10                  15

ttc tct aac cat cca aaa atg gag atg atc ctg tca gga gtt gtc gcc 96
Phe Ser Asn His Pro Lys Met Glu Met Ile Leu Ser Gly Val Val Ala
            20                  25                  30

atc ttc tac tta att aca ttg gtg ggt aac aca gcc atc att ctt gca 144
Ile Phe Tyr Leu Ile Thr Leu Val Gly Asn Thr Ala Ile Ile Leu Ala
        35                  40                  45

tct ctc ctg gat tcc cag ctt cat aca cca atg tac ttt ttc ctc aga 192
Ser Leu Leu Asp Ser Gln Leu His Thr Pro Met Tyr Phe Phe Leu Arg
    50                  55                  60

aat tta tct ttc cta gat cta tgt ttc aca acc agc atc atc cct cag 240
Asn Leu Ser Phe Leu Asp Leu Cys Phe Thr Thr Ser Ile Ile Pro Gln
65                  70                  75                  80

atg ctg gtc aac ttg tgg gga cct gat aag acc atc agc tat gtg ggt 288
Met Leu Val Asn Leu Trp Gly Pro Asp Lys Thr Ile Ser Tyr Val Gly
                85                  90                  95

tgt atc atc caa ctc tat gtt tac atg tgg ttg ggc tca gtt gag tgc 336
Cys Ile Ile Gln Leu Tyr Val Tyr Met Trp Leu Gly Ser Val Glu Cys
            100                 105                 110

ctt ctc ctg gct gtt atg tcc tat gat cgt ttt aca gct ata tgt aag 384
Leu Leu Leu Ala Val Met Ser Tyr Asp Arg Phe Thr Ala Ile Cys Lys
        115                 120                 125

ccc ttg cat tat ttt gta gtc atg aac cca cat cta tgt cta aag atg 432

Pro Leu His Tyr Phe Val Val Met Asn Pro His Leu Cys Leu Lys Met
    130                 135                 140

att atc atg atc tgg agt att agt ttg gcc aat tct gta gta tta tgt         480
Ile Ile Met Ile Trp Ser Ile Ser Leu Ala Asn Ser Val Val Leu Cys
145                 150                 155                 160

aca ctc act ctg aat ttg ccc aca tgt gga aac aac att ctg gat cat         528
Thr Leu Thr Leu Asn Leu Pro Thr Cys Gly Asn Asn Ile Leu Asp His
                165                 170                 175

ttc ttg tgt gag ttg cca gct ctg gtc aag ata gct tgt gta gac acc         576
Phe Leu Cys Glu Leu Pro Ala Leu Val Lys Ile Ala Cys Val Asp Thr
            180                 185                 190

aca aca gtt gaa atg tct gtt ttc gct tta ggc att ata att gtc ctc         624
Thr Thr Val Glu Met Ser Val Phe Ala Leu Gly Ile Ile Ile Val Leu
        195                 200                 205

aca cct ctc atc ctt att ctt ata tcc tat ggc tac att gcc aaa gct         672
Thr Pro Leu Ile Leu Ile Leu Ile Ser Tyr Gly Tyr Ile Ala Lys Ala
    210                 215                 220

gtg ctg aga acg aag tca aaa gca agc cag cga aaa gca atg aat acc         720
Val Leu Arg Thr Lys Ser Lys Ala Ser Gln Arg Lys Ala Met Asn Thr
225                 230                 235                 240

tgt gga tct cat ctt act gta gtg tct atg ttc tat gga act att atc         768
Cys Gly Ser His Leu Thr Val Val Ser Met Phe Tyr Gly Thr Ile Ile
                245                 250                 255

tac atg tac ctg caa cca ggt aac agg gct tcc aaa gac cag ggc aag         816
Tyr Met Tyr Leu Gln Pro Gly Asn Arg Ala Ser Lys Asp Gln Gly Lys
            260                 265                 270

ttc ctc acc ctc ttt tac acc gtc atc act cca agt ctc aac ccg ctc         864
Phe Leu Thr Leu Phe Tyr Thr Val Ile Thr Pro Ser Leu Asn Pro Leu
        275                 280                 285

att tac acc tta aga aat aag gac atg aag gat gcc ctg aag aaa ctg         912
Ile Tyr Thr Leu Arg Asn Lys Asp Met Lys Asp Ala Leu Lys Lys Leu
    290                 295                 300

atg aga ttt cac cac aaa tct aca aaa ata aag agg aat tgc aag tca         960
Met Arg Phe His His Lys Ser Thr Lys Ile Lys Arg Asn Cys Lys Ser
305                 310                 315                 320

tag                                                                     963

<210>  58
<211>  320
<212>  PRT
<213>  Homo sapiens

<400>  58

Met Asp Gln Ser Asn Tyr Ser Ser Leu His Gly Phe Ile Leu Leu Gly
1               5                   10                  15

Phe Ser Asn His Pro Lys Met Glu Met Ile Leu Ser Gly Val Val Ala
            20                  25                  30

Ile Phe Tyr Leu Ile Thr Leu Val Gly Asn Thr Ala Ile Ile Leu Ala
        35                  40                  45

Ser Leu Leu Asp Ser Gln Leu His Thr Pro Met Tyr Phe Phe Leu Arg
    50                  55                  60

Asn Leu Ser Phe Leu Asp Leu Cys Phe Thr Thr Ser Ile Ile Pro Gln
65                  70                  75                  80

Met Leu Val Asn Leu Trp Gly Pro Asp Lys Thr Ile Ser Tyr Val Gly
                85                  90                  95

Cys Ile Ile Gln Leu Tyr Val Tyr Met Trp Leu Gly Ser Val Glu Cys
            100                 105                 110

Leu Leu Leu Ala Val Met Ser Tyr Asp Arg Phe Thr Ala Ile Cys Lys
        115                 120                 125

Pro Leu His Tyr Phe Val Val Met Asn Pro His Leu Cys Leu Lys Met
    130                 135                 140

Ile Ile Met Ile Trp Ser Ile Ser Leu Ala Asn Ser Val Val Leu Cys
145                 150                 155                 160

Thr Leu Thr Leu Asn Leu Pro Thr Cys Gly Asn Asn Ile Leu Asp His
                165                 170                 175

Phe Leu Cys Glu Leu Pro Ala Leu Val Lys Ile Ala Cys Val Asp Thr
            180                 185                 190

Thr Thr Val Glu Met Ser Val Phe Ala Leu Gly Ile Ile Ile Val Leu
        195                 200                 205

Thr Pro Leu Ile Leu Ile Leu Ile Ser Tyr Gly Tyr Ile Ala Lys Ala
    210                 215                 220

Val Leu Arg Thr Lys Ser Lys Ala Ser Gln Arg Lys Ala Met Asn Thr
225                 230                 235                 240

Cys Gly Ser His Leu Thr Val Val Ser Met Phe Tyr Gly Thr Ile Ile
                245                 250                 255

Tyr Met Tyr Leu Gln Pro Gly Asn Arg Ala Ser Lys Asp Gln Gly Lys
            260                 265                 270

Phe Leu Thr Leu Phe Tyr Thr Val Ile Thr Pro Ser Leu Asn Pro Leu

275                 280                 285

Ile Tyr Thr Leu Arg Asn Lys Asp Met Lys Asp Ala Leu Lys Lys Leu
    290                 295                 300

Met Arg Phe His His Lys Ser Thr Lys Ile Lys Arg Asn Cys Lys Ser
305                 310                 315                 320

<210>  59
<211>  2526
<212>  DNA
<213>  Homo sapiens

<220>
<221>  CDS
<222>  (1)..(2526)

<400>  59
atg ctg ctc tgc acg gct cgc ctg gtc ggc ctg cag ctt ctc att tcc      48
Met Leu Leu Cys Thr Ala Arg Leu Val Gly Leu Gln Leu Leu Ile Ser
1               5                   10                  15

tgc tgc tgg gcc ttt gcc tgc cat agc acg gag tct tct cct gac ttc      96
Cys Cys Trp Ala Phe Ala Cys His Ser Thr Glu Ser Ser Pro Asp Phe
            20                  25                  30

acc ctc ccc gga gat tac ctc ctg gca ggc ctg ttc cct ctc cat tct      144
Thr Leu Pro Gly Asp Tyr Leu Leu Ala Gly Leu Phe Pro Leu His Ser
        35                  40                  45

ggc tgt ctg cag gtg agg cac aga ccc gag gtg acc ctg tgt gac agg      192
Gly Cys Leu Gln Val Arg His Arg Pro Glu Val Thr Leu Cys Asp Arg
    50                  55                  60

tct tgt agc ttc aat gag cat ggc tac cac ctc ttc cag gct atg cgg      240
Ser Cys Ser Phe Asn Glu His Gly Tyr His Leu Phe Gln Ala Met Arg
65                  70                  75                  80

ctt ggg gtt gag gag ata aac aac tcc acg gcc ctg ctg ccc aac atc      288
Leu Gly Val Glu Glu Ile Asn Asn Ser Thr Ala Leu Leu Pro Asn Ile
                85                  90                  95

acc ctg ggg tac cag ctg tat gat gtg tgt tct gac tct gcc aat gtg      336
Thr Leu Gly Tyr Gln Leu Tyr Asp Val Cys Ser Asp Ser Ala Asn Val
            100                 105                 110

tat gcc acg ctg aga gtg ctc tcc ctg cca ggg caa cac cac ata gag      384
Tyr Ala Thr Leu Arg Val Leu Ser Leu Pro Gly Gln His His Ile Glu
        115                 120                 125

ctc caa gga gac ctt ctc cac tat tcc cct acg gtg ctg gca gtg att      432
Leu Gln Gly Asp Leu Leu His Tyr Ser Pro Thr Val Leu Ala Val Ile
    130                 135                 140

ggg cct gac agc acc aac cgt gct gcc acc aca gcc gcc ctg ctg agc      480
Gly Pro Asp Ser Thr Asn Arg Ala Ala Thr Thr Ala Ala Leu Leu Ser
145                 150                 155                 160

cct ttc ctg gtg ccc atg att agc tat gcg gcc agc agc gag acg ctc      528

Pro Phe Leu Val Pro Met Ile Ser Tyr Ala Ala Ser Ser Glu Thr Leu
                165                 170                 175

agc gtg aag cgg cag tat ccc tct ttc ctg cgc acc atc ccc aat gac      576
Ser Val Lys Arg Gln Tyr Pro Ser Phe Leu Arg Thr Ile Pro Asn Asp
            180                 185                 190

aag tac cag gtg gag acc atg gtg ctg ctg ctg cag aag ttc ggg tgg      624
Lys Tyr Gln Val Glu Thr Met Val Leu Leu Leu Gln Lys Phe Gly Trp
        195                 200                 205

acc tgg atc tct ctg gtt ggc agc agt gac gac tat ggg cag cta ggg      672
Thr Trp Ile Ser Leu Val Gly Ser Ser Asp Asp Tyr Gly Gln Leu Gly
    210                 215                 220

gtg cag gca ctg gag aac cag gcc act ggt cag ggg atc tgc att gct      720
Val Gln Ala Leu Glu Asn Gln Ala Thr Gly Gln Gly Ile Cys Ile Ala
225                 230                 235                 240

ttc aag gac atc atg ccc ttc tct gcc cag gtg ggc gat gag agg atg      768
Phe Lys Asp Ile Met Pro Phe Ser Ala Gln Val Gly Asp Glu Arg Met
                245                 250                 255

cag tgc ctc atg cgc cac ctg gcc cag gcc ggg gcc acc gtc gtg gtt      816
Gln Cys Leu Met Arg His Leu Ala Gln Ala Gly Ala Thr Val Val Val
            260                 265                 270

gtt ttt tcc agc cgg cag ttg gcc agg gtg ttt ttc gag tcc gtg gtg      864
Val Phe Ser Ser Arg Gln Leu Ala Arg Val Phe Phe Glu Ser Val Val
        275                 280                 285

ctg acc aac ctg act ggc aag gtg tgg gtc gcc tca gaa gcc tgg gcc      912
Leu Thr Asn Leu Thr Gly Lys Val Trp Val Ala Ser Glu Ala Trp Ala
    290                 295                 300

ctc tcc agg cac atc act ggg gtg ccc ggg atc cag cgc att ggg atg      960
Leu Ser Arg His Ile Thr Gly Val Pro Gly Ile Gln Arg Ile Gly Met
305                 310                 315                 320

gtg ctg ggc gtg gcc atc cag aag agg gct gtc cct ggc ctg aag gcg     1008
Val Leu Gly Val Ala Ile Gln Lys Arg Ala Val Pro Gly Leu Lys Ala
                325                 330                 335

ttt gaa gaa gcc tat gcc cgg gca gac aag aag gcc cct agg cct tgc     1056
Phe Glu Glu Ala Tyr Ala Arg Ala Asp Lys Lys Ala Pro Arg Pro Cys
            340                 345                 350

cac aag ggc tcc tgg tgc agc agc aat cag ctc tgc aga gaa tgc caa     1104
His Lys Gly Ser Trp Cys Ser Ser Asn Gln Leu Cys Arg Glu Cys Gln
        355                 360                 365

gct ttc atg gca cac acg atg ccc aag ctc aaa gcc ttc tcc atg agt     1152
Ala Phe Met Ala His Thr Met Pro Lys Leu Lys Ala Phe Ser Met Ser
    370                 375                 380

tct gcc tac aac gca tac cgg gct gtg tat gcg gtg gcc cat ggc ctc     1200
Ser Ala Tyr Asn Ala Tyr Arg Ala Val Tyr Ala Val Ala His Gly Leu
385                 390                 395                 400

cac cag ctc ctg ggc tgt gcc tct gga gct tgt tcc agg ggc cga gtc     1248
His Gln Leu Leu Gly Cys Ala Ser Gly Ala Cys Ser Arg Gly Arg Val
                405                 410                 415

tac ccc tgg cag ctt ttg gag cag atc cac aag gtg cat ttc ctt cta 1296
Tyr Pro Trp Gln Leu Leu Glu Gln Ile His Lys Val His Phe Leu Leu
            420                 425                 430

cac aag gac act gtg gcg ttt aat gac aac aga gat ccc ctc agt agc 1344
His Lys Asp Thr Val Ala Phe Asn Asp Asn Arg Asp Pro Leu Ser Ser
        435                 440                 445

tat aac ata att gcc tgg gac tgg aat gga ccc aag tgg acc ttc acg 1392
Tyr Asn Ile Ile Ala Trp Asp Trp Asn Gly Pro Lys Trp Thr Phe Thr
    450                 455                 460

gtc ctc ggt tcc tcc aca tgg tct cca gtt cag cta aac ata aat gag 1440
Val Leu Gly Ser Ser Thr Trp Ser Pro Val Gln Leu Asn Ile Asn Glu
465                 470                 475                 480

acc aaa atc cag tgg cac gga aag gac aac cag gtg cct aag tct gtg 1488
Thr Lys Ile Gln Trp His Gly Lys Asp Asn Gln Val Pro Lys Ser Val
                485                 490                 495

tgt tcc agc gac tgt ctt gaa ggg cac cag cga gtg gtt acg ggt ttc 1536
Cys Ser Ser Asp Cys Leu Glu Gly His Gln Arg Val Val Thr Gly Phe
            500                 505                 510

cat cac tgc tgc ttt gag tgt gtg ccc tgt ggg gct ggg acc ttc ctc 1584
His His Cys Cys Phe Glu Cys Val Pro Cys Gly Ala Gly Thr Phe Leu
        515                 520                 525

aac aag agt gac ctc tac aga tgc cag cct tgt ggg aaa gaa gag tgg 1632
Asn Lys Ser Asp Leu Tyr Arg Cys Gln Pro Cys Gly Lys Glu Glu Trp
    530                 535                 540

gca cct gag gga agc cag acc tgc ttc ccg cgc act gtg gtg ttt ttg 1680
Ala Pro Glu Gly Ser Gln Thr Cys Phe Pro Arg Thr Val Val Phe Leu
545                 550                 555                 560

gct ttg cgt gag cac acc tct tgg gtg ctg ctg gca gct aac acg ctg 1728
Ala Leu Arg Glu His Thr Ser Trp Val Leu Leu Ala Ala Asn Thr Leu
                565                 570                 575

ctg ctg ctg ctg ctg ctt ggg act gct ggc ctg ttt gcc tgg cac cta 1776
Leu Leu Leu Leu Leu Leu Gly Thr Ala Gly Leu Phe Ala Trp His Leu
            580                 585                 590

gac acc cct gtg gtg agg tca gca ggg ggc cgc ctg tgc ttt ctt atg 1824
Asp Thr Pro Val Val Arg Ser Ala Gly Gly Arg Leu Cys Phe Leu Met
        595                 600                 605

ctg ggc tcc ctg gca gca ggt agt ggc agc ctc tat ggc ttc ttt ggg 1872
Leu Gly Ser Leu Ala Ala Gly Ser Gly Ser Leu Tyr Gly Phe Phe Gly
    610                 615                 620

gaa ccc aca agg cct gcg tgc ttg cta cgc cag gcc ctc ttt gcc ctt 1920
Glu Pro Thr Arg Pro Ala Cys Leu Leu Arg Gln Ala Leu Phe Ala Leu
625                 630                 635                 640

ggt ttc acc atc ttc ctg tcc tgc ctg aca gtt cgc tca ttc caa cta 1968
Gly Phe Thr Ile Phe Leu Ser Cys Leu Thr Val Arg Ser Phe Gln Leu
                645                 650                 655

atc atc atc ttc aag ttt tcc acc aag gta cct aca ttc tac cac gcc 2016
Ile Ile Ile Phe Lys Phe Ser Thr Lys Val Pro Thr Phe Tyr His Ala
            660                 665                 670

tgg gtc caa aac cac ggt gct ggc ctg ttt gtg atg atc agc tca gcg     2064
Trp Val Gln Asn His Gly Ala Gly Leu Phe Val Met Ile Ser Ser Ala
        675                 680                 685

gcc cag ctg ctt atc tgt cta act tgg ctg gtg gtg tgg acc cca ctg     2112
Ala Gln Leu Leu Ile Cys Leu Thr Trp Leu Val Val Trp Thr Pro Leu
    690                 695                 700

cct gct agg gaa tac cag cgc ttc ccc cat ctg gtg atg ctt gag tgc     2160
Pro Ala Arg Glu Tyr Gln Arg Phe Pro His Leu Val Met Leu Glu Cys
705                 710                 715                 720

aca gag acc aac tcc ctg ggc ttc ata ctg gcc ttc ctc tac aat ggc     2208
Thr Glu Thr Asn Ser Leu Gly Phe Ile Leu Ala Phe Leu Tyr Asn Gly
                725                 730                 735

ctc ctc tcc atc agt gcc ttt gcc tgc agc tac ctg ggt aag gac ttg     2256
Leu Leu Ser Ile Ser Ala Phe Ala Cys Ser Tyr Leu Gly Lys Asp Leu
            740                 745                 750

cca gag aac tac aac gag gcc aaa tgt gtc acc ttc agc ctg ctc ttc     2304
Pro Glu Asn Tyr Asn Glu Ala Lys Cys Val Thr Phe Ser Leu Leu Phe
        755                 760                 765

aac ttc gtg tcc tgg atc gcc ttc ttc acc acg gcc agc gtc tac gac     2352
Asn Phe Val Ser Trp Ile Ala Phe Phe Thr Thr Ala Ser Val Tyr Asp
    770                 775                 780

ggc aag tac ctg cct gcg gcc aac atg atg gct ggg ctg agc agc ctg     2400
Gly Lys Tyr Leu Pro Ala Ala Asn Met Met Ala Gly Leu Ser Ser Leu
785                 790                 795                 800

agc agc ggc ttc ggt ggg tat ttt ctg cct aag tgc tac gtg atc ctc     2448
Ser Ser Gly Phe Gly Gly Tyr Phe Leu Pro Lys Cys Tyr Val Ile Leu
                805                 810                 815

tgc cgc cca gac ctc aac agc aca gag cac ttc cag gcc tcc att cag     2496
Cys Arg Pro Asp Leu Asn Ser Thr Glu His Phe Gln Ala Ser Ile Gln
            820                 825                 830

gac tac acg agg cgc tgc ggc tcc acc tga                             2526
Asp Tyr Thr Arg Arg Cys Gly Ser Thr
        835                 840

<210>  60
<211>  841
<212>  PRT
<213>  Homo sapiens

<400>  60

Met Leu Leu Cys Thr Ala Arg Leu Val Gly Leu Gln Leu Leu Ile Ser
1               5                   10                  15

Cys Cys Trp Ala Phe Ala Cys His Ser Thr Glu Ser Ser Pro Asp Phe
            20                  25                  30

Thr Leu Pro Gly Asp Tyr Leu Leu Ala Gly Leu Phe Pro Leu His Ser
        35                  40                  45

Gly Cys Leu Gln Val Arg His Arg Pro Glu Val Thr Leu Cys Asp Arg
    50                  55                  60

Ser Cys Ser Phe Asn Glu His Gly Tyr His Leu Phe Gln Ala Met Arg
65                  70                  75                  80

Leu Gly Val Glu Glu Ile Asn Asn Ser Thr Ala Leu Leu Pro Asn Ile
                85                  90                  95

Thr Leu Gly Tyr Gln Leu Tyr Asp Val Cys Ser Asp Ser Ala Asn Val
            100                 105                 110

Tyr Ala Thr Leu Arg Val Leu Ser Leu Pro Gly Gln His His Ile Glu
        115                 120                 125

Leu Gln Gly Asp Leu Leu His Tyr Ser Pro Thr Val Leu Ala Val Ile
    130                 135                 140

Gly Pro Asp Ser Thr Asn Arg Ala Ala Thr Thr Ala Ala Leu Leu Ser
145                 150                 155                 160

Pro Phe Leu Val Pro Met Ile Ser Tyr Ala Ala Ser Ser Glu Thr Leu
                165                 170                 175

Ser Val Lys Arg Gln Tyr Pro Ser Phe Leu Arg Thr Ile Pro Asn Asp
            180                 185                 190

Lys Tyr Gln Val Glu Thr Met Val Leu Leu Leu Gln Lys Phe Gly Trp
        195                 200                 205

Thr Trp Ile Ser Leu Val Gly Ser Ser Asp Asp Tyr Gly Gln Leu Gly
    210                 215                 220

Val Gln Ala Leu Glu Asn Gln Ala Thr Gly Gln Gly Ile Cys Ile Ala
225                 230                 235                 240

Phe Lys Asp Ile Met Pro Phe Ser Ala Gln Val Gly Asp Glu Arg Met
                245                 250                 255

Gln Cys Leu Met Arg His Leu Ala Gln Ala Gly Ala Thr Val Val Val
            260                 265                 270

Val Phe Ser Ser Arg Gln Leu Ala Arg Val Phe Phe Glu Ser Val Val
        275                 280                 285

Leu Thr Asn Leu Thr Gly Lys Val Trp Val Ala Ser Glu Ala Trp Ala

290                 295                 300

Leu Ser Arg His Ile Thr Gly Val Pro Gly Ile Gln Arg Ile Gly Met
305                 310                 315                 320

Val Leu Gly Val Ala Ile Gln Lys Arg Ala Val Pro Gly Leu Lys Ala
                325                 330                 335

Phe Glu Glu Ala Tyr Ala Arg Ala Asp Lys Lys Ala Pro Arg Pro Cys
            340                 345                 350

His Lys Gly Ser Trp Cys Ser Ser Asn Gln Leu Cys Arg Glu Cys Gln
        355                 360                 365

Ala Phe Met Ala His Thr Met Pro Lys Leu Lys Ala Phe Ser Met Ser
    370                 375                 380

Ser Ala Tyr Asn Ala Tyr Arg Ala Val Tyr Ala Val Ala His Gly Leu
385                 390                 395                 400

His Gln Leu Leu Gly Cys Ala Ser Gly Ala Cys Ser Arg Gly Arg Val
                405                 410                 415

Tyr Pro Trp Gln Leu Leu Glu Gln Ile His Lys Val His Phe Leu Leu
            420                 425                 430

His Lys Asp Thr Val Ala Phe Asn Asp Asn Arg Asp Pro Leu Ser Ser
        435                 440                 445

Tyr Asn Ile Ile Ala Trp Asp Trp Asn Gly Pro Lys Trp Thr Phe Thr
    450                 455                 460

Val Leu Gly Ser Ser Thr Trp Ser Pro Val Gln Leu Asn Ile Asn Glu
465                 470                 475                 480

Thr Lys Ile Gln Trp His Gly Lys Asp Asn Gln Val Pro Lys Ser Val
                485                 490                 495

Cys Ser Ser Asp Cys Leu Glu Gly His Gln Arg Val Val Thr Gly Phe
            500                 505                 510

His His Cys Cys Phe Glu Cys Val Pro Cys Gly Ala Gly Thr Phe Leu
        515                 520                 525

Asn Lys Ser Asp Leu Tyr Arg Cys Gln Pro Cys Gly Lys Glu Glu Trp
    530                 535                 540

Ala Pro Glu Gly Ser Gln Thr Cys Phe Pro Arg Thr Val Val Phe Leu
545                 550                 555                 560

Ala Leu Arg Glu His Thr Ser Trp Val Leu Leu Ala Ala Asn Thr Leu
                565                 570                 575

Leu Leu Leu Leu Leu Leu Gly Thr Ala Gly Leu Phe Ala Trp His Leu
            580                 585                 590

Asp Thr Pro Val Val Arg Ser Ala Gly Gly Arg Leu Cys Phe Leu Met
        595                 600                 605

Leu Gly Ser Leu Ala Ala Gly Ser Gly Ser Leu Tyr Gly Phe Phe Gly
    610                 615                 620

Glu Pro Thr Arg Pro Ala Cys Leu Leu Arg Gln Ala Leu Phe Ala Leu
625                 630                 635                 640

Gly Phe Thr Ile Phe Leu Ser Cys Leu Thr Val Arg Ser Phe Gln Leu
                645                 650                 655

Ile Ile Ile Phe Lys Phe Ser Thr Lys Val Pro Thr Phe Tyr His Ala
            660                 665                 670

Trp Val Gln Asn His Gly Ala Gly Leu Phe Val Met Ile Ser Ser Ala
        675                 680                 685

Ala Gln Leu Leu Ile Cys Leu Thr Trp Leu Val Val Trp Thr Pro Leu
    690                 695                 700

Pro Ala Arg Glu Tyr Gln Arg Phe Pro His Leu Val Met Leu Glu Cys
705                 710                 715                 720

Thr Glu Thr Asn Ser Leu Gly Phe Ile Leu Ala Phe Leu Tyr Asn Gly
                725                 730                 735

Leu Leu Ser Ile Ser Ala Phe Ala Cys Ser Tyr Leu Gly Lys Asp Leu
            740                 745                 750

Pro Glu Asn Tyr Asn Glu Ala Lys Cys Val Thr Phe Ser Leu Leu Phe
        755                 760                 765

Asn Phe Val Ser Trp Ile Ala Phe Phe Thr Thr Ala Ser Val Tyr Asp
    770                 775                 780

Gly Lys Tyr Leu Pro Ala Ala Asn Met Met Ala Gly Leu Ser Ser Leu
785                 790                 795                 800

Ser Ser Gly Phe Gly Gly Tyr Phe Leu Pro Lys Cys Tyr Val Ile Leu
                805                 810                 815

Cys Arg Pro Asp Leu Asn Ser Thr Glu His Phe Gln Ala Ser Ile Gln
            820                 825                 830

Asp Tyr Thr Arg Arg Cys Gly Ser Thr
        835                 840

<210>  61
<211>  2520
<212>  DNA
<213>  Homo sapiens

<220>
<221>  CDS
<222>  (1)..(2520)

<400>  61
atg ggg ccc agg gca aag acc atc tcc tcc ctg ttc ttc ctc cta tgg      48
Met Gly Pro Arg Ala Lys Thr Ile Ser Ser Leu Phe Phe Leu Leu Trp
1               5                   10                  15

gtc ctg gct gag ccg gct gag aac tcg gac ttc tac ctg cct ggg gat      96
Val Leu Ala Glu Pro Ala Glu Asn Ser Asp Phe Tyr Leu Pro Gly Asp
            20                  25                  30

tac ctc ctg ggt ggc ctc ttc tcc ctc cat gcc aac atg aag ggc att      144
Tyr Leu Leu Gly Gly Leu Phe Ser Leu His Ala Asn Met Lys Gly Ile
        35                  40                  45

gtt cac ctt aac ttc ctg cag gtg ccc atg tgc aag gag tat gaa gtg      192
Val His Leu Asn Phe Leu Gln Val Pro Met Cys Lys Glu Tyr Glu Val
    50                  55                  60

aag gtg ata ggc tac aac ctc atg cag gcc atg cgc ttt gcg gtg gag      240
Lys Val Ile Gly Tyr Asn Leu Met Gln Ala Met Arg Phe Ala Val Glu
65                  70                  75                  80

gag atc aac aat gac agc agc ctg ctg cct ggt gtg ctg ctg ggc tat      288
Glu Ile Asn Asn Asp Ser Ser Leu Leu Pro Gly Val Leu Leu Gly Tyr
                85                  90                  95

gag atc gtg gat gtg tgc tac atc tcc aac aat gtc cag ccg gtg ctc      336
Glu Ile Val Asp Val Cys Tyr Ile Ser Asn Asn Val Gln Pro Val Leu
            100                 105                 110

tac ttc ctg gca cac gag gac aac ctc ctt ccc atc caa gag gac tac      384
Tyr Phe Leu Ala His Glu Asp Asn Leu Leu Pro Ile Gln Glu Asp Tyr
        115                 120                 125

agt aac tac att tcc cgt gtg gtg gct gtc att ggc cct gac aac tcc      432
Ser Asn Tyr Ile Ser Arg Val Val Ala Val Ile Gly Pro Asp Asn Ser
    130                 135                 140

gag tct gtc atg act gtg gcc aac ttc ctc tcc cta ttt ctc ctt cca      480
Glu Ser Val Met Thr Val Ala Asn Phe Leu Ser Leu Phe Leu Leu Pro
145                 150                 155                 160

cag atc acc tac agc gcc atc agc gat gag ctg cga gac aag gtg cgc 528
Gln Ile Thr Tyr Ser Ala Ile Ser Asp Glu Leu Arg Asp Lys Val Arg
                165                 170                 175

ttc ccg gct ttg ctg cgt acc aca ccc agc gcc gac cac cac atc gag 576
Phe Pro Ala Leu Leu Arg Thr Thr Pro Ser Ala Asp His His Ile Glu
            180                 185                 190

gcc atg gtg cag ctg atg ctg cac ttc cgc tgg aac tgg atc att gtg 624
Ala Met Val Gln Leu Met Leu His Phe Arg Trp Asn Trp Ile Ile Val
        195                 200                 205

ctg gtg agc agc gac acc tat ggc cgc gac aat ggc cag ctg ctt ggc 672
Leu Val Ser Ser Asp Thr Tyr Gly Arg Asp Asn Gly Gln Leu Leu Gly
    210                 215                 220

gag cgc gtg gcc cgg cgc gac atc tgc atc gcc ttc cag gag acg ctg 720
Glu Arg Val Ala Arg Arg Asp Ile Cys Ile Ala Phe Gln Glu Thr Leu
225                 230                 235                 240

ccc aca ctg cag ccc aac cag aac atg acg tca gag gag cgc cag cgc 768
Pro Thr Leu Gln Pro Asn Gln Asn Met Thr Ser Glu Glu Arg Gln Arg
                245                 250                 255

ctg gtg acc att gtg gac aag ctg cag cag agc aca gcg cgc gtc gtg 816
Leu Val Thr Ile Val Asp Lys Leu Gln Gln Ser Thr Ala Arg Val Val
            260                 265                 270

gtc gtg ttc tcg ccc gac ctg acc ctg tac cac ttc ttc aat gag gtg 864
Val Val Phe Ser Pro Asp Leu Thr Leu Tyr His Phe Phe Asn Glu Val
        275                 280                 285

ctg cgc cag aac ttc act ggc gcc gtg tgg atc gcc tcc gag tcc tgg 912
Leu Arg Gln Asn Phe Thr Gly Ala Val Trp Ile Ala Ser Glu Ser Trp
    290                 295                 300

gcc atc gac ccg gtc ctg cac aac ctc acg gag ctg cgc cac ttg ggc 960
Ala Ile Asp Pro Val Leu His Asn Leu Thr Glu Leu Arg His Leu Gly
305                 310                 315                 320

acc ttc ctg ggc atc acc atc cag agc gtg ccc atc ccg ggc ttc agt 1008
Thr Phe Leu Gly Ile Thr Ile Gln Ser Val Pro Ile Pro Gly Phe Ser
                325                 330                 335

gag ttc cgc gag tgg ggc cca cag gct ggg ccg cca ccc ctc agc agg 1056
Glu Phe Arg Glu Trp Gly Pro Gln Ala Gly Pro Pro Pro Leu Ser Arg
            340                 345                 350

acc agc cag agc tat acc tgc aac cag gag tgc gac aac tgc ctg aac 1104
Thr Ser Gln Ser Tyr Thr Cys Asn Gln Glu Cys Asp Asn Cys Leu Asn
        355                 360                 365

gcc acc ttg tcc ttc aac acc att ctc agg ctc tct ggg gag cgt gtc 1152
Ala Thr Leu Ser Phe Asn Thr Ile Leu Arg Leu Ser Gly Glu Arg Val
    370                 375                 380

gtc tac agc gtg tac tct gcg gtc tat gct gtg gcc cat gcc ctg cac 1200
Val Tyr Ser Val Tyr Ser Ala Val Tyr Ala Val Ala His Ala Leu His
385                 390                 395                 400

agc ctc ctc ggc tgt gac aaa agc acc tgc acc aag agg gtg gtc tac 1248
Ser Leu Leu Gly Cys Asp Lys Ser Thr Cys Thr Lys Arg Val Val Tyr

405                 410                 415

ccc tgg cag ctg ctt gag gag atc tgg aag gtc aac ttc act ctc ctg        1296
Pro Trp Gln Leu Leu Glu Glu Ile Trp Lys Val Asn Phe Thr Leu Leu
            420                 425                 430

gac cac caa atc ttc ttc gac ccg caa ggg gac gtg gct ctg cac ttg        1344
Asp His Gln Ile Phe Phe Asp Pro Gln Gly Asp Val Ala Leu His Leu
        435                 440                 445

gag att gtc cag tgg caa tgg gac cgg agc cag aat ccc ttc cag agc        1392
Glu Ile Val Gln Trp Gln Trp Asp Arg Ser Gln Asn Pro Phe Gln Ser
    450                 455                 460

gtc gcc tcc tac tac ccc ctg cag cga cag ctg aag aac atc caa gac        1440
Val Ala Ser Tyr Tyr Pro Leu Gln Arg Gln Leu Lys Asn Ile Gln Asp
465                 470                 475                 480

atc tcc tgg cac acc atc aac aac acg atc cct atg tcc atg tgt tcc        1488
Ile Ser Trp His Thr Ile Asn Asn Thr Ile Pro Met Ser Met Cys Ser
                485                 490                 495

aag agg tgc cag tca ggg caa aag aag aag cct gtg ggc atc cac gtc        1536
Lys Arg Cys Gln Ser Gly Gln Lys Lys Lys Pro Val Gly Ile His Val
            500                 505                 510

tgc tgc ttc gag tgc atc gac tgc ctt ccc ggc acc ttc ctc aac cac        1584
Cys Cys Phe Glu Cys Ile Asp Cys Leu Pro Gly Thr Phe Leu Asn His
        515                 520                 525

act gaa gat gaa tat gaa tgc cag gcc tgc ccg aat aac gag tgg tcc        1632
Thr Glu Asp Glu Tyr Glu Cys Gln Ala Cys Pro Asn Asn Glu Trp Ser
    530                 535                 540

tac cag agt gag acc tcc tgc ttc aag cgg cag ctg gtc ttc ctg gaa        1680
Tyr Gln Ser Glu Thr Ser Cys Phe Lys Arg Gln Leu Val Phe Leu Glu
545                 550                 555                 560

tgg cat gag gca ccc acc atc gct gtg gcc ctg ctg gcc gcc ctg ggc        1728
Trp His Glu Ala Pro Thr Ile Ala Val Ala Leu Leu Ala Ala Leu Gly
                565                 570                 575

ttc ctc agc acc ctg gcc atc ctg gtg ata ttc tgg agg cac ttc cag        1776
Phe Leu Ser Thr Leu Ala Ile Leu Val Ile Phe Trp Arg His Phe Gln
            580                 585                 590

aca ccc ata gtt cgc tcg gct ggg ggc ccc atg tgc ttc ctg atg ctg        1824
Thr Pro Ile Val Arg Ser Ala Gly Gly Pro Met Cys Phe Leu Met Leu
        595                 600                 605

aca ctg ctg ctg gtg gca tac atg gtg gtc ccg gtg tac gtg ggg ccg        1872
Thr Leu Leu Leu Val Ala Tyr Met Val Val Pro Val Tyr Val Gly Pro
    610                 615                 620

ccc aag gtc tcc acc tgc ctc tgc cgc cag gcc ctc ttt ccc ctc tgc        1920
Pro Lys Val Ser Thr Cys Leu Cys Arg Gln Ala Leu Phe Pro Leu Cys
625                 630                 635                 640

ttc aca atc tgc atc tcc tgt atc gcc gtg cgt tct ttc cag atc gtc        1968
Phe Thr Ile Cys Ile Ser Cys Ile Ala Val Arg Ser Phe Gln Ile Val
                645                 650                 655

tgc gcc ttc aag atg gcc agc cgc ttc cca cgc gcc tac agc tac tgg        2016

Cys Ala Phe Lys Met Ala Ser Arg Phe Pro Arg Ala Tyr Ser Tyr Trp
            660                 665                 670

gtc cgc tac cag ggg ccc tac gtc tct atg gca ttt atc acg gta ctc 2064
Val Arg Tyr Gln Gly Pro Tyr Val Ser Met Ala Phe Ile Thr Val Leu
        675                 680                 685

aaa atg gtc att gtg gta att ggc atg ctg gcc acg ggc ctc agt ccc 2112
Lys Met Val Ile Val Val Ile Gly Met Leu Ala Thr Gly Leu Ser Pro
    690                 695                 700

acc acc cgt act gac ccc gat gac ccc aag atc aca att gtc tcc tgt 2160
Thr Thr Arg Thr Asp Pro Asp Asp Pro Lys Ile Thr Ile Val Ser Cys
705                 710                 715                 720

aac ccc aac tac cgc aac agc ctg ctg ttc aac acc agc ctg gac ctg 2208
Asn Pro Asn Tyr Arg Asn Ser Leu Leu Phe Asn Thr Ser Leu Asp Leu
                725                 730                 735

ctg ctc tca gtg gtg ggt ttc agc ttc gcc tac atg ggc aaa gag ctg 2256
Leu Leu Ser Val Val Gly Phe Ser Phe Ala Tyr Met Gly Lys Glu Leu
            740                 745                 750

ccc acc aac tac aac gag gcc aag ttc atc acc ctc agc atg acc ttc 2304
Pro Thr Asn Tyr Asn Glu Ala Lys Phe Ile Thr Leu Ser Met Thr Phe
        755                 760                 765

tat ttc acc tca tcc gtc tcc ctc tgc acc ttc atg tct gcc tac agc 2352
Tyr Phe Thr Ser Ser Val Ser Leu Cys Thr Phe Met Ser Ala Tyr Ser
    770                 775                 780

ggg gtg ctg gtc acc atc gtg gac ctc ttg gtc act gtg ctc aac ctc 2400
Gly Val Leu Val Thr Ile Val Asp Leu Leu Val Thr Val Leu Asn Leu
785                 790                 795                 800

ctg gcc atc agc ctg ggc tac ttc ggc ccc aag tgc tac atg atc ctc 2448
Leu Ala Ile Ser Leu Gly Tyr Phe Gly Pro Lys Cys Tyr Met Ile Leu
                805                 810                 815

ttc tac ccg gag cgc aac acg ccc gcc tac ttc aac agc atg atc cag 2496
Phe Tyr Pro Glu Arg Asn Thr Pro Ala Tyr Phe Asn Ser Met Ile Gln
            820                 825                 830

ggc tac acc atg agg agg gac tag 2520
Gly Tyr Thr Met Arg Arg Asp
        835

<210> 62
<211> 839
<212> PRT
<213> Homo sapiens

<400> 62

Met Gly Pro Arg Ala Lys Thr Ile Ser Ser Leu Phe Phe Leu Leu Trp
1               5                   10                  15

Val Leu Ala Glu Pro Ala Glu Asn Ser Asp Phe Tyr Leu Pro Gly Asp
            20                  25                  30

Tyr Leu Leu Gly Gly Leu Phe Ser Leu His Ala Asn Met Lys Gly Ile
        35                  40                  45

Val His Leu Asn Phe Leu Gln Val Pro Met Cys Lys Glu Tyr Glu Val
    50                  55                  60

Lys Val Ile Gly Tyr Asn Leu Met Gln Ala Met Arg Phe Ala Val Glu
65                  70                  75                  80

Glu Ile Asn Asn Asp Ser Ser Leu Leu Pro Gly Val Leu Leu Gly Tyr
                85                  90                  95

Glu Ile Val Asp Val Cys Tyr Ile Ser Asn Asn Val Gln Pro Val Leu
            100                 105                 110

Tyr Phe Leu Ala His Glu Asp Asn Leu Leu Pro Ile Gln Glu Asp Tyr
        115                 120                 125

Ser Asn Tyr Ile Ser Arg Val Val Ala Val Ile Gly Pro Asp Asn Ser
    130                 135                 140

Glu Ser Val Met Thr Val Ala Asn Phe Leu Ser Leu Phe Leu Leu Pro
145                 150                 155                 160

Gln Ile Thr Tyr Ser Ala Ile Ser Asp Glu Leu Arg Asp Lys Val Arg
                165                 170                 175

Phe Pro Ala Leu Leu Arg Thr Thr Pro Ser Ala Asp His His Ile Glu
            180                 185                 190

Ala Met Val Gln Leu Met Leu His Phe Arg Trp Asn Trp Ile Ile Val
        195                 200                 205

Leu Val Ser Ser Asp Thr Tyr Gly Arg Asp Asn Gly Gln Leu Leu Gly
    210                 215                 220

Glu Arg Val Ala Arg Arg Asp Ile Cys Ile Ala Phe Gln Glu Thr Leu
225                 230                 235                 240

Pro Thr Leu Gln Pro Asn Gln Asn Met Thr Ser Glu Glu Arg Gln Arg
                245                 250                 255

Leu Val Thr Ile Val Asp Lys Leu Gln Gln Ser Thr Ala Arg Val Val
            260                 265                 270

Val Val Phe Ser Pro Asp Leu Thr Leu Tyr His Phe Phe Asn Glu Val
        275                 280                 285

Leu Arg Gln Asn Phe Thr Gly Ala Val Trp Ile Ala Ser Glu Ser Trp
    290                 295                 300

Ala Ile Asp Pro Val Leu His Asn Leu Thr Glu Leu Arg His Leu Gly
305                 310                 315                 320

Thr Phe Leu Gly Ile Thr Ile Gln Ser Val Pro Ile Pro Gly Phe Ser
                325                 330                 335

Glu Phe Arg Glu Trp Gly Pro Gln Ala Gly Pro Pro Pro Leu Ser Arg
            340                 345                 350

Thr Ser Gln Ser Tyr Thr Cys Asn Gln Glu Cys Asp Asn Cys Leu Asn
        355                 360                 365

Ala Thr Leu Ser Phe Asn Thr Ile Leu Arg Leu Ser Gly Glu Arg Val
    370                 375                 380

Val Tyr Ser Val Tyr Ser Ala Val Tyr Ala Val Ala His Ala Leu His
385                 390                 395                 400

Ser Leu Leu Gly Cys Asp Lys Ser Thr Cys Thr Lys Arg Val Val Tyr
                405                 410                 415

Pro Trp Gln Leu Leu Glu Glu Ile Trp Lys Val Asn Phe Thr Leu Leu
            420                 425                 430

Asp His Gln Ile Phe Phe Asp Pro Gln Gly Asp Val Ala Leu His Leu
        435                 440                 445

Glu Ile Val Gln Trp Gln Trp Asp Arg Ser Gln Asn Pro Phe Gln Ser
    450                 455                 460

Val Ala Ser Tyr Tyr Pro Leu Gln Arg Gln Leu Lys Asn Ile Gln Asp
465                 470                 475                 480

Ile Ser Trp His Thr Ile Asn Asn Thr Ile Pro Met Ser Met Cys Ser
                485                 490                 495

Lys Arg Cys Gln Ser Gly Gln Lys Lys Lys Pro Val Gly Ile His Val
            500                 505                 510

Cys Cys Phe Glu Cys Ile Asp Cys Leu Pro Gly Thr Phe Leu Asn His
        515                 520                 525

Thr Glu Asp Glu Tyr Glu Cys Gln Ala Cys Pro Asn Asn Glu Trp Ser
    530                 535                 540

Tyr Gln Ser Glu Thr Ser Cys Phe Lys Arg Gln Leu Val Phe Leu Glu
545                 550                 555                 560

Trp His Glu Ala Pro Thr Ile Ala Val Ala Leu Leu Ala Ala Leu Gly
                565                 570                 575

Phe Leu Ser Thr Leu Ala Ile Leu Val Ile Phe Trp Arg His Phe Gln
            580                 585                 590

Thr Pro Ile Val Arg Ser Ala Gly Gly Pro Met Cys Phe Leu Met Leu
        595                 600                 605

Thr Leu Leu Leu Val Ala Tyr Met Val Val Pro Val Tyr Val Gly Pro
    610                 615                 620

Pro Lys Val Ser Thr Cys Leu Cys Arg Gln Ala Leu Phe Pro Leu Cys
625                 630                 635                 640

Phe Thr Ile Cys Ile Ser Cys Ile Ala Val Arg Ser Phe Gln Ile Val
                645                 650                 655

Cys Ala Phe Lys Met Ala Ser Arg Phe Pro Arg Ala Tyr Ser Tyr Trp
            660                 665                 670

Val Arg Tyr Gln Gly Pro Tyr Val Ser Met Ala Phe Ile Thr Val Leu
        675                 680                 685

Lys Met Val Ile Val Val Ile Gly Met Leu Ala Thr Gly Leu Ser Pro
    690                 695                 700

Thr Thr Arg Thr Asp Pro Asp Asp Pro Lys Ile Thr Ile Val Ser Cys
705                 710                 715                 720

Asn Pro Asn Tyr Arg Asn Ser Leu Leu Phe Asn Thr Ser Leu Asp Leu
                725                 730                 735

Leu Leu Ser Val Val Gly Phe Ser Phe Ala Tyr Met Gly Lys Glu Leu
            740                 745                 750

Pro Thr Asn Tyr Asn Glu Ala Lys Phe Ile Thr Leu Ser Met Thr Phe
        755                 760                 765

Tyr Phe Thr Ser Ser Val Ser Leu Cys Thr Phe Met Ser Ala Tyr Ser
    770                 775                 780

Gly Val Leu Val Thr Ile Val Asp Leu Leu Val Thr Val Leu Asn Leu

785                 790                 795                 800

Leu Ala Ile Ser Leu Gly Tyr Phe Gly Pro Lys Cys Tyr Met Ile Leu
                805                 810                 815

Phe Tyr Pro Glu Arg Asn Thr Pro Ala Tyr Phe Asn Ser Met Ile Gln
            820                 825                 830

Gly Tyr Thr Met Arg Arg Asp
        835

<210>  63
<211>  2559
<212>  DNA
<213>  Homo sapiens

<220>
<221>  CDS
<222>  (1)..(2559)

<400>  63
atg ctg ggc cct gct gtc ctg ggc ctc agc ctc tgg gct ctc ctg cac      48
Met Leu Gly Pro Ala Val Leu Gly Leu Ser Leu Trp Ala Leu Leu His
1               5                   10                  15

cct ggg acg ggg gcc cca ttg tgc ctg tca cag caa ctt agg atg aag      96
Pro Gly Thr Gly Ala Pro Leu Cys Leu Ser Gln Gln Leu Arg Met Lys
            20                  25                  30

ggg gac tac gtg ctg ggg ggg ctg ttc ccc ctg ggc gag gcc gag gag      144
Gly Asp Tyr Val Leu Gly Gly Leu Phe Pro Leu Gly Glu Ala Glu Glu
        35                  40                  45

gct ggc ctc cgc agc cgg aca cgg ccc agc agc cct gtg tgc acc agg      192
Ala Gly Leu Arg Ser Arg Thr Arg Pro Ser Ser Pro Val Cys Thr Arg
    50                  55                  60

ttc tcc tca aac ggc ctg ctc tgg gca ctg gcc atg aaa atg gcc gtg      240
Phe Ser Ser Asn Gly Leu Leu Trp Ala Leu Ala Met Lys Met Ala Val
65                  70                  75                  80

gag gag atc aac aac aag tcg gat ctg ctg ccc ggg ctg cgc ctg ggc      288
Glu Glu Ile Asn Asn Lys Ser Asp Leu Leu Pro Gly Leu Arg Leu Gly
                85                  90                  95

tac gac ctc ttt gat acg tgc tcg gag cct gtg gtg gcc atg aag ccc      336
Tyr Asp Leu Phe Asp Thr Cys Ser Glu Pro Val Val Ala Met Lys Pro
            100                 105                 110

agc ctc atg ttc ctg gcc aag gca ggc agc cgc gac atc gcc gcc tac      384
Ser Leu Met Phe Leu Ala Lys Ala Gly Ser Arg Asp Ile Ala Ala Tyr
        115                 120                 125

tgc aac tac acg cag tac cag ccc cgt gtg ctg gct gtc atc ggg ccc      432
Cys Asn Tyr Thr Gln Tyr Gln Pro Arg Val Leu Ala Val Ile Gly Pro
    130                 135                 140

cac tcg tca gag ctc gcc atg gtc acc ggc aag ttc ttc agc ttc ttc      480

His Ser Ser Glu Leu Ala Met Val Thr Gly Lys Phe Phe Ser Phe Phe
145                 150                 155                 160

ctc atg ccc cag gtc agc tac ggt gct agc atg gag ctg ctg agc gcc     528
Leu Met Pro Gln Val Ser Tyr Gly Ala Ser Met Glu Leu Leu Ser Ala
                165                 170                 175

cgg gag acc ttc ccc tcc ttc ttc cgc acc gtg ccc agc gac cgt gtg     576
Arg Glu Thr Phe Pro Ser Phe Phe Arg Thr Val Pro Ser Asp Arg Val
            180                 185                 190

cag ctg acg gcc gcc gcg gag ctg ctg cag gag ttc ggc tgg aac tgg     624
Gln Leu Thr Ala Ala Ala Glu Leu Leu Gln Glu Phe Gly Trp Asn Trp
        195                 200                 205

gtg gcc gcc ctg ggc agc gac gac gag tac ggc cgg cag ggc ctg agc     672
Val Ala Ala Leu Gly Ser Asp Asp Glu Tyr Gly Arg Gln Gly Leu Ser
    210                 215                 220

atc ttc tcg gcc ctg gcc gcg gca cgc ggc atc tgc atc gcg cac gag     720
Ile Phe Ser Ala Leu Ala Ala Ala Arg Gly Ile Cys Ile Ala His Glu
225                 230                 235                 240

ggc ctg gtg ccg ctg ccc cgt gcc gat gac tcg cgg ctg ggg aag gtg     768
Gly Leu Val Pro Leu Pro Arg Ala Asp Asp Ser Arg Leu Gly Lys Val
                245                 250                 255

cag gac gtc ctg cac cag gtg aac cag agc agc gtg cag gtg gtg ctg     816
Gln Asp Val Leu His Gln Val Asn Gln Ser Ser Val Gln Val Val Leu
            260                 265                 270

ctg ttc gcc tcc gtg cac gcc gcc cac gcc ctc ttc aac tac agc atc     864
Leu Phe Ala Ser Val His Ala Ala His Ala Leu Phe Asn Tyr Ser Ile
        275                 280                 285

agc agc agg ctc tcg ccc aag gtg tgg gtg gcc agc gag gcc tgg ctg     912
Ser Ser Arg Leu Ser Pro Lys Val Trp Val Ala Ser Glu Ala Trp Leu
    290                 295                 300

acc tct gac ctg gtc atg ggg ctg ccc ggc atg gcc cag atg ggc acg     960
Thr Ser Asp Leu Val Met Gly Leu Pro Gly Met Ala Gln Met Gly Thr
305                 310                 315                 320

gtg ctt ggc ttc ctc cag agg ggt gcc cag ctg cac gag ttc ccc cag    1008
Val Leu Gly Phe Leu Gln Arg Gly Ala Gln Leu His Glu Phe Pro Gln
                325                 330                 335

tac gtg aag acg cac ctg gcc ctg gcc acc gac ccg gcc ttc tgc tct    1056
Tyr Val Lys Thr His Leu Ala Leu Ala Thr Asp Pro Ala Phe Cys Ser
            340                 345                 350

gcc ctg ggc gag agg gag cag ggt ctg gag gag gac gtg gtg ggc cag    1104
Ala Leu Gly Glu Arg Glu Gln Gly Leu Glu Glu Asp Val Val Gly Gln
        355                 360                 365

cgc tgc ccg cag tgt gac tgc atc acg ctg cag aac gtg agc gca ggg    1152
Arg Cys Pro Gln Cys Asp Cys Ile Thr Leu Gln Asn Val Ser Ala Gly
    370                 375                 380

cta aat cac cac cag acg ttc tct gtc tac gca gct gtg tat agc gtg    1200
Leu Asn His His Gln Thr Phe Ser Val Tyr Ala Ala Val Tyr Ser Val
385                 390                 395                 400

gcc cag gcc ctg cac aac act ctt cag tgc aac gcc tca ggc tgc ccc          1248
Ala Gln Ala Leu His Asn Thr Leu Gln Cys Asn Ala Ser Gly Cys Pro
                405                 410                 415

gcg cag gac ccc gtg aag ccc tgg cag ctc ctg gag aac atg tac aac          1296
Ala Gln Asp Pro Val Lys Pro Trp Gln Leu Leu Glu Asn Met Tyr Asn
            420                 425                 430

ctg acc ttc cac gtg ggc ggg ctg ccg ctg cgg ttc gac agc agc gga          1344
Leu Thr Phe His Val Gly Gly Leu Pro Leu Arg Phe Asp Ser Ser Gly
        435                 440                 445

aac gtg gac atg gag tac gac ctg aag ctg tgg gtg tgg cag ggc tca          1392
Asn Val Asp Met Glu Tyr Asp Leu Lys Leu Trp Val Trp Gln Gly Ser
    450                 455                 460

gtg ccc agg ctc cac gac gtg ggc agg ttc aac ggc agc ctc agg aca          1440
Val Pro Arg Leu His Asp Val Gly Arg Phe Asn Gly Ser Leu Arg Thr
465                 470                 475                 480

gag cgc ctg aag atc cgc tgg cac acg tct gac aac cag aag ccc gtg          1488
Glu Arg Leu Lys Ile Arg Trp His Thr Ser Asp Asn Gln Lys Pro Val
                485                 490                 495

tcc cgg tgc tcg cgg cag tgc cag gag ggc cag gtg cgc cgg gtc aag          1536
Ser Arg Cys Ser Arg Gln Cys Gln Glu Gly Gln Val Arg Arg Val Lys
            500                 505                 510

ggg ttc cac tcc tgc tgc tac gac tgt gtg gac tgc gag gcg ggc agc          1584
Gly Phe His Ser Cys Cys Tyr Asp Cys Val Asp Cys Glu Ala Gly Ser
        515                 520                 525

tac cgg caa aac cca gac gac atc gcc tgc acc ttt tgt ggc cag gat          1632
Tyr Arg Gln Asn Pro Asp Asp Ile Ala Cys Thr Phe Cys Gly Gln Asp
    530                 535                 540

gag tgg tcc ccg gag cga agc aca cgc tgc ttc cgc cgc agg tct cgg          1680
Glu Trp Ser Pro Glu Arg Ser Thr Arg Cys Phe Arg Arg Arg Ser Arg
545                 550                 555                 560

ttc ctg gca tgg ggc gag ccg gct gtg ctg ctg ctg ctc ctg ctg ctg          1728
Phe Leu Ala Trp Gly Glu Pro Ala Val Leu Leu Leu Leu Leu Leu Leu
                565                 570                 575

agc ctg gcg ctg ggc ctt gtg ctg gct gct ttg ggg ctg ttc gtt cac          1776
Ser Leu Ala Leu Gly Leu Val Leu Ala Ala Leu Gly Leu Phe Val His
            580                 585                 590

cat cgg gac agc cca ctg gtt cag gcc tcg ggg ggg ccc ctg gcc tgc          1824
His Arg Asp Ser Pro Leu Val Gln Ala Ser Gly Gly Pro Leu Ala Cys
        595                 600                 605

ttt ggc ctg gtg tgc ctg ggc ctg gtc tgc ctc agc gtc ctc ctg ttc          1872
Phe Gly Leu Val Cys Leu Gly Leu Val Cys Leu Ser Val Leu Leu Phe
    610                 615                 620

cct ggc cag ccc agc cct gcc cga tgc ctg gcc cag cag ccc ttg tcc          1920
Pro Gly Gln Pro Ser Pro Ala Arg Cys Leu Ala Gln Gln Pro Leu Ser
625                 630                 635                 640

cac ctc ccg ctc acg ggc tgc ctg agc aca ctc ttc ctg cag gcg gcc          1968
His Leu Pro Leu Thr Gly Cys Leu Ser Thr Leu Phe Leu Gln Ala Ala
                645                 650                 655

gag atc ttc gtg gag tca gaa ctg cct ctg agc tgg gca gac cgg ctg 2016
Glu Ile Phe Val Glu Ser Glu Leu Pro Leu Ser Trp Ala Asp Arg Leu
            660                 665                 670

agt ggc tgc ctg cgg ggg ccc tgg gcc tgg ctg gtg gtg ctg ctg gcc 2064
Ser Gly Cys Leu Arg Gly Pro Trp Ala Trp Leu Val Val Leu Leu Ala
        675                 680                 685

atg ctg gtg gag gtc gca ctg tgc acc tgg tac ctg gtg gcc ttc ccg 2112
Met Leu Val Glu Val Ala Leu Cys Thr Trp Tyr Leu Val Ala Phe Pro
    690                 695                 700

ccg gag gtg gtg acg gac tgg cac atg ctg ccc acg gag gcg ctg gtg 2160
Pro Glu Val Val Thr Asp Trp His Met Leu Pro Thr Glu Ala Leu Val
705                 710                 715                 720

cac tgc cgc aca cgc tcc tgg gtc agc ttc ggc cta gcg cac gcc acc 2208
His Cys Arg Thr Arg Ser Trp Val Ser Phe Gly Leu Ala His Ala Thr
                725                 730                 735

aat gcc acg ctg gcc ttt ctc tgc ttc ctg ggc act ttc ctg gtg cgg 2256
Asn Ala Thr Leu Ala Phe Leu Cys Phe Leu Gly Thr Phe Leu Val Arg
            740                 745                 750

agc cag ccg ggc cgc tac aac cgt gcc cgt ggc ctc acc ttt gcc atg 2304
Ser Gln Pro Gly Arg Tyr Asn Arg Ala Arg Gly Leu Thr Phe Ala Met
        755                 760                 765

ctg gcc tac ttc atc acc tgg gtc tcc ttt gtg ccc ctc ctg gcc aat 2352
Leu Ala Tyr Phe Ile Thr Trp Val Ser Phe Val Pro Leu Leu Ala Asn
    770                 775                 780

gtg cag gtg gtc ctc agg ccc gcc gtg cag atg ggc gcc ctc ctg ctc 2400
Val Gln Val Val Leu Arg Pro Ala Val Gln Met Gly Ala Leu Leu Leu
785                 790                 795                 800

tgt gtc ctg ggc atc ctg gct gcc ttc cac ctg ccc agg tgt tac ctg 2448
Cys Val Leu Gly Ile Leu Ala Ala Phe His Leu Pro Arg Cys Tyr Leu
                805                 810                 815

ctc atg cgg cag cca ggg ctc aac acc ccc gag ttc ttc ctg gga ggg 2496
Leu Met Arg Gln Pro Gly Leu Asn Thr Pro Glu Phe Phe Leu Gly Gly
            820                 825                 830

ggc cct ggg gat gcc caa ggc cag aat gac ggg aac aca gga aat cag 2544
Gly Pro Gly Asp Ala Gln Gly Gln Asn Asp Gly Asn Thr Gly Asn Gln
        835                 840                 845

ggg aaa cat gag tga 2559
Gly Lys His Glu
    850

<210> 64
<211> 852
<212> PRT
<213> Homo sapiens

<400> 64

Met Leu Gly Pro Ala Val Leu Gly Leu Ser Leu Trp Ala Leu Leu His
1               5                   10                  15

Pro Gly Thr Gly Ala Pro Leu Cys Leu Ser Gln Gln Leu Arg Met Lys
            20                  25                  30

Gly Asp Tyr Val Leu Gly Gly Leu Phe Pro Leu Gly Glu Ala Glu Glu
        35                  40                  45

Ala Gly Leu Arg Ser Arg Thr Arg Pro Ser Ser Pro Val Cys Thr Arg
    50                  55                  60

Phe Ser Ser Asn Gly Leu Leu Trp Ala Leu Ala Met Lys Met Ala Val
65                  70                  75                  80

Glu Glu Ile Asn Asn Lys Ser Asp Leu Leu Pro Gly Leu Arg Leu Gly
                85                  90                  95

Tyr Asp Leu Phe Asp Thr Cys Ser Glu Pro Val Val Ala Met Lys Pro
            100                 105                 110

Ser Leu Met Phe Leu Ala Lys Ala Gly Ser Arg Asp Ile Ala Ala Tyr
        115                 120                 125

Cys Asn Tyr Thr Gln Tyr Gln Pro Arg Val Leu Ala Val Ile Gly Pro
    130                 135                 140

His Ser Ser Glu Leu Ala Met Val Thr Gly Lys Phe Phe Ser Phe Phe
145                 150                 155                 160

Leu Met Pro Gln Val Ser Tyr Gly Ala Ser Met Glu Leu Leu Ser Ala
                165                 170                 175

Arg Glu Thr Phe Pro Ser Phe Phe Arg Thr Val Pro Ser Asp Arg Val
            180                 185                 190

Gln Leu Thr Ala Ala Ala Glu Leu Leu Gln Glu Phe Gly Trp Asn Trp
        195                 200                 205

Val Ala Ala Leu Gly Ser Asp Asp Glu Tyr Gly Arg Gln Gly Leu Ser
    210                 215                 220

Ile Phe Ser Ala Leu Ala Ala Ala Arg Gly Ile Cys Ile Ala His Glu
225                 230                 235                 240

Gly Leu Val Pro Leu Pro Arg Ala Asp Asp Ser Arg Leu Gly Lys Val
                245                 250                 255

Gln Asp Val Leu His Gln Val Asn Gln Ser Ser Val Gln Val Val Leu

            260                 265                 270

Leu Phe Ala Ser Val His Ala Ala His Ala Leu Phe Asn Tyr Ser Ile
        275                 280                 285

Ser Ser Arg Leu Ser Pro Lys Val Trp Val Ala Ser Glu Ala Trp Leu
    290                 295                 300

Thr Ser Asp Leu Val Met Gly Leu Pro Gly Met Ala Gln Met Gly Thr
305                 310                 315                 320

Val Leu Gly Phe Leu Gln Arg Gly Ala Gln Leu His Glu Phe Pro Gln
                325                 330                 335

Tyr Val Lys Thr His Leu Ala Leu Ala Thr Asp Pro Ala Phe Cys Ser
            340                 345                 350

Ala Leu Gly Glu Arg Glu Gln Gly Leu Glu Glu Asp Val Val Gly Gln
        355                 360                 365

Arg Cys Pro Gln Cys Asp Cys Ile Thr Leu Gln Asn Val Ser Ala Gly
    370                 375                 380

Leu Asn His His Gln Thr Phe Ser Val Tyr Ala Ala Val Tyr Ser Val
385                 390                 395                 400

Ala Gln Ala Leu His Asn Thr Leu Gln Cys Asn Ala Ser Gly Cys Pro
                405                 410                 415

Ala Gln Asp Pro Val Lys Pro Trp Gln Leu Leu Glu Asn Met Tyr Asn
            420                 425                 430

Leu Thr Phe His Val Gly Gly Leu Pro Leu Arg Phe Asp Ser Ser Gly
        435                 440                 445

Asn Val Asp Met Glu Tyr Asp Leu Lys Leu Trp Val Trp Gln Gly Ser
    450                 455                 460

Val Pro Arg Leu His Asp Val Gly Arg Phe Asn Gly Ser Leu Arg Thr
465                 470                 475                 480

Glu Arg Leu Lys Ile Arg Trp His Thr Ser Asp Asn Gln Lys Pro Val
                485                 490                 495

Ser Arg Cys Ser Arg Gln Cys Gln Glu Gly Gln Val Arg Arg Val Lys
            500                 505                 510

Gly Phe His Ser Cys Cys Tyr Asp Cys Val Asp Cys Glu Ala Gly Ser
        515                 520                 525

Tyr Arg Gln Asn Pro Asp Asp Ile Ala Cys Thr Phe Cys Gly Gln Asp
    530                 535                 540

Glu Trp Ser Pro Glu Arg Ser Thr Arg Cys Phe Arg Arg Arg Ser Arg
545                 550                 555                 560

Phe Leu Ala Trp Gly Glu Pro Ala Val Leu Leu Leu Leu Leu Leu Leu
                565                 570                 575

Ser Leu Ala Leu Gly Leu Val Leu Ala Ala Leu Gly Leu Phe Val His
            580                 585                 590

His Arg Asp Ser Pro Leu Val Gln Ala Ser Gly Gly Pro Leu Ala Cys
        595                 600                 605

Phe Gly Leu Val Cys Leu Gly Leu Val Cys Leu Ser Val Leu Leu Phe
    610                 615                 620

Pro Gly Gln Pro Ser Pro Ala Arg Cys Leu Ala Gln Gln Pro Leu Ser
625                 630                 635                 640

His Leu Pro Leu Thr Gly Cys Leu Ser Thr Leu Phe Leu Gln Ala Ala
                645                 650                 655

Glu Ile Phe Val Glu Ser Glu Leu Pro Leu Ser Trp Ala Asp Arg Leu
            660                 665                 670

Ser Gly Cys Leu Arg Gly Pro Trp Ala Trp Leu Val Val Leu Leu Ala
        675                 680                 685

Met Leu Val Glu Val Ala Leu Cys Thr Trp Tyr Leu Val Ala Phe Pro
    690                 695                 700

Pro Glu Val Val Thr Asp Trp His Met Leu Pro Thr Glu Ala Leu Val
705                 710                 715                 720

His Cys Arg Thr Arg Ser Trp Val Ser Phe Gly Leu Ala His Ala Thr
                725                 730                 735

Asn Ala Thr Leu Ala Phe Leu Cys Phe Leu Gly Thr Phe Leu Val Arg
            740                 745                 750

Ser Gln Pro Gly Arg Tyr Asn Arg Ala Arg Gly Leu Thr Phe Ala Met
        755                 760                 765

Leu Ala Tyr Phe Ile Thr Trp Val Ser Phe Val Pro Leu Leu Ala Asn
    770                 775                 780

Val Gln Val Val Leu Arg Pro Ala Val Gln Met Gly Ala Leu Leu Leu
785                 790                 795                 800

Cys Val Leu Gly Ile Leu Ala Ala Phe His Leu Pro Arg Cys Tyr Leu
                805                 810                 815

Leu Met Arg Gln Pro Gly Leu Asn Thr Pro Glu Phe Phe Leu Gly Gly
            820                 825                 830

Gly Pro Gly Asp Ala Gln Gly Gln Asn Asp Gly Asn Thr Gly Asn Gln
        835                 840                 845

Gly Lys His Glu
    850

<210>  65
<211>  1125
<212>  DNA
<213>  Homo sapiens

<220>
<221>  CDS
<222>  (1)..(1125)

<400>  65
atg gcc cgc tcg ctg acc tgg cgc tgc tgc ccc tgg tgc ctg acg gag      48
Met Ala Arg Ser Leu Thr Trp Arg Cys Cys Pro Trp Cys Leu Thr Glu
1               5                   10                  15

gat gag aag gcc gcc gcc cgg gtg gac cag gag atc aac agg atc ctc      96
Asp Glu Lys Ala Ala Ala Arg Val Asp Gln Glu Ile Asn Arg Ile Leu
            20                  25                  30

ttg gag cag aag aag cag gac cgc ggg gag ctg aag ctg ctg ctt ttg      144
Leu Glu Gln Lys Lys Gln Asp Arg Gly Glu Leu Lys Leu Leu Leu Leu
        35                  40                  45

ggc cca ggc gag agc ggg aag agc acc ttc atc aag cag atg cgg atc      192
Gly Pro Gly Glu Ser Gly Lys Ser Thr Phe Ile Lys Gln Met Arg Ile
    50                  55                  60

atc cac ggc gcc ggc tac tcg gag gag gag cgc aag ggc ttc cgg ccc      240
Ile His Gly Ala Gly Tyr Ser Glu Glu Glu Arg Lys Gly Phe Arg Pro
65                  70                  75                  80

ctg gtc tac cag aac atc ttc gtg tcc atg cgg gcc atg atc gag gcc      288
Leu Val Tyr Gln Asn Ile Phe Val Ser Met Arg Ala Met Ile Glu Ala
                85                  90                  95

atg gag cgg ctg cag att cca ttc agc agg ccc gag agc aag cac cac      336
Met Glu Arg Leu Gln Ile Pro Phe Ser Arg Pro Glu Ser Lys His His
            100                 105                 110

gct agc ctg gtc atg agc cag gac ccc tat aaa gtg acc acg ttt gag 384
Ala Ser Leu Val Met Ser Gln Asp Pro Tyr Lys Val Thr Thr Phe Glu
        115                 120                 125

aag cgc tac gct gcg gcc atg cag tgg ctg tgg agg gat gcc ggc atc 432
Lys Arg Tyr Ala Ala Ala Met Gln Trp Leu Trp Arg Asp Ala Gly Ile
    130                 135                 140

cgg gcc tgc tat gag cgt cgg cgg gaa ttc cac ctg ctc gat tca gcc 480
Arg Ala Cys Tyr Glu Arg Arg Arg Glu Phe His Leu Leu Asp Ser Ala
145                 150                 155                 160

gtg tac tac ctg tcc cac ctg gag cgc atc acc gag gag ggc tac gtc 528
Val Tyr Tyr Leu Ser His Leu Glu Arg Ile Thr Glu Glu Gly Tyr Val
                165                 170                 175

ccc aca gct cag gac gtg ctc cgc agc cgc atg ccc acc act ggc atc 576
Pro Thr Ala Gln Asp Val Leu Arg Ser Arg Met Pro Thr Thr Gly Ile
            180                 185                 190

aac gag tac tgc ttc tcc gtg cag aaa acc aac ctg cgg atc gtg gac 624
Asn Glu Tyr Cys Phe Ser Val Gln Lys Thr Asn Leu Arg Ile Val Asp
        195                 200                 205

gtc ggg ggc cag aag tca gag cgt aag aaa tgg atc cat tgt ttc gag 672
Val Gly Gly Gln Lys Ser Glu Arg Lys Lys Trp Ile His Cys Phe Glu
    210                 215                 220

aac gtg atc gcc ctc atc tac ctg gcc tca ctg agt gaa tac gac cag 720
Asn Val Ile Ala Leu Ile Tyr Leu Ala Ser Leu Ser Glu Tyr Asp Gln
225                 230                 235                 240

tgc ctg gag gag aac aac cag gag aac cgc atg aag gag agc ctc gca 768
Cys Leu Glu Glu Asn Asn Gln Glu Asn Arg Met Lys Glu Ser Leu Ala
                245                 250                 255

ttg ttt ggg act atc ctg gaa cta ccc tgg ttc aaa agc aca tcc gtc 816
Leu Phe Gly Thr Ile Leu Glu Leu Pro Trp Phe Lys Ser Thr Ser Val
            260                 265                 270

atc ctc ttt ctc aac aaa acc gac atc ctg gag gag aaa atc ccc acc 864
Ile Leu Phe Leu Asn Lys Thr Asp Ile Leu Glu Glu Lys Ile Pro Thr
        275                 280                 285

tcc cac ctg gct acc tat ttc ccc agt ttc cag ggc cct aag cag gat 912
Ser His Leu Ala Thr Tyr Phe Pro Ser Phe Gln Gly Pro Lys Gln Asp
    290                 295                 300

gct gag gca gcc aag agg ttc atc ctg gac atg tac acg agg atg tac 960
Ala Glu Ala Ala Lys Arg Phe Ile Leu Asp Met Tyr Thr Arg Met Tyr
305                 310                 315                 320

acc ggg tgc gtg gac ggc ccc gag ggc agc aag aag ggc gca cga tcc 1008
Thr Gly Cys Val Asp Gly Pro Glu Gly Ser Lys Lys Gly Ala Arg Ser
                325                 330                 335

cga cgc ctt ttc agc cac tac aca tgt gcc aca gac aca cag aac atc 1056
Arg Arg Leu Phe Ser His Tyr Thr Cys Ala Thr Asp Thr Gln Asn Ile
            340                 345                 350

cgc aag gtc ttc aag gac gtg cgg gac tcg gtg ctc gcc cgc tac ctg 1104
Arg Lys Val Phe Lys Asp Val Arg Asp Ser Val Leu Ala Arg Tyr Leu

355                 360                 365

gac gag atc aac ctg ctg tga                                         1125
Asp Glu Ile Asn Leu Leu
    370

<210>  66
<211>  374
<212>  PRT
<213>  Homo sapiens

<400>  66

Met Ala Arg Ser Leu Thr Trp Arg Cys Cys Pro Trp Cys Leu Thr Glu
1               5                   10                  15

Asp Glu Lys Ala Ala Ala Arg Val Asp Gln Glu Ile Asn Arg Ile Leu
            20                  25                  30

Leu Glu Gln Lys Lys Gln Asp Arg Gly Glu Leu Lys Leu Leu Leu Leu
        35                  40                  45

Gly Pro Gly Glu Ser Gly Lys Ser Thr Phe Ile Lys Gln Met Arg Ile
    50                  55                  60

Ile His Gly Ala Gly Tyr Ser Glu Glu Glu Arg Lys Gly Phe Arg Pro
65                  70                  75                  80

Leu Val Tyr Gln Asn Ile Phe Val Ser Met Arg Ala Met Ile Glu Ala
                85                  90                  95

Met Glu Arg Leu Gln Ile Pro Phe Ser Arg Pro Glu Ser Lys His His
            100                 105                 110

Ala Ser Leu Val Met Ser Gln Asp Pro Tyr Lys Val Thr Thr Phe Glu
        115                 120                 125

Lys Arg Tyr Ala Ala Ala Met Gln Trp Leu Trp Arg Asp Ala Gly Ile
    130                 135                 140

Arg Ala Cys Tyr Glu Arg Arg Arg Glu Phe His Leu Leu Asp Ser Ala
145                 150                 155                 160

Val Tyr Tyr Leu Ser His Leu Glu Arg Ile Thr Glu Glu Gly Tyr Val
                165                 170                 175

Pro Thr Ala Gln Asp Val Leu Arg Ser Arg Met Pro Thr Thr Gly Ile
            180                 185                 190

Asn Glu Tyr Cys Phe Ser Val Gln Lys Thr Asn Leu Arg Ile Val Asp

195                 200                 205

Val Gly Gly Gln Lys Ser Glu Arg Lys Lys Trp Ile His Cys Phe Glu
    210                 215                 220

Asn Val Ile Ala Leu Ile Tyr Leu Ala Ser Leu Ser Glu Tyr Asp Gln
225                 230                 235                 240

Cys Leu Glu Glu Asn Asn Gln Glu Asn Arg Met Lys Glu Ser Leu Ala
                245                 250                 255

Leu Phe Gly Thr Ile Leu Glu Leu Pro Trp Phe Lys Ser Thr Ser Val
            260                 265                 270

Ile Leu Phe Leu Asn Lys Thr Asp Ile Leu Glu Glu Lys Ile Pro Thr
        275                 280                 285

Ser His Leu Ala Thr Tyr Phe Pro Ser Phe Gln Gly Pro Lys Gln Asp
    290                 295                 300

Ala Glu Ala Ala Lys Arg Phe Ile Leu Asp Met Tyr Thr Arg Met Tyr
305                 310                 315                 320

Thr Gly Cys Val Asp Gly Pro Glu Gly Ser Lys Lys Gly Ala Arg Ser
                325                 330                 335

Arg Arg Leu Phe Ser His Tyr Thr Cys Ala Thr Asp Thr Gln Asn Ile
            340                 345                 350

Arg Lys Val Phe Lys Asp Val Arg Asp Ser Val Leu Ala Arg Tyr Leu
        355                 360                 365

Asp Glu Ile Asn Leu Leu
    370

<210>  67
<211>  1065
<212>  DNA
<213>  Rattus norvegicus

<220>
<221>  CDS
<222>  (1)..(1065)

<400>  67
atg gga agt gga att agt tca gag agc aag gag tca gcc aaa agg tcc      48
Met Gly Ser Gly Ile Ser Ser Glu Ser Lys Glu Ser Ala Lys Arg Ser
1               5                   10                  15

aaa gaa ctg gag aag aag ctt cag gaa gat gct gaa cga gat gca aga      96

Lys Glu Leu Glu Lys Lys Leu Gln Glu Asp Ala Glu Arg Asp Ala Arg
            20                  25                  30

act gtg aag ttg ctg cta tta gga gca ggt gaa tct gga aaa agt act      144
Thr Val Lys Leu Leu Leu Leu Gly Ala Gly Glu Ser Gly Lys Ser Thr
        35                  40                  45

att gtt aaa caa atg aag atc atc cac aag aat ggt tac agt aaa caa      192
Ile Val Lys Gln Met Lys Ile Ile His Lys Asn Gly Tyr Ser Lys Gln
    50                  55                  60

gaa tgc atg gag ttt aaa gca gtg gtt tac agt aac acg ttg cag tcc      240
Glu Cys Met Glu Phe Lys Ala Val Val Tyr Ser Asn Thr Leu Gln Ser
65                  70                  75                  80

atc ctg gcc att gtg aaa gcc atg act aca cta ggg att gat tat gtc      288
Ile Leu Ala Ile Val Lys Ala Met Thr Thr Leu Gly Ile Asp Tyr Val
                85                  90                  95

aat ccg aga agt aga gag gac caa caa ctg ctt ctc tcc atg gca aac      336
Asn Pro Arg Ser Arg Glu Asp Gln Gln Leu Leu Leu Ser Met Ala Asn
            100                 105                 110

aca cta gaa gat ggt gac atg acg cct cag ttg gct gaa ata att aaa      384
Thr Leu Glu Asp Gly Asp Met Thr Pro Gln Leu Ala Glu Ile Ile Lys
        115                 120                 125

cgt ctg tgg ggc gat cca gga att caa gcc tgc ttc gaa agg gca tct      432
Arg Leu Trp Gly Asp Pro Gly Ile Gln Ala Cys Phe Glu Arg Ala Ser
    130                 135                 140

gaa tac cag ctc aat gac tct gca gct tac tac ctt aat gac tta gat      480
Glu Tyr Gln Leu Asn Asp Ser Ala Ala Tyr Tyr Leu Asn Asp Leu Asp
145                 150                 155                 160

aga ctc aca gcc cct ggg tat gtg cca aat gaa caa gac gtt cta cat      528
Arg Leu Thr Ala Pro Gly Tyr Val Pro Asn Glu Gln Asp Val Leu His
                165                 170                 175

tcc cgg gtg aaa acc act ggt atc att gaa act caa ttc tcc ttt aaa      576
Ser Arg Val Lys Thr Thr Gly Ile Ile Glu Thr Gln Phe Ser Phe Lys
            180                 185                 190

gac ttg aac ttc aga atg ttt gat gta ggt ggc cag aga tca gaa aga      624
Asp Leu Asn Phe Arg Met Phe Asp Val Gly Gly Gln Arg Ser Glu Arg
        195                 200                 205

aag aaa tgg atc cac tgc ttt gaa gga gtg acg tgc att ata ttt tgt      672
Lys Lys Trp Ile His Cys Phe Glu Gly Val Thr Cys Ile Ile Phe Cys
    210                 215                 220

gca gcc cta agt gcc tac gac atg gta ctt gta gaa gat gaa gag gtg      720
Ala Ala Leu Ser Ala Tyr Asp Met Val Leu Val Glu Asp Glu Glu Val
225                 230                 235                 240

aac aga atg cat gaa agt ctt cac ctc ttc aac agc atc tgt aat cac      768
Asn Arg Met His Glu Ser Leu His Leu Phe Asn Ser Ile Cys Asn His
                245                 250                 255

aag tat ttt gca acc acc tcc att gtt ctg ttt ctt aac aag aaa gat      816
Lys Tyr Phe Ala Thr Thr Ser Ile Val Leu Phe Leu Asn Lys Lys Asp
            260                 265                 270

ctc ttc cag gag aaa gtg acc aag gtg cac ctc agc atc tgt ttc cca 864
Leu Phe Gln Glu Lys Val Thr Lys Val His Leu Ser Ile Cys Phe Pro
        275                 280                 285

gaa tac act gga cca aat aca ttc gaa gat gca ggg aac tac atc aag 912
Glu Tyr Thr Gly Pro Asn Thr Phe Glu Asp Ala Gly Asn Tyr Ile Lys
    290                 295                 300

aac cag ttc cta gac ctg aac tta aaa aaa gaa gat aag gaa atc tat 960
Asn Gln Phe Leu Asp Leu Asn Leu Lys Lys Glu Asp Lys Glu Ile Tyr
305                 310                 315                 320

tct cac atg acc tgc gct act gac aca caa aac gtc aaa ttc gtg ttt 1008
Ser His Met Thr Cys Ala Thr Asp Thr Gln Asn Val Lys Phe Val Phe
                325                 330                 335

gat gcc gtg aca gat ata ata ata aaa gag aac ctc aaa gac tgt ggg 1056
Asp Ala Val Thr Asp Ile Ile Ile Lys Glu Asn Leu Lys Asp Cys Gly
            340                 345                 350

ctc ttc tga 1065
Leu Phe

<210> 68
<211> 354
<212> PRT
<213> Rattus norvegicus

<400> 68

Met Gly Ser Gly Ile Ser Ser Glu Ser Lys Glu Ser Ala Lys Arg Ser
1               5                   10                  15

Lys Glu Leu Glu Lys Lys Leu Gln Glu Asp Ala Glu Arg Asp Ala Arg
            20                  25                  30

Thr Val Lys Leu Leu Leu Leu Gly Ala Gly Glu Ser Gly Lys Ser Thr
        35                  40                  45

Ile Val Lys Gln Met Lys Ile Ile His Lys Asn Gly Tyr Ser Lys Gln
    50                  55                  60

Glu Cys Met Glu Phe Lys Ala Val Val Tyr Ser Asn Thr Leu Gln Ser
65                  70                  75                  80

Ile Leu Ala Ile Val Lys Ala Met Thr Thr Leu Gly Ile Asp Tyr Val
                85                  90                  95

Asn Pro Arg Ser Arg Glu Asp Gln Gln Leu Leu Leu Ser Met Ala Asn
            100                 105                 110

Thr Leu Glu Asp Gly Asp Met Thr Pro Gln Leu Ala Glu Ile Ile Lys
        115                 120                 125

Arg Leu Trp Gly Asp Pro Gly Ile Gln Ala Cys Phe Glu Arg Ala Ser
    130                 135                 140

Glu Tyr Gln Leu Asn Asp Ser Ala Ala Tyr Tyr Leu Asn Asp Leu Asp
145                 150                 155                 160

Arg Leu Thr Ala Pro Gly Tyr Val Pro Asn Glu Gln Asp Val Leu His
                165                 170                 175

Ser Arg Val Lys Thr Thr Gly Ile Ile Glu Thr Gln Phe Ser Phe Lys
            180                 185                 190

Asp Leu Asn Phe Arg Met Phe Asp Val Gly Gly Gln Arg Ser Glu Arg
        195                 200                 205

Lys Lys Trp Ile His Cys Phe Glu Gly Val Thr Cys Ile Ile Phe Cys
    210                 215                 220

Ala Ala Leu Ser Ala Tyr Asp Met Val Leu Val Glu Asp Glu Glu Val
225                 230                 235                 240

Asn Arg Met His Glu Ser Leu His Leu Phe Asn Ser Ile Cys Asn His
                245                 250                 255

Lys Tyr Phe Ala Thr Thr Ser Ile Val Leu Phe Leu Asn Lys Lys Asp
            260                 265                 270

Leu Phe Gln Glu Lys Val Thr Lys Val His Leu Ser Ile Cys Phe Pro
        275                 280                 285

Glu Tyr Thr Gly Pro Asn Thr Phe Glu Asp Ala Gly Asn Tyr Ile Lys
    290                 295                 300

Asn Gln Phe Leu Asp Leu Asn Leu Lys Lys Glu Asp Lys Glu Ile Tyr
305                 310                 315                 320

Ser His Met Thr Cys Ala Thr Asp Thr Gln Asn Val Lys Phe Val Phe
                325                 330                 335

Asp Ala Val Thr Asp Ile Ile Ile Lys Glu Asn Leu Lys Asp Cys Gly
            340                 345                 350

Leu Phe

<210>  69
<211>  1146

<212>  DNA
<213>  Homo sapiens

<220>
<221>  CDS
<222>  (1)..(1146)

<400>  69
atg ggg tgt ttg ggc ggc aac agc aag acg acg gaa gac cag ggc gtc      48
Met Gly Cys Leu Gly Gly Asn Ser Lys Thr Thr Glu Asp Gln Gly Val
1               5                   10                  15

gat gaa aaa gaa cga cgc gag gcc aac aaa aag atc gag aag cag ttg      96
Asp Glu Lys Glu Arg Arg Glu Ala Asn Lys Lys Ile Glu Lys Gln Leu
            20                  25                  30

cag aaa gag cgc ctg gct tac aag gct acc cac cgc ctg ctg ctc ctg      144
Gln Lys Glu Arg Leu Ala Tyr Lys Ala Thr His Arg Leu Leu Leu Leu
        35                  40                  45

ggg gct ggt gag tct ggg aaa agc act atc gtc aaa cag atg agg atc      192
Gly Ala Gly Glu Ser Gly Lys Ser Thr Ile Val Lys Gln Met Arg Ile
    50                  55                  60

ctg cac gtc aat ggg ttt aat ccc gag gaa aag aaa cag aaa att ctg      240
Leu His Val Asn Gly Phe Asn Pro Glu Glu Lys Lys Gln Lys Ile Leu
65                  70                  75                  80

gac atc cgg aaa aat gtt aaa gat gct atc gtg aca att gtt tca gca      288
Asp Ile Arg Lys Asn Val Lys Asp Ala Ile Val Thr Ile Val Ser Ala
                85                  90                  95

atg agt act ata ata cct cca gtt ccg ctg gcc aac cct gaa aac caa      336
Met Ser Thr Ile Ile Pro Pro Val Pro Leu Ala Asn Pro Glu Asn Gln
            100                 105                 110

ttt cga tca gac tac atc aag agc ata gcc cct atc act gac ttt gaa      384
Phe Arg Ser Asp Tyr Ile Lys Ser Ile Ala Pro Ile Thr Asp Phe Glu
        115                 120                 125

tat tcc cag gaa ttc ttt gac cat gtg aaa aaa ctt tgg gac gat gaa      432
Tyr Ser Gln Glu Phe Phe Asp His Val Lys Lys Leu Trp Asp Asp Glu
    130                 135                 140

ggc gtg aag gca tgc ttt gag aga tcc aac gaa tac cag ctg att gac      480
Gly Val Lys Ala Cys Phe Glu Arg Ser Asn Glu Tyr Gln Leu Ile Asp
145                 150                 155                 160

tgt gca caa tac ttc ctg gaa aga atc gac agc gtc agc ttg gtt gac      528
Cys Ala Gln Tyr Phe Leu Glu Arg Ile Asp Ser Val Ser Leu Val Asp
                165                 170                 175

tac aca ccc aca gac cag gac ctc ctc aga tgc aga gtt ctg aca tct      576
Tyr Thr Pro Thr Asp Gln Asp Leu Leu Arg Cys Arg Val Leu Thr Ser
            180                 185                 190

ggg att ttt gag aca cga ttc caa gtg gac aaa gta aac ttc cac atg      624
Gly Ile Phe Glu Thr Arg Phe Gln Val Asp Lys Val Asn Phe His Met
        195                 200                 205

ttt gat gtt ggt ggc cag agg gat gag agg aga aaa tgg atc cag tgc      672
Phe Asp Val Gly Gly Gln Arg Asp Glu Arg Arg Lys Trp Ile Gln Cys

210                 215                 220

ttt aac gat gtc aca gct atc att tac gtc gca gcc tgc agt agc tac       720
Phe Asn Asp Val Thr Ala Ile Ile Tyr Val Ala Ala Cys Ser Ser Tyr
225                 230                 235                 240

aac atg gtg att cga gaa gat aac aac acc aac agg ctg aga gag tcc       768
Asn Met Val Ile Arg Glu Asp Asn Asn Thr Asn Arg Leu Arg Glu Ser
                245                 250                 255

ctg gat ctt ttt gaa agc atc tgg aac aac agg tgg tta cgg acc att       816
Leu Asp Leu Phe Glu Ser Ile Trp Asn Asn Arg Trp Leu Arg Thr Ile
            260                 265                 270

tct atc atc ttg ttc ttg aac aaa caa gat atg ctg gca gaa aaa gtc       864
Ser Ile Ile Leu Phe Leu Asn Lys Gln Asp Met Leu Ala Glu Lys Val
        275                 280                 285

ttg gca ggg aaa tca aaa att gaa gac tat ttc cca gaa tat gca aat       912
Leu Ala Gly Lys Ser Lys Ile Glu Asp Tyr Phe Pro Glu Tyr Ala Asn
    290                 295                 300

tat act gtt cct gaa gac gca aca cca gat gca gga gaa gat ccc aaa       960
Tyr Thr Val Pro Glu Asp Ala Thr Pro Asp Ala Gly Glu Asp Pro Lys
305                 310                 315                 320

gtt aca aga gcc aag ttc ttt atc cgg gac ctg ttt ttg agg atc agc      1008
Val Thr Arg Ala Lys Phe Phe Ile Arg Asp Leu Phe Leu Arg Ile Ser
                325                 330                 335

acg gcc acc ggt gac ggc aaa cat tac tgc tac ccg cac ttc acc tgc      1056
Thr Ala Thr Gly Asp Gly Lys His Tyr Cys Tyr Pro His Phe Thr Cys
            340                 345                 350

gcc gtg gac aca gag aac atc cgc agg gtg ttc aac gac tgc cgc gac      1104
Ala Val Asp Thr Glu Asn Ile Arg Arg Val Phe Asn Asp Cys Arg Asp
        355                 360                 365

atc atc cag cgg atg cac ctc aag cag tat gag ctc ttg tga              1146
Ile Ile Gln Arg Met His Leu Lys Gln Tyr Glu Leu Leu
    370                 375                 380


<210>  70
<211>  381
<212>  PRT
<213>  Homo sapiens

<400>  70

Met Gly Cys Leu Gly Gly Asn Ser Lys Thr Thr Glu Asp Gln Gly Val
1               5                   10                  15


Asp Glu Lys Glu Arg Arg Glu Ala Asn Lys Lys Ile Glu Lys Gln Leu
            20                  25                  30


Gln Lys Glu Arg Leu Ala Tyr Lys Ala Thr His Arg Leu Leu Leu Leu
        35                  40                  45


Gly Ala Gly Glu Ser Gly Lys Ser Thr Ile Val Lys Gln Met Arg Ile

50                  55                  60

Leu His Val Asn Gly Phe Asn Pro Glu Glu Lys Lys Gln Lys Ile Leu
65                  70                  75                  80

Asp Ile Arg Lys Asn Val Lys Asp Ala Ile Val Thr Ile Val Ser Ala
                85                  90                  95

Met Ser Thr Ile Ile Pro Pro Val Pro Leu Ala Asn Pro Glu Asn Gln
            100                 105                 110

Phe Arg Ser Asp Tyr Ile Lys Ser Ile Ala Pro Ile Thr Asp Phe Glu
        115                 120                 125

Tyr Ser Gln Glu Phe Phe Asp His Val Lys Lys Leu Trp Asp Asp Glu
    130                 135                 140

Gly Val Lys Ala Cys Phe Glu Arg Ser Asn Glu Tyr Gln Leu Ile Asp
145                 150                 155                 160

Cys Ala Gln Tyr Phe Leu Glu Arg Ile Asp Ser Val Ser Leu Val Asp
                165                 170                 175

Tyr Thr Pro Thr Asp Gln Asp Leu Leu Arg Cys Arg Val Leu Thr Ser
            180                 185                 190

Gly Ile Phe Glu Thr Arg Phe Gln Val Asp Lys Val Asn Phe His Met
        195                 200                 205

Phe Asp Val Gly Gly Gln Arg Asp Glu Arg Arg Lys Trp Ile Gln Cys
    210                 215                 220

Phe Asn Asp Val Thr Ala Ile Ile Tyr Val Ala Ala Cys Ser Ser Tyr
225                 230                 235                 240

Asn Met Val Ile Arg Glu Asp Asn Asn Thr Asn Arg Leu Arg Glu Ser
                245                 250                 255

Leu Asp Leu Phe Glu Ser Ile Trp Asn Asn Arg Trp Leu Arg Thr Ile
            260                 265                 270

Ser Ile Ile Leu Phe Leu Asn Lys Gln Asp Met Leu Ala Glu Lys Val
        275                 280                 285

Leu Ala Gly Lys Ser Lys Ile Glu Asp Tyr Phe Pro Glu Tyr Ala Asn
    290                 295                 300

Tyr Thr Val Pro Glu Asp Ala Thr Pro Asp Ala Gly Glu Asp Pro Lys
305                 310                 315                 320

Val Thr Arg Ala Lys Phe Phe Ile Arg Asp Leu Phe Leu Arg Ile Ser
                325                 330                 335

Thr Ala Thr Gly Asp Gly Lys His Tyr Cys Tyr Pro His Phe Thr Cys
            340                 345                 350

Ala Val Asp Thr Glu Asn Ile Arg Arg Val Phe Asn Asp Cys Arg Asp
        355                 360                 365

Ile Ile Gln Arg Met His Leu Lys Gln Tyr Glu Leu Leu
    370                 375                 380

<210>  71
<211>  588
<212>  DNA
<213>  Encephalomyocarditis virus

<400>  71
gcccctctcc ctcccccccc cctaacgtta ctggccgaag ccgcttggaa taaggccggt      60

gtgcgtttgt ctatatgtta ttttccacca tattgccgtc ttttggcaat gtgagggccc     120

ggaaacctgg ccctgtcttc ttgacgagca ttcctagggg tctttcccct ctcgccaaag     180

gaatgcaagg tctgttgaat gtcgtgaagg aagcagttcc tctggaagct tcttgaagac     240

aaacaacgtc tgtagcgacc ctttgcaggc agcggaaccc cccacctggc gacaggtgcc     300

tctgcggcca aaagccacgt gtataagata cacctgcaaa ggcggcacaa ccccagtgcc     360

acgttgtgag ttggatagtt gtggaaagag tcaaatggct ctcctcaagc gtattcaaca     420

aggggctgaa ggatgcccag aaggtacccc attgtatggg atctgatctg gggcctcggt     480

gcacatgctt tacatgtgtt tagtcgaggt taaaaaaacg tctaggcccc ccgaaccacg     540

gggacgtggt tttcctttga aaaacacgat gataatatgg ccacaacc                  588

<210>  72
<211>  135
<212>  DNA
<213>  Rattus norvegicus

<220>
<221>  CDS
<222>  (1)..(135)

<400>  72
atg gcc gct gtt acc tat cct tca tcc gtg cct acg acc ttg gac cct       48
Met Ala Ala Val Thr Tyr Pro Ser Ser Val Pro Thr Thr Leu Asp Pro
1               5                   10                  15

ggg aat gca tcc tca gcc tgg ccc ctg gac acg tcc ctg ggg aat gca       96

Gly Asn Ala Ser Ser Ala Trp Pro Leu Asp Thr Ser Leu Gly Asn Ala
            20                  25                  30

tct gct ggc act agc ctg gca gga ctg gct gtc agt ggc                135
Ser Ala Gly Thr Ser Leu Ala Gly Leu Ala Val Ser Gly
        35                  40                  45

<210>  73
<211>  45
<212>  PRT
<213>  Rattus norvegicus

<400>  73

Met Ala Ala Val Thr Tyr Pro Ser Ser Val Pro Thr Thr Leu Asp Pro
1               5                   10                  15

Gly Asn Ala Ser Ser Ala Trp Pro Leu Asp Thr Ser Leu Gly Asn Ala
            20                  25                  30

Ser Ala Gly Thr Ser Leu Ala Gly Leu Ala Val Ser Gly
        35                  40                  45

<210>  74
<211>  1047
<212>  DNA
<213>  Homo sapiens

<220>
<221>  CDS
<222>  (1)..(1047)

<400>  74
atg aat ggc aca gaa ggc cct aac ttc tac gtg ccc ttc tcc aat gcg      48
acg ggt gtg gta cgc agc ccc ttc gag tac cca cag tac tac ctg gct      96
gag cca tgg cag ttc tcc atg ctg gcc gcc tac atg ttt ctg ctg atc      144
gtg ctg ggc ttc ccc atc aac ttc ctc acg ctc tac gtc acc gtc cag      192
cac aag aag ctg cgc acg cct ctc aac tac atc ctg ctc aac cta gcc      240
gtg gct gac ctc ttc atg gtc cta ggt ggc ttc acc agc acc ctc tac      288
acc tct ctg cat gga tac ttc gtc ttc ggg ccc aca gga tgc aat ttg      336
gag ggc ttc ttt gcc acc ctg ggc ggt gaa att gcc ctg tgg tcc ttg 384
gtg gtc ctg gcc atc gag cgg tac gtg gtg gtg tgt aag ccc atg agc 432
aac ttc cgc ttc ggg gag aac cat gcc atc atg ggc gtt gcc ttc acc 480
tgg gtc atg gcg ctg gcc tgc gcc gca ccc cca ctc gcc ggc tgg tcc 528
agg tac atc ccc gag ggc ctg cag tgc tcg tgt gga atc gac tac tac 576
acg ctc aag ccg gag gtc aac aac gag tct ttt gtc atc tac atg ttc 624
gtg gtc cac ttc acc atc ccc atg att atc atc ttt ttc tgc tat ggg 672
cag ctc gtc ttc acc gtc aag gag gcc gct gcc cag cag cag gag tca 720
gcc acc aca cag aag gca gag aag gag gtc acc cgc atg gtc atc atc 768
atg gtc atc gct ttc ctg atc tgc tgg gtg ccc tac gcc agc gtg gca 816
ttc tac atc ttc acc cac cag ggc tcc aac ttc ggt ccc atc ttc atg 864
acc atc cca gcg ttc ttt gcc aag agc gcc gcc atc tac aac cct gtc 912
atc tat atc atg atg aac aag cag ttc cgg aac tgc atg ctc acc acc 960
atc tgc tgc ggc aag aac cca ctg ggt gac gat gag gcc tct gct acc 1008
gtg tcc aag acg gag acg agc cag gtg gcc ccg gcc taa 1047

<210>  75
<211>  348
<212>  PRT
<213>  Homo sapiens

<400>  75

<210>  76
<211>  45
<212>  DNA
<213>  Artificial Sequence

<220>
<223>  V5 epitope

<400>  76
ggtaagccta tccctaaccc tctcctcggt ctcgattcta cgtga                        45

<210>  77
<211>  1125
<212>  DNA
<213>  Artificial Sequence

<220>
<223>  coding Garufa16/Garufagustducin chimera protein

<400>  77
atggcccgct cgctgacctg gcgctgctgc ccctggtgcc tgacggagga tgagaaggcc        60

gccgcccggg tggaccagga gatcaacagg atcctcttgg agcagaagaa gcaggaccgc       120

ggggagctga agctgctgct tttgggccca ggcgagagcg ggaagagcac cttcatcaag       180

cagatgcgga tcatccacgg cgccggctac tcggaggagg agcgcaaggg cttccggccc       240

ctggtctacc agaacatctt cgtgtccatg cgggccatga tcgaggccat ggagcggctg       300

cagattccat tcagcaggcc cgagagcaag caccacgcta gcctggtcat gagccaggac 360

ccctataaag tgaccacgtt tgagaagcgc tacgctgcgg ccatgcagtg gctgtggagg 420

gatgccggca tccgggcctg ctatgagcgt cggcgggaat tccacctgct cgattcagcc 480

gtgtactacc tgtcccacct ggagcgcatc accgaggagg gctacgtccc cacagctcag 540

gacgtgctcc gcagccgcat gcccaccact ggcatcaacg agtactgctt ctccgtgcag 600

aaaaccaacc tgcggatcgt ggacgtcggg ggccagaagt cagagcgtaa gaaatggatc 660

cattgtttcg agaacgtgat cgccctcatc tacctggcct cactgagtga atacgaccag 720

tgcctggagg agaacaacca ggagaaccgc atgaaggaga gcctcgcatt gtttgggact 780

atcctggaac taccctggtt caaaagcaca tccgtcatcc tctttctcaa caaaaccgac 840

atcctggagg agaaaatccc cacctcccac ctggctacct atttccccag tttccagggc 900

cctaagcagg atgctgaggc agccaagagg ttcatcctgg acatgtacac gaggatgtac 960

accgggtgcg tggacggccc cgagggcagc aagttaaaaa aagaagataa ggaaatctat 1020

tctcacatga cctgcgctac tgacacacaa aacgtcaaat tcgtgtttga tgccgtgaca 1080

gatataataa taaaagagaa cctcaaagac tgtgggctct tctga 1125

<210> 78
<211> 720
<212> DNA
<213> Homo sapiens

<220>
<221> CDS
<222> (1)..(720)

<400> 78
atg gtg agc aag ggc gag gag ctg ttc acc ggg gtg gtg ccc atc ctg 48
Met Val Ser Lys Gly Glu Glu Leu Phe Thr Gly Val Val Pro Ile Leu
1               5                   10                  15

gtc gag ctg gac ggc gac gta aac ggc cac aag ttc agc gtg tcc ggc 96
Val Glu Leu Asp Gly Asp Val Asn Gly His Lys Phe Ser Val Ser Gly
            20                  25                  30

gag ggc gag ggc gat gcc acc tac ggc aag ctg acc ctg aag ttc atc 144
Glu Gly Glu Gly Asp Ala Thr Tyr Gly Lys Leu Thr Leu Lys Phe Ile
        35                  40                  45

tgc acc acc ggc aag ctg ccc gtg ccc tgg ccc acc ctc gtg acc acc 192
Cys Thr Thr Gly Lys Leu Pro Val Pro Trp Pro Thr Leu Val Thr Thr
    50                  55                  60

ctg acc tac ggc gtg cag tgc ttc agc cgc tac ccc gac cac atg aag 240
Leu Thr Tyr Gly Val Gln Cys Phe Ser Arg Tyr Pro Asp His Met Lys
65                  70                  75                  80

cag cac gac ttc ttc aag tcc gcc atg ccc gaa ggc tac gtc cag gag 288
Gln His Asp Phe Phe Lys Ser Ala Met Pro Glu Gly Tyr Val Gln Glu

85                  90                  95

cgc acc atc ttc ttc aag gac gac ggc aac tac aag acc cgc gcc gag          336
Arg Thr Ile Phe Phe Lys Asp Asp Gly Asn Tyr Lys Thr Arg Ala Glu
            100                 105                 110

gtg aag ttc gag ggc gac acc ctg gtg aac cgc atc gag ctg aag ggc          384
Val Lys Phe Glu Gly Asp Thr Leu Val Asn Arg Ile Glu Leu Lys Gly
        115                 120                 125

atc gac ttc aag gag gac ggc aac atc ctg ggg cac aag ctg gag tac          432
Ile Asp Phe Lys Glu Asp Gly Asn Ile Leu Gly His Lys Leu Glu Tyr
    130                 135                 140

aac tac aac agc cac aac gtc tat atc atg gcc gac aag cag aag aac          480
Asn Tyr Asn Ser His Asn Val Tyr Ile Met Ala Asp Lys Gln Lys Asn
145                 150                 155                 160

ggc atc aag gtg aac ttc aag atc cgc cac aac atc gag gac ggc agc          528
Gly Ile Lys Val Asn Phe Lys Ile Arg His Asn Ile Glu Asp Gly Ser
                165                 170                 175

gtg cag ctc gcc gac cac tac cag cag aac acc ccc atc ggc gac ggc          576
Val Gln Leu Ala Asp His Tyr Gln Gln Asn Thr Pro Ile Gly Asp Gly
            180                 185                 190

ccc gtg ctg ctg ccc gac aac cac tac ctg agc acc cag tcc gcc ctg          624
Pro Val Leu Leu Pro Asp Asn His Tyr Leu Ser Thr Gln Ser Ala Leu
        195                 200                 205

agc aaa gac ccc aac gag aag cgc gat cac atg gtc ctg ctg gag ttc          672
Ser Lys Asp Pro Asn Glu Lys Arg Asp His Met Val Leu Leu Glu Phe
    210                 215                 220

gtg acc gcc gcc ggg atc act ctc ggc atg gac gag ctg tac aag taa          720
Val Thr Ala Ala Gly Ile Thr Leu Gly Met Asp Glu Leu Tyr Lys
225                 230                 235


<210>  79
<211>  239
<212>  PRT
<213>  Homo sapiens

<400>  79

Met Val Ser Lys Gly Glu Glu Leu Phe Thr Gly Val Val Pro Ile Leu
1               5                   10                  15


Val Glu Leu Asp Gly Asp Val Asn Gly His Lys Phe Ser Val Ser Gly
            20                  25                  30


Glu Gly Glu Gly Asp Ala Thr Tyr Gly Lys Leu Thr Leu Lys Phe Ile
        35                  40                  45


Cys Thr Thr Gly Lys Leu Pro Val Pro Trp Pro Thr Leu Val Thr Thr
    50                  55                  60


Leu Thr Tyr Gly Val Gln Cys Phe Ser Arg Tyr Pro Asp His Met Lys

65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  80
<211>  5529
<212>  DNA
<213>  Artificial Sequence

<220>
<223>  pcDNA3.1 Hygro(+)

<400>  80
gacggatcgg gagatctccc gatcccctat ggtgcactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttctcg agtctagagg gcccgtttaa acccgctgat cagcctcgac 960

tgtgccttct agttgccagc catctgttgt ttgcccctcc cccgtgcctt ccttgaccct 1020

ggaaggtgcc actcccactg tcctttccta ataaaatgag gaaattgcat cgcattgtct 1080

gagtaggtgt cattctattc tggggggtgg ggtggggcag gacagcaagg gggaggattg 1140

ggaagacaat agcaggcatg ctggggatgc ggtgggctct atggcttctg aggcggaaag 1200

aaccagctgg ggctctaggg ggtatcccca cgcgccctgt agcggcgcat taagcgcggc 1260

gggtgtggtg gttacgcgca gcgtgaccgc tacacttgcc agcgccctag cgcccgctcc 1320

tttcgctttc ttcccttcct ttctcgccac gttcgccggc tttccccgtc aagctctaaa 1380

tcgggggctc cctttagggt tccgatttag tgctttacgg cacctcgacc ccaaaaaact 1440

tgattagggt gatggttcac gtagtgggcc atcgccctga tagacggttt ttcgcccttt 1500

gacgttggag tccacgttct ttaatagtgg actcttgttc caaactggaa caacactcaa 1560

ccctatctcg gtctattctt ttgatttata agggattttg ccgatttcgg cctattggtt 1620

aaaaaatgag ctgatttaac aaaaatttaa cgcgaattaa ttctgtggaa tgtgtgtcag 1680

ttagggtgtg gaaagtcccc aggctcccca gcaggcagaa gtatgcaaag catgcatctc 1740

aattagtcag caaccaggtg tggaaagtcc ccaggctccc cagcaggcag aagtatgcaa 1800

agcatgcatc tcaattagtc agcaaccata gtcccgcccc taactccgcc catcccgccc 1860

ctaactccgc ccagttccgc ccattctccg ccccatggct gactaatttt ttttatttat 1920

gcagaggccg aggccgcctc tgcctctgag ctattccaga agtagtgagg aggctttttt 1980

ggaggcctag gcttttgcaa aaagctcccg ggagcttgta tatccatttt cggatctgat 2040

cagcacgtga tgaaaaagcc tgaactcacc gcgacgtctg tcgagaagtt tctgatcgaa 2100

aagttcgaca gcgtctccga cctgatgcag ctctcggagg gcgaagaatc tcgtgctttc 2160

agcttcgatg taggagggcg tggatatgtc ctgcgggtaa atagctgcgc cgatggtttc 2220

tacaaagatc gttatgttta tcggcacttt gcatcggccg cgctcccgat tccggaagtg 2280
cttgacattg gggaattcag cgagagcctg acctattgca tctcccgccg tgcacagggt 2340
gtcacgttgc aagacctgcc tgaaaccgaa ctgcccgctg ttctgcagcc ggtcgcggag 2400
gccatggatg cgatcgctgc ggccgatctt agccagacga gcgggttcgg cccattcgga 2460
ccgcaaggaa tcggtcaata cactacatgg cgtgatttca tatgcgcgat tgctgatccc 2520
catgtgtatc actggcaaac tgtgatggac gacaccgtca gtgcgtccgt cgcgcaggct 2580
ctcgatgagc tgatgctttg ggccgaggac tgccccgaag tccggcacct cgtgcacgcg 2640
gatttcggct ccaacaatgt cctgacggac aatggccgca taacagcggt cattgactgg 2700
agcgaggcga tgttcgggga ttcccaatac gaggtcgcca acatcttctt ctggaggccg 2760
tggttggctt gtatggagca gcagacgcgc tacttcgagc ggaggcatcc ggagcttgca 2820
ggatcgccgc ggctccgggc gtatatgctc cgcattggtc ttgaccaact ctatcagagc 2880
ttggttgacg gcaatttcga tgatgcagct tgggcgcagg gtcgatgcga cgcaatcgtc 2940
cgatccggag ccgggactgt cgggcgtaca caaatcgccc gcagaagcgc ggccgtctgg 3000
accgatggct gtgtagaagt actcgccgat agtggaaacc gacgccccag cactcgtccg 3060
agggcaaagg aatagcacgt gctacgagat ttcgattcca ccgccgcctt ctatgaaagg 3120
ttgggcttcg gaatcgtttt ccgggacgcc ggctggatga tcctccagcg cggggatctc 3180
atgctggagt tcttcgccca ccccaacttg tttattgcag cttataatgg ttacaaataa 3240
agcaatagca tcacaaattt cacaaataaa gcattttttt cactgcattc tagttgtggt 3300
ttgtccaaac tcatcaatgt atcttatcat gtctgtatac cgtcgacctc tagctagagc 3360
ttggcgtaat catggtcata gctgtttcct gtgtgaaatt gttatccgct cacaattcca 3420
cacaacatac gagccggaag cataaagtgt aaagcctggg gtgcctaatg agtgagctaa 3480
ctcacattaa ttgcgttgcg ctcactgccc gctttccagt cgggaaacct gtcgtgccag 3540
ctgcattaat gaatcggcca acgcgcgggg agaggcggtt tgcgtattgg gcgctcttcc 3600
gcttcctcgc tcactgactc gctgcgctcg gtcgttcggc tgcggcgagc ggtatcagct 3660
cactcaaagg cggtaatacg gttatccaca gaatcagggg ataacgcagg aaagaacatg 3720
tgagcaaaag gccagcaaaa ggccaggaac cgtaaaaagg ccgcgttgct ggcgtttttc 3780
cataggctcc gcccccctga cgagcatcac aaaaatcgac gctcaagtca gaggtggcga 3840
aacccgacag gactataaag ataccaggcg tttccccctg gaagctccct cgtgcgctct 3900
cctgttccga ccctgccgct taccggatac ctgtccgcct ttctcccttc gggaagcgtg 3960
gcgctttctc atagctcacg ctgtaggtat ctcagttcgg tgtaggtcgt tcgctccaag 4020
ctgggctgtg tgcacgaacc ccccgttcag cccgaccgct gcgccttatc cggtaactat 4080
cgtcttgagt ccaacccggt aagacacgac ttatcgccac tggcagcagc cactggtaac 4140

aggattagca gagcgaggta tgtaggcggt gctacagagt tcttgaagtg gtggcctaac 4200

tacggctaca ctagaagaac agtatttggt atctgcgctc tgctgaagcc agttaccttc 4260

ggaaaaagag ttggtagctc ttgatccggc aaacaaacca ccgctggtag cggttttttt 4320

gtttgcaagc agcagattac gcgcagaaaa aaaggatctc aagaagatcc tttgatcttt 4380

tctacggggt ctgacgctca gtggaacgaa aactcacgtt aagggatttt ggtcatgaga 4440

ttatcaaaaa ggatcttcac ctagatcctt ttaaattaaa aatgaagttt taaatcaatc 4500

taaagtatat atgagtaaac ttggtctgac agttaccaat gcttaatcag tgaggcacct 4560

atctcagcga tctgtctatt tcgttcatcc atagttgcct gactccccgt cgtgtagata 4620

actacgatac gggagggctt accatctggc cccagtgctg caatgatacc gcgagaccca 4680

cgctcaccgg ctccagattt atcagcaata aaccagccag ccggaagggc cgagcgcaga 4740

agtggtcctg caactttatc cgcctccatc cagtctatta attgttgccg ggaagctaga 4800

gtaagtagtt cgccagttaa tagtttgcgc aacgttgttg ccattgctac aggcatcgtg 4860

gtgtcacgct cgtcgtttgg tatggcttca ttcagctccg gttcccaacg atcaaggcga 4920

gttacatgat cccccatgtt gtgcaaaaaa gcggttagct ccttcggtcc tccgatcgtt 4980

gtcagaagta agttggccgc agtgttatca ctcatggtta tggcagcact gcataattct 5040

cttactgtca tgccatccgt aagatgcttt tctgtgactg gtgagtactc aaccaagtca 5100

ttctgagaat agtgtatgcg gcgaccgagt tgctcttgcc cggcgtcaat acgggataat 5160

accgcgccac atagcagaac tttaaaagtg ctcatcattg gaaaacgttc ttcggggcga 5220

aaactctcaa ggatcttacc gctgttgaga tccagttcga tgtaacccac tcgtgcaccc 5280

aactgatctt cagcatcttt tactttcacc agcgtttctg ggtgagcaaa aacaggaagg 5340

caaaatgccg caaaaaaggg aataagggcg acacggaaat gttgaatact catactcttc 5400

ctttttcaat attattgaag catttatcag ggttattgtc tcatgagcgg atacatattt 5460

gaatgtattt agaaaaataa acaaataggg gttccgcgca catttccccg aaaagtgcca 5520

cctgacgtc 5529

<210> 81
<211> 6069
<212> DNA
<213> Artificial Sequence

<220>
<223> pEAK10 vector

<400> 81
ggcgtaatct gctgcttgca aacaaaaaaa ccaccgctac cagcggtggt ttgtttgccg 60

gatcaagagc taccaactct ttttccgaag gtaactggct tcagcagagc gcagatacca 120

aatactgtcc ttctagtgta gccgtagtta ggccaccact tcaagaactc tgtagcaccg 180

cctacatacc tcgctctgct gaagccagtt accagtggct gctgccagtg gcgataagtc 240

gtgtcttacc gggttggact caagagatag ttaccggata aggcgcagcg gtcgggctga 300

acggggggtt cgtgcacaca gcccagcttg gagcgaacga cctacaccga actgagatac 360

ctacagcgtg agctatgaga aagcgccacg cttcccgaag ggagaaaggc ggacaggtat 420

ccggtaagcg gcagggtcgg aacaggagag cgcacgaggg agcttccagg gggaaacgcc 480

tggtatcttt atagtcctgt cgggtttcgc cacctctgac ttgagcgtcg atttttgtga 540

tgctcgtcag gggggcggag cctatggaaa aacgccagca acgcaagcta gagtttaaac 600

ttgacagatg agacaataac cctgataaat gcttcaataa tattgaaaaa ggaaaagtat 660

gagtattcaa catttccgtg tcgcccttat tccctttttt gcggcatttt gccttcctgt 720

ttttgctcac ccagaaacgc tggtgaaagt aaaagatgca gaagatcact tgggtgcgcg 780

agtgggttac atcgaactgg atctcaacag cggtaagatc cttgagagtt ttcgccccga 840

agaacgtttc ccaatgatga gcacttttaa agttctgcta tgtggcgcgg tattatcccg 900

tattgatgcc gggcaagagc aactcggtcg ccgcatacac tattctcaga atgacttggt 960

tgaatactca ccagtcacag aaaagcatct tacggatggc atgacagtaa gagaattatg 1020

cagtgctgcc ataaccatga gtgataacac tgcggccaac ttacttctga caactatcgg 1080

aggaccgaag gagctaaccg cttttttgca caacatgggg gatcatgtaa ctcgccttga 1140

tcgttgggaa ccggagctga atgaagccat accaaacgac gagcgtgaca ccacgatgcc 1200

tgtagcaatg gcaacaacgt tgcgaaaact attaactggc gaactactta ctctagcttc 1260

ccggcaacaa ctaatagact ggatggaggc ggataaagtt gcaggaccac ttctgcgctc 1320

ggcacttccg gctggctggt ttattgctga taaatcagga gccggtgagc gtgggtcacg 1380

cggtatcatt gcagcactgg ggccggatgg taagccctcc cgtatcgtag ttatctacac 1440

tacggggagt caggcaacta tggatgaacg aaatagacag atcgctgaga taggtgcctc 1500

actgattaag cattggtaag gataaatttc tggtaaggag gacacgtatg gaagtgggca 1560

agttggggaa gccgtatccg ttgctgaatc tggcatatgt gggagtataa gacgcgcagc 1620

gtcgcatcag gcattttttt ctgcgccaat gcaaaaaggc catccgtcag gatggccttt 1680

cggcataact agtgaggctc cggtgcccgt cagtgggcag agcgcacatc gcccacagtc 1740

cccgagaagt tggggggagg ggtcggcaat tgaaccggtg cctagagaag gtggcgcggg 1800

gtaaactggg aaagtgatgt cgtgtactgg ctccgccttt ttcccgaggg tgggggagaa 1860

ccgtatataa gtgcagtagt cgccgtgaac gttctttttc gcaacgggtt tgccgccaga 1920

acacaggtaa gtgccgtgtg tggttcccgc gggcctggcc tctttacggg ttatggccct 1980

tgcgtgcctt gaattacttc cacctggctg cagtacgtga ttcttgatcc cgagcttcgg 2040

gttggaagtg ggtgggagag ttcgaggcct tgcgcttaag gagccccttc gcctcgtgct 2100

tgagttgagg cctggcctgg gcgctggggc cgccgcgtgc gaatctggtg gcaccttcgc 2160

gcctgtctcg ctgctttcga taagtctcta gccatttaaa atttttgatg acctgctgcg 2220

acgctttttt tctggcaaga tagtcttgta aatgcgggcc aagacgatct gcacactggt 2280

atttcggttt ttggggccgc gggcggcgac ggggcccgtg cgtcccagcg cacatgcatg 2340

ttcggcgagg cggggcctgc gagcgcggcc accgagaatc ggacgggggt agtctcaagc 2400

tggccggcct gctctggtgc ctggcctcgc gccgccgtgt atcgccccgc cctgggcggc 2460

aaggctggga gctcaaaatg gaggacgcgg cgctcgggag agcgggcggg tgagtcaccc 2520

acacaaagga aaagggcctt tccgtcctca gccgtcgctt catgtgactc cacggagtac 2580

cgggcgccgt ccaggcacct cgattagttc tcgagctttt ggagtacgtc gtctttaggt 2640

tggggggagg ggttttatgc gatggagttt ccccacactg agtgggtgga gactgaagtt 2700

aggccagctt ggcacttgat gtaattctcc ttggaatttg ccctttttga gtttggatct 2760

tggttcattc tcaagcctca gacagtggtt caaagttttt ttcttccatt tcaggtgtcg 2820

tgaaaagctt gaattcggcg cgccgcggcc gcaggtaagc cagcccaggc ctcgccctcc 2880

agctcaaggc gggacaggtg ccctagagta gcctgcatcc agggacaggc cccagccggg 2940

tgctgacacg tccacctcca tctcttcctc aggtctgccc gggtggcatc cctgtgaccc 3000

ctccccagtg cctctcctgg tcgtggaagg tgctactcca gtgcccacca gccttgtcct 3060

aataaaatta agttgcatca ttttgtttga ctaggtgtcc ttgtataata ttatggggtg 3120

gaggcgggtg gtatggagca aggggcccaa gttaacttgt ttattgcagc ttataatggt 3180

tacaaataaa gcaatagcat cacaaatttc acaaataaag catttttttc actgcattct 3240

agttgtggtt tgtccaaact catcaatgta tcttatcatg tctggatccg cttcaggcac 3300

cgggcttgcg ggtcatgcac caggtgcgcg gtccttcggg cacctcgacg tcggcggtga 3360

cggtgaagcc gagccgctcg tagaagggga ggttgcgggg cgcggaggtc tccaggaagg 3420

cgggcacccc ggcgcgctcg gccgcctcca ctccggggag cacgacggcg ctgcccagac 3480

ccttgccctg gtggtcgggc gagacgccga cggtggccag gaaccacgcg ggctccttgg 3540

gccggtgcgg cgccaggagg ccttccatct gttgctgcgc ggccagcctg gaaccgctca 3600

actcggccat gcgcgggccg atctcggcga acaccgcccc cgcttcgacg ctctccggcg 3660

tggtccagac cgccaccgcg gcgccgtcgt ccgcgaccca caccttgccg atgtcgagcc 3720

cgacgcgcgt gaggaagagt tcttgcagct cggtgacccg ctcgatgtgg cggtccgggt 3780

cgacggtgtg gcgcgtggcg gggtagtcgg cgaacgcggc ggcgagggtg cgtacggccc 3840

gggggacgtc gtcgcgggtg gcgaggcgca ccgtgggctt gtactcggtc atggtggcct 3900

gcagagtcgc tctgtgttcg aggccacacg cgtcacctta atatgcgaag tggacctggg 3960

accgcgccgc cccgactgca tctgcgtgtt ttcgccaatg acaagacgct gggcggggtt 4020

tgtgtcatca tagaactaaa gacatgcaaa tatatttctt ccggggacac cgccagcaaa 4080

cgcgagcaac gggccacggg gatgaagcag ctgcgccact ccctgaagat ccatcgtctc 4140

ctaacaagtt acatcactcc tgcccttcct caccctcatc tccatcacct ccttcatctc 4200

cgtcatctcc gtcatcaccc tccgcggcag ccccttccac cataggtgga aaccagggag 4260

gcaaatctac tccatcgtca aagctgcaca cagtcaccct gatattgcag gtaggagcgg 4320

gctttgtcat aacaaggtcc ttaatcgcat ccttcaaaac ctcagcaaat atatgagttt 4380

gtaaaaagac catgaaataa cagacaatgg actcccttag cgggccaggt tgtgggccgg 4440

gtccaggggc cattccaaag gggagacgac tcaatggtgt aagacgacat tgtggaatag 4500

caagggcagt tcctcgcctt aggttgtaaa gggaggtctt actacctcca tatacgaaca 4560

caccggcgac ccaagttcct tcgtcggtag tcctttctac gtgactccta gccaggagag 4620

ctcttaaacc ttctgcaatg ttctcaaatt tcgggttgga acctccttga ccacgatgct 4680

ttccaaacca ccctcctttt ttgcgcctgc ctccatcacc ctgacccccg ctgcgcgggg 4740

gcacgtcagg ctcaccatct gggccgcctt cttggtggta ttcaaaataa tcggcttccc 4800

ctacagggtg gaaaaatggc cttctacctg gagggggcct gcgcggtgga gacccggatg 4860

atgatgactg actactggga ctcctgggcc tcttttctcc acgtccacga cctctccccc 4920

tggctctttc acgacttccc cccctggctc tttcacgtcc tctaccccgg cggcctccac 4980

tacctcctcg accccggcct ccactacctc ctcgaccccg gcctccactg cctcctcgac 5040

cccggcctcc acctcctgct cctgcccctc ccgctcctgc tcctgctcct gttccaccgt 5100

gggtcccttt gcagccaatg caacttggac gtttttgggg tctccggaca ccatctctat 5160

gtcttggccc tgatcctgag ccgcccgggg ctcctggtct tccgcctcct cgtcctcgtc 5220

ctcttccccg tcctcgtcca tgtgccatga tggcggcctg cagctgtgtt cgaggccgcg 5280

cgtgtcacct taatatgcga agtggacctg ggaccgcgcc gccccgactg catctgcgtg 5340

ttcgagttcg ccaatgacaa gacgctgggc ggggagatcc cccttattaa ccctaaacgg 5400

gtagcatatg cttcccgggt agtagtatat actatccaga ctaaccctaa ttcaatagca 5460

tatgttaccc aacgggaagc atatgctatc gaattagggt tagtaaaagg gtcctaagga 5520

acagcgatct ggatagcata tgctatccta atctatatct gggtagcata tgctatccta 5580

atctatatct gggtagcata ggctatccta atctatatct gggtagcata tgctatccta 5640

atctatatct gggtagtata tgctatccta atttatatct gggtagcata ggctatccta 5700

atctatatct gggtagcata tgctatccta atctatatct gggtagtata tgctatccta 5760

atctgtatcc gggtagcata tgctatcctc atgcatatac agtcagcata tgatacccag 5820

tagtagagtg ggagtgctat cctttgcata tgccgccacc tcccaaggag atctgtcgac 5880

atcgatgggc gcgggtgtac actccgccca tcccgcccct aactccgccc agttccgccc 5940

attctccgcc tcatggctga ctaatttttt ttatttatgc agaggccgag gccgcctcgg 6000

cctctgagct attccagaag tagtgaggag gcttttttgg aggcctaggc ttttgcaaaa 6060

agctaattc 6069

## Description

### TECHNICAL FIELD

The present invention relates to a construct for expressing a G protein-coupled receptor or a subunit thereof, transformed cells prepared by gene transfer of the construct of the present invention, a method for measuring a physiological response to a substance using the transformed cells of the present invention, a use of the transformed cells of the present invention for measuring a physiological response to a substance, and a kit for use in a method for measuring a physiological response to a substance, which comprises the transformed cells of the present invention.

### BACKGROUND ART

### 1. G protein-coupled receptor and G protein-mediated signaling

G protein-coupled receptors are a form of receptors present on eukaryotic cytoplasmic membrane or intracellular constitutive membrane. When various signals (neurotransmitters, hormones, chemical substances, light, etc.) are received from outside the cells (outside the membrane), G protein-coupled receptors undergo a structural change to activate trimeric G proteins bound to the inner side of the membrane for signaling.

G proteins are heterotrimers bound to the inner surface of cell membranes, in which Gβγ dimers are tightly bound to Gα subunits. When activated upon binding of ligands to G protein-coupled receptors, G proteins bound to the G protein-coupled receptors dissociate their own GDP from Gα subunits and bind to new GTP molecules (Conversion from GDP type → GTP type). This conversion dissociates Gα subunits, Gβγ dimers and G protein-coupled receptors, respectively. The thus dissociated Gα subunits activate effector proteins downstream thereof.

G protein-coupled receptors and G proteins downstream thereof control many basic physiochemical reactions in intracellular signal networks such as olfactory sense, gustatory sense, vision, neurotransmission, metabolism, cell differentiation and cell proliferation, and inflammatory response and immune response.

### 2. Findings on gustatory sense and olfactory sense

Gustatory sense is sensation that especially results from binding of a substance to specific receptors present on the surface of the tongue, when the substance is in the mouth. The mammalian gustatory sense is composed of five basic tastes, that is, salty taste, sour taste, sweet taste, umami taste and bitter taste, and is considered to be formed by integration of these basic tastes. At present, salty taste and sour taste are said to be sensed via several ionotropic receptors expressed on the cell membrane on the proximal side of taste cells present in taste buds on the surface of the tongue.

Sweet taste, umami taste and bitter taste are considered to be sensed by signaling via G protein-coupled receptors present in taste cells and G proteins coupled thereto. Specifically, it has been revealed that bitter taste is accepted by molecules (bitter taste receptors) (25 types in human) designated as the T2R family, sweet taste is accepted by a T1R2 + T1R3 heterodimer (sweet taste receptor), and umami taste is accepted by a T1R1 + T1R3 heterodimer (umami taste receptor).

The signal transduction mechanism of gustatory sense information is generally understood as follows. Specifically, it is commonly considered that: first, a taste substance is bound at receptors of taste cells to activate a signal transduction process through mediation by intracellular second messengers (IP3, DAG) and the like, increasing the intracellular calcium concentration; calcium ions supplied into the cells cause synapses to release neurotransmitters, so as to cause neurons to generate an action potential; gustatory sense signals originating from the receptors are transmitted from the gustatory nerve to the brain; and thus the gustatory sense information is identified and determined.

Olfactory sense receptors are also G protein-coupled receptors, as are gustatory sense receptors such as bitter taste receptors, sweet taste receptors and umami taste receptors. Olfactory sense receptors are present on olfactory cells of the olfactory epithelium. The location of olfactory cells is the only place in the human body where neurons are in direct contact with the outside world, and the other end thereof is directly connected to a region called the olfactory bulb of the brain. "Odorant" is a low molecular weight compound having a molecular weight of about 30 to 300, and hundreds of thousands of odorants are said to exist on the earth. As many as 400 or more olfactory sense receptors have been identified in humans. When an odorant is bound at an olfactory sense receptor, in a manner similar to that of gustatory sense receptors, such as bitter taste receptors, sweet taste receptors, and umami taste receptors, intracellular calcium concentration increases through the signal transduction process mediated by intracellular second messengers, etc.

### 3. Evaluation of G protein-coupled receptor agonists

Chemical substances, neurotransmitters, hormones, etc., contained in foods and beverages, etc., can be G protein-coupled receptor agonists in a series of signaling, whereby their binding at G protein-coupled receptors causes a structural change to activate G proteins bound to the inside of the membrane. It is desirable to evaluate conveniently and efficiently the physiological responses to these candidate G protein-coupled receptor agonists. If it is possible to appropriately evaluate the physiological responses to such candidate protein-coupled receptor agonists, beneficial effects such as improvement of the taste, quality, etc., of foods and beverages, medicines, and the like, and regulation of the dosages of protein-coupled receptor agonists, can be achieved.

On the other hand, substances contained in the above foods, beverages and the like have also been evaluated by sensory tests. However, the evaluation by such sensory tests is known to be intrinsically problematic in that ensuring the objectivity of the evaluation is not easy, such evaluation is burdensome in terms of human factors, time and money, and a large amount of substances cannot be evaluated in a short time, for example.

Therefore, as an evaluation method that is an alternative to the sensory tests, a method that involves preparing a cell line expressing a G protein-coupled receptor and a Gα protein, directly adding a G protein-coupled receptor agonist (candidate compound) to such a cell line, and then evaluating the effects has been reported.

In cell lines for evaluation of G protein-coupled receptor agonists, a G protein-coupled receptor protein and a Gα protein are required to be expressed in the same cell. In order to express the two protein types, a double-gene transfer method is generally widely used in which two vector types comprising nucleic acids encoding respective proteins are introduced simultaneously or sequentially. This is an embodiment as follows, for example:
(a) cotransfection of host cells with a vector comprising a nucleic acid encoding a Gα protein and a vector comprising a nucleic acid encoding the G protein-coupled receptor protein or a subunit thereof; and
(b) transformation of a cell line stably expressing a Gα protein with a vector comprising a nucleic acid encoding the G protein-coupled receptor protein or a subunit thereof.

Examples of using such double-gene transfer method for bitter taste receptors are described in Chem. Senses 35, p.157-170, 2010; The Journal of Neuroscience, 23(19), p.7376-7380, 2003; Biochem Biophys Res Commun, 365(4): p.851-855, 2008. Further, examples of the same for sweet taste receptors are described in Japanese Unexamined Patent Publication No. 2008-13570, Japanese Unexamined Patent Publication No.2008-228690, and WO2007-121604.

However, in any of these cases, the preparation difficulty is high (the success rate is low), and stable data cannot be acquired. Therefore, these cell lines cannot be said as having sufficient quality for use in evaluation of G protein-coupled receptor agonists.

As a method for expressing two protein types, a method in which a co-expression vector prepared by insertion of nucleic acids expressing two protein types, respectively, into one vector is known in addition to the double-gene transfer method. In general, even if two nucleic acid types expressing two protein types are directly inserted in tandem into a single vector, the more distant the nucleic acid located from a promoter, the more difficult it is to be translated, resulting in the decreased protein expression level. Therefore, ingenuity is required for increasing the translational efficiency of nucleic acids located more distant from a promoter.

Japanese Patent No. 5905187 describes "a sweet taste receptor expression construct obtained by insertion of the genes encoding gustatory receptor subunits T1R2 and T1R3 and a G protein α subunit, respectively, into the same plasmid, wherein a gene encoding the G protein α subunit is linked via an IRES sequence to a site immediately following the gene encoding the sweet taste receptor subunit T1R2 located downstream of an EF-1α promoter, and, a gene encoding the G protein α subunit is linked via an IRES sequence to a site immediately following the gene encoding the sweet taste receptor subunit T1R3 located downstream of a CMV promoter located downstream of the above-mentioned G protein α subunit-encoding gene". The construct of Japanese Patent No. 5905187 has a specific structure comprising, from 5' in this order, EF-1α promoter-T1R2-IRES-Gα-CMV promoter-TIR3-IRES-Gα. In this construct, T1R2 and T1R3 are located downstream of particular promoters, respectively, and located upstream of the IRESs, respectively.

IRES is an RNA factor that enables cap-independent translational initiation to further increase the efficiency of protein synthesis. It is known that IRES-mediated translation tends to be less efficient compared to that of upstream proteins that generally undergo cap-dependent translation.

Prior to the present invention, cell lines having sufficient quality for use in evaluation of G protein-coupled receptor agonists have not been obtained.

### CITATION LIST

### PATENT LITERATURE

PTL 1: Japanese Unexamined Patent Publication No. 2008-13570
PTL 2: Japanese Unexamined Patent Publication No. 2008-228690
PTL 3: WO2007-121604

### NON PATENT LITERATURE

NPL1: Chem. Senses 35, p.157-170, 2010;
NPL2: The Journal of Neuroscience, 23(19), p.7376-7380, 2003;
NPL3: Biochem Biophys Res Commun, 365(4): p.851-855,2008
NPL4: Molecular BioSystems, 7, p.911-919, 2011
NPL5: Proc.Natl.Acad.Sci.USA, Vol.88, p.5587-5591, 1991
NPL6: Journal of Virology, Vol.86, No.3, p.1468-1486, 2012
NPL7: GENES & DEVELOPMENT, Vol, 15, p.1593-1612, 20
NPL8: Archives of Physiology and Biochemistry Vol.110 p.137-45,2002
NPL9: Nature Genetics, Vol.32, p.397-401, 2002; Cell, Vol.100, p.703-711, 2000

### SUMMARY OF INVENTION

### TECHNICAL PROBLEM

As a result of intensive efforts to solve the above problems, the present inventors have discovered that a construct for expressing a G protein-coupled receptor protein or a subunit thereof comprising: a nucleic acid encoding a G protein α subunit protein; a nucleic acid comprising an IRES sequence; and a nucleic acid encoding the G protein-coupled receptor protein or a subunit thereof in the stated order from the 5' side in a single vector, provides high efficiency expression of the G protein-coupled receptor protein or a subunit thereof and high efficiency response to a G protein-coupled receptor agonist. Hence, the present inventors have conceived of the present invention.

The expression level of a protein encoded by a gene placed on the 3' side of IRES is thought to be low. Accordingly, in the present invention, the fact that a G protein-coupled receptor protein or a subunit thereof is expressed with high efficiency is contrary to conventional knowledges and is characteristic.

### SOLUTION TO PROBLEM

The present invention encompasses, but is not limited to, the following embodiments.

### [Embodiment 1]

A construct for expressing a G protein-coupled receptor protein or a subunit thereof, comprising: a nucleic acid encoding a G protein α subunit protein; a nucleic acid comprising an IRES sequence; and a nucleic acid encoding the G protein-coupled receptor protein or a subunit thereof in the stated order from the 5' side in a single vector.

### [Embodiment 2]

The construct according to embodiment 1, wherein the G protein-coupled receptor or a subunit thereof is a G protein-coupled receptor of a rhodopsin-like receptor class or a metabotropic glutamate receptor/metabotropic pheromone receptor class.

### [Embodiment 3]

The construct according to embodiment 1, wherein the G protein-coupled receptor is a G protein-coupled receptor of the rhodopsin-like receptor class.

### [Embodiment 4]

The construct according to embodiment 1, wherein the G protein-coupled receptor is a bitter taste receptor.

### [Embodiment 5]

The construct according to any one of embodiments 1 to 4, wherein the vector is a plasmid vector.

### [Embodiment 6]

The construct according to any one of embodiments 1 to 4, wherein the vector is a plasmid vector comprising a CMV promoter.

### [Embodiment 7]

The construct according to any one of embodiments 1 to 4, wherein the vector is a plasmid vector comprising a CMV promoter and a hygromycin resistance gene.

### [Embodiment 8]

The construct according to any one of embodiments 1 to 4, wherein the vector is a plasmid vector comprising an EF1α promoter.

### [Embodiment 9]

The construct according to any one of embodiments 1 to 4, wherein the vector is a plasmid vector comprising an EF1α promoter and a puromycin resistance gene.

### [Embodiment 10]

The construct according to any one of embodiments 1 to 9, wherein the IRES sequence is a type 2 IRES sequence.

### [Embodiment 11]

The construct according to any one of embodiments 1 to 10, wherein the IRES sequence is an IRES sequence derived from the encephalomyocarditis virus.

### [Embodiment 12]

The construct according to any one of embodiments 1 to 7, comprising a nucleic acid encoding a polypeptide having a function of enhancing the translocation of the G protein-coupled receptor protein or a subunit thereof to a cell membrane, 5' to the nucleic acid encoding the G protein-coupled receptor protein or a subunit thereof.

### [Embodiment 13]

The construct according to embodiment 12, wherein the polypeptide having a function of enhancing the translocation of the G protein-coupled receptor protein or a subunit thereof to a cell membrane is a polypeptide comprising N-terminal 45 amino acids of a rat somatostatin receptor or a polypeptide comprising N-terminal 39 amino acids of bovine rhodopsin.

### [Embodiment 14]

The construct according to any one of embodiments 1 to 13, wherein the G protein α subunit protein is a G protein α subunit protein of the Gq family, a G protein α subunit protein of the Gi family, a G protein α subunit protein of the Gs family, or a chimeric protein thereof.

### [Embodiment 15]

The construct according to any one of embodiments 1 to 14, wherein the G protein α subunit protein is Gα16, Gαgust or Gαolf, or a chimeric protein thereof.

### [Embodiment 16]

The construct according to any one of embodiments 1 to 15, wherein the G protein α subunit protein is human Gα16/rat Gαgust.

### [Embodiment 17]

The construct according to embodiment 16, wherein the G protein α subunit protein is human Gα16/rat Gαgust43.

### [Embodiment 18]

The construct according to any one of embodiments 1 to 17, wherein the G protein-coupled receptor protein is a human G protein-coupled receptor protein.

### [Embodiment 19]

Transformed cells, into which the construct according to any one of embodiments 1 to 18 is introduced.

### [Embodiment 20]

A method for measuring a physiological response to a substance, comprising adding a substance to the transformed cells according to embodiment 19, and then measuring a physiological response induced by the substance.

### [Embodiment 21]

The measurement method according to embodiment 20, wherein the substance is a bitter substance, an odorant, an umami substance or a sweet substance, or a modulator for these substances.

### [Embodiment 22]

Use of the transformed cells according to embodiment 19, for measurement of a physiological response to a substance.

### [Embodiment 23]

A kit comprising the transformed cells according to embodiment 19, which is used for a method for measuring a physiological response to a substance by adding a substance to the transformed cells, and then measuring a physiological response induced by the substance.

### ADVANTAGEOUS EFFECTS OF INVENTION

Cells transformed with the construct of the present invention express a G protein-coupled receptor protein or a subunit thereof with high efficiency and respond with high efficiency to a G protein-coupled receptor agonist. The transformed cells of the present invention provide cell lines having sufficient quality to be used for evaluation of G protein-coupled receptor agonists.

### BRIEF DESCRIPTION OF DRAWINGS

Fig. 1 illustrates the construction of a construct prepared in Example 1. In Fig. 1, the term "CMV" refers to a CMV promoter.
Fig. 2 depicts the results of measuring the response of a T2R16 stable expression cell line to a bitter substance, salicin.
Fig. 3 depicts the results of measuring the response of a T2R40 stable expression cell line to a bitter substance, diphenydol, and the response of a T2R43 stable expression cell line to a bitter substance, quinine.
Fig. 4 depicts the results of measuring the response of T2R16 transiently expressing cells to the bitter substance, salicin.
Fig. 5 depicts the results of measuring the response to a T2R-independent Gα16 agonist (isoproterenol) in T2R16 transiently expressing cells.
Fig. 6 depicts the results of measuring the expression level of T2R16 in T2R16 transiently expressing cells.
Fig. 7 depicts the results of comparing response intensity before and the same after cryopreservation of the T2R16 stable expression cell line.
Fig. 8 depicts the results of examining the correlation between the response intensity of a Gα-T2R40 stable expression cell line to a T2R40 agonist and the response intensity of the same to a Gα agonist.
Fig. 9 depicts the results of measuring the expression level of T1R2 in a cell line (Gα-T1R2 + T1R3) transiently expressing Gα-T1R2 and T1R3 after double-gene transfer thereof.
Fig. 10 depicts the results of measuring the response to a T2R-independent Gα16 agonist (isoproterenol) in a cell line (Gα-T1R2 + T1R3) transiently expressing Gα-T1R2 and T1R3 after double-gene transfer thereof.
Fig. 11 depicts the results of measuring the response to a sweetener, aspartame, in a cell line (Gα-T1R2 + T1R3) transiently expressing Gα-T1R2 and T1R3 after double-gene transfer thereof.
Fig. 12 depicts the results of measuring the expression level of T1R3 in a cell line (Gα-T1R3 + T1R2) transiently expressing Gα-T1R3 and T1R2 after double-gene transfer thereof.
Fig. 13 depicts the results of measuring the response to a T2R-independent Gα16 agonist (isoproterenol) in a cell line (Gα-T1R3 + T1R2) transiently expressing Gα-T1R3 and T1R2 after double-gene transfer thereof.
Fig. 14 depicts the results of measuring the response to a sweetener, cyclamate, in a cell line (Gα-T1R3 + T1R2) transiently expressing Gα-T1R3 and T1R2 after double-gene transfer thereof.
Fig. 15 depicts the results of measuring the expression level of EmGFP when a green fluorescent protein (EmGFP) was expressed upstream and downstream of IRES.
Fig. 16 depicts the results of examining the expression level of T2R16 in HEK293T cells transiently expressing pEAK10-T2R16-Gα and pEAK10-Gα-T2R16.
Fig. 17 depicts the results of examining seven T2R subtypes (bitter taste receptors) for the response of transiently expressing cells to each agonist (bitter substance).
Fig. 18 depicts the results of examining a T2R38 subtype (bitter taste receptor) for the response of stably expressing cells to an agonist (6-n-propylthiouracil). Black bars indicate the result of T2R38, and diagonally shaded bars indicate the results of a control (a cell line (HEK293T) not incorporating the expression vector Gα-T2R38).
Fig. 19 depicts the results of examining the expression levels of fourteen T2R subtypes (bitter taste receptors) in transiently expressing cells (compared to T2R4).
Fig. 20 depicts the results of examining the response in cells transiently expressing an olfactory receptor (OR3A1). The diagonally shaded bars indicate the results of introducing OR3A1-Gαolf and black bars indicate the results of introducing Gαolf-OR3A1. The dotted bars (OR3A1 + Gαolf) indicate the results of introducing OR3A1 and Gαolf genes separately into pcDNA for double-gene transfer thereof into HEK293T.
Fig. 21 depicts the expression levels of OR3A1 and Gαolf in cells transiently expressing the olfactory receptor (OR3A1). The diagonally shaded bars indicate the results of OR3A1, and the black bars indicate the results of Gαolf. "OR3A1 + Gαolf" indicates the result of introducing the genes of OR3A1 and Gαolf separately into pcDNA for double-gene transfer thereof into HEK293T.

### DESCRIPTION OF EMBODIMENTS

The present invention relates to a construct for expressing a G protein-coupled receptor or a subunit thereof, transformed cells containing the construct of the present invention through gene transfer, a method for measuring a physiological response to a substance using the transformed cells of the present invention, a use of the transformed cells of the present invention for measuring a physiological response to a substance, and a kit comprising the transformed cells of the present invention, which is used for a method for measuring a physiological response to a substance. Hereinafter, embodiments of the present invention will be described without limitation.

### I. Construct for expressing G protein-coupled receptor or subunit thereof

The present invention relates to, in one embodiment, a construct for expressing a G protein-coupled receptor or a subunit thereof. The construct of the present invention comprises: a nucleic acid encoding a G protein α subunit protein; a nucleic acid comprising an IRES sequence; and a nucleic acid encoding the G protein-coupled receptor protein or a subunit thereof in the stated order from the 5' side in a single vector.

### (1) G protein-coupled receptor or subunit thereof

"G protein-coupled receptors (GPCR)" are a form of receptors present on eukaryotic cytoplasmic membrane or intracellular constitutive membrane. Seven helical structures penetrate the cell membrane (or intracellular constituent membrane), and the N-terminus is located outside the membrane, and the C-terminus is located inside the membrane. Because of such a characteristic structure, these receptors are also referred to as another name "7-transmembrane receptors". The extramembrane loop contains two well-conserved cysteine residues, and the receptor structure is stabilized by disulfide bonds. When G protein-coupled receptors accept various signals (neurotransmitters, hormones, chemical substances, light, etc.) from outside the cells (outside the membrane), the receptors undergo a structural change to activate trimeric G proteins bound to the inner side of the membrane, so that signaling is performed. G protein-coupled receptors control many basic physiochemical reactions in intracellular signal networks such as olfactory sense, gustatory sense, vision, neurotransmission, metabolism, cell differentiation and cell proliferation, and inflammatory responses and immune responses.

G protein-coupled receptors are classified into six classes based on similarities in amino acid sequences, pharmacological dynamics, functions, etc. (Molecular BioSystems, 7, p. 911-919, 2011).
Class A: rhodopsin-like receptor
Class B: secretin receptor family
Class C: metabotropic glutamate receptor/metabotropic pheromone receptor
Class D: fungal mating factor receptor
Class E: cyclic AMP (cAMP) receptor
Class F: frizzled, smoothened

### (i) Class A: rhodopsin-like receptor

The rhodopsin-like receptors of class A have a relatively short N-terminal extracellular domain, wherein a plurality of transmembrane domains form a ligand binding site. Class A rhodopsin-like receptors account for 80% or more of G protein-coupled receptors, and include bitter taste receptors, olfactory sense receptors, rhodopsin, adrenoceptors, muscarinic acetylcholine receptors and the like. An amino acid sequence conserved in the rhodopsin-like receptor is the E/DRY (Asp/Glu, Arg, Tyr) motif located inside the membrane of the third transmembrane domain.

Bitter taste receptors are G protein-coupled receptors belonging to rhodopsin-like receptors, and have been identified as members of the T2R family on human and mouse chromosome 6. At least 35 types of T2R in mice and 25 types of T2R in humans function as bitter taste receptors. Examples of the relationship between human T2R and ligands are as follows.
T2R4: denatonium benzoate;
T2R10: strychnine;
T2R14: nonspecific binding of bitter substances with many different structural features
T2R16: β-glucopyranoside (salicin)
T2R31: aristolochic acid
T2R38: bitter substances having isothiocyanate groups, for example, phenylthiocarbamide (PTC) and propylthiouracil (PROP)
T2R40: diphenydol
T2R43: quinine
T2R46: absinthin, strychnine, denatonium

The amino acid sequences of the proteins of and the nucleic acid sequences of the genes of mouse T2R and human T2R are known. For example, the amino acid sequences and the gene sequences of 25 types of human T2R bitter taste receptors are registered in the National Center for Biotechnology Information (NCBI) or GenBank under the accession numbers described in Table 1 below.

**[Table 1-1]**

| Nucleotide sequence | Amino acid sequence | Gene name | Another name (former name) | Data source | ACCESSION No. | Title | Difference between a sequence used in Example and a sequence in DB |
|---|---|---|---|---|---|---|---|
| 1 | 2 | hTAS2R1 | | NCBI Reference Sequence | NM_019599.2 | Homo sapiens taste 2 receptor member 1 (TAS2R1), mRNA | |
| 3 | 4 | hTAS2R3 | | GenBank | AF227130.1 | Homo sapiens candidate taste receptor T2R3 gene, complete cds | |
| 5 | 6 | hTAS2R4 | | NCBI Reference Sequence | NM016944 | Homo sapiens taste 2 receptor member 4 (TAS2R4), mRNA | |
| 7 | 8 | hTAS2R5 | | GenBank | BC101588.1 | Homo sapiens taste receptor, type 2, member 5, mRNA (cDNA clone MGC:126637 IMAGE:8069094), complete cds | |
| 9 | 10 | hTAS2R7 | | NCBI Reference Sequence | NM_023919.2 | Homo sapiens taste 2 receptor member 7 (TAS2R7), mRNA | |
| 11 | 12 | hTAS2R8 | | NCBI Reference Sequence | NM_023918.1 | Homo sapiens taste 2 receptor member 8 (TAS2R8), mRNA | |
| 13 | 14 | hTAS2R9 | | NCBI Reference Sequence | NM_023917.2 | Homo sapiens taste 2 receptor member 9 (TAS2R9), mRNA | |
| 15 | 16 | hTAS2R10 | | NCBI Reference Sequence | NM_023921.1 | Homo sapiens taste 2 receptor member 10 (TAS2R10), mRNA | Regarding those subjected to insertion of V5, the 3' terminal stop codon, a nucleotide triplet, was deleted and V5 (including a stop codon) was inserted. |
| 17 | 18 | hTAS2R13 | | NCBI Reference Sequence | NM_023920.2 | Homo sapiens taste 2 receptor member 13 (TAS2R13), mRNA | |
| 19 | 20 | hTAS2R14 | | NCBI Reference Sequence | NM_023922.1 | Homo sapiens taste 2 receptor member 14 (TAS2R14), mRNA | |
| 21 | 22 | hTAS2R16 | | GenBank | AF227139.1 | Homo sapiens candidate taste receptor T2R16 gene, complete cds | |
| 23 | 24 | hTAS2R38 | | GenBank | AY258597.1 | Homo sapiens PTC bitter taste receptor (PTC) gene, PTC-taster allele, complete cds | |
| 25 | 26 | hTAS2R39 | | NCBI Reference Sequence | NM_176881.2 | Homo sapiens taste 2 receptor member 39 (TAS2R39), mRNA | |
| 27 | 28 | hTAS2R40 | | NCBI Reference Sequence | NM_176882.1 | Homo sapiens taste 2 receptor member 40 (TAS2R40), mRNA | |
| 29 | 30 | hTAS2R41 | | GenBank | AF494232.1 | Homo sapiens candidate taste receptor TAS2R41 gene, complete cds | |
| 31 | 32 | hTAS2R42 | | NCBI Reference Sequence | NM_181429.2 | Homo sapiens taste 2 receptor member 42 (TAS2R42), mRNA | |
| 33 | 34 | hTAS2R43 | | GenBank | BC117423.1 | Homo sapiens taste receptor, type 2, member 43, mRNA (cDNA clone MGC:151032 IMAGE:40125974), complete cds | |

**[Table 1-2]**

| Nucleotide sequence | Amino acid sequence | Gene name | Another name (former name) | Data source | ACCESSION No. | Title | Difference between a sequence used in Example and a sequence in DB |
|---|---|---|---|---|---|---|---|
| 35 | 36 | hTAS2R44 | hTAS2R31 | GenBank | AF494228 | Homo sapiens candidate taste receptor TAS2R44 gene, complete cds | Regarding those subjected to insertion of V5, the 3' terminal stop codon, a nucleotide triplet, was deleted and V5 (including a stop codon) was inserted. |
| 37 | 38 | hTAS2R45 | | GenBank | AF494226.1 | Homo sapiens candidate taste receptor TAS2R45 gene, complete cds | |
| 39 | 40 | hTAS2R46 | | NCBI Reference Sequence | NM_176887.2 | Homo sapiens taste 2 receptor member 46 (TAS2R46), mRNA | |
| 41 | 42 | hTAS2R47 | hTAS2R30 | NCBI Reference Sequence | NM_001097643.1 | Homo sapiens taste 2 receptor member 30 (TAS2R30), mRNA | |
| 43 | 44 | hTAS2R48 | hTAS2R19 | NCBI Reference Sequence | NM_176888.2 | Homo sapiens taste 2 receptor member 19 (TAS2R19), mRNA | |
| 45 | 46 | hTAS2R49 | hTAS2R20 | NCBI Reference Sequence | NM_176889.3 | Homo sapiens taste 2 receptor member 20 (TAS2R20), mRNA | |
| 47 | 48 | hTAS2R50 | | NCBI Reference Sequence | NM_176890.2 | Homo sapiens taste 2 receptor member 50 (TAS2R50), mRNA | |
| 49 | 50 | hTAS2R60 | | NCBI Reference Sequence | NM_177437.1 | Homo sapiens taste 2 receptor member 60 (TAS2R60), mRNA | |

In the description, the term "gene" is used synonymously with "nucleic acid" unless otherwise specified, and the present invention includes an embodiment in which a control sequence such as a promoter is not contained. Nucleic acids also include DNA (including both genomic DNA and cDNA), RNA (mRNA), and chimeras of DNA and RNA. The term "gene sequence" of a particular protein refers to a DNA sequence (containing no intron) encoding the amino acid sequence of the protein.

Olfactory sense receptors are also G protein-coupled receptors belonging to rhodopsin-like receptors as in the case of bitter taste receptors. Olfactory sense receptors have an average length of about 310 amino acid residues and share several motif sequences. A particularly characteristic motif sequence is MAYDRYVAIC which is present from the third transmembrane site to the fourth intramembrane loop. Among these motif sequences, three amino acid residues of DRY (Asp/Glu, Arg, Tyr) are common with respect to other rhodopsin-like receptors and are considered to be important for G protein activation. The amino acid of the proteins and the nucleic acid sequences of the genes of many olfactory sense receptors are known.

For example, the amino acid sequences and the gene sequences of human olfactory sense receptors are registered in the NCBI or the GenBank under the accession numbers described in Table 2 below.

**[Table 2]**

| Nucleotide sequence | Amino acid sequence | Gene name | Another name (former name) | Data source | ACCESSION No. | Title | Difference between a sequence used in Example and a sequence in DB |
|---|---|---|---|---|---|---|---|
| 51 | 52 | hOR3A1 | | NCBI Reference Sequence | NM_002550.2 | Homo sapiens olfactory receptor family 3 subfamily A member 1 (OR3A1), mRNA | A start codon, a nucleotide triplet, was deleted and the 5' terminal 63 nucleotides of Rho (including a start codon) were inserted. |
| 53 | 54 | hOR1D2 | | NCBI Reference Sequence | NM_002548.2 | Homo sapiens olfactory receptor family 1 subfamily D member 2 (OR1D2), mRNA | |
| 55 | 56 | hOR2J2 | | NCBI Reference Sequence | NM_030905.2 | Homo sapiens olfactory receptor family 2 subfamily J member 2 (OR2J2), mRNA | |
| 57 | 58 | hOR2W1 | | NCBI Reference Sequence | NM_030903.3 | Homo sapiens olfactory receptor family 2 subfamily W member 1 (OR2W1), mRNA | |

### (ii) Class C: Metabotropic glutamate receptor/metabotropic pheromone receptor

Class C includes, in addition to a metabotropic glutamate receptor and a metabotropic pheromone receptor, a sweet taste receptor, an umami taste receptor, a GABA (type B) receptor, and a calcium sensing receptor etc. When referred to as "metabotropic glutamate receptor/metabotropic pheromone receptor class" in the description and claims, the term refers to the whole members of Class C, unless otherwise specified. Class C receptors function as homo- or hetero-dimers and have the largest extracellular domain of about 500 to 600 residues among G protein-coupled receptors. The ligand binding domain of the extracellular domain has a structure called the Venus Flytrap module (VFTM) structure.

The sweet taste receptors are receptors belonging to class C and function as T1R2/T1R3 heterodimers. It has been reported that the N-terminal domain of human T1R2 is important for acceptance of artificial sweeteners, aspartame and neotame, the transmembrane domain of human T1R3 is important for acceptance of an artificial sweetener, cyclamate, and a sweetness suppressant, lactisole, and the N-terminal domain of human T1R3 is important for acceptance of a sweetness modifier, neoculin. It has been reported that the human T1R3 C-rich domain is important for sensitivity to a sweet taste protein, brazzein.

Umami taste receptors are receptors belonging to class C and function as T1R1/T1R3 heterodimers. It has been revealed that umami substances, glutamic acid and inosinic acid (IMP), bind to the N-terminal domain of T1R1.

The amino acid sequences and the gene sequences of human T1R1, T1R2 and T1R3 constituting the sweet taste receptors and the umami taste receptors are registered in the NCBI under the accession numbers described in Table 3 below.

**[Table 3]**

| Nucleotide sequence | Amino acid sequence | Gene name | Another name (former name) | Data source | ACCESSION No. | Title | Difference between a sequence used in Example and a sequence in DB |
|---|---|---|---|---|---|---|---|
| 59 | 60 | hT1R1 | | GenBank | BK000153.1 | TPA_exp: Homo sapiens taste receptor (TAS1R1) mRNA, complete cds | Regarding those subjected to insertion of V5, the 3' terminal stop codon, a nucleotide triplet, was deleted and V5 (including a stop codon) was inserted. |
| 61 | 62 | hT1R2 | | NCBI Reference Sequence | NM_152232.2 | Homo sapiens taste 1 receptor member 2 (TAS1R2), mRNA | |
| 63 | 64 | hT1R3 | | NCBI Reference Sequence | NM_152228.2 | Homo sapiens taste 1 receptor member 3 (TAS1R3), mRNA | |

The entire G protein-coupled receptor protein or a subunit thereof may be expressed using the construct of the present invention. For example, a construct that expresses only one subunit type, T1R2, T1R3 or T1R1, which constitutes a dimeric sweet taste receptor or umami taste receptor, is included. In that case, the other subunit constituting the dimer may be co-transfected with the use of another construct. Alternatively, a construct may be constructed such that nucleic acids encoding multiple subunits constituting a G protein-coupled receptor protein are contained in a single vector. In the present invention, a construct is constructed such that a nucleic acid encoding at least one subunit is located on the 3' side of IRES.

### (iii) Class B: Secretin receptor family

The class B secretin receptor family has a long N-terminal extracellular domain to form a ligand binding site. The family is classified into two subgroups: secretin-like receptors (in a narrow sense) and adhesion-type G protein-coupled receptors. The secretin-like receptors (in a narrow sense) include receptors that bind to peptide hormones such as secretin, glucagon, glucagon-like peptide (GLP), calcitonin, and parathyroid hormone. The adhesion-type G protein-coupled receptors have various domain structures at the large N-terminal site, suggesting an interaction with extracellular matrix etc.

### (iv) Class F: Frizzled, smoothened

Class F G-protein coupled receptors include 10 types of frizzled proteins that function in the Wnt signaling pathway and Smoothened proteins that function in the hedgehog signaling pathway. The class F receptors have a domain which is referred to as a cysteine-rich domain (CRD) outside the cell, comprises about 120 amino acid residues, and serves as a binding domain of a ligand such as Wnt.

In the present invention, the type of a G protein-coupled receptor or a subunit thereof is not particularly limited. The advantageous effects of the present invention have been confirmed for a plurality of types of bitter taste receptor T2R belonging to at least class A, and subunits T1R2 (constituting sweet taste receptors) and T1R3 (constituting sweet taste receptors and umami taste receptors) belonging to class C different therefrom.

In one embodiment of the present invention, preferably, the G protein-coupled receptor or a subunit thereof is, without limitation, a G protein-coupled receptor of class A rhodopsin-like receptors or a G protein-coupled receptor of class C metabotropic glutamate receptors/metabotropic pheromone receptors. In one embodiment of the present invention, the G protein-coupled receptor is a G protein-coupled receptor of the class of rhodopsin-like receptors. More preferably, the G protein-coupled receptor is a bitter taste receptor.

The biological species from which the G protein-coupled receptor protein is derived is not particularly limited. Preferably it is derived from an animal, more preferably a mammalian species. Examples of mammals include humans, rats, monkeys, dogs, pigs, sheep, horses, and mice. In one embodiment of the present invention, the G protein-coupled receptor protein is a human G protein-coupled receptor protein.

### (2) G protein α subunit

The term "G protein (guanidine nucleotide binding protein)" is a protein belonging to a group of GTPases, and refers to a membrane receptor-related heterotrimer that is generally composed of three subunits, α, β, and γ.

G proteins bind to the inner surface of the cell membrane, wherein Gβγ dimers are tightly bound to Gα subunits. When activated upon binding of ligands to G protein-coupled receptors, G proteins bound to the G protein-coupled receptors dissociate their own GDP from the Gα subunits and bind to new GTP molecules (conversion from GDP type -> GTP type). This conversion dissociates the Gα subunits, the Gβγ dimers and the G protein-coupled receptors, respectively. The thus dissociated Gα subunits activate effector proteins downstream thereof.

The G protein α subunit consists of two domains, a GTPase domain and an α helical domain. At present, at least 20 Gα subunit types are known and mainly classified into four families.

**[Table 4]**

| G protein family | α subunit | Gene | Signaling | Examples of effects |
|---|---|---|---|---|
| Gi Family | | | | |
| Gi/o | αi,αo | GNAO1, GNAI1, GNAI2, GNAI3 | Suppression of adenylate cyclase, (β/γ subunit-mediated) K⁺ channel opening, Ca²⁺ channel closing | Smooth muscle contraction, decreased neuronal activity |
| Gt | αt (transducin) | IGNAT1, GNAT2 | Activation of phosphodiesterase 6 | Vision |
| Ggust | agust (gustducin) | GNAT3 | Activation of phosphodiesterase 6 | Gustatory sense |
| Gz | αz | GNAZ | Suppression of adenylate cyclase | Ion balance maintenance in cochlear duct fluid of external lymph and internal lymph |

| Gs Family | | | | |
|---|---|---|---|---|
| Gs | αs | GNAS | Activation of adenylate cyclase | Increased heart rate, relaxed smooth muscle, stimulation of neuronal activity |
| Golf | αolf | GNAL | Activation of adenylate cyclase | Sense of smell |

| Gq Family | | | | |
|---|---|---|---|---|
| Gq | αq,α11, α14,α15, α16 | GNAQ, GNA11, GNA14, GNA15 | Activation of phospholipase C | Smooth muscle contraction, Ca²⁺ flow |

| G12/13 Family | | | | |
|---|---|---|---|---|
| G12/13 | α12,α13 | GNA12, GNA13 | Activation of GTPase Rho family | Cytoskeletal function, smooth muscle contraction |

In the present invention, the type of G protein α subunit is not particularly limited. In one embodiment of the present invention, the G protein α subunit protein is, without limitation, a G protein α subunit protein of the Gq family, a G protein α subunit protein of the Gi family, a G protein α subunit protein of the Gs family, or a chimeric protein thereof. In one embodiment of the present invention, the G protein α subunit protein is Gα16, Gαgust or Gαolf, or a chimeric protein thereof.

The biological species from which the G protein subunit is derived is not particularly limited. Preferably the G protein subunit is derived from an animal, more preferably a mammalian species. Examples of mammals include humans, rats, monkeys, dogs, pigs, sheep, horses, and mice. In one embodiment of the present invention, the G protein is a human G protein-coupled receptor protein.

Human Gα16 is described, for example, in Proc. Natl. Acad. Sci. USA, Vol. 88, p. 5587-5591, 1991, and the mRNA sequence is registered in the GenBank under the accession number of M63904.1. The nucleotide sequence and the amino acid sequence of human Gα16 are also represented by SEQ ID NOs: 65 and 66 in the sequence listing of the present application. The gene encoding human Gα16 can also be obtained, for example, from cultured cells such as HL60 cells.

The mRNA sequence of rat Gastducin is registered in the NCBI under the accession number of NM_173139.1. The nucleotide sequence and the amino acid sequence of rat Gastducin are also represented by SEQ ID NOs: 67 and 68 in the sequence listing of the present application.

The mRNA sequence of human Gαolf is registered in the GenBank under the accession number of AF493893.1. The nucleotide sequence and the amino acid sequence of human Gαolf are also represented by SEQ ID NOS: 69 and 70 in the sequence listing of the present application.

The G protein α subunit protein may be a chimeric protein. The chimeric protein may be a fusion protein of different types of G protein α subunits derived from the same biological species, or a fusion protein of G protein α subunits of the same type derived from different biological species, or a fusion protein of G protein α subunits of different types derived from different biological species. The chimeric protein of G protein α subunit protein n may differ from the original G protein α subunit protein in characteristics such as binding specificity to a G protein-coupled receptor, binding strength or stability. With the use of a construct comprising a nucleic acid encoding such a chimeric protein, transformed cells having desired characteristics can be obtained. Further, the transformed cells having desired characteristics can be appropriately used in a method for measuring a physiological response to a substance.

In one embodiment of the present invention, the G protein α subunit protein is human Gα16/rat Gαgust. Human Gα16/rat Gαgust is a chimeric protein in which the C-terminal sequence of human Gα16 is substituted with the C-terminal sequence of rat Gαgust. It has been reported that a chimeric protein in which the C-terminal sequence of Gα16 is substituted with the C-terminal sequence of Gαgust having 37 or more amino acid residues responds to bitter taste receptor T2R (The Journal of Neuroscience, 23(19), p. 7376-7380, 2003).

The C-terminal sequence of rat Gαgust that is substituted with human Gα16 is composed of preferably at least 25 or more amino acid groups, 30 or more amino acid groups, 35 or more amino acid residues, 37 or more amino acid residues, 40 or more amino acid groups, or 43 or more amino acid residues. The C-terminal sequence of rat Gαgust that is substituted with human Gα16 is composed of preferably 60 or less amino acid residues, 50 or less amino acid groups, 45 or less amino acid groups, or 43 or less amino acid groups. In one embodiment of the present invention, the G protein α subunit protein is human Gα16/rat Gαgust43.

In the examples herein, the 3' terminal 132 nucleotides (corresponding to a sequence encoding C terminal 43 amino acids including stop and start codons) of SEQ ID NO: 65 of human Gα16 were substituted with the corresponding sequence of rat Gastducin; that is, the 3' terminal 132 nucleotides (corresponding to a sequence encoding C terminal 43 amino acids including stop and start codons) of SEQ ID NO: 67 (Gα16/Gαgust43 (Gα)).

### (3) IRES

The construct of the present invention comprises a nucleic acid comprising an IRES sequence downstream (3' side) of the nucleic acid encoding a G protein α subunit protein and an IRES sequence upstream of (5' to) the nucleic acid encoding a G protein-coupled receptor protein or a subunit thereof in a single vector. IRES is an abbreviation of Internal Ribosome Entry Site (site for the entry of an internal ribosome), and is an RNA factor that enables cap-independent translation initiation in order to further increase the efficiency of protein synthesis. Although many IRESs are present in the 5'UTR (5 'untranslated region) of RNA viruses, it has been suggested that IRESs are also present in mammalian cells cellular mRNAs.

Viral IRESs discovered to date are roughly divided into five classes (Journal of Virology, Vol. 86, No. 3, p. 1468-1486, 2012).

The first class includes the dicistrovirus IRES group. The second class includes the IRES of the hepatitis C virus (HCV) and viruses of the genus *Pestivirus,* as well as the IRES of a number of structurally similar viruses.

Furthermore, three classes of IRES, referred to as type 1 to type 3, are known. These are all identified at the 5' UTR of the family Picornaviridae.

Among these, members of type 2 are IRESs mainly found in the genera *Aphthovirus, Cardiovirus,* and *Parechovirus.* Furthermore, divergent members also exist in the genera *Erbovirus* and *Cosavirus.* Viruses of the genus *Cardiovirus* include Encephalomyocarditis virus (EMCV) and Theilovirus. Type 2 IRESs have a length of about 450 nucleotides, and have 3'-terminal Yn-Xm-AUG (Yn is a pyrimidine tract, n is an integer of 8-10, and m is 18-20) motif, wherein the AUG triplet may be a start codon. Type 2 IRESs contain five major domains: H, I, J, K and L. Type 2 IRESs function without the involvement of eukaryotic translation initiation factor IF4E and factors involved in ribosomal scanning, such as eIF1 and eIF1A. However, some of the type 2 IRESs require one or more IRES trans-activating factors (ITAFs). ITAF is a cellular RNA binding protein such as a pyrimidine tract binding protein (PTB).

Type 1 IRESs are those found only in the genus *Enterovirus.* Type 1 IRESs contain five major domains of II-VI. Type 2 IRESs have a length of about 450 nucleotides and have a 3'-terminal Yn-Xm-AUG motif like type 2. However, this motif of the type 1 IRES is separated from the start codon by a non-conserved spacer. Type 3 IRESs are those found only in hepatitis A virus.

Cellular IRES is described, for example, in GENES & DEVELOPMENT, Vol, 15, p. 1593-1612, 2001.

In the present invention, the type of IRES is not particularly limited. In one embodiment of the present invention, IRES is, but is not limited to, a viral IRES. In one embodiment of the present invention, the IRES sequence is a type 2 IRES sequence, such as an IRES sequence derived from encephalomyocarditis virus. The nucleic acid sequence of the IRES derived from encephalomyocarditis virus is registered in the GenBank under the accession number of M81861.1. The IRES sequence used in the examples herein is represented by SEQ ID NO: 71, which is partially modified based on the sequence registered in the GenBank under the accession number of M81861.1, simulating the IRES site of the pIRES2-EGFP vector (Clontech). Specifically, in SEQ ID NO: 71, G is added to nucleotide number 1 and A is added to nucleotide number 512.

### (4) Polypeptide having a function of enhancing translocation of G protein-coupled receptor protein or subunit thereof to cell membrane

In one embodiment, the construct of the present invention comprises a nucleic acid encoding a polypeptide having a function of enhancing the translocation of the G protein-coupled receptor protein or a subunit thereof to the cell membrane, 5' to the nucleic acid encoding the G protein-coupled receptor protein or a subunit thereof.

When such a construct is used, a fusion protein, in which such a polypeptide is bound to the N'-terminal side of a G protein-coupled receptor protein or a subunit thereof, is expressed. Therefore, in cells transformed with the construct, the translocation of the G protein-coupled receptor protein or a subunit thereof to the cell membrane is improved. Transformed cells, in which more G protein-coupled receptors are translocated to the cell membrane, will be more accessible by a method for measuring a physiological response to a substance.

In the present invention, the type of a polypeptide having a function of enhancing the translocation of the G protein-coupled receptor protein or a subunit thereof to the cell membrane is not particularly limited, as long as it has a function of "enhance the translocation of the G protein-coupled receptor protein or a subunit thereof to a cell membrane". The expression "enhance the translocation of the G protein-coupled receptor protein or a subunit thereof to a cell membrane" means that when the polypeptide is bound to the N-terminal side of each G protein-coupled receptor protein or a subunit thereof, the proportion of the thus expressed G protein-coupled receptor proteins or subunits thereof translocated from the nuclei of transformed cells to a cell membrane is (significantly) increased, compared with a case where no polypeptide is bound.

Examples of such a polypeptide that enhances the translocation of the G protein-coupled receptor proteins or a subunit thereof to a cell membrane include, but are not limited to, a polypeptide comprising the whole or a portion of a rat somatostatin receptor, and a polypeptide comprising the whole or a portion of bovine rhodopsin (Archives of Physiology and Biochemistry Vol. 110 p.137-45,2002; Nature Genetics, Vol.32, p.397-401, 2002; Cell, Vol.100, p.703-711, 2000). In one embodiment of the present invention, a polypeptide that enhances the translocation of the G protein-coupled receptor protein or a subunit thereof to a cell membrane is a polypeptide comprising the N-terminal 45 amino acids of a rat somatostatin receptor, a polypeptide comprising N-terminal 39 amino acids of bovine rhodopsin, or a polypeptide comprising N-terminal 21 amino acids of human rhodopsin.

The mRNA sequence of the rat somatostatin receptor is registered in the NCBI under the accession number of NM_133522.1. The nucleotide sequence and the amino acid sequence of the rat somatostatin receptor are also represented by SEQ ID NOs: 72 and 73 in the sequence listing of the present application. In the examples of the description, the 5' terminal 135 nucleotides of SEQ ID NO: 72 were linked 5' to the nucleotide sequence encoding a G protein-coupled receptor.

The mRNA sequence of human rhodopsin is registered in the NCBI under the accession number of NM_000539.1. The nucleotide sequence and the amino acid sequence of human rhodopsin are also represented by SEQ ID NOs: 74 and 75 in the sequence listing of the present application.

### (5) Vector

The construct of the present invention comprises: a nucleic acid encoding a G protein α subunit protein; a nucleic acid comprising an IRES sequence; and a nucleic acid encoding the G protein-coupled receptor protein or a subunit thereof in the stated order from the 5' side in a single vector. The construct comprising the above nucleic acids in the order in a single vector provides effects of the increased expression level of the G protein-coupled receptor protein or a subunit thereof in transformed cells, and/or the increased responsiveness of the G protein-coupled receptor protein or a subunit thereof to a ligand substance. In the vector, it is important that the nucleic acid encoding the G protein α subunit protein, the nucleic acid comprising the IRES sequence, and the nucleic acid encoding the G protein-coupled receptor protein or a subunit thereof are arranged from 5' in this order. It is a conventional theory that the expression level of a protein encoded by the nucleic acid on the 3' side of IRES is not high. The present inventors conceived of the present invention based on the discovery that in the case of the G protein-coupled receptor protein or a subunit thereof, the expression level is increased by arranging the protein or a subunit thereof 3' rather than 5' to IRES.

Further, when the G protein-coupled receptor is a dimer such as a sweet taste receptor (T1R2/T1R3) or a umami taste receptor (T1R1/T1R3), for example, only a nucleic acid encoding one subunit constituting the dimer may be contained together with a nucleic acid encoding the G protein αsubunit protein and IRES in a vector, as described above. A nucleic acid encoding the other subunit may be contained in another vector and this vector and the afore-mentioned vector may be co-transfected.

The type of a vector is not particularly limited as long as it is an expression vector that enables the expression of a protein in desired cells. Examples thereof include, but are not limited to, plasmid vectors, phage vectors, cosmid vectors, and viral vectors. Persons skilled in the art can appropriately select a vector depending on the type of cells in which the protein is expressed.

In one embodiment of the present invention, the vector is a plasmid vector. Examples of the plasmid vector include pFRT/lacZeo, pUC12, pUC13, pUC19, pBR322, pBR325, pSH15, pSH19, pUB110, and pC194. The vectors of the pcDNA™ series (Thermo Fisher Scientific Inc., pcDNA3 and pcDNA5) can be used as expression vectors in mammalian cells. In addition to these vectors, pEAK10 (Edge biosystem) can also be used as an expression vector in mammalian cells.

The vector of the present invention preferably comprises a promoter. The type of the promoter is not particularly limited, and examples thereof include a human cytomegalovirus-derived promoter (CMV promoter) and an EF1α promoter. In one embodiment of the present invention, the vector is a plasmid vector comprising a CMV promoter or a plasmid vector comprising an EF1α promoter.

The vector of the present invention preferably comprises a selection marker for selecting transformed cells. The type of the selection marker is not particularly limited, and examples thereof include known promoters such as a drug resistance gene and a fluorescent protein gene. Containing such a selection marker makes it possible to select a cell line that stably expresses the protein.

In one embodiment of the present invention, the selection marker is a drug resistance gene. Examples of the drug resistance gene include, but are not limited to, a hygromycin resistance gene, a puromycin resistance gene, a neomycin resistance gene, and a blasticidin resistance gene.

In one embodiment of the present invention, the vector is a plasmid vector comprising a CMV promoter and a hygromycin resistance gene. An example of such a vector is the pcDNA3.1 Hygro(+) vector (Thermo Fisher Scientific Inc.) used in the examples herein.

In another embodiment of the present invention, the vector is a plasmid vector comprising an EF1α promoter and a puromycin resistance gene. An example of such a vector is pEAK10 (Edge biosystem).

The sequences of the pcDNA3.1 Hygro(+) vector and the pEAK10 vector used in the examples herein are represented by SEQ ID NOs: 80 and 81, respectively, in the sequence listing of the present application. Nucleotides 895 to 942 of SEQ ID NO: 80 correspond to a multiple cloning site (MCS) into which a target gene is inserted. Nucleotides 1892-2823 of SEQ ID NO: 81 correspond to the EF-1α promoter sequence, nucleotides 2844 to 2845 correspond to a site where the target gene is inserted, and nucleotides 3293 to 3892 correspond to the puromycin resistance gene sequence.

### II. Transformed cells

The present invention also relates to transformed cells into which the construct of the present invention is introduced. The type of host cells for preparation of transformed cells by gene transfer of the construct of the present invention is not particularly limited as long as the cells can be transformed by introducing an exogenous nucleic acid via gene transfer.

For example, the cells are selected from the group consisting of animal cells, insect cells, plant cells, yeasts and bacteria. The origin of animal cells to be used herein is not particularly limited. Preferably, such cells refer to cells derived from an animal belonging to the phylum Chordata. The phylum Chordata includes Agnatha and Gnathostomata, and the Gnathostomata includes Mammalia, Aves, Amphibia, Reptilia and the like. Preferably, the animal cells are generally cells derived from an animal belonging to the Mammalia referred to as mammals. Examples of mammals include, but are not limited to, preferably mice, rats, humans, monkeys, pigs, dogs, sheep, and goats.

Cells derived from mammals include "established cell lines", which are cultured cells maintained outside the body for a long period of time to have certain stable properties, and thus the semi-permanent passage thereof can be performed. For example, there are established cell lines derived from various tissues of various biological species including humans, such as PC12 cells (derived from rat adrenal medulla), CHO cells (derived from Chinese hamster ovary), HEK293 cells (derived from human fetal kidney), HL-60 cells (derived from human white blood cells), HeLa cells (derived from human cervical cancer), Vero cells (derived from African green monkey kidney epithelial cells), MDCK cells (derived from canine renal tubular epithelial cells) and HepG2 cells (derived from human liver cancer).

The origin of plant cells herein is not particularly limited. The cells of plants including bryophytes, pteridophytes, and spermatophytes are target plant cells. Plants from which cells of spermatophytes are derived include both monocotyledonous and dicotyledonous plants. The origin of insect cells herein is also not particularly limited. The types of archaebacteria and bacteria to be used herein are also not particularly limited.

Examples of host cells for preparation of the transformed cells of the present invention, which can be used herein, include, but are not limited to, eukaryotic cells such as HEK 293 cells, CHO cells, 32D cells, HeLa cells, COS cells, and BHK cells. In one embodiment of the present invention, the host cells are HEK 293 cells.

Methods for the gene transfer (transformation) into host cells using the construct of the present invention are not particularly limited. Methods suitable for gene transfer of nucleic acids into animal cells, plant cells, and bacteria, are known for each of these cells (e.g., MOLECULAR CLONING: A Laboratory Manual (Fourth Edition), Michael R Green and Joseph Sambrook, 2012, (Cold Spring Harbor Laboratory Press)). Persons skilled in the art can employ appropriate gene transfer methods depending on the type of a vector, the type of cells, and the like. For example, in the case of non-viral vectors such as plasmid vectors, gene transfer methods such as a lipofection method, an electroporation method and a microinjection method are known.

The transformed cells may be cells (stable expression cell line) stably expressing a G protein-coupled receptor protein or a subunit thereof or cells transiently expressing the same (transiently expressing cells). The "stable expression cell line" is, for example, a cell line stably expressing the G protein-coupled receptor protein or a subunit thereof, as confirmed using a selection marker that a nucleic acid contained in the construct has been stably incorporated by gene transfer into the genome of host cells. The "transiently expressing cells" are not confirmed to undergo stable incorporation of the nucleic acid contained in the construct into the genome of the host cells, and thus are not confirmed to stably express the G protein-coupled receptor protein or a subunit thereof.

The transformed cells of the present invention have the increased expression level of a G protein-coupled receptor protein or a subunit thereof, and/or the increased responsiveness of the G protein-coupled receptor protein or a subunit thereof to a ligand substance. The expression "the increased expression level of a G protein-coupled receptor protein or a subunit thereof' means that the expression level of the G protein-coupled receptor protein or a subunit thereof is increased compared to a case where the same is expressed using conventional techniques. The expression "the increased responsiveness of a G protein-coupled receptor protein or a subunit thereof to a ligand substance" means that the responsiveness of the G protein-coupled receptor protein or a subunit thereof to a ligand substance is increased compared to a case where the same is expressed using conventional techniques prior to the present invention.

The "conventional techniques" includes, for example, the following embodiments.

### (1) Double-gene transfer

(1a) Host cells are co-transfected with a vector comprising a nucleic acid encoding a Gα protein and a vector comprising a nucleic acid encoding the G protein-coupled receptor protein or a subunit thereof;
(1b) A cell line in which a Gα protein is stably expressed is transformed with a vector comprising a nucleic acid encoding the G protein-coupled receptor protein or a subunit thereof.

### (2) Co-expression vector comprising IRES

Transformation is performed using a single vector comprising, from 5' in this order: a promoter; a nucleic acid encoding the G protein-coupled receptor protein or a subunit thereof; IRES; and a nucleic acid encoding a Gα protein.

The transformed cells of the present invention are superior in stability to a case where a G protein-coupled receptor protein or a subunit thereof is expressed using conventional techniques. Examples of the stability include stability under normal culture conditions for cells and stability under storage conditions such as conditions for cryopreservation. For example, in the examples herein, even in the case of about 1 month of cryopreservation, the response intensity to a ligand was hardly decreased and was 0.8 or more as compared to that before cryopreservation. On the other hand, in the case of double-gene transfer (double stable), the response intensity to a ligand was decreased to less than 0.6 after freeze-drying.

### III. Method for measuring physiological response to substance

The present invention further relates to a method for measuring a physiological response to a substance. The measuring method of the present invention comprises a step of adding a substance to the transformed cells of the present invention and measuring the physiological response to the substance.

The "substance" is not particularly limited as long as it is a substance that can bind to a G protein-coupled receptor and function as an agonist. Such substance causes a structural change in a G protein-coupled receptor, and activates a G protein bound to the inside of the membrane to perform signaling, and examples thereof include chemical substances contained in foods and beverages, odor molecules, neurotransmitters, hormones, etc. Signals induce physiological responses in intracellular signal networks such as olfactory sense, gustatory sense, vision, neurotransmission, metabolism, cell differentiation and cell proliferation, and inflammatory responses and immune responses. Substances differ depending on the types of G protein-coupled receptors.

In one embodiment, the substance is a bitter substance, an odorant, an umami substance or a sweet substance, or a modulator to these substances.

The "bitter substance" is not particularly limited as long as it is a substance that a human can sense its bitter taste. Examples thereof include denatonium benzoate, strychnine, β-glucopyranoside (salicin), aristolochic acid, a bitter substance having an isothiocyanate group (e.g., phenylthiocarbamide (PTC), propylthiouracil (PROP)), diphenydol, quinine, absinthin and denatonium.

The "odorant" is a low molecular weight compound having a molecular weight of about 30 to 300. For example, the basic skeleton of serotonin, which is a brain hormone, is a substance with a fecal odor called indole, and the skeleton of dopamine or adrenalin resembles vanillin, which is the odor of vanilla. Any substance can be an odorant, as long as it is a low molecular weight organic compound having the property of volatility. Odors are emitted from various foods, plants and animals, and even humans said to have deteriorated sense of smell can identify about 10,000 odor types. Examples of "odorant", and specific examples of "odorant" include muscone which is a main component of musk (mush) odor, civetone which is a main component of civet, and α-ionone which exudes a flower scent of violet, α-terpineol which is a main component of a flower scent of lilac, (-) 5R-carvone which produces a scent of spearmint, and further, methylamine, dimethylamine, trimethylamine, etc., which produce fish odor.

The "umami substance" is not particularly limited as long as it is a substance, the umami taste of which can be detected by humans, for example. Examples thereof include glutamic acid and inosinic acid (IMP).

The "sweet substance" is not particularly limited as long as it is a substance the sweetness of which can be sensed by humans. Examples thereof broadly include saccharide sweeteners such as glucose, fructose, galactose, raffinose, xylose, sucrose, maltose, lactose, starch syrup, isomerized sugar, isomaltooligosaccharide, fructooligosaccharide, galactooligosaccharide, xylooligosaccharide, lactosucrose, soy oligosaccharide, trehalose, sorbitol, mannitol, maltitol, xylitol, erythritol, lactitol, isomaltol, reduced sugar syrup, reduced palatinose, Wasanbon (Japanese cured sugar), black sugar, soft brown sugar, honey, molasses, licorice extract, and maple syrup, and non-saccharide sweeteners such as aspartame, saccharin, dulcin, stevioside, stevia extract, glycyrrhizin, acesulfame-K, sucralose (registered trademark; San-Ei Gen F. F. I., Inc.), cyclamate, alitame, neotame, perillartin, monellin, and curculin (registered trademark; ADEKA).

The term "a bitter substance, an odorant, an umami substance or a modulator of a sweet substance" refers to a substance that does not independently produce bitterness, smell, umami taste or sweetness, but enhances, modifies or suppresses the gustatory sense and the olfactory sense of each substance when accepted together with a bitter substance, an odorant, an umami substance or a sweetener.

Examples of "a modulator of a bitter substance" include Benecort BMI (Kao) and probenecid. Examples of "a modulator of an odorant" include olfactory inhibitors such as 2,4,6-trichloroanisole and geraniol. Examples of "a modulator of a sweetener" include a sweetness suppressant, lactisole, a sweetness suppressant, gymnemic acid, and a sweetness modifier, neoculin.

A physiological response to a substance is measured using the transformed cells of the present invention as follows, for example.

A predetermined number of the transformed cells of the present invention are seeded in each well of a microplate having a large number of wells (24 wells, 48 wells, 96 wells, 384 wells, etc.) (e.g., 10,000 to 500,000 cells/well) and then the cells are cultured in a predetermined medium (e.g., DMEM medium). After cell culture, when a specific substance is added, a physiological response generated in a stable culture cell line is measured, and the physiological response to the substance is evaluated based on the measurement result. In measuring physiological responses, events that change with activation of G protein-coupled receptors are appropriately selected depending on the type of a substance.

For example, when a gustatory sense receptor such as a bitter taste receptor, a sweet taste receptor, and an umami taste receptor is activated, intracellular calcium concentration is increased through the signal transduction process mediated by intracellular second messengers (IP3 (inositol triphosphate), DAG), etc. Therefore, physiological responses to be measured include changes in intracellular second messengers, changes in intracellular calcium concentration, and the like, which take place in association with activation of taste receptors. Thus, it is possible to measure a physiological response by measuring changes in intracellular second messengers or calcium concentration.

For example, such measurement can be performed by a calcium imaging method that involves observing a change in intracellular calcium concentration caused by stimulation of gustatory senses such as bitter taste, sweet taste or umami taste using a fluorescent calcium indicator. The fluorescent calcium indicator requires a change in fluorescence characteristics at a calcium concentration that can be physiologically changed, and induction of such a change in a calcium-specific manner. Examples of the fluorescent calcium indicator include Fluo-8 (registered trademark) (AAT Bioquest, Inc.) (excitation wavelength of 490 nm, fluorescence wavelength of 514 nm), Fluo-4 AM (AAT Bioquest, Inc.) (excitation wavelength of 490 nm, fluorescence wavelength of 525 nm), and Fura 2 AM (Thermo Fisher, Inc.) (excitation wavelength of 340 nm, fluorescence wavelength of 380 nm).

For measuring a physiological response to an odorant, for example, a technique for measuring a change in intracellular cAMP concentration induced by the stimulation of olfactory sense by an odorant is employed for measuring a physiological response to the odorant. Examples of a method for measuring intracellular cAMP include techniques that involve measuring by a fluorescent polarization immunoassay or a competitive immunoassay using cAMP labeled with a fluorescence indicator or an enzyme such as HRP and an anti-cAMP antibody, and techniques that involve performing gene transfer of a vector containing luciferase inserted therein to a site downstream of a cAMP response element (CRE) and then measuring by luciferase assay.

The present invention comprises the use of the transformed cells according to the present invention for measuring a physiological response to a substance.

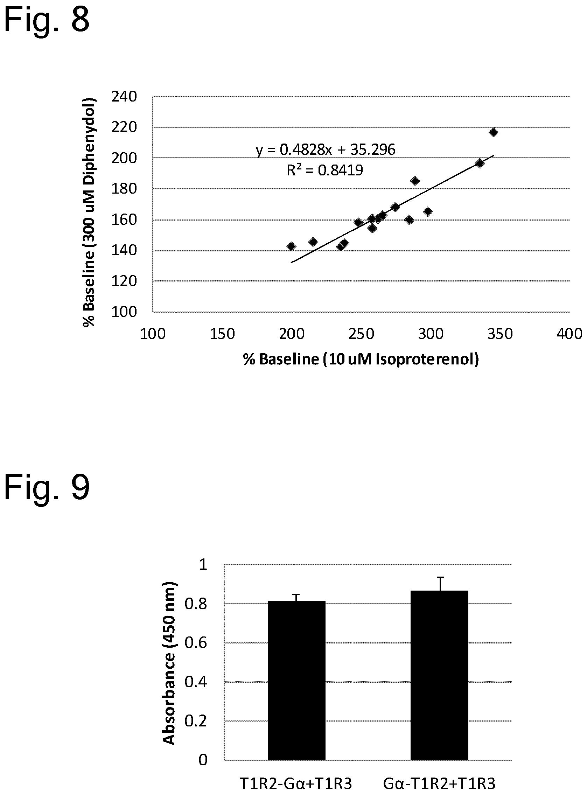

The present invention comprises, in a single vector, a nucleic acid encoding a G protein α subunit protein and a nucleic acid encoding a G protein-coupled receptor protein or a subunit thereof. Therefore, it is presumed that the expression levels of the G protein α subunit protein and the G protein-coupled receptor protein in the transformed cells are positively correlated. In the transformed cells of the present invention, the responsiveness of the G protein-coupled receptor to a G protein-coupled receptor agonist and the response intensity to a Gα agonist exhibit a high positive correlation (Example 7, Fig. 8). Therefore, it is also possible to estimate the physiological response intensity to a substance by measuring the response intensity of a G protein α subunit to a Gα agonist in the transformed cells to which the substance (G protein-coupled receptor agonist) has been added.

### IV. Kit for use in a method for measuring physiological response to substance

The present invention further relates to a kit comprising the transformed cells of the present invention, which is used for a method for measuring a physiological response to a substance by adding a substance to the transformed cells, and then measuring a physiological response induced by the substance.

The kit of the present invention may comprise, in addition to the transformed cells of the present invention, materials necessary for use in a method for measuring a physiological response to a substance, instructions for use, and the like. Examples of materials necessary for use in the method for measuring a physiological response to a substance include, a medium for maintaining and culturing transformed cells, and reagents (e.g., a fluorescent calcium indicator for measuring calcium concentration etc.) for measuring second messengers or the concentration of calcium resulting from a physiological response to a substance.

The present invention relates to an apparatus comprising transformed cells, which is used for a method for measuring a physiological response to a substance by adding a substance to the transformed cells and measuring a physiological response induced by the substance. The apparatus of the present invention includes the kit of the present invention, and equipment for measuring second messengers or the concentration of calcium resulting from a physiological response to a substance.

As an example of the apparatus for measuring a physiological response to a taste substance such as a bitter substance, an odorant, an umami substance or a sweet substance, or modulators of these substances, an "artificial lipid membrane taste sensor" has been developed by Intelligent Sensor Technology Inc., (http://www.insent.co.jp/products/taste_sensor_index.html), for example. This taste sensor is a device for detecting a displacement in the membrane potential of the artificial lipid membrane due to electrostatic interaction or hydrophobic interaction with various taste substances in the form of sensor outputs by a computer. For example, a taste sensing system TS-5000Z is marketed by the Intelligent Sensor Technology Inc. The apparatus of the present invention utilizes the transformed cells of the present invention instead of the artificial lipid membrane in such a sensor. Specifically, the transformed cells of the present invention are immobilized in the apparatus instead of an artificial membrane, a substance is allowed to act, a change in intracellular second messenger or calcium concentration is measured, and thus a physiological response to the substance is measured. The apparatus of the present invention can be used repeatedly for measurement by removing test substances by washing or the like after use.

### EXAMPLES

Hereinafter, the present invention will be described in detail based on examples, but the present invention is not limited to these examples. Persons skilled in the art can easily make modifications and changes to the present invention based on the description, and these are included in the technical scope of the present invention.

### Example 1 Construction of construct and method for preparing construct

In this example, a construct for expressing a G protein-coupled receptor protein was prepared. The construct of the present invention comprises: a nucleic acid encoding a G protein α subunit protein; a nucleic acid comprising an IRES sequence; and a nucleic acid encoding the G protein-coupled receptor protein in the stated order from the 5' side in a single vector.

The following three gene types were amplified by PCR respectively.

A human bitter taste receptor (T2R16) expression gene (ssr/T2R16/V5) prepared by the addition of a gene sequence ranging from the start codon to the 135th nucleotide of rat somatostatin receptor 3 (ssr3) to the 5' end and a V5 epitope sequence to the 3' end.

Note that the V5 epitope sequence is an antibody recognition tag for examining the protein expression level of T2R (nucleotide sequence: SEQ ID NO: 76).
- Encephalomyocarditis virus-derived IRES, and
- Gα16/gust expression gene (Gα16/Gαgust43) (SEQ ID NO: 77) prepared by substitution of terminal 129 nulceotides of human G protein αsubunit G16 with terminal 129 nucleotides of rat gustducin (Ggust).

The thus obtained gene fragment was inserted into the multiple cloning site of pcDNA3.1 Hygro(+) vector (Thermo Fisher Scientific Inc.) using an In-Fusion HD Cloning Kit (Clontech). The insertion order is as follows.
Vector of the present invention: Gα-T2R16
Gα16/Gαgust43-IRES-ssr/T2R16/V5
   Vector of a comparative example: T2R16-Gα
   ssr/T2R16/V5-IRES-Gα16/Gαgust43

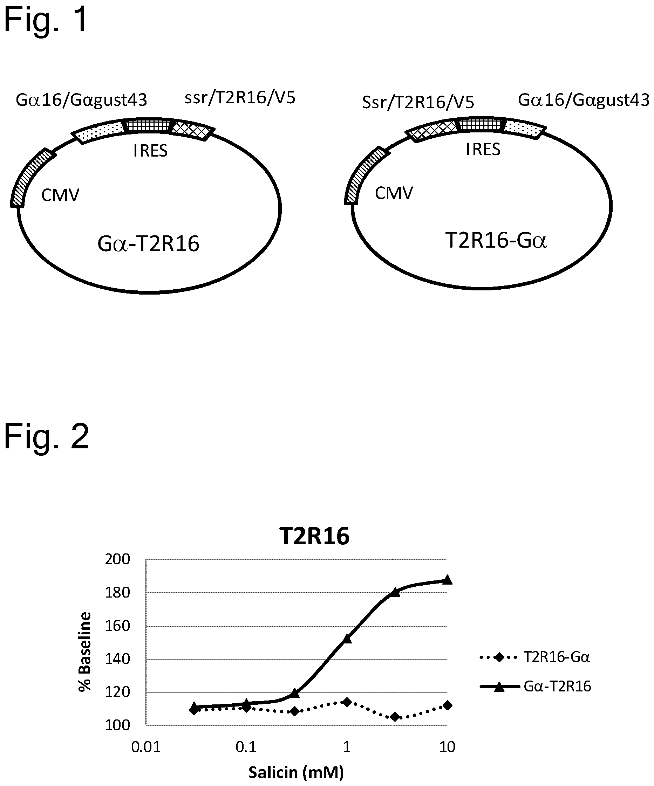

The construction of each vector is depicted in Fig. 1.

### Example 2 Preparation of stable expression cell line containing the construct of the present invention

In this example, a cell line (T2R16 stable expression cell line) stably expressing the bitter taste receptor T2R16 was prepared by incorporating the construct (Gα-T2R16) of the present invention.

Specifically, into 1 × 10⁶ cells of the human embryonic kidney cell line HEK293T (obtained from RIKEN) seeded in a culture vessel, 5 µg of each of the Gα-T2R16 and T2R16-Gα plasmid vectors prepared in Example 1 was introduced through the use of lipofectamine (registered trademark) 3000 (Thermo Fisher Scientific Inc.). After 48 hours of culture, drug resistance screening was performed by adding 100 µg/mL hygromycin B to the medium and then culturing for about 15 days. Cells that had acquired drug resistance were cloned by seeding these cells in each well of a 96-well plate at one cell per well. After about 10 days of culture, a stable expression cell line was obtained.

### Example 3 Measurement of response of stable expression cell line to bitter substance

### (1) Measurement of response of Gα-T2R16 stable expression cell line

In this example, the response of the T2R16 stable expression cell line of Example 2 to a bitter substance was measured.

The T2R16 stable expression cell line prepared based on the method described in Example 2 was seeded at 2 × 10⁴ cells/well in a 96-well plate. The culture media for cells contained a green fluorescent calcium indicator Fluo-8 (registered trademark) (AAT Bioquest, Inc.), and the uptake of Fluo-8 into the cell lines was examined. Fluo-8 (registered trademark) is suitable for detection of intracellular calcium dynamics and is an indicator of GPCR activity.

Cells incorporating Fluo-8 (registered trademark) were excited at 490 nm and the fluorescence intensity at 514 nm was measured. During measurement, T2R16 agonist, salicin (10, 3, 1, 0.3 or 0.1 mM), was added, and the maximum fluorescence intensity during 40 seconds after addition was obtained. The increase rate of the maximum fluorescence intensity relative to the fluorescence intensity at the start of the measurement was calculated as the response intensity of T2R16 to the agonist. The results are depicted in Fig. 2.

As depicted in Fig. 2, only the cell line containing the Gα-T2R16 plasmid exhibited responses to salicin. In the cell line containing the T2R16-Gα plasmid, almost no response to salicin was observed, and a clone exhibiting a response could not be isolated.

### (2) Measurement of response of Gα-T2R40 and Gα-T2R43 stable expression cell lines

Responses of cell lines stably expressing bitter taste receptor subtypes, T2R40 and T2R43, other than T2R16, were also measured by the same method as in Example 3(1).

Specifically, by the same method as in Example 1 and Example 2, a Gα-T2R40 stable expression cell line (containing Gα16/Gαgust43-IRES-ssr/T2R40/V5) and a Gα-T2R43 stable expression cell line (containing Gα16/Gαgust43-IRES-ssr/T2R43/V5) were prepared.

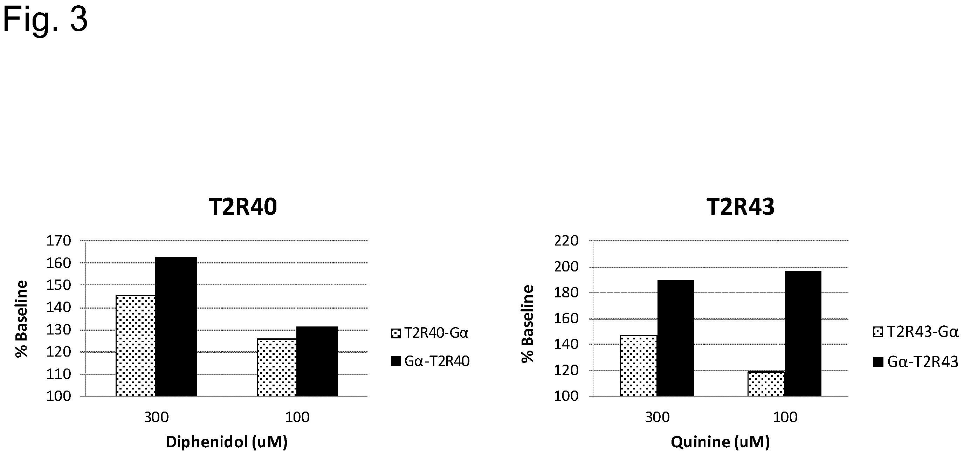

The Gα-T2R40 stable expression cell line and the Gα-T2R43 stable expression cell line were prepared by the same method as in Example 2. Each agonist (T2R40: diphenydol, T2R43: quinine) was allowed to act on these cell lines at 100 µM or 300 µM, and then the maximum fluorescence intensity during 40 seconds after addition was obtained. The increase rate of the maximum fluorescence intensity relative to the fluorescence intensity at the start of measurement was calculated as the response intensity of T2R40 and T2R43 to the agonists. The results are depicted in Fig. 3. As compared with T2R40-Gα and T2R43-Gα, Gα-T2R40 and Gα-T2R43 exhibited higher response intensity to each agonist.

### Example 4 Measurement of response of transiently expressing cells to bitter substance

Into HEK293T seeded at 2 × 10⁴ in a 96-well plate, 0.1 µg of each of the Gα-T2R16 and the T2R16-Gα plasmid vectors was introduced using lipofectamine (registered trademark) 3000. After 30 hours of culture, response intensity to salicin was measured using the same technique as in Example 3 (1) (maximum fluorescence intensity during 40 seconds after the addition of salicin). Note that drug resistance screening with hygromycin B as in Example 2 was not performed (transiently expressing cells).

Furthermore, as a comparative example, the following cells were also prepared other than T2R16-Gα expressing cells, and then the response to salicin was measured:
cells (cotransfection), prepared by simultaneous gene transfer of a total of 0.1 µg of a Gα16/Gαgust43gust expression vector and a ssr-T2R16-V5 expression vector comprising a Gα16/Gαgust43 expression gene and a ssr-T2R16-V5 expression gene, respectively, via incorporation thereof into pcDNA3.1 Hygro(+) vectors for transient expression of the genes; and
cells (Gα stable), prepared by the gene transfer of 0.1 µg of a ssr-T2R16-V5 expression vector into a Gα16/Gαgust43 stable expression cell line (subjected in advance to drug resistance screening using hygromycin B) prepared by the same technique as in Example 2 for transient expression of the genes.

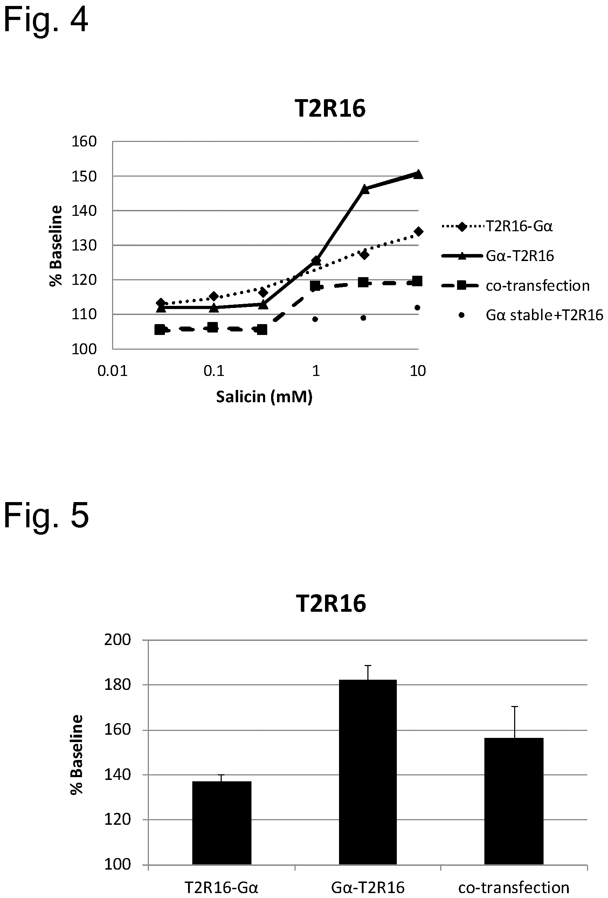

The results are depicted in Fig. 4. As can be seen from the results in Fig. 4, it was confirmed that Gα-T2R16 exhibited response intensity higher than that of T2R16-Gα even in the transient expression system, and also higher than that of the previously reported transient expression systems (co-transfection and Gα stable).

### Example 5 Expression levels of Gα16/Gαgust43 and bitter taste receptor T2R16

In general, IRES-mediated translation of mRNA is considered to be less efficient compared to cap-dependent translation from the 5' end. In this example, the expression levels of Gα16/Gαgust43 and bitter taste receptor T2R16 were examined for cap-dependent translation and IRES-dependent translation.

### (1) Gα16/Gαgust43 expression level

Transiently expressing cells, "Gα-T2R16 (the present invention)", transiently expressing cells, "T2R16-Gα", and transiently expressing cells, "co-transfection" prepared in Example 4, were subjected to measurement and comparison of response intensity to a T2R independent Gα16 agonist, isoproterenol (3 µM) by the same method as in Example 3. The results are depicted in Fig. 5.

The response intensity to isoproterenol is considered to be proportional to the expression level of Gα16. As depicted in Fig. 5, it was confirmed that the expression level of T2R16-Gα resulting from IRES-mediated translation of Gα16/Gαgust43 is lower than that of "Gα-T2R16" and "co-transfection" resulting from cap-dependent translation of Gα16/Gαgust43. Specifically, it was demonstrated that the expression level of Gα16/Gαgust43 is cap-dependent > IRES-dependent.

### (1) T2R16 expression level

T2R16-Gα, Gα-T2R16 (the present invention) and ssr-T2R16-V5 alone (T2R16 single)-transiently expressing cells were compared for the expression level of T2R16 by an In cell ELISA method. For the In cell ELISA, an In Cell ELISA Kit (BOO Bioscientific) was used, and for detection of T2R16, an anti-V5-HRP antibody (Thermo Fisher Scientific Inc.) for recognizing V5 epitope added to the C-terminus was used. The relative expression level of T2R16 was obtained by measuring the absorbance at 450 nm to determine the color development of the HRP substrate.

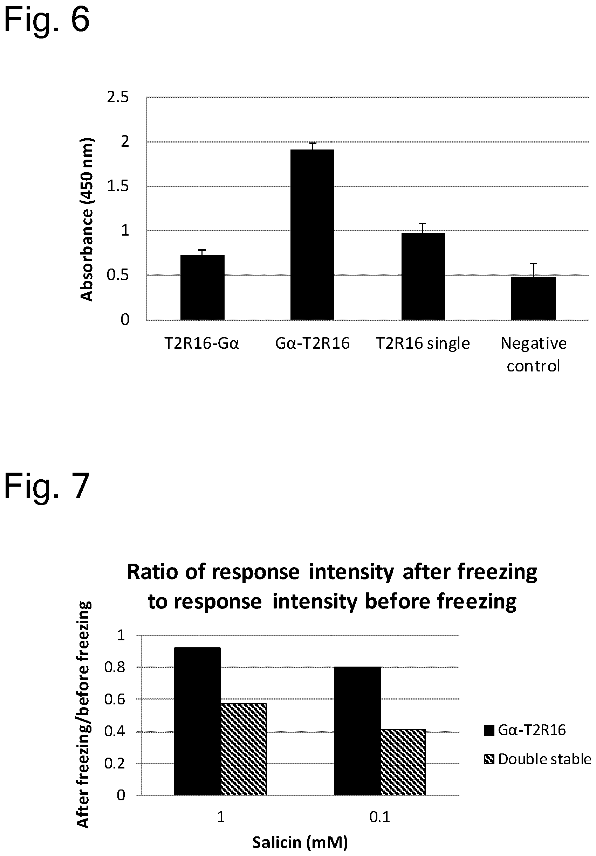

The results are depicted in Fig. 6. As a result, it was confirmed that the expression level of T2R16 of Gα-T2R16 resulting from IRES-mediated translation of "T2R16" was higher than that of "T2R16-Gα and T2R16 single" resulting from cap-dependent translation of T2R16. Specifically, it was demonstrated that the expression level of T2R16 was cap-dependent < IRES-dependent. Similar results were also obtained upon the use of Gα16 having a property of binding with T2R lower than that of Gα16/Gαgust43 (data not shown).

Therefore, it was demonstrated that the Gα-T2R16 of the present invention causes the high levels of expression of both Gα16/Gαgust43 (and Gα16) and T2R16. Furthermore, similar results were observed for subtypes (T2R40 and T2R43) other than the bitter taste receptor T2R16 (data not shown).

### Example 6 Storage Stability of Gα-T2R16 stable expression cell line

In this example, response intensity before and that after cryopreservation of the Gα-T2R16 stable expression cell line were examined.

Gα-T2R16 in this example was prepared in the same manner as in the case of the Gα-T2R16 stable expression cell line in Example 2, but pEAK10 (Edge biosystem, Inc.) was used as an expression vector and "ssr/T2R16" (without V5) was inserted instead of "ssr/T2R16/V5". Further, drug resistance screening was performed with 1 µg/mL puromycin instead of hygromycin B.

As a comparative example, into the Gα16/Gαgust43 stable expression cell line (subjected in advance to drug resistance screening with puromycin) prepared using the same technique as in Example 2,5 µg of ssr-T2R16 expression vector was introduced and then drug resistance screening was performed using puromycin. The thus prepared stable expression cell line (Double stable) stably expressing Gα16/Gαgust43 + T2R16 was used.

Before and after cryopreservation of the cell line, the response intensity to salicin (1 or 0.1 mM) was examined in the same manner as in Example 3. For cryopreservation, CELLBANKER (Nippon Zenyaku Kogyo Co., Ltd.) was used, and cells were stored under liquid nitrogen for 1 month. The temperature for the cryopreserved cells was brought back to normal temperature, cells were passaged at least twice, and then the response intensity after cryopreservation was measured. The results are depicted in Fig. 7.

As a result, almost no effect due to freezing was confirmed among the Gα-T2R16 stable expression cell line. On the other hand, in the case of Double stable, cell lines exhibiting a significant decrease in response intensity after freezing were occasionally observed. An example of the results is depicted in Fig. 7. In Fig. 7, the Gα-T2R16 stable expression cell line exhibited the ratio of response intensity after freezing/response intensity before freezing of 0.8 or more in cases of both salicin concentrations of 1 mM and 0.1 mM. On the contrary, in "Double stable", the ratio was less than 0.6 (in particular, 0.4 or less in the case of 0.1 mM salicin).

### Example 7 Response intensity of Gα-T2R40 stable expression cell line to T2R40 agonist and response intensity of the same to Gα agonist

In this example, the Gα-T2R40 stable expression cell line of the present invention was examined for the correlation between the response intensity to a T2R40 agonist (diphenydol) and the response intensity to a Gα agonist (isoproterenol).

As described in Example 3 (2), a Gα-T2R40 stable expression cell line was prepared by the same method as in Example 1 and Example 2, and 15 clones were prepared by cloning. The correlation between responses to isoproterenol (10 µM) and responses to diphenydol (300 µM) in these 15 clones is depicted in Fig. 8. As a result, a high correlation was obtained between the responses to the T2R40 agonist, diphenydol, and the responses to the Gα16 agonist, isoproterenol.

Therefore, through the method using the construct of the present invention, a desired cell line that expresses both Gα subunit and T2R proteins can be obtained by only one evaluation of either T2R or Gα subunit protein. On the other hand, when a conventional method is employed, a gene for expressing the Gα subunit protein and a gene for expressing the T2R protein are successively introduced into cells, and after every introduction, screening should be performed while evaluating whether or not each protein is stably expressed. Specifically, after a Gα stable expression cell line is prepared and then screened for (evaluation 1), a gene encoding the T2R protein is introduced and thus a cell line expressing the T2R protein at a high level (evaluation 3) while maintaining the high-level expression of the Gα protein (evaluation 2) is obtained. Hence, evaluation should be performed 3 times to obtain a desired cell line.

According to the present invention, the working efficiency for obtaining a Gα-T2R40 stable expression cell line is remarkably improved.

### Example 8 IRES-mediated expression system 1 for sweet taste receptor T1R2/T1R3: examination of T1R2

A sweet taste receptor is a G protein-coupled receptor of the metabotropic glutamate receptor/metabotropic pheromone receptor class consisting of a combination of two subunit types of T1R2 and T1R3. In this example, T1R2 of sweet taste receptor T1R2/T1R3 in the IRES-mediated expression system was examined.

### (1) Preparation of expression vector

Human sweet taste receptor subunit T1R2 and human sweet taste receptor subunit T1R3 and IRES and Gα16/Gαgust43 (Gα) described in Example 1 were prepared. They were inserted into the multiple cloning site of pcDNA3.1 Hygro (+) vectors (Thermo Fisher Scientific Inc.) as follows, thereby obtaining each construct.
Construct Gα-T1R2
   Gα16/Gαgust43-IRES-T1R2/V5
   Construct T1R2-Gα
   ssr/T1R2/V5-IRES-Gα16/Gαgust43
Construct T1R3
   T1R3

### (2) T1R2 expression level

Through double-gene transfer of Gα-T1R2 and T1R3 by the same method as in Example 4, a cell line (Gα-T1R2 + T1R3) transiently expressing Gα-T1R2 and T1R3 was obtained. As a comparative example, a cell line (T1R2-Gα + T1R3) transiently expressing T1R2-Gα and T1R3 was obtained by double-gene transfer thereof.

The expression level of T1R2 was examined by the In cell ELISA using an In Cell ELISA Kit (BOO Bioscientific) by the same method as in Example 5 (2). The results are depicted in Fig. 9. The expression level of T1R2 tended to be higher in "Gα-T1R2 + T1R3" than that in "T1R2-Gα + T1R3". As in the bitter taste receptor (T2R), the expression level was cap-dependent < IRES-dependent.

### (3) Gα16/Gαgust43 expression level

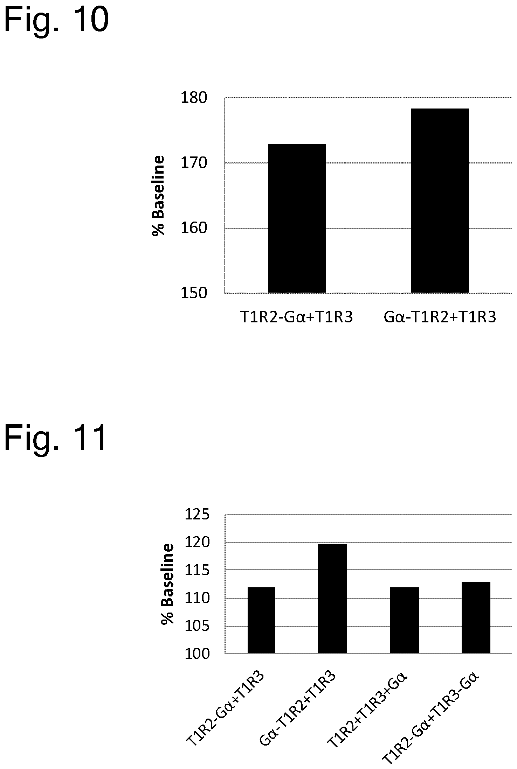

The expression level of Gα16/Gαgust43 was examined for "Gα-T1R2 + T1R3" and "T1R2-Gα + T1R3" by the same method as in Example 5(1). 10 µM of isoproterenol, which is a Gα16 agonist, was used. As depicted in Fig. 10, the expression level of Gα16/Gαgust43 was higher in "Gα-T1R2 + T1R3" than the other. As in the bitter taste receptor (T2R), the expression level was cap-dependent > IRES-dependent.

### (4) Response to T1R2 agonist

"Gα-T1R2 + T1R3" and "T1R2-Gα + T1R3" were examined for the response to a T1R2 agonist. Aspartame (10 mM) was added and the maximum fluorescence intensity during 40 seconds after addition was obtained. The increase rate of the maximum fluorescence intensity relative to the fluorescence intensity at the start of measurement was calculated as the response intensity of T1R2 to the agonist.

As a comparative example, in addition to "T1R2-Gα + T1R3", "cells (T1R2 + T1R3 + Gα) co-transfected with three types of vectors each comprising a gene encoding each protein of T1R2, T1R3 and Gα16/Gα gust43", and "cells (T1R2-Gα + T1R3-Gα) co-transfected with T1R2-Gα and T1R3-Gα" were used. As depicted in Fig. 11, "Gα-T1R2 + T1R3" exhibited the highest response to the T1R2 agonist.

### Example 9 IRES-mediated expression system 2 for sweet taste receptor T1R2/T1R3: Examination of T1R3

In this example, T1R3 of sweet taste receptor T1R2/T1R3 in the IRES-mediated expression system was examined.

### (1) Preparation of expression vector

Human sweet taste receptor subunit T1R2 and human sweet taste receptor subunit T1R3 and IRES and Gα16/Gαgust43 (Gα) described in Example 1 were prepared. They were inserted into the multiple cloning site of pcDNA3.1 Hygro (+) vectors (Thermo Fisher Scientific Inc.) as follows, thereby obtaining each construct.
Construct Gα-T1R3
   Gα16/Gαgust43-IRES-ssr/T1R3/V5
   Construct T1R3-Gα
   ssr/T1R3/V5-IRES-Gα16/Gαgust43
Construct T1R2
   T1R2

### (2) T1R3 expression level

Through double-gene transfer of Gα-T1R3 and T1R2 by the same method as in Example 4, a cell line (Gα-T1R3 + T1R2) transiently expressing Gα-T1R3 and T1R2 was obtained. As a comparative example, a cell line (T1R3-Gα + T1R2) transiently expressing T1R3-Gα and T1R2 was obtained by double-gene transfer thereof.

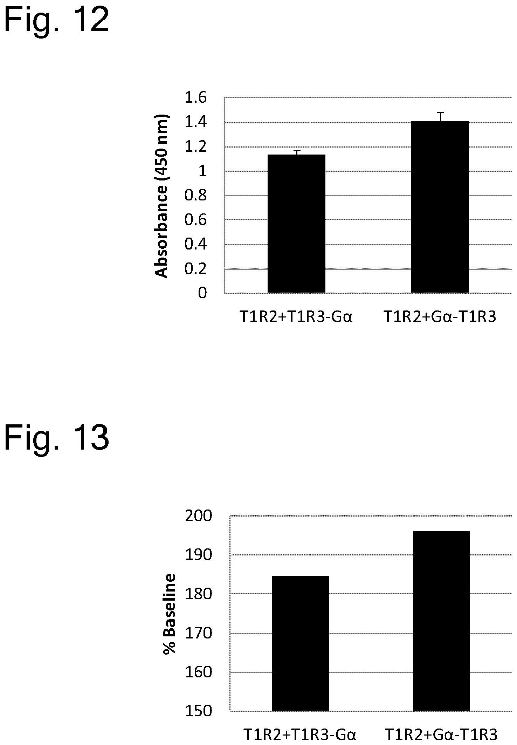

The expression level of T1R3 was examined by the In cell ELISA using an In Cell ELISA Kit (BOO Bioscientific) by the same method as in Example 5(2). The results are depicted in Fig. 12. The expression level of T1R3 was higher in "Gα-T1R3 + T1R2" than in "T1R3-Gα + T1R2". The expression level of T1R3 was cap-dependent < IRES-dependent.

### (3) Gα16/Gαgust43 expression level

"Gα-T1R3 + T1R2" and "T1R3-Gα + T1R2" were examined for the expression level of Gα16/Gαgust43 by the same method as in Example 5(1). 10 µM of isoproterenol, which is a Gα16 agonist, was used. As depicted in Fig. 13, the expression level of Gα16/Gαgust43 was higher in "Gα-T1R3 + T1R2", and was cap-dependent > IRES-dependent, as in the case of the bitter taste receptor (T2R).

Taken together with the results of Example 9(2), it was demonstrated that the Gα-T1R3 of the present invention causes the high-level expression of both T1R3 and Gα16/Gαgust43.

### (4) Response to T1R3 agonist

"Gα-T1R3 + T1R2" and "T1R3-Gα + T1R2" were examined for the response to a T1R3 agonist. Cyclamate (10 mM) was added, and the maximum fluorescence intensity during 40 seconds after addition was obtained. The increase rate of the maximum fluorescence intensity relative to the fluorescence intensity at the start of measurement was calculated as the response intensity of T1R3 to the agonist.

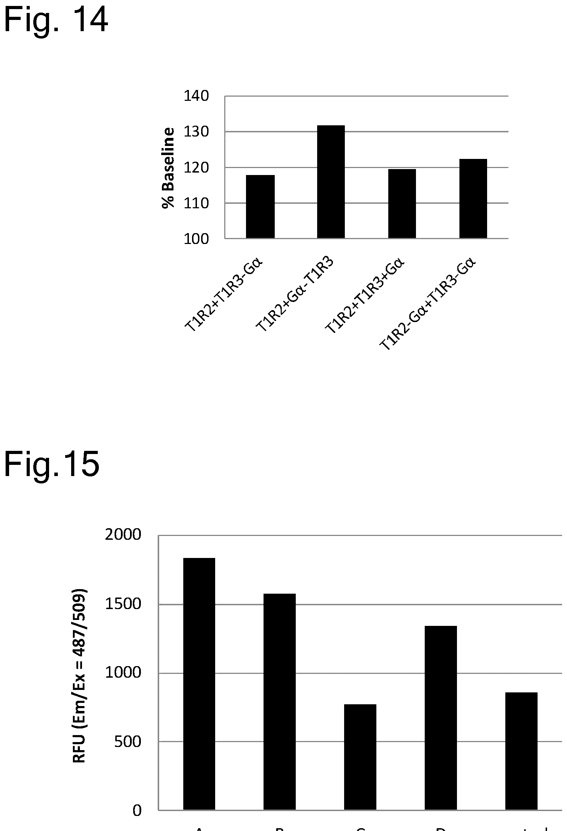

As a comparative example, in addition to "T1R3-Gα + T1R2", "cells co-transfected with three types of vectors each containing a gene encoding each protein of T1R2, T1R3 and Gα16/Gαgust43", and "cells co-transfected with T1R2-Gα and T1R3-Gα" were used. As depicted in Fig. 14, "Gα-T1R3 + T1R2" exhibited the highest response to the T1R3 agonist.

### Example 10 IRES-mediated protein expression

In order to examine the effect of IRES on the expression level of a protein, proteins other than a GPCR protein were expressed upstream and downstream of IRES. As the protein, a green fluorescent protein (EmGFP) (nucleotide sequence: SEQ ID NO: 78 (GenBank Accession No. EU056363.1); amino acid sequence: SEQ ID NO: 79) was used. Constructs were prepared using pcDNA vectors comprising the following nucleic acid sequences A to D, and then introduced into cell line HEK293T for transient expression thereof.
A: ssr- EmGFP - IRES- Gα
B: EmGFP - IRES - Gα
C: Gα - IRES - ssr - EmGFP
D: Gα - IRES - EmGFP
Control: pcDNA vector containing no inserted gene

Cells were excited at the wavelength of 487 nm and the fluorescence intensity at the wavelength of 509 nm was measured. The fluorescence intensity is proportional to the expression level of EmGFP. The results are depicted in Fig. 15. The expression level of EmGFP protein was higher in cases (A, B) where the EmGFP gene had been placed upstream of IRES, than in cases (C, D) where the same was placed downstream of IRES. The expression level of EmGFP protein was cap-dependent > IRES-dependent as in the case of Gα protein.

### Example 11 T2R16 expression level in cells transiently expressing pEAK10-T2R16-Gα and pEAK10-Gα-T2R16

In this example, T2R16 expression level in HEL293T cells transiently expressing pEAK10-IRES-T2R16-Gα and pEAK10-Gα-IRES-T2R16 after gene transfer thereof was examined. Hereinafter, the description of "IRES" may be omitted in Examples 11-15.

A gene sequence encoding T2R16-IRES-Gα and Gα-IRES-T2R16 was subcloned into pEAK10 vectors (Edge Biosystem) to prepare expression vectors pEAK10-T2R16-Gα and pEAK10-Gα-T2R16. The pEAK10 vectors are plasmid vectors each comprising an EF1α promoter and a puromycin resistance gene. The thus prepared expression vectors were introduced into HEK293T cells by the same method as in Example 4 to prepare transiently expressing cells.

The expression level of T2R16 in transiently expressing cells was evaluated by the In cell ELISA using the same technique as in Example 5 (2). As a control, HEK293T cells into which a pEAK expression vector containing no gene inserted therein were used. For detection, as in Example 5 (2), an anti-V5-HRP antibody (Thermo Fisher Scientific Inc.) recognizing the V5 epitope added to the C-terminus was used.

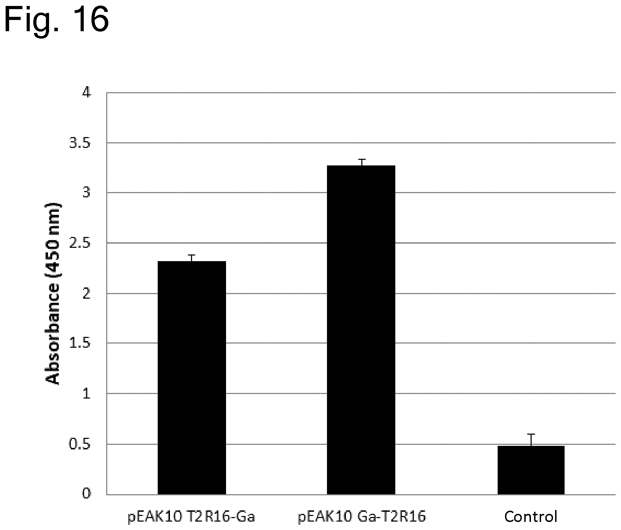

As depicted in Fig. 16, the cells into which the pEAK10-Gα-T2R16 expression vector had been introduced exhibited a higher T2R16 expression level than that exhibited by the cells into which the pEAK10-T2R16-Gα expression vector had been introduced. This demonstrates that the effect of the present invention is not influenced by the gene sequence unique to the vector, such as a promoter and a non-translated region.

### Example 12 Measurement of response of transiently expressing cells to bitter substance (2)

In this example, with respect to seven T2R subtypes (bitter taste receptors), responses of transiently expressing cells to bitter substances were measured.

Seven types of expression vectors comprising each of the genes encoding the seven T2R subtypes listed in Table 5 instead of the gene encoding T2R16 of Gα-T2R16 in Example 4 were prepared, and then the response of transiently expressing cells to known agonists (Table 5) for each subtype was measured by the same method as in Example 4.

**[Table 5]**

| Subtype | Agonist | Concentration for addition |
|---|---|---|
| T2R4 | Amarogentin | 300 uM |
| T2R8 | Denatonium benzoate | 300 uM |
| T2R9 | Ofloxacin | 100 uM |
| T2R10 | Chloroquine | 3 mM |
| T2R13 | Diphenidole | 100 uM |
| T2R20 | Cromolyn | 1 mM |
| T2R39 | Chloroquinine | 1 mM |

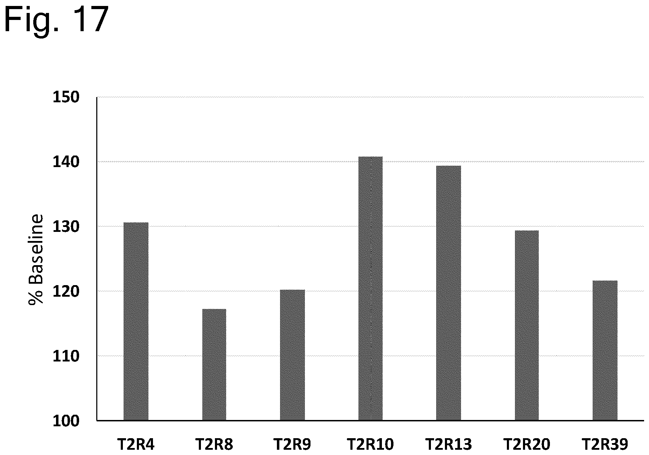

As a result, all T2R subtypes exhibited responses to agonists (Fig. 17).

### Example 13 Measurement of response of stably expressing cells to bitter substance (2)

In this example, with regard to a T2R38 subtype (bitter taste receptor), the response of stably expressing cells to a bitter substance was measured.

A Gα-T2R38 expression vector comprising a gene encoding a T2R38 subtype (bitter taste receptor) instead of the gene encoding T2R16 of Gα-T2R16 in Examples 1 and 2 was prepared, and thus a stable expression cell line was prepared by the same method as in Examples 1 and 2.

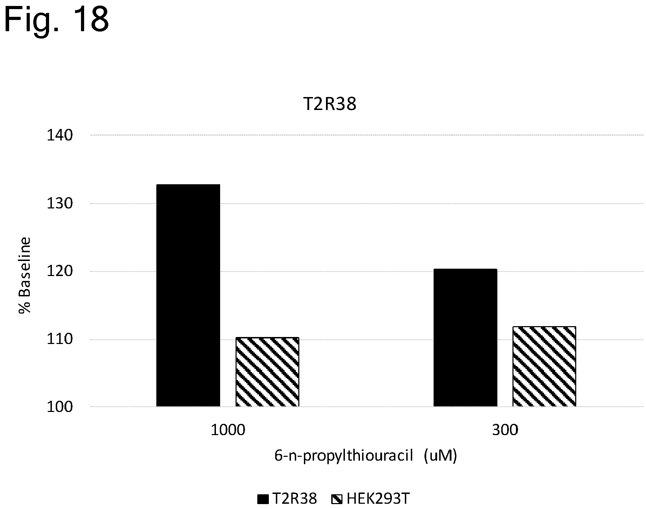

Subsequently, using the same method as in Example 3, the response to a known T2R38 agonist, 6-n-propylthiouracil (1000 µM, 300 µM), was measured. As a control, a cell line (HEK293T) not incorporating any Gα-T2R38 expression vector was used. As a result, responses to 6-n-propylthiouracil (at both 1000 µM and 300 µM) were exhibited (Fig. 18).

### Example 14 Expression Level of bitter taste receptor in transiently expressing cells

In this example, the expression levels of fourteen T2R subtypes (bitter taste receptors) in transiently expressing cells were examined.

Instead of the gene encoding T2R16 of Gα-T2R16 in Example 4, fourteen expression vector types comprising genes encoding each of fourteen T2R subtypes listed in Table 6 were prepared, and thus cells transiently expressing each of T2R and Gα were prepared by the same method as in Example 4.

**[Table 6]**

| T2R Subtype | | |
|---|---|---|
| T2R4 | T2R14 | T2R45 |
| T2R1 | T2R19 | T2R46 |
| T2R3 | T2R41 | T2R47 |
| T2R5 | T2R42 | T2R50 |
| T2R7 | T2R44 | T2R60 |

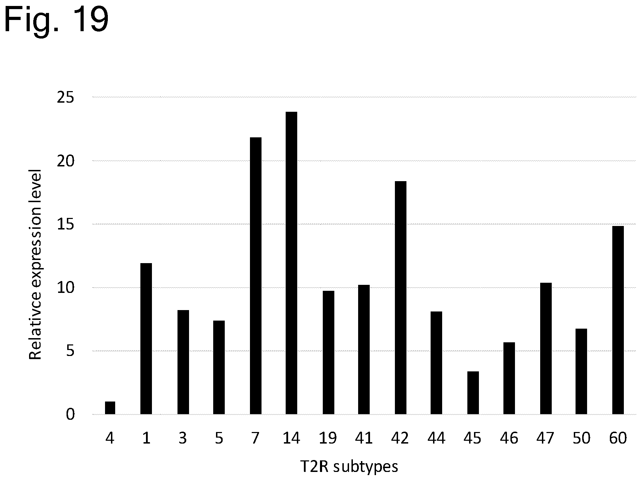

Next, the expression level of T2R in each cell was measured by the In cell ELISA using the same technique as in Example 5 (2). The thus obtained each T2R expression level was compared with the T2R4 expression level in cells transiently expressing Gα-T2R4, the response of which to a known agonist was confirmed in Example 12. As a result, cells exhibited the expression levels of the fourteen T2R subtypes higher than that of T2R4 (Fig. 19). These results demonstrated that the expression levels of the 14 types of T2R were all sufficient for measurement of the response.

### Example 15 Measurement of response and expression level in cells transiently expressing olfactory receptor

In this example, the measurement of the response and the expression level in cells transiently expressing an olfactory receptor (OR3A1) were examined.

Genes encoding Rho-OR3A1, in which N-terminal 21 amino acids (Rho) of human rhodopsin had been added to the N-terminus of OR3A1, and Gαolf were introduced together with IRES into pcDNA vectors by the same method as in Example 1, thereby preparing expression vectors, OR3A1-Gαolf and Gαolf-OR3A1. Using these expression vectors, transiently expressing HEK293T cells were prepared by the same method as in Example 4. As a control, HEK293T cells prepared by separate introduction of each gene of OR3A1 and Gαolf into pcDNA and then double-gene transfer thereof were used.

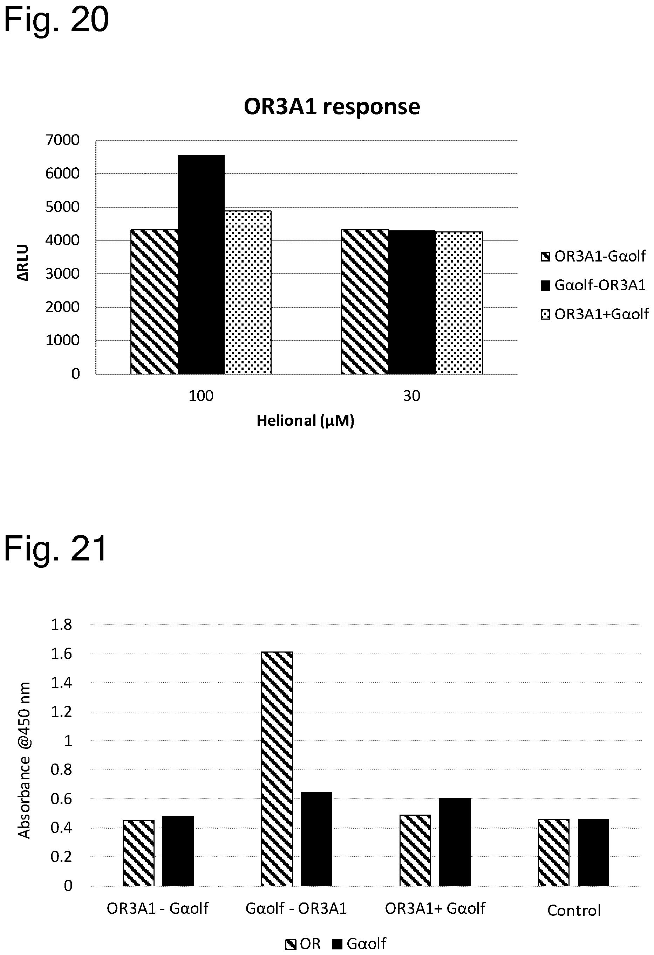

Next, helional, a known agonist for OR3A1, was added onto the prepared cells (100 µM, 30 µM). The amount of cAMP produced in association with the response of OR3A1 20 minutes after the addition of helional was detected by luminescence measurement using a cAMP-GloMax assay kit (Promega). As a result, cells into which the Gαolf-OR3A1 expression vector had been introduced exhibited an increase in cAMP after the addition of 100 µM helional. In the case of introducing the OR3A1-Gαolf expression vector and in the case of separately introducing the OR3A1 and Gαolf genes, no response (increase in cAMP) was observed due to the addition of helional (Fig. 20).

Furthermore, the expression levels of Rho-OR3A1 and Gαolf were compared by In Cell ELISA using an anti-Rho antibody (Merck) and an anti-Gαolf antibody (SANTA CRUZ). As a control, HEK293T cells into which pcDNA containing no such gene inserted therein had been introduced were used. As a result, cells into which Gαolf-OR3A1 had been introduced exhibited higher expression levels of Gαolf and OR3A1 compared to cells into which OR3A1-Gαolf had been introduced (Fig. 21).

## Claims

1. A construct for expressing a G protein-coupled receptor protein or a subunit thereof, comprising: a nucleic acid encoding a G protein α subunit protein; a nucleic acid comprising an IRES sequence; and a nucleic acid encoding the G protein-coupled receptor protein or a subunit thereof in the stated order from the 5' side in a single vector.

2. The construct according to claim 1, wherein the G protein-coupled receptor or a subunit thereof is a G protein-coupled receptor of a rhodopsin-like receptor class or a metabotropic glutamate receptor/metabotropic pheromone receptor class.

3. The construct according to claim 1, wherein the G protein-coupled receptor is a G protein-coupled receptor of the rhodopsin-like receptor class.

4. The construct according to claim 1, wherein the G protein-coupled receptor is a bitter taste receptor.

5. The construct according to any one of claims 1 to 4, wherein the vector is a plasmid vector.

6. The construct according to any one of claims 1 to 4, wherein the vector is a plasmid vector comprising a CMV promoter.

7. The construct according to any one of claims 1 to 4, wherein the vector is a plasmid vector comprising a CMV promoter and a hygromycin resistance gene.

8. The construct according to any one of claims 1 to 4, wherein the vector is a plasmid vector comprising an EF1α promoter.

9. The construct according to any one of claims 1 to 4, wherein the vector is a plasmid vector comprising an EF1α promoter and a puromycin resistance gene.

10. The construct according to any one of claims 1 to 9, wherein the IRES sequence is a type 2 IRES sequence.

11. The construct according to any one of claims 1 to 10, wherein the IRES sequence is an IRES sequence derived from encephalomyocarditis virus.

12. The construct according to any one of claims 1 to 7, comprising a nucleic acid encoding a polypeptide having a function of enhancing the translocation of the G protein-coupled receptor protein or a subunit thereof to a cell membrane, 5' to the nucleic acid encoding the G protein-coupled receptor protein or a subunit thereof.

13. The construct according to claim 12, wherein the polypeptide having a function of enhancing the translocation of the G protein-coupled receptor protein or a subunit thereof to a cell membrane is a polypeptide comprising N-terminal 45 amino acids of a rat somatostatin receptor or a polypeptide comprising N-terminal 39 amino acids of bovine rhodopsin.

14. The construct according to any one of claims 1 to 13, wherein the G protein α subunit protein is a G protein α subunit protein of the Gq family, a G protein α subunit protein of the Gi family, a G protein α subunit protein of the Gs family, or a chimeric protein thereof.

15. The construct according to any one of claims 1 to 14, wherein the G protein α subunit protein is Gα16, Gαgust or Gαolf, or a chimeric protein thereof.

16. The construct according to any one of claims 1 to 15, wherein the G protein α subunit protein is human Gα16/rat Gαgust.

17. The construct according to claim 16, wherein the G protein α subunit protein is human Gα16/rat Gαgust43.

18. The construct according to any one of claims 1 to 17, wherein the G protein-coupled receptor protein is a human G protein-coupled receptor protein.

19. Transformed cells, into which the construct according to any one of claims 1 to 18 is introduced.

20. A method for measuring a physiological response to a substance, comprising adding the substance to the transformed cells according to claim 19, and then measuring a physiological response induced by the substance.

21. The measurement method according to claim 20, wherein the substance is a bitter substance, an odorant, an umami substance or a sweet substance, or a modulator for these substances.

22. Use of the transformed cells according to claim 19, for measurement of a physiological response to a substance.

23. A kit comprising the transformed cells according to claim 19, which is used for a method for measuring a physiological response to a substance by adding the substance to the transformed cells and then measuring a physiological response induced by the substance.
